# EUROPÄISCHE PATENTSCHRIFT

(11) **EP 2 176 433 B1**
(45) Veröffentlichungstag und Bekanntmachung des Hinweises auf die Patenterteilung: **12.10.2011**
(21) Anmeldenummer: 08802946.7
(22) Anmeldetag: 30.07.2008
(51) Int. Cl.: C12Q 1/68, C12N 15/82

(54) **DESATURASEN UND VERFAHREN ZUR HERSTELLUNG MEHRFACH UNGESÄTTIGTER FETTSÄUREN IN TRANSGENEN ORGANISMEN**
DESATURASES AND METHODS FOR PRODUCING POLYUNSATURATED FATTY ACIDS IN TRANSGENIC ORGANISMS
DÉSATURASES ET PROCÉDÉS DE PRODUCTION D'ACIDES GRAS POLYINSATURÉS DANS DES ORGANISMES TRANSGÉNIQUES

(30) Priorität: 31.07.2007 EP 07113506; 20.12.2007 EP 07123864; 01.04.2008 EP 08103294
(43) Veröffentlichungstag der Anmeldung: 21.04.2010
(73) Patentinhaber: BASF Plant Science GmbH, 67056 Ludwigshafen (DE)
(72) Erfinder: BAUER, Jörg, 67117 Limburgerhof (DE); SENGER, Toralf, 69469 Weinheim (DE)
(86) Internationale Anmeldenummer: PCT/EP2008/059999
(87) Internationale Veröffentlichungsnummer: WO 2009/016202

(56) Entgegenhaltungen:
- WO-A-03/099216
- US-A- 6 075 183
- US-A1- 2006 094 090
- US-A1- 2006 115 881
- OURA TAKAHIRO ET AL: "Saccharomyces kluyveri FAD3 encodes an omega3 fatty acid desaturase" MICROBIOLOGY (READING), Bd. 150, Nr. Part 6, Juni 2004 (2004-06), Seiten 1983-1990, XP002503090 ISSN: 1350-0872
- WONGWATHANARAT P ET AL: "Two fatty acid DELTA9-desaturase genes, ole1 and ole2, from Mortierella alpina complement the yeast ole1 mutation" MICROBIOLOGY, SOCIETY FOR GENERAL MICROBIOLOGY, READING, GB, Bd. 145, Nr. 10, 1. Oktober 1999 (1999-10-01), Seiten 2939-2946, XP002352108 ISSN: 1350-0872

### SEQUENCE LISTING

<110> BASF PLANT SCIENCE GMBH
<120> Desaturasen und verfahren zur Herstellung von mehrfach
   ungesättigten Fettsäuren in transgenen organismen
<130> BPS65693PC
<150> EP 07113506.5 <151> 2007-07-31
<150> EP 07123864.6 <151> 2007-12-20
<150> EP 08103294.8 <151> 2008-04-01
<160> 184
<170> PatentIn version 3.4
<210> 1
   <211> 2258
   <212> DNA
   <213> Nectria haematococca
<400> 1
   tgtgcctggt ctgcttcact gcttgcctgt ccgttctccc gtggctgcca ggctttgagt     60
   gggctccacg gcacttcccc caacaaaacg ttcctgccga gcaagcggaa tcgcattgca    120
   tccataaaac ttcaaccccc cccttattat aacacactcc cgtcagcctc ttccttcttc    180
   ttcacctccc ctctttctct cttcgactct tgtcctttgg ccaatccgac caattgactc    240
   ttctctcttt gtcggtcgtc ctcccttccc ccccgcaaca atggcttcta cggctgtgcc    300
   caggcagaac cctgcccttc gacgcaccgt cacctctacc acggctaccg actccgaaac    360
   gtcagctgcc gtctctccct cggactctcc tcgtccctcg gcttcctcta cttcgctctc    420
   ctccctgtct gaggtggaca ttgcgaagcc caagtccagc tatggccgcc tgatcgacac    480
   atacggcaac gagttcgagg tgcccgactt taccatcaag gacatccacg atgccatccc    540
   caagcactgc ttcaagcgct ccgccatcag gggctacggc tacatcctcc gcgacattgt    600
   cctcctcacc accactttca ccatctggca caactttgtg acccccgagt acatcccctc    660
   cactcccgcc cgtgctggtc tctgggccgt ctacaccgtc ctccagggtc tctttggaac    720
   tggtctctgg gtcattgccc acgagtgcgg ccacggcgcc ttctccgact ctcgcctcgt    780
   caacgacatc accggctggg tcctccactc gtccctgctc gtcccctact tcagctggca    840
   gatctcgcac cgaaagcacc acaaggccac cggcaacatg gagcgtgaca tggtctttgt    900
   tccccgaacc agggagcagc aggctactcg tctcggcaag atggctcacg agctcgctca    960
   cctcaccgag gagacccccg ccttcaccct gctcatgctg gtcatgcagc agctcgttgg   1020
   ctggcccaat tacctcctga ccaacgtcac tggccacaac ttccacgagc gccagcgcga   1080
   gggtcgcggc aagggcaaga agaacggcct cggcggtggt gtcaaccact tcgatccccg   1140
   cagccctctg tacgagaacc gtgatgctaa gctcatcgtc ctgagcgata tcggtattgg   1200
   tctggtcgct accggtctgt acttcctcgt gcagaagttt ggcttctaca acatggccat   1260
   ctggtacttt gttccctacc tctgggtcaa ccactggctg ggtaagctcc tctcgtcttc   1320
   tttcacttgt taatcaatac tgacctgtct agttgccatc actttccttc agcacaccga   1380
   ccctaccctc cctcactacc acaacgagga gtggaacttt gtccgcggcg ccgctgccac   1440
   catcgatcgt gagatgggct tcatcggccg acacctgctc cacggcatca tcgagaccca   1500
   cgtcctccac cactacgtca gcagcatccc cttctacaac gctgatgagg ccaccgaggc   1560
   tatcaagcct gtcatgggca agcactaccg cgctgacgtc aaggacggtc ctcgtggttt   1620
   catccgtgcc atgtacaaca gcgcccgtat gtgccagtgg gttgagccca gtgctgaggc   1680
   tgagggtgct ggcaagaaca ttctcttctt ccgcaaccgc aacaacctgg gcactaagcc   1740
   tgccatcatc gaccctcccg ccgcctaagg gtgctatatc ggtccgctcg cagggtgtct   1800
   gcacacccac agatgttttc agcgttgatt gttccaagtt tggtcatggg agtcgtcgga   1860
   ttcggttgtt tgggtcggag caagaccgta catgaaacag gtcttgccag tcggttaaat   1920
   tgatatggcc catggacatg gtggaggatg gaagaggagg taaaataatc gaaggaacaa   1980
   agactcaaag agtgatgaac tagatgatgg agagcagcaa ggatatgaaa cctaatgaag   2040
   ccactcaatt tcttggatca aggactgtct tgtgatgttt ctcgaggttt gttgacgctg   2100
   aaatgacgga tgccccggac tatcaagtga tctgatgcgg ggaaagccac acgtgctcac   2160
   gggtcatatt tggagtccga ccgccgaagg tctttatctt gaagctgtcg agattgtgat   2220
   ttggtggttt ctgtggggtt tgagaactgc cgttgggt                           2258
<210> 2
   <211> 1437
   <212> DNA
   <213> Nectria haematococca
<400> 2
   atggcttcta cggctgtgcc caggcagaac cctgcccttc gacgcaccgt cacctctacc 60
   acggctaccg actccgaaac gtcagctgcc gtctctccct cggactctcc tcgtccctcg 120
   gcttcctcta cttcgctctc ctccctgtct gaggtggaca ttgcgaagcc caagtccagc 180
   tatggccgcc tgatcgacac atacggcaac gagttcgagg tgcccgactt taccatcaag 240
   gacatccacg atgccatccc caagcactgc ttcaagcgct ccgccatcag gggctacggc 300
   tacatcctcc gcgacattgt cctcctcacc accactttca ccatctggca caactttgtg 360
   acccccgagt acatcccctc cactcccgcc cgtgctggtc tctgggccgt ctacaccgtc 420
   ctccagggtc tctttggaac tggtctctgg gtcattgccc acgagtgcgg ccacggcgcc 480
   ttctccgact ctcgcctcgt caacgacatc accggctggg tcctccactc gtccctgctc 540
   gtcccctact tcagctggca gatctcgcac cgaaagcacc acaaggccac cggcaacatg 600
   gagcgtgaca tggtctttgt tccccgaacc agggagcagc aggctactcg tctcggcaag 660
   atggctcacg agctcgctca cctcaccgag gagacccccg ccttcaccct gctcatgctg 720
   gtcatgcagc agctcgttgg ctggcccaat tacctcctga ccaacgtcac tggccacaac 780
   ttccacgagc gccagcgcga gggtcgcggc aagggcaaga agaacggcct cggcggtggt 840
   gtcaaccact tcgatccccg cagccctctg tacgagaacc gtgatgctaa gctcatcgtc 900
   ctgagcgata tcggtattgg tctggtcgct accggtctgt acttcctcgt gcagaagttt 960
   ggcttctaca acatggccat ctggtacttt gttccctacc tctgggtcaa ccactggctg 1020
   gttgccatca ctttccttca gcacaccgac cctaccctcc ctcactacca caacgaggag 1080
   tggaactttg tccgcggcgc cgctgccacc atcgatcgtg agatgggctt catcggccga 1140
   cacctgctcc acggcatcat cgagacccac gtcctccacc actacgtcag cagcatcccc 1200
   ttctacaacg ctgatgaggc caccgaggct atcaagcctg tcatgggcaa gcactaccgc 1260
   gctgacgtca aggacggtcc tcgtggtttc atccgtgcca tgtacaacag cgcccgtatg 1320
   tgccagtggg ttgagcccag tgctgaggct gagggtgctg gcaagaacat tctcttcttc 1380
   cgcaaccgca acaacctggg cactaagcct gccatcatcg accctcccgc cgcctaa 1437
<210> 3
   <211> 478
   <212> PRT
   <213> Nectria haematococca
<400> 3
   Met Ala Ser Thr Ala Val Pro Arg Gln Asn Pro Ala Leu Arg Arg Thr
   1               5                   10                  15
   Val Thr Ser Thr Thr Ala Thr Asp Ser Glu Thr Ser Ala Ala Val Ser
               20                  25                  30
   Pro Ser Asp Ser Pro Arg Pro Ser Ala Ser Ser Thr Ser Leu Ser Ser
           35                  40                  45
   Leu Ser Glu Val Asp Ile Ala Lys Pro Lys Ser Ser Tyr Gly Arg Leu
       50                  55                  60
   Ile Asp Thr Tyr Gly Asn Glu Phe Glu Val Pro Asp Phe Thr Ile Lys
   65                  70                  75                  80
   Asp Ile His Asp Ala Ile Pro Lys His Cys Phe Lys Arg Ser Ala Ile
                   85                  90                  95
   Arg Gly Tyr Gly Tyr Ile Leu Arg Asp Ile Val Leu Leu Thr Thr Thr
               100                 105                 110
   Phe Thr Ile Trp His Asn Phe Val Thr Pro Glu Tyr Ile Pro Ser Thr
           115                 120                 125
   Pro Ala Arg Ala Gly Leu Trp Ala Val Tyr Thr Val Leu Gln Gly Leu
       130                 135                 140
   Phe Gly Thr Gly Leu Trp Val Ile Ala His Glu Cys Gly His Gly Ala
   145                 150                 155                 160
   Phe Ser Asp Ser Arg Leu Val Asn Asp Ile Thr Gly Trp Val Leu His
                   165                 170                 175
   Ser Ser Leu Leu Val Pro Tyr Phe Ser Trp Gln Ile Ser His Arg Lys
               180                 185                 190
   His His Lys Ala Thr Gly Asn Met Glu Arg Asp Met Val Phe Val Pro
           195                 200                 205
   Arg Thr Arg Glu Gln Gln Ala Thr Arg Leu Gly Lys Met Ala His Glu
       210                 215                 220
   Leu Ala His Leu Thr Glu Glu Thr Pro Ala Phe Thr Leu Leu Met Leu
   225                 230                 235                 240
   Val Met Gln Gln Leu Val Gly Trp Pro Asn Tyr Leu Leu Thr Asn Val
                   245                 250                 255
   Thr Gly His Asn Phe His Glu Arg Gln Arg Glu Gly Arg Gly Lys Gly
               260                 265                 270
   Lys Lys Asn Gly Leu Gly Gly Gly Val Asn His Phe Asp Pro Arg Ser
           275                 280                 285
   Pro Leu Tyr Glu Asn Arg Asp Ala Lys Leu Ile Val Leu Ser Asp Ile
       290                 295                 300
   Gly Ile Gly Leu Val Ala Thr Gly Leu Tyr Phe Leu Val Gln Lys Phe
   305                 310                 315                 320
   Gly Phe Tyr Asn Met Ala Ile Trp Tyr Phe Val Pro Tyr Leu Trp Val
                   325                 330                 335
   Asn His Trp Leu Val Ala Ile Thr Phe Leu Gln His Thr Asp Pro Thr
               340                 345                 350
   Leu Pro His Tyr His Asn Glu Glu Trp Asn Phe Val Arg Gly Ala Ala
           355                 360                 365
   Ala Thr Ile Asp Arg Glu Met Gly Phe Ile Gly Arg His Leu Leu His
       370                 375                 380
   Gly Ile Ile Glu Thr His Val Leu His His Tyr Val Ser Ser Ile Pro
   385                 390                 395                 400
   Phe Tyr Asn Ala Asp Glu Ala Thr Glu Ala Ile Lys Pro Val Met Gly
                   405                 410                 415
   Lys His Tyr Arg Ala Asp Val Lys Asp Gly Pro Arg Gly Phe Ile Arg
               420                 425                 430
   Ala Met Tyr Asn Ser Ala Arg Met Cys Gln Trp Val Glu Pro Ser Ala
   435 440 445
   Glu Ala Glu Gly Ala Gly Lys Asn Ile Leu Phe Phe Arg Asn Arg Asn
   450 455 460
   Asn Leu Gly Thr Lys Pro Ala Ile Ile Asp Pro Pro Ala Ala
   465 470 475
<210> 4
   <211> 1996
   <212> DNA
   <213> Nectria haematococca
<400> 4
   ggggaggagg agagagggac aagctatggg tttaaagcta ggaggagagc cgcgcaaaca      60
   gggagggagc ccaaggagaa gaaaaatttc ccacctttct ttctcttccc ttttctctct     120
   agcaaacatc aattggtcag aggtcgctgg ctcgtcttct acgtctccca attgcattgt     180
   tctgtctaga tcagctcaac atggctactc gacagcgtac ttcgaccact gttgtggttg     240
   agaagccttc tgccaaggta agtttctact gtctcaccct cttgtctctt caggtggctg     300
   actctgtcgc aaaaggttac tctcgagcct cagcagcagc ctcaattccc cgatatcaag     360
   accatcaagg atgccatccc cgcccactgc ttccagccct cgctcttcac ctccttctac     420
   tatgtcttcc gcgactttgc catggtggcc accctcgtct gggctgccct gacctacatt     480
   cccgccattc ctgaccagag gctgcgcgtc gccgcctgga tggtctacgg ctttgtccaa     540
   ggcctcgtgt gcactggtgt ctggatcctg ggccacgagt gcggtcacgg tgctttctct     600
   acccacggaa agctcaacaa tgtcgtcgga tggttcctcc actcgttcct cctcgtgccc     660
   tacttctcgt ggaagtactc tcaccaccgc caccaccgct tcaccggcca catggacctc     720
   gacatggcct ttgtccccgc cacccagccc aagaagcaca gcatccttgc cggcatcgac     780
   ctgaacgagc tgtttgagga cacccccatc gcccagctca tcaggatcgt cttccaccag     840
   ctgttcggct ggcaggtgta cctcctgttc aacgccagcg ctggtaaggg cagcaagcag     900
   tgggagccta ctggtctggc caagtggttc cgtgtcagcc actttgagcc caccagcgcc     960
   gtgttccgac ccagcgaggc catcttcatc ttcatctctg acctcggcct ggccatcacc    1020
   ttcaccgccc tgtactttgc ctccaaggct gtgggaacct cgactgttct gttcctctac    1080
   gccgtcccct acttctgggt gcaccactgg ctgggtaaga tttctgatgg caacaaaatt    1140
   gaatagccgc tgacgtgatt tttagtcgcc atcacctacc tccaccacca ccacaccgag    1200
   gtccctcact acactaacga gggctggacc tatgtcaagg gcgctctcgc taccgttgac    1260
   cgtgagtttg gcttcattgg caagcacctg ttccacggca tcattgagaa gcacgttgtc    1320
   caccaccttt tcccgtgagt actccccgct atttggggtg aaccaatcta accatctcag    1380
   ccgcatcccc ttctacaagg ccgacgaggc taccgaggcc atcaagcctc tcctgggcga    1440
   cctctactac cacgacgagc gcaacttcat cggccagctc tggtccgtct ttggctcgct    1500
   caagtacgtc gagcacgacc ccaccaacca gggtgccatg cgatgggcca aggagtagag    1560
   ctccaaggct gtcggagccc catgcgaaga gacatagaga gattagacga tgggattaga 1620
   cacgatagac caaacgacta gacacacaat gctatagatc tagagcaatt gctttctcgg 1680
   ctggtatcgg atttagacgc tctgagataa aggtcaaatc ggatgatgaa acaaaatgga 1740
   ggcttgcttg tcctgttgga ctgcaatgta ttataccaat agaagctgaa tcaatgacct 1800
   gacttgtgca acatcttgag ttggtgttca aattggctca atttgaggca tcctctgcga 1860
   ttgggcagag atgaacttgc tttatcgatc tagatccatc caatcctgag acaatctctt 1920
   gcatgagatt gagatggtca tggaggtgtg gagactctct cctcaacaac gccaagacca 1980
   atcggcttga ttagta 1996
<210> 5
   <211> 1203
   <212> DNA
   <213> Nectria haematococca
<400> 5
   atggctactc gacagcgtac ttcgaccact gttgtggttg agaagccttc tgccaaggtt 60
   actctcgagc ctcagcagca gcctcaattc cccgatatca agaccatcaa ggatgccatc 120
   cccgcccact gcttccagcc ctcgctcttc acctccttct actatgtctt ccgcgacttt 180
   gccatggtgg ccaccctcgt ctgggctgcc ctgacctaca ttcccgccat tcctgaccag 240
   aggctgcgcg tcgccgcctg gatggtctac ggctttgtcc aaggcctcgt gtgcactggt 300
   gtctggatcc tgggccacga gtgcggtcac ggtgctttct ctacccacgg aaagctcaac 360
   aatgtcgtcg gatggttcct ccactcgttc ctcctcgtgc cctacttctc gtggaagtac 420
   tctcaccacc gccaccaccg cttcaccggc cacatggacc tcgacatggc ctttgtcccc 480
   gccacccagc ccaagaagca cagcatcctt gccggcatcg acctgaacga gctgtttgag 540
   gacaccccca tcgcccagct catcaggatc gtcttccacc agctgttcgg ctggcaggtg 600
   tacctcctgt tcaacgccag cgctggtaag ggcagcaagc agtgggagcc tactggtctg 660
   gccaagtggt tccgtgtcag ccactttgag cccaccagcg ccgtgttccg acccagcgag 720
   gccatcttca tcttcatctc tgacctcggc ctggccatca ccttcaccgc cctgtacttt 780
   gcctccaagg ctgtgggaac ctcgactgtt ctgttcctct acgccgtccc ctacttctgg 840
   gtgcaccact ggctggtcgc catcacctac ctccaccacc accacaccga ggtccctcac 900
   tacactaacg agggctggac ctatgtcaag ggcgctctcg ctaccgttga ccgtgagttt 960
   ggcttcattg gcaagcacct gttccacggc atcattgaga agcacgttgt ccaccacctt 1020
   ttcccccgca tccccttcta caaggccgac gaggctaccg aggccatcaa gcctctcctg 1080
   ggcgacctct actaccacga cgagcgcaac ttcatcggcc agctctggtc cgtctttggc 1140
   tcgctcaagt acgtcgagca cgaccccacc aaccagggtg ccatgcgatg ggccaaggag 1200
   tag 1203
<210> 6
   <211> 400
   <212> PRT
   <213> Nectria haematococca
<400> 6
   Met Ala Thr Arg Gln Arg Thr Ser Thr Thr Val Val Val Glu Lys Pro
   1               5                   10                  15
   Ser Ala Lys Val Thr Leu Glu Pro Gln Gln Gln Pro Gln Phe Pro Asp
               20                  25                  30
   Ile Lys Thr Ile Lys Asp Ala Ile Pro Ala His Cys Phe Gln Pro Ser
           35                  40                  45
   Leu Phe Thr Ser Phe Tyr Tyr Val Phe Arg Asp Phe Ala Met Val Ala
       50                  55                  60
   Thr Leu Val Trp Ala Ala Leu Thr Tyr Ile Pro Ala Ile Pro Asp Gln
   65                  70                  75                  80
   Arg Leu Arg Val Ala Ala Trp Met Val Tyr Gly Phe Val Gln Gly Leu
                   85                  90                  95
   Val Cys Thr Gly Val Trp Ile Leu Gly His Glu Cys Gly His Gly Ala
               100                 105                 110
   Phe Ser Thr His Gly Lys Leu Asn Asn Val Val Gly Trp Phe Leu His
           115                 120                 125
   Ser Phe Leu Leu Val Pro Tyr Phe Ser Trp Lys Tyr Ser His His Arg
       130                 135                 140
   His His Arg Phe Thr Gly His Met Asp Leu Asp Met Ala Phe Val Pro
   145                 150                 155                 160
   Ala Thr Gln Pro Lys Lys His Ser Ile Leu Ala Gly Ile Asp Leu Asn
                   165                 170                 175
   Glu Leu Phe Glu Asp Thr Pro Ile Ala Gln Leu Ile Arg Ile Val Phe
               180                 185                 190
   His Gln Leu Phe Gly Trp Gln Val Tyr Leu Leu Phe Asn Ala Ser Ala
           195                 200                 205
   Gly Lys Gly Ser Lys Gln Trp Glu Pro Thr Gly Leu Ala Lys Trp Phe
       210                 215                 220
   Arg Val Ser His Phe Glu Pro Thr Ser Ala Val Phe Arg Pro Ser Glu
   225                 230                 235                 240
   Ala Ile Phe Ile Phe Ile Ser Asp Leu Gly Leu Ala Ile Thr Phe Thr
                   245                 250                 255
   Ala Leu Tyr Phe Ala Ser Lys Ala Val Gly Thr Ser Thr Val Leu Phe
                   260                     265                     270
   Leu Tyr Ala Val Pro Tyr Phe Trp Val His His Trp Leu Val Ala Ile
           275                     280                     285
   Thr Tyr Leu His His His His Thr Glu Val Pro His Tyr Thr Asn Glu
       290                     295                     300
   Gly Trp Thr Tyr Val Lys Gly Ala Leu Ala Thr Val Asp Arg Glu Phe
   305                     310                     315                     320
   Gly Phe Ile Gly Lys His Leu Phe His Gly Ile Ile Glu Lys His Val
                   325                     330                     335
   Val His His Leu Phe Pro Arg Ile Pro Phe Tyr Lys Ala Asp Glu Ala
               340                     345                     350
   Thr Glu Ala Ile Lys Pro Leu Leu Gly Asp Leu Tyr Tyr His Asp Glu
           355                     360                     365
   Arg Asn Phe Ile Gly Gln Leu Trp Ser Val Phe Gly Ser Leu Lys Tyr
       370                     375                     380
   Val Glu His Asp Pro Thr Asn Gln Gly Ala Met Arg Trp Ala Lys Glu
   385                     390                     395                     400
<210> 7
   <211> 1988
   <212> DNA
   <213> Trichoderma resii
<400> 7
   ccccgttagc agggggtacc tcccaacgcc agcagtgccg ccaccactcc gcaggtcaca     60
   ggtgttaacg ccaccaacca gatgccgcca gataagggac acgacacgaa aaatggatca   120
   ggacgaccaa gctagagggc gaaaggggaa gtggaagtgg aaaatttgat gccgtgcctg   180
   actatggagc tccctttttc ccagcgtttt ctgccttaca gttgggctgc tggtgtgggt   240
   ttaaaaggca atcaggacgg ctgttctcgc tcaggaagag tcctcagttc atactcccaa   300
   ctttcttcat ccattgagtt gctggccttt gtccacttca cgatggctac taccacgacg   360
   gtcaccaaga ggggaggcgc cgtgggctca atgtcagtag tcctgctagt ctcatgaagg   420
   ctttttctgc cccgaaaagt tttcacccgt tgctctgttt tttttgctga ctccagatac   480
   agcaagctgc ctgaggagga cagtcggttc cccgatatca acacgattcg ggccgccatc   540
   cccaagcact gctttgagcc gtctgtggcc atctccatgg gctacctggt gcgcgatgtg   600
   gtcatgattg gagcccttgg ctgggctgcc cttacttaca ttcccaccat tcctgatgcc   660
   acccttcgga ccattgcctg gatcgtctac ggcttcgtcc agggcctcgt ctgcactggt   720
   ctctggattc tgggccacga ggccggccac ggcgcctttt cccagcactc tctgctgaac   780
   cacgtcgtcg gcttcttctc tcactccgtc ctgctggtgc cgtactactc ctggaagttc   840
   tctcaccacc gccaccacaa gtacaccggc cacatggaga aggacatggc ctttgtcccc     900
   cggaccaagc ccgactacgc caagcgaacc ctggccgccc ttgagatgct cgaggacacg     960
   cccgcctacc agttcgtgac gctcatgttc caccagctgt ttgcgtggca gatctacctg    1020
   ctgttcaaca tctccgccgg ccgcaacagc ctgcagaagt ctgccacgac catgttcggc    1080
   aagagccact ttggccccaa cagcgccgtc ttccgccgca gcgaggctcc cttcatcttc    1140
   ctgtctgacg tcggccttgc catgacggcg tttgcgctgt acaagctcgc gggagccgtg    1200
   ggaacgggca ccatgctgct tgtttacgcg cagccttact tctgggttca ccactggttg    1260
   agtatgtttt ctcctaaaca aacgcccaag caaacgcaca gtcacactca agcctgatgc    1320
   aaacgcaaac acggcgtgtc aatgtcaaaa cagggaataa gattgctgac gggagtgcca    1380
   ttttttagtt gccatcactt accttcacca cactcacatg gaggtgcctc actatgatgc     1440
   cgagaactgg acctttgtca agggtgctct ggccactgtt gaccgcgagt ttggctttgt    1500
   cggccgccac ctcttccact gcattattga gcaccacgtt gttcaccacc ttttcccgta    1560
   agtcttcagc tggacctata gctgccaatt gccattcact aacattcttc tagtcgcatt    1620
   cccttctact atgccggaga agccaccgag gccatcaagc ccgtgcttgg cgacctgtac    1680
   taccgcgatg agcgatcttt cctgggccag ctctggagca actttaccaa gtgcaagtac    1740
   gtcgtggccg atgagaagac gcccggagct ctgcactggg cgcaatgatt gaatgaatga    1800
   gaacatggtt taaatggatc gcgttgggtt gctctgcttt gtctttctcg gtgggaagga    1860
   taatggcttt caaaagggga taaaagtatc tcggattaga ctcttctgtg tgctctctcg    1920
   tatgatgaga tgggccttgg ggggaaatct ttgttttgcg gttttagatg atacacgata    1980
   gacttcgt                                                             1988

<210> 8
   <211> 1173
   <212> DNA
   <213> Trichoderma resii
<400> 8
   atggctacta ccacgacggt caccaagagg ggaggcgccg tgggctcaat caagctgcct 60
   gaggaggaca gtcggttccc cgatatcaac acgattcggg ccgccatccc caagcactgc 120
   tttgagccgt ctgtggccat ctccatgggc tacctggtgc gcgatgtggt catgattgga 180
   gcccttggct gggctgccct tacttacatt cccaccattc ctgatgccac ccttcggacc 240
   attgcctgga tcgtctacgg cttcgtccag ggcctcgtct gcactggtct ctggattctg 300
   ggccacgagg ccggccacgg cgccttttcc cagcactctc tgctgaacca cgtcgtcggc 360
   ttcttctctc actccgtcct gctggtgccg tactactcct ggaagttctc tcaccaccgc 420
   caccacaagt acaccggcca catggagaag gacatggcct ttgtcccccg gaccaagccc 480
   gactacgcca agcgaaccct ggccgccctt gagatgctcg aggacacgcc cgcctaccag 540
   ttcgtgacgc tcatgttcca ccagctgttt gcgtggcaga tctacctgct gttcaacatc 600
   tccgccggcc gcaacagcct gcagaagtct gccacgacca tgttcggcaa gagccacttt 660
   ggccccaaca gcgccgtctt ccgccgcagc gaggctccct tcatcttcct gtctgacgtc 720
   ggccttgcca tgacggcgtt tgcgctgtac aagctcgcgg gagccgtggg aacgggcacc 780
   atgctgcttg tttacgcgca gccttacttc tgggttcacc actggttgat tgccatcact 840
   taccttcacc acactcacat ggaggtgcct cactatgatg ccgagaactg gacctttgtc 900
   aagggtgctc tggccactgt tgaccgcgag tttggctttg tcggccgcca cctcttccac 960
   tgcattattg agcaccacgt tgttcaccac cttttccctc gcattccctt ctactatgcc 1020
   ggagaagcca ccgaggccat caagcccgtg cttggcgacc tgtactaccg cgatgagcga 1080
   tctttcctgg gccagctctg gagcaacttt accaagtgca agtacgtcgt ggccgatgag 1140
   aagacgcccg gagctctgca ctgggcgcaa tga 1173
<210> 9
   <211> 390
   <212> PRT
   <213> Trichoderma resii
<400> 9
   Met Ala Thr Thr Thr Thr Val Thr Lys Arg Gly Gly Ala Val Gly Ser
   1               5                   10                  15
   Ile Lys Leu Pro Glu Glu Asp Ser Arg Phe Pro Asp Ile Asn Thr Ile
               20                  25                  30
   Arg Ala Ala Ile Pro Lys His Cys Phe Glu Pro Ser Val Ala Ile Ser
           35                  40                  45
   Met Gly Tyr Leu Val Arg Asp Val Val Met Ile Gly Ala Leu Gly Trp
       50                  55                  60
   Ala Ala Leu Thr Tyr Ile Pro Thr Ile Pro Asp Ala Thr Leu Arg Thr
   65                  70                  75                  80
   Ile Ala Trp Ile Val Tyr Gly Phe Val Gln Gly Leu Val Cys Thr Gly
                   85                  90                  95
   Leu Trp Ile Leu Gly His Glu Ala Gly His Gly Ala Phe Ser Gln His
               100                 105                 110
   Ser Leu Leu Asn His Val Val Gly Phe Phe Ser His Ser Val Leu Leu
           115                 120                 125
   Val Pro Tyr Tyr Ser Trp Lys Phe Ser His His Arg His His Lys Tyr
       130                 135                 140
   Thr Gly His Met Glu Lys Asp Met Ala Phe Val Pro Arg Thr Lys Pro
   145                 150                 155                 160
   Asp Tyr Ala Lys Arg Thr Leu Ala Ala Leu Glu Met Leu Glu Asp Thr
                   165                 170                 175
   Pro Ala Tyr Gln Phe Val Thr Leu Met Phe His Gln Leu Phe Ala Trp
   180 185 190
   Gln Ile Tyr Leu Leu Phe Asn Ile Ser Ala Gly Arg Asn Ser Leu Gln
   195 200 205
   Lys Ser Ala Thr Thr Met Phe Gly Lys Ser His Phe Gly Pro Asn Ser
   210 215 220
   Ala Val Phe Arg Arg Ser Glu Ala Pro Phe Ile Phe Leu Ser Asp Val
   225 230 235 240
   Gly Leu Ala Met Thr Ala Phe Ala Leu Tyr Lys Leu Ala Gly Ala Val
   245 250 255
   Gly Thr Gly Thr Met Leu Leu Val Tyr Ala Gln Pro Tyr Phe Trp Val
   260 265 270
   His His Trp Leu Ile Ala Ile Thr Tyr Leu His His Thr His Met Glu
   275 280 285
   Val Pro His Tyr Asp Ala Glu Asn Trp Thr Phe Val Lys Gly Ala Leu
   290 295 300
   Ala Thr Val Asp Arg Glu Phe Gly Phe Val Gly Arg His Leu Phe His
   305 310 315 320
   Cys Ile Ile Glu His His Val Val His His Leu Phe Pro Arg Ile Pro
   325 330 335
   Phe Tyr Tyr Ala Gly Glu Ala Thr Glu Ala Ile Lys Pro Val Leu Gly
   340 345 350
   Asp Leu Tyr Tyr Arg Asp Glu Arg Ser Phe Leu Gly Gln Leu Trp Ser
   355 360 365
   Asn Phe Thr Lys Cys Lys Tyr Val Val Ala Asp Glu Lys Thr Pro Gly
   370 375 380
   Ala Leu His Trp Ala Gln
   385 390
<210> 10
   <211> 2520
   <212> DNA
   <213> Monosiga brevicollis
<400> 10
   agatcttgca tcaccggtcg ctctcgtgtg cagagggcag ccggggtctg cgcgttgagg     60
   ccgtggaaga agacgtactt gttgtgcagg gggaaaaact aaaaagctgg ctggttgtat    120
   caagccggct tcagggttca gcagatcatg cacggctgcg ctgccgtttt gacaggttgc    180
   gcgttgtccg tgtcatacag ggagatggtg caaggtgcgt atgcaggtgt gatcacttca 240
   aggtatagtc aagctaaacg tcacgtgcat gtgtgcaagc cacccaaagt gaggaaagca 300
   tgtgatgaga tcgcacatgt cacgccattg cagtctctca cttgtctctg gtttagatct 360
   ggctacagga ctattctctg aaatcaacgt tatatcccat tgccacgacg acactttgat 420
   gactcacgtc tccctctctc tctgcttgaa ccactcgccc cccggccttt ctctgacacg 480
   acactgtcaa cgacttcact tttttttttc tctcgacgcc gacgccgacg ccgacgccga 540
   cgccgaaccc ttcaaggtca gtcccgccac cccggcccgg ccagatccgc gccgcaaacg 600
   ggccttgtcg taatcgggtt cttgagtatc attcgccacg gcctggcttg gtttcctgtc 660
   gtcgaatcta cacggctgct ctgccatcgc ttctcgagtg tcgctcacgt gctcgtgcgg 720
   gtttcgcttg tggggaatgt agatgacggt ggcttcccag gtgaccgagg aggtggcagc 780
   cgtgcccacc aagcaccccc gcatgcccac ggacactccg ccttttaccc ttggccaact 840
   caaggtgatt caccgcgtct ttccgcgtct ttccttgtta ctcgtcatgc tctcgctgga 900
   cgtaaactca tcttcttgca tcatccggtt ccgcaaccat ggcccgttag gcagccatcc 960
   cgccccactg ctttgagcgc aacctcttca cttcgatgcg ctatgtgttc caagaccttg 1020
   ctggtgttgc cactctctac tacctctcca cctttattga tcaactgccc tcgttcgccg 1080
   gccttttcct ctggcctctc tattggtacc tccaggtcag tctctgtttc ccctcttctt    1140
   cttcttctcg acgttggccc ttccttttca atccctcccc aagcgccatc cctccccttt    1200
   gtcttgccat gcaccaattg ctgcccacgc acatgctcgc tggtttgcgt ttcctctatt 1260
   cctcaacttt gaacccgcct tttcccttcg ttcatagggt gccgtcatga ctggtttgtg 1320
   ggttctggcc catgagtgtg gccaccgcgc gttttgcgac aacgagacca ttggcgatgc 1380
   cgtgggcatg atcctccact ctgtacgtgc tttctgccat tggacccccg catgcacttg 1440
   atctcccccc cctctttctt tttctcggcc gtcctcaaat accatggtgg tcgggcacga 1500
   tgaagccaaa cgcatcctcg agtcctctca ttgacttttt gttttggtct ccctctacct 1560
   tgacaggctc tcttggtgcc ctaccacccc tggcgtatct cccaccgcaa gcaccactcg 1620
   cgcaccaacc acatgaccga ggatgaggtc ttcatccctg acacccgtgg tcacgagcac 1680
   attgtcccct acgatgagat ggtcggcccc gtctcggtcg ccctgcgcgt cttcttctgc 1740
   gctcgtatgc ttctctttgg ctggcccgcc tacctcttga cccatgttac tggtcgtcaa 1800
   tacggtcgcc gcaccaacca ttttgagccc gagtctcctc tcttcgacaa gaaggagcgc 1860
   ggtggcgtgg tcctcagtga cctggtcctc ttctcctgga tcggctgcct tctctacgct 1920
   ggtcagaccg ctggctgggg ctggctcttc aagtcttact ttgttcctta catcatcgtc 1980
   aacttttggc tcgtcctcat cacccacctc caacacacgg acgtccgcct tccccactat 2040
   cgctcccaag agtggacctg gctcaagggt gccctctgca ccatggaccg cgactatggt 2100
   cacctcaaca tccttcacca tcacatctcc gatacccacg tcgtccatca cctcttcagc 2160
   tacctccccc actatcatgc cgaagaggct ttggccgcca tcaagcccat tctgggcgag 2220
   tattacctca aggactccgt ttcccccggt ctgcagggcg ttatggaggc cctctggaac 2280
   agcatgacct actgccgcgt cgtcgagaac accggtgagg tcctttggtt caaggacagg 2340
   taaaacggct tgccctcttt gtcttcttgt gccccaaacc cgatttgcgg ttggatgagc 2400
   atgagagaat cggtggcagt accggttgcc ggagaatgag cgcctctgta catgaccaca 2460
   cccacccatg ttcttttcct actctgactt gcgttgccgg tgatagctgc agcgctctct 2520
<210> 11
   <211> 1149
   <212> DNA
   <213> Monosiga brevicollis
<400> 11
   atgacggtgg cttcccaggt gaccgaggag gtggcagccg tgcccaccaa gcacccccgc 60
   atgcccacgg acactccgcc ttttaccctt ggccaactca aggcagccat cccgccccac 120
   tgctttgagc gcaacctctt cacttcgatg cgctatgtgt tccaagacct tgctggtgtt 180
   gccactctct actacctctc cacctttatt gatcaactgc cctcgttcgc cggccttttc 240
   ctctggcctc tctattggta cctccagggt gccgtcatga ctggtttgtg ggttctggcc 300
   catgagtgtg gccaccgcgc gttttgcgac aacgagacca ttggcgatgc cgtgggcatg 360
   atcctccact ctgctctctt ggtgccctac cacccctggc gtatctccca ccgcaagcac 420
   cactcgcgca ccaaccacat gaccgaggat gaggtcttca tccctgacac ccgtggtcac 480
   gagcacattg tcccctacga tgagatggtc ggccccgtct cggtcgccct gcgcgtcttc 540
   ttctgcgctc gtatgcttct ctttggctgg cccgcctacc tcttgaccca tgttactggt 600
   cgtcaatacg gtcgccgcac caaccatttt gagcccgagt ctcctctctt cgacaagaag 660
   gagcgcggtg gcgtggtcct cagtgacctg gtcctcttct cctggatcgg ctgccttctc 720
   tacgctggtc agaccgctgg ctggggctgg ctcttcaagt cttactttgt tccttacatc 780
   atcgtcaact tttggctcgt cctcatcacc cacctccaac acacggacgt ccgccttccc 840
   cactatcgct cccaagagtg gacctggctc aagggtgccc tctgcaccat ggaccgcgac 900
   tatggtcacc tcaacatcct tcaccatcac atctccgata cccacgtcgt ccatcacctc 960
   ttcagctacc tcccccacta tcatgccgaa gaggctttgg ccgccatcaa gcccattctg 1020
   ggcgagtatt acctcaagga ctccgtttcc cccggtctgc agggcgttat ggaggccctc 1080
   tggaacagca tgacctactg ccgcgtcgtc gagaacaccg gtgaggtcct ttggttcaag 1140
   gacaggtaa 1149
<210> 12
   <211> 382
   <212> PRT
   <213> Monosiga brevicollis
<400> 12
   Met Thr Val Ala Ser Gln Val Thr Glu Glu Val Ala Ala Val Pro Thr
   1               5                   10                  15
   Lys His Pro Arg Met Pro Thr Asp Thr Pro Pro Phe Thr Leu Gly Gln
   20 25 30
   Leu Lys Ala Ala Ile Pro Pro His Cys Phe Glu Arg Asn Leu Phe Thr
   35 40 45
   Ser Met Arg Tyr Val Phe Gln Asp Leu Ala Gly Val Ala Thr Leu Tyr
   50 55 60
   Tyr Leu Ser Thr Phe Ile Asp Gln Leu Pro Ser Phe Ala Gly Leu Phe
   65 70 75 80
   Leu Trp Pro Leu Tyr Trp Tyr Leu Gln Gly Ala Val Met Thr Gly Leu
   85 90 95
   Trp Val Leu Ala His Glu Cys Gly His Arg Ala Phe Cys Asp Asn Glu
   100 105 110
   Thr Ile Gly Asp Ala Val Gly Met Ile Leu His Ser Ala Leu Leu Val
   115 120 125
   Pro Tyr His Pro Trp Arg Ile Ser His Arg Lys His His Ser Arg Thr
   130 135 140
   Asn His Met Thr Glu Asp Glu Val Phe Ile Pro Asp Thr Arg Gly His
   145 150 155 160
   Glu His Ile Val Pro Tyr Asp Glu Met Val Gly Pro Val Ser Val Ala
   165 170 175
   Leu Arg Val Phe Phe Cys Ala Arg Met Leu Leu Phe Gly Trp Pro Ala
   180 185 190
   Tyr Leu Leu Thr His Val Thr Gly Arg Gln Tyr Gly Arg Arg Thr Asn
   195 200 205
   His Phe Glu Pro Glu Ser Pro Leu Phe Asp Lys Lys Glu Arg Gly Gly
   210 215 220
   Val Val Leu Ser Asp Leu Val Leu Phe Ser Trp Ile Gly Cys Leu Leu
   225 230 235 240
   Tyr Ala Gly Gln Thr Ala Gly Trp Gly Trp Leu Phe Lys Ser Tyr Phe
   245 250 255
   Val Pro Tyr Ile Ile Val Asn Phe Trp Leu Val Leu Ile Thr His Leu
   260 265 270
   Gln His Thr Asp Val Arg Leu Pro His Tyr Arg Ser Gln Glu Trp Thr
   275 280 285
   Trp Leu Lys Gly Ala Leu Cys Thr Met Asp Arg Asp Tyr Gly His Leu
   290 295 300
   Asn Ile Leu His His His Ile Ser Asp Thr His Val Val His His Leu
   305                 310                 315                 320
   Phe Ser Tyr Leu Pro His Tyr His Ala Glu Glu Ala Leu Ala Ala Ile
                   325                 330                 335
   Lys Pro Ile Leu Gly Glu Tyr Tyr Leu Lys Asp Ser Val Ser Pro Gly
               340                 345                 350
   Leu Gln Gly Val Met Glu Ala Leu Trp Asn Ser Met Thr Tyr Cys Arg
           355                 360                 365
   Val Val Glu Asn Thr Gly Glu Val Leu Trp Phe Lys Asp Arg
       370                 375                 380
<210> 13
   <211> 1779
   <212> DNA
   <213> Mycospaerella graminicola
<400> 13
   ttttgccctt gccaagggac ccgtggcaga atggatcatc ttctgcggtg aattgggaga      60
   gtcgggggag agaggacgtg caccgcctat cacttgcggg tctttctgcc tgtaagcctg     120
   ggaaggggtc cgcctcacca ataaatttgt ctctggagct tcttctatct ccacgtctcc     180
   ttcttcttgc gacatcctgt tccaccctct cccacacatt ctcagacgcc attatgagca     240
   gaacagtcac attagaagcc gctccggcga cgacgctgtc ggtcgagaag aaaactccga     300
   ccattcgcca aatccgcaat gccatccccg agcactgctt caagcccaca gcgtggaaat     360
   cctccgccca cgccatcttc gactgctccg tcgccgcgct gatcgccttc gccgcctaca     420
   agaccatccc tcttgtcgag tactggcccg ctcgctgggc attgtgggcg ctgtacggtt     480
   acatcgaagg tctcgtcttc acgggcatct ggatcgtggc ccacgagtgc ggacacggcg     540
   gtctctacac cagcaactgg gccaacgaca tcgtcggata caccctccac acatccctct     600
   tggtgccgta ctttccctgg aagtacaccc acgcccgtca tcatcgctac accggacaca     660
   tggagaagga caccgcgttc gtgccgcacc gagcgggcga gaagagtatt gggagcaaga     720
   ttgcggaggt gattggacac gctgaggacg ctcctctgta tatgtttggt ggacttgtga     780
   tgcatcagct tttgggctgg caggcgtacc tcctcttcta cgtgagcgcc ggggcgcggt     840
   ccactcctaa ggctttggag ggctcgagct gggcgggaag ccatttcgac ccgatggcca     900
   acttgtggac gccttcgcag cggccattcg tgttcttgtc gacagtcggt ctcggtgcgg     960
   tcatgttcgc tctgtaccag ctctccggcg tgattggcgt agccaacaca ttcctgttat    1020
   acggcctgcc ctacctctgg gtcaacaact ggttgggtat gttatcctgc gatcctcgct    1080
   gggttgtctc ttcaattgct gacctgtgct ctttagtcgc catcacttac ctccaccaca    1140
   ctcacccgga cagccaccac tacgaggcct cgcgctggac cttcctcgac ggcgccttga    1200
   ccactgtcga ccgaccattt ggcttcatcg gccgcaaggt cttccacggc atcatcgact    1260
   tccacgtcgt tcaccacctc ttcccgtgag cattatcgta gcaactcctt tctgtgatcc    1320
   agaactgact tccctcctct tccagatcca tgccattcta ccacgccgaa gaagccacca    1380
   aagccatgag gcccgttctc ggcgactact accgccgtga cgacaccccg ttctggatcg    1440
   cattgtggaa gacattctca agctgccagg cggtgcagcc caaagaggga gaggagggag    1500
   ttctcgaatg ggagacgaag aagacgcatg ttaagagcgc gtgatcgttt ggcatattga    1560
   gcgaggcgtt gcatatcgtg actctctcga atgagtcttc gtggattctg aaaagcatta    1620
   gaatgagaac ggatacatta aaggcgaatt atagaggcta gatgatgtat tacatgagga    1680
   atttcatgat catgcggatg atgtgtcgcg tgaggggtct gtgacactgt tgccgctgag    1740
   atccgtcgag ctgatgcaat gtggtggtct gtgcttttg                            1779
<210> 14
   <211> 1191
   <212> DNA
   <213> Mycospaerella graminicola
<400> 14 <210> 15

   <211> 396
   <212> PRT
   <213> Mycospaerella graminicola
<400> 15
<210> 16
   <211> 2301
   <212> DNA
   <213> Mycospaerella graminicola
<400> 16
   actgtctccg atcaatggca aagagtgagc aaagaaagag tcggtgtatg ggaatctggt     60
   cggaccgctg ggaccaggtc tagaagaccc ttggttcagt cccatctcac tctaatgcga    120
   gccccggcgc tagctcaaaa tctcccaaat catacaacaa aactgccatt ccccgaggcc    180
   acagttcctc ctttgctaca caatccgctc gtccttggtc tcgtctgcgt tgtgttcttg    240
   tcttctgcca ttcgacgtct tgcagctgca gtatcgcctt accaccgtct ctatcctcag    300
   ctccccacct ctcccactgg gcgacctctt tcccaacgcg ccaagtttca agagctgtgc    360
   tctcaaccac ttgagacggc agcgacatct ctcgccagaa cttcgaccga gaagacactc    420
   ttctctcgaa ataactccac cacacggcac gccgcagtct atacgataca gtcgccaaaa    480
   tgagcaccac cgccctctcc cagaagccgg ccatgagacg ccatgtcacg gcggagtcct    540
   ctccctcgac cgccgctggt tcccccaatg attcgccggc agcctccgcc tcgtcgacat    600
   ccctctcatc gatgggcgac gacgactacc agcaaaagcc agcctccagc aagctcatcg    660
   atacctacgg caacgagttc aagatgccag actacacaat caaggacatc cgtgatgcca    720
   ttcccaagca ttgctttgag cgatccgccg caacgggcct gtactatgtc gccagggata    780
   tcgtgctcct gagcaccaca tttttcttgt tcaacaagtt ctgcacaccg gaatacgttc    840
   cctcataccc agctcgtgca gctctgtggg cagcatacac attcattcag ggtcttttcg     900
   gcacaggtct gtgggtcttg gcccacgagt gcggtcacca gtccttctcg cccagcaaga     960
   ctctcaatga caccgttgga tggatctgcc actctgctct tcttgtcccg tacttcagct    1020
   ggaaaatctc ccacggcaag caccacaagg cgactggcaa catggagcgc gacatggtct    1080
   ttctcccacg cactcgcgat gagcacgcta cccgcactgg cgcccttctc cacgagatgc    1140
   acgagctgat ggaggagact ccaatctaca ccgccgtctc catgatcgtc cagcagctcg    1200
   gcggatggcc aatgtacctg atccagaacc tgaccggcca caacaaccac gagaagcagc    1260
   ctgagggcaa gggtgtcggc aaaaagaatg gcaacggcag cgtcaaccac ttcttgccaa    1320
   gcagccctct ctacgagaag aaggacgagc acctcatcct cctctccgac ttgggtcttg    1380
   ccattaccgc ttccgtcttg acctacgtcg gcaagaccta cggcttcacc aacctcctcg    1440
   tctggtacat cctcccatac ctctgggtga accactggct cgtggccatc acctacctcc    1500
   agcacaccga tccttctctc cctcactaca ctggtgacgc ctggaacttc gcccgcggtg    1560
   ccgccgcgac catcgaccgc gaattcggct tcatcggccg caacctcctc cacggtatcg    1620
   tcgagaccca cgttctccac cactacgtct ctaccatccc cttctaccac gccgacgagg    1680
   cgaccgaggc catcaagcca atcatgggcc agcactaccg cgccgatgtc gagggcggct    1740
   cgatcggatt cttgaaggca atgtggaagt ccgctcgttg gtgccagtgg gtcgagcctc    1800
   tgcctggtac cactggcgag gaatccaagg tgctgttctt caggaacagg aacggtcttg    1860
   gtgtgccgcc ggcgaagctc tctcccaagg ccggcaagag ggccatggtt gaggatgatt    1920
   ccgaatagac tccgaataga ttagaacatc gaaagagatg gacatggaaa tgaatgggtg    1980
   gttttgaaag tgtctttagc cggctttatt taggggttta aggtggcgtt ttccgacgag    2040
   gtggtatgtc gaggcagatg acgcttagaa gcaagttatt gtatacatct tgttcgtcaa    2100
   gatcactttc cgcacctgat cgatttgatg taatgcaggg caagtgttga acgtggacag    2160
   gttcgatgcg acatgacttg agcttgagat gcccagttgc aaagtcacat ggttaagcag    2220
   gttacgcaag ctctcgttgc aggaacgtcg gtttggatcg atcgatgagt gtccattact    2280
   ggctttcacg ccacgccgta t                                               2301

<210> 17
   <211> 1449
   <212> DNA
   <213> Mycospaerella graminicola
<400> 17
   atgagcacca ccgccctctc ccagaagccg gccatgagac gccatgtcac ggcggagtcc      60
   tctccctcga ccgccgctgg ttcccccaat gattcgccgg cagcctccgc ctcgtcgaca     120
   tccctctcat cgatgggcga cgacgactac cagcaaaagc cagcctccag caagctcatc     180
   gatacctacg gcaacgagtt caagatgcca gactacacaa tcaaggacat ccgtgatgcc     240
   attcccaagc attgctttga gcgatccgcc gcaacgggcc tgtactatgt cgccagggat     300
   atcgtgctcc tgagcaccac atttttcttg ttcaacaagt tctgcacacc ggaatacgtt     360
   ccctcatacc cagctcgtgc agctctgtgg gcagcataca cattcattca gggtcttttc     420
   ggcacaggtc tgtgggtctt ggcccacgag tgcggtcacc agtccttctc gcccagcaag     480
   actctcaatg acaccgttgg atggatctgc cactctgctc ttcttgtccc gtacttcagc     540
   tggaaaatct cccacggcaa gcaccacaag gcgactggca acatggagcg cgacatggtc     600
   tttctcccac gcactcgcga tgagcacgct acccgcactg gcgcccttct ccacgagatg     660
   cacgagctga tggaggagac tccaatctac accgccgtct ccatgatcgt ccagcagctc     720
   ggcggatggc caatgtacct gatccagaac ctgaccggcc acaacaacca cgagaagcag     780
   cctgagggca agggtgtcgg caaaaagaat ggcaacggca gcgtcaacca cttcttgcca     840
   agcagccctc tctacgagaa gaaggacgag cacctcatcc tcctctccga cttgggtctt     900
   gccattaccg cttccgtctt gacctacgtc ggcaagacct acggcttcac caacctcctc     960
   gtctggtaca tcctcccata cctctgggtg aaccactggc tcgtggccat cacctacctc    1020
   cagcacaccg atccttctct ccctcactac actggtgacg cctggaactt cgcccgcggt    1080
   gccgccgcga ccatcgaccg cgaattcggc ttcatcggcc gcaacctcct ccacggtatc    1140
   gtcgagaccc acgttctcca ccactacgtc tctaccatcc ccttctacca cgccgacgag    1200
   gcgaccgagg ccatcaagcc aatcatgggc cagcactacc gcgccgatgt cgagggcggc    1260
   tcgatcggat tcttgaaggc aatgtggaag tccgctcgtt ggtgccagtg ggtcgagcct    1320
   ctgcctggta ccactggcga ggaatccaag gtgctgttct tcaggaacag gaacggtctt    1380
   ggtgtgccgc cggcgaagct ctctcccaag gccggcaaga gggccatggt tgaggatgat    1440
   tccgaatag                                                            1449
<210> 18
   <211> 482
   <212> PRT
   <213> Mycospaerella graminicola
<400> 18
   Met Ser Thr Thr Ala Leu Ser Gln Lys Pro Ala Met Arg Arg His Val
   1               5                   10                  15
   Thr Ala Glu Ser Ser Pro Ser Thr Ala Ala Gly Ser Pro Asn Asp Ser
               20                  25                  30
   Pro Ala Ala Ser Ala Ser Ser Thr Ser Leu Ser Ser Met Gly Asp Asp
           35                  40                  45
   Asp Tyr Gln Gln Lys Pro Ala Ser Ser Lys Leu Ile Asp Thr Tyr Gly
       50                  55                  60
   Asn Glu Phe Lys Met Pro Asp Tyr Thr Ile Lys Asp Ile Arg Asp Ala
   65                  70                  75                  80
   Ile Pro Lys His Cys Phe Glu Arg Ser Ala Ala Thr Gly Leu Tyr Tyr
                   85                  90                  95
   Val Ala Arg Asp Ile Val Leu Leu Ser Thr Thr Phe Phe Leu Phe Asn
   100 105 110
   Lys Phe Cys Thr Pro Glu Tyr Val Pro Ser Tyr Pro Ala Arg Ala Ala
   115 120 125
   Leu Trp Ala Ala Tyr Thr Phe Ile Gln Gly Leu Phe Gly Thr Gly Leu
   130 135 140
   Trp Val Leu Ala His Glu Cys Gly His Gln Ser Phe Ser Pro Ser Lys
   145 150 155 160
   Thr Leu Asn Asp Thr Val Gly Trp Ile Cys His Ser Ala Leu Leu Val
   165 170 175
   Pro Tyr Phe Ser Trp Lys Ile Ser His Gly Lys His His Lys Ala Thr
   180 185 190
   Gly Asn Met Glu Arg Asp Met Val Phe Leu Pro Arg Thr Arg Asp Glu
   195 200 205
   His Ala Thr Arg Thr Gly Ala Leu Leu His Glu Met His Glu Leu Met
   210 215 220
   Glu Glu Thr Pro Ile Tyr Thr Ala Val Ser Met Ile Val Gln Gln Leu
   225 230 235 240
   Gly Gly Trp Pro Met Tyr Leu Ile Gln Asn Leu Thr Gly His Asn Asn
   245 250 255
   His Glu Lys Gln Pro Glu Gly Lys Gly Val Gly Lys Lys Asn Gly Asn
   260 265 270
   Gly Ser Val Asn His Phe Leu Pro Ser Ser Pro Leu Tyr Glu Lys Lys
   275 280 285
   Asp Glu His Leu Ile Leu Leu Ser Asp Leu Gly Leu Ala Ile Thr Ala
   290 295 300
   Ser Val Leu Thr Tyr Val Gly Lys Thr Tyr Gly Phe Thr Asn Leu Leu
   305 310 315 320
   Val Trp Tyr Ile Leu Pro Tyr Leu Trp Val Asn His Trp Leu Val Ala
   325 330 335
   Ile Thr Tyr Leu Gln His Thr Asp Pro Ser Leu Pro His Tyr Thr Gly
   340 345 350
   Asp Ala Trp Asn Phe Ala Arg Gly Ala Ala Ala Thr Ile Asp Arg Glu
   355 360 365
   Phe Gly Phe Ile Gly Arg Asn Leu Leu His Gly Ile Val Glu Thr His
       370                 375                 380
   Val Leu His His Tyr Val Ser Thr Ile Pro Phe Tyr His Ala Asp Glu
   385                 390                 395                 400
   Ala Thr Glu Ala Ile Lys Pro Ile Met Gly Gln His Tyr Arg Ala Asp
                   405                 410                 415
   Val Glu Gly Gly Ser Ile Gly Phe Leu Lys Ala Met Trp Lys Ser Ala
               420                 425                 430
   Arg Trp Cys Gln Trp Val Glu Pro Leu Pro Gly Thr Thr Gly Glu Glu
           435                 440                 445
   Ser Lys Val Leu Phe Phe Arg Asn Arg Asn Gly Leu Gly Val Pro Pro
       450                 455                 460
   Ala Lys Leu Ser Pro Lys Ala Gly Lys Arg Ala Met Val Glu Asp Asp
   465                 470                 475                 480
   Ser Glu
<210> 19
   <211> 1906
   <212> DNA
   <213> Naegleria gruberi
<400> 19
   gcatcctaaa cagtatgaca gagttactgt tcggatagca atccggtctt tttatccatc      60
   gcactaggat atgcttgaaa tttccgtatt ttcaaaaaga tctcgaaaga aaaaaaatgt     120
   cagctgccac atcagaatct tgcgctcaac aagctgaaaa gtatgacaaa ctctcttcac     180
   tctaccacgg agatgccatt cccttcactc ttcgtgaatt aaaggaagcc atcccaaaac     240
   attgcttcca aagacctgtc atgaccagta tgctccatct catttcagat ttggttcaat     300
   taattgccta tactgttgcc tattattact tttccaattt gttagaatcg tggtggtttg     360
   ctaatttgaa tcaaattgta gtgttgagtc caatttatta cttgttgcag ggtgtcttgt     420
   tttgtggtta tgtttttatt gaaggtttta catttactgg attgtgggtt cttcaggtag     480
   gttaattatt attcgattag gtttatatat tatggatttc atctttatgt ttcgaataat     540
   tcgtgtgaaa ttgtgtgaaa tatttcgaac aatttgtgtg aatattttat tatgattgta     600
   gcatgaatgt ggacattatg cttttgctga tagtcctctt gtttgtgata ttgtcggtta     660
   cattgtcgga agtgccttgt tggttccata ttttgcttgg caagtaagtt tagtcaacca     720
   gagaatttgt taatttttta ctggaatatt tgaatatttt ttgaaattct tcagaaatca     780
   catgctattc accatgcaaa taccaatcac atgacaagag atcaaacctg ggttcctcaa     840
   aatgtccaac caattccttc taataatcca gctcaaaaat ctcaagttga caaacaagtt     900
   gaaatgaaac caaaatctga atcatctctt tctgccatta ctgatatgat tgttatggca     960
   actattggtt ggccacttca cttggtgctc aatgtttctg gtccagttaa ggaaaatgtt    1020
   gtggcctatt atgcaaaggg cattcgttat gaaccaacta ttattaccga aagtgatgga    1080
   aactctcaag atgatgaaaa tgaatattct gactataagc aaatttcaca aaagtatcca    1140
   atcaaattca cctctcactt tttaccatcc tctccaatct ttaatagtag agaaacctta    1200
   aagatttact tgtctaattt tggagtaatt gccatgttat tcattctcta tcaattattc    1260
   caaatttatg gatggcaagt gatggtcact gtctatgtct taccactcag tattaatttc    1320
   ttcctattga cctcaattac ctttttacaa cacgtccacg atgacgttcc tcatttggat    1380
   gaaggtgagt ggaattggtt gaagggagct ttgtgtacca ttgatcgttc gatgggttca    1440
   ttcttggatt ccaaattgca tcacattact gatacacatg tgtgtcatca tgtcttttca    1500
   aagattccat tctatcatgc tgaggaagca acgaaagcaa ttaagggaaa acttggaaat    1560
   tattatagag atgagactga taagagtttc tttggtgctt tattcgaaaa tttgaagaat    1620
   tgtgttgcct tgaagagaaa tgaaaagttt agaggaattt tgtggtggga tcattaaact    1680
   tgaataaata gcagattcat ttacaatatt ctaaaggttt cttctttttc aataatattt    1740
   aactccttct tgaaagattg atatcaaaga cgaaaacata ataagcaaaa ggttcagata    1800
   agtttgatga aatactttct gtttattgag attcaaaatt aatttagaaa gcaatgaaga    1860
   aggatgaaac caaagcaaca ataaccatga tgagcatatt cattcc                   1906
<210> 20
   <211> 1197
   <212> DNA
   <213> Naegleria gruberi
<400> 20
   atgtcagctg ccacatcaga atcttgcgct caacaagctg aaaagtatga caaactctct     60
   tcactctacc acggagatgc cattcccttc actcttcgtg aattaaagga agccatccca    120
   aaacattgct tccaaagacc tgtcatgacc agtatgctcc atctcatttc agatttggtt    180
   caattaattg cctatactgt tgcctattat tacttttcca atttgttaga atctccaatt    240
   tattacttgt tgcagggtgt cttgttttgt ggttatgttt ttattgaagg ttttacattt    300
   actggattgt gggttcttca gcatgaatgt ggacattatg cttttgctga tagtcctctt    360
   gtttgtgata ttgtcggtta cattgtcgga agtgccttgt tggttccata ttttgcttgg    420
   caaaaatcac atgctattca ccatgcaaat accaatcaca tgacaagaga tcaaacctgg    480
   gttcctcaaa atgtccaacc aattccttct aataatccag ctcaaaaatc tcaagttgac    540
   aaacaagttg aaatgaaacc aaaatctgaa tcatctcttt ctgccattac tgatatgatt    600
   gttatggcaa ctattggttg gccacttcac ttggtgctca atgtttctgg tccatatcca    660
   atcaaattca cctctcactt tttaccatcc tctccaatct ttaatagtag agaaacctta    720
   aagatttact tgtctaattt tggagtaatt gccatgttat tcattctcta tcaattattc    780
   caaatttatg gatggcaagt gatggtcact gtctatgtct taccactcag tattaatttc    840
   ttcctattga cctcaattac ctttttacaa cacgtccacg atgacgttcc tcatttggat    900
   gaaggtgagt ggaattggtt gaagggagct ttgtgtacca ttgatcgttc gatgggttca    960
   ttcttggatt ccaaattgca tcacattact gatacacatg tgtgtcatca tgtcttttca   1020
   aagattccat tctatcatgc tgaggaagca acgaaagcaa ttaagggaaa acttggaaat   1080
   tattatagag atgagactga taagagtttc tttggtgctt tattcgaaaa tttgaagaat   1140
   tgtgttgcct tgaagagaaa tgaaaagttt agaggaattt tgtggtggga tcattaa      1197
<210> 21
   <211> 398
   <212> PRT
   <213> Naegleria gruberi
<400> 21
   Met Ser Ala Ala Thr Ser Glu Ser Cys Ala Gln Gln Ala Glu Lys Tyr
   1 5 10 15
   Asp Lys Leu Ser Ser Leu Tyr His Gly Asp Ala Ile Pro Phe Thr Leu
   20 25 30
   Arg Glu Leu Lys Glu Ala Ile Pro Lys His Cys Phe Gln Arg Pro Val
   35 40 45
   Met Thr Ser Met Leu His Leu Ile Ser Asp Leu Val Gln Leu Ile Ala
   50 55 60
   Tyr Thr Val Ala Tyr Tyr Tyr Phe Ser Asn Leu Leu Glu Ser Pro Ile
   65 70 75 80
   Tyr Tyr Leu Leu Gln Gly Val Leu Phe Cys Gly Tyr Val Phe Ile Glu
   85 90 95
   Gly Phe Thr Phe Thr Gly Leu Trp Val Leu Gln His Glu Cys Gly His
   100 105 110
   Tyr Ala Phe Ala Asp Ser Pro Leu Val Cys Asp Ile Val Gly Tyr Ile
   115 120 125
   Val Gly Ser Ala Leu Leu Val Pro Tyr Phe Ala Trp Gln Lys Ser His
   130 135 140
   Ala Ile His His Ala Asn Thr Asn His Met Thr Arg Asp Gln Thr Trp
   145 150 155 160
   Val Pro Gln Asn Val Gln Pro Ile Pro Ser Asn Asn Pro Ala Gln Lys
   165 170 175
   Ser Gln Val Asp Lys Gln Val Glu Met Lys Pro Lys Ser Glu Ser Ser
   180 185 190
   Leu Ser Ala Ile Thr Asp Met Ile Val Met Ala Thr Ile Gly Trp Pro
           195                 200                 205
   Leu His Leu Val Leu Asn Val Ser Gly Pro Tyr Pro Ile Lys Phe Thr
       210                 215                 220
   Ser His Phe Leu Pro Ser Ser Pro Ile Phe Asn Ser Arg Glu Thr Leu
   225                 230                 235                 240
   Lys Ile Tyr Leu Ser Asn Phe Gly Val Ile Ala Met Leu Phe Ile Leu
                   245                 250                 255
   Tyr Gln Leu Phe Gln Ile Tyr Gly Trp Gln Val Met Val Thr Val Tyr
               260                 265                 270
   Val Leu Pro Leu Ser Ile Asn Phe Phe Leu Leu Thr Ser Ile Thr Phe
           275                 280                 285
   Leu Gln His Val His Asp Asp Val Pro His Leu Asp Glu Gly Glu Trp
       290                 295                 300
   Asn Trp Leu Lys Gly Ala Leu Cys Thr Ile Asp Arg Ser Met Gly Ser
   305                 310                 315                 320
   Phe Leu Asp Ser Lys Leu His His Ile Thr Asp Thr His Val Cys His
                   325                 330                 335
   His Val Phe Ser Lys Ile Pro Phe Tyr His Ala Glu Glu Ala Thr Lys
               340                 345                 350
   Ala Ile Lys Gly Lys Leu Gly Asn Tyr Tyr Arg Asp Glu Thr Asp Lys
           355                 360                 365
   Ser Phe Phe Gly Ala Leu Phe Glu Asn Leu Lys Asn Cys Val Ala Leu
       370                 375                 380
   Lys Arg Asn Glu Lys Phe Arg Gly Ile Leu Trp Trp Asp His
   385                 390                 395
<210> 22
   <211> 1740
   <212> DNA
   <213> Phycomyces blakesleeanus
<400> 22
   caagggggga gaagggggga gaaggaaaca tacggttact atattctgat aggtaaatct     60
   tgctgttcaa tgtctgtgta ataaaaccgg agttatttta acttttgtat attcctacaa   120
   aaaaaacttg tttaaaagac actggtttaa ccaaagcttt tcgtttttta tttgtgtctt   180
   tcatccttat attatttctt atgtcggata acactgaatc aatcaagaca gaaaagcaag   240
   cactcaattt agacgaggca attgccaaag gatgggagat tccggatttt actatcaagg   300
   aaatccgtga tgctattcct tcccactgct tccgtattga cacttttcgg tcctttggat   360
   atgttttaca cgatttcttt tttgtcgcac tactaatgta cggtgccagc aagattgaca   420
   ctctgtcatc tccatatatt cgatttgtcc tatgggctac ctacagtgtc ttacaaggaa   480
   ttgtgggtac tggtctttgg gttatcggtc atgaatgtgg acatcaagca ttcagtcctt   540
   caaaggtaca tggatcaatt atatatttat agtatgcgct tttttcttac taacactaac   600
   attattatta ttattatata tagacaataa acaatagcgt tggtataatt atccacagcc   660
   tattgcttgt tccatactac tcttggaaga tttctcattc aaagcatcac aagtctaatg   720
   ggcacttgta caatgatatg gtaactatct cttctatata aaagctatat tatcgcaaac   780
   agccatatta ttaaactatt acttatataa atacatgccc atctttttgc aggtatatgt   840
   tcctagaaca cgatcgcata gaaagctagg acctaaagaa gaggaccatg agctcgacgg   900
   acctcacagt gctcttatgg aatcccctat tgtagaaaca gcaaagatct tcagactggt   960
   tgtgttcggg tgggtttgtt atgtcttact tgacgtaacc ggaaagcgtc caaagaaaac  1020
   ctggacatca catttcaact tcaattgcca catctttgag gaacataact atgcagactt  1080
   tgtcaagtca aacattggat tagctgtcgc aatctctggc ctagtctgtg ctggccaaat  1140
   ttacggctcg atgactgtaa tgaagtatta tgtgttccct tatctttttg tcaactgctg  1200
   gcttgtcttg atcacctatc tccagcacac cgactcagcg attccccgtt atagtcccaa  1260
   tgtgtggaac ttccaacgtg gcgctgctct cacagttgat cgctcatatg ggcccatact  1320
   gaatcatttc caccaccaca tttcggacac tcatgtagcc catcatttct tttctaatat  1380
   gccacactat catgcagtag aagccactaa gcacatcaag aaggttttgg gaaaacatta  1440
   tatgtctgat gacaccccta ttcttcaggc attgtacaaa tcatacagag aatgtaaatt  1500
   tattgaagac gaaggcgacg ttcgtttcct caagaattaa agaagaagaa aaagaaaata  1560
   atcacacaca cacacacaca cacacatata tatatatata tatccattac acatacacac  1620
   acgatataca catcacaaat cacatatcat acattagata tcgcttttgt atttctatta  1680
   ttattattca atacatgtat tcaaaataaa ataaacggaa atatttaacc acaaatcaaa  1740
<210> 23
   <211> 1170
   <212> DNA
   <213> Phycomyces blakesleeanus
<400> 23
   atgtcggata acactgaatc aatcaagaca gaaaagcaag cactcaattt agacgaggca     60
   attgccaaag gatgggagat tccggatttt actatcaagg aaatccgtga tgctattcct    120
   tcccactgct tccgtattga cacttttcgg tcctttggat atgttttaca cgatttcttt    180
   tttgtcgcac tactaatgta cggtgccagc aagattgaca ctctgtcatc tccatatatt    240
   cgatttgtcc tatgggctac ctacagtgtc ttacaaggaa ttgtgggtac tggtctttgg    300
   gttatcggtc atgaatgtgg acatcaagca ttcagtcctt caaagacaat aaacaatagc    360
   gttggtataa ttatccacag cctattgctt gttccatact actcttggaa gatttctcat    420
   tcaaagcatc acaagtctaa tgggcacttg tacaatgata tggtatatgt tcctagaaca    480
   cgatcgcata gaaagctagg acctaaagaa gaggaccatg agctcgacgg acctcacagt    540
   gctcttatgg aatcccctat tgtagaaaca gcaaagatct tcagactggt tgtgttcggg    600
   tgggtttgtt atgtcttact tgacgtaacc ggaaagcgtc caaagaaaac ctggacatca    660
   catttcaact tcaattgcca catctttgag gaacataact atgcagactt tgtcaagtca    720
   aacattggat tagctgtcgc aatctctggc ctagtctgtg ctggccaaat ttacggctcg    780
   atgactgtaa tgaagtatta tgtgttccct tatctttttg tcaactgctg gcttgtcttg    840
   atcacctatc tccagcacac cgactcagcg attccccgtt atagtcccaa tgtgtggaac    900
   ttccaacgtg gcgctgctct cacagttgat cgctcatatg ggcccatact gaatcatttc    960
   caccaccaca tttcggacac tcatgtagcc catcatttct tttctaatat gccacactat   1020
   catgcagtag aagccactaa gcacatcaag aaggttttgg gaaaacatta tatgtctgat   1080
   gacaccccta ttcttcaggc attgtacaaa tcatacagag aatgtaaatt tattgaagac   1140
   gaaggcgacg ttcgtttcct caagaattaa                                    1170

<210> 24
   <211> 389
   <212> PRT
   <213> Phycomyces blakesleeanus
<400> 24
   Met Ser Asp Asn Thr Glu Ser Ile Lys Thr Glu Lys Gln Ala Leu Asn
   1               5                   10                  15
   Leu Asp Glu Ala Ile Ala Lys Gly Trp Glu Ile Pro Asp Phe Thr Ile
               20                  25                  30
   Lys Glu Ile Arg Asp Ala Ile Pro Ser His Cys Phe Arg Ile Asp Thr
           35                  40                  45
   Phe Arg Ser Phe Gly Tyr Val Leu His Asp Phe Phe Phe Val Ala Leu
       50                  55                  60
   Leu Met Tyr Gly Ala Ser Lys Ile Asp Thr Leu Ser Ser Pro Tyr Ile
   65                  70                  75                  80
   Arg Phe Val Leu Trp Ala Thr Tyr Ser Val Leu Gln Gly Ile Val Gly
                   85                  90                  95
   Thr Gly Leu Trp Val Ile Gly His Glu Cys Gly His Gln Ala Phe Ser
               100                 105                 110
   Pro Ser Lys Thr Ile Asn Asn Ser Val Gly Ile Ile Ile His Ser Leu
           115                 120                 125
   Leu Leu Val Pro Tyr Tyr Ser Trp Lys Ile Ser His Ser Lys His His
       130                 135                 140
   Lys Ser Asn Gly His Leu Tyr Asn Asp Met Val Tyr Val Pro Arg Thr
   145 150 155 160
   Arg Ser His Arg Lys Leu Gly Pro Lys Glu Glu Asp His Glu Leu Asp
   165 170 175
   Gly Pro His Ser Ala Leu Met Glu Ser Pro Ile Val Glu Thr Ala Lys
   180 185 190
   Ile Phe Arg Leu Val Val Phe Gly Trp Val Cys Tyr Val Leu Leu Asp
   195 200 205
   Val Thr Gly Lys Arg Pro Lys Lys Thr Trp Thr Ser His Phe Asn Phe
   210 215 220
   Asn Cys His Ile Phe Glu Glu His Asn Tyr Ala Asp Phe Val Lys Ser
   225 230 235 240
   Asn Ile Gly Leu Ala Val Ala Ile Ser Gly Leu Val Cys Ala Gly Gln
   245 250 255
   Ile Tyr Gly Ser Met Thr Val Met Lys Tyr Tyr Val Phe Pro Tyr Leu
   260 265 270
   Phe Val Asn Cys Trp Leu Val Leu Ile Thr Tyr Leu Gln His Thr Asp
   275 280 285
   Ser Ala Ile Pro Arg Tyr Ser Pro Asn Val Trp Asn Phe Gln Arg Gly
   290 295 300
   Ala Ala Leu Thr Val Asp Arg Ser Tyr Gly Pro Ile Leu Asn His Phe
   305 310 315 320
   His His His Ile Ser Asp Thr His Val Ala His His Phe Phe Ser Asn
   325 330 335
   Met Pro His Tyr His Ala Val Glu Ala Thr Lys His Ile Lys Lys Val
   340 345 350
   Leu Gly Lys His Tyr Met Ser Asp Asp Thr Pro Ile Leu Gln Ala Leu
   355 360 365
   Tyr Lys Ser Tyr Arg Glu Cys Lys Phe Ile Glu Asp Glu Gly Asp Val
   370 375 380
   Arg Phe Leu Lys Asn
   385
<210> 25
   <211> 2243
   <212> DNA
   <213> Nematostella vectensis
<400> 25
   tatgcgctga ataaaatcat aaaaatagtt tgataacact tgattttgta aaacatagag     60
   ttccgatgga tgatataaag tattctacat ttaaaccacc attttaaaga gaaaaaagat    120
   acattttata tggaaaaata ttattccctc aatattggcg atacttgtaa ttatccatta    180
   cattatttat caattgtttc aatgttttaa atccgattat cttccagctt ttggcagtta    240
   tgttcacttt ttgtgtgagc gcgaactgtt tttttttaga cgtaaacatt gcgtgcgttg     300
   ttacactctc tttcacacac aactcgtgct ttccatcaga cccgacaaac gcgccaaaca    360
   tgccgccgtg tcacgcaaca accggaagtg gcgagcagca agtcatgcaa tccacatctg    420
   atgacaccaa aaacgaacac gagaaaacct tgccattcaa gccgaaaatc gtttggttaa    480
   acgtgggttt tattctggtt cttcacgtta tggccttcta tgggatattt cttcttccac    540
   agctgaaatt ctggacgatg atttggacga tagcttgtca ctttgccggc ggctatggcg    600
   tgacaatagg tgcgcaccga ctgtgggctc acaggacctt caaggcaaaa tggccgcttc    660
   gcctcgtgat tatgctaatg aacagcatgg ctgcgcagaa cgacatcttc gagtggtcac    720
   gtgatcatcg cgtgcatcac aagtactcgg agaccgatgc tgacccccat aatgcaaagc    780
   gcgggttctt tttctctcac gtgggctggc tgatgcagag aaaacacccg gatgttatac    840
   ggaaagggaa aggaattgac ctgagcgact tgtacgccga cagcgtggtc atgttccaaa    900
   ggaggtaagt tggagaggaa ctggatttgg gggggggggg gggggtattt aggggaggga    960
   gagggggggg ggtactcccg tatatagctg ggtgtctaaa taactttttt caccaccgct   1020
   gccagcctcc ttcttaagaa aaaaatcttg ctcgctgtga acaacagccc acattttccc   1080
   gaaagctctg ccactgatcc ttctcaaaac tgcggccagt tcccgtttaa cccggtagat   1140
   tccctgtaaa caggggtacg caagtgtata aggcagatat tcagatagac ggtgaaaaca   1200
   gacaaaagga ataaacagat aaaagcaaaa ttgtttggaa aacgaataga aaaaagatga   1260
   cacttagaaa actaagggga cttaatgtaa cttagagtcc gttcaagatc gttgtattaa   1320
   aaatggctgt gacagaccgc taaaaatagg caaatgcaga caggcagaaa actgactggc   1380
   gaacagacgg aaagggtata tttacgtcaa tgacactatg tttcttcgtt cttaaggcat   1440
   tacaagaaaa tctccatgtt gatgtgtgtg cttatcccta cgctggtacc cagtctctgg   1500
   ggcgagtctc tgtggaacgc ctacttcaca tcattcgcac tccgttacgt catcaccctc   1560
   aacgtcacgt ggtgtgtcaa cagcatcgcg cacatgtggg gcgacaagcc ttatgacgtc   1620
   accattaacc cagcagagaa cctcttcgtt accttggcaa cgtcaggcga gggttaccat   1680
   aactaccatc acacttttcc ccaggactac gcggctagcg agtttggtac tcgcctcaac   1740
   atgagtacga ggcttattga tatgtgggcc gcgatgggac tagtcagcga tcgaaaggtg   1800
   gttcccaagg agacgattcg taagaggatg atgcggactg gcgcgctaag gagcttggga   1860
   gaaaactgtg aaaaggacta aatacttatc agacttcata cacgcatttg caattaaaga   1920
   atataactta ttggatatat agattttttg tatcataggc atgacttttt tatatcaagt   1980
   ccggataatc gagaaaaaat aatttattta attttgctga atttgtattt tgcgccctaa   2040
   tgtgcaacgg caacggccac agttttcaga aactaacata acgatgcggg agacgtactt   2100
   tttgattgac attcacttca attagccaat gaagagctca ggaagccaat gaagtgccca 2160
   gaggctcgct tcgaaatgag aatgatgtaa ccaacatcag tgatattgat aagctattgt 2220
   tgttatttat tttcgttggt tgc 2243
<210> 26
   <211> 990
   <212> DNA
   <213> Nematostella vectensis
<400> 26
   atgccgccgt gtcacgcaac aaccggaagt ggcgagcagc aagtcatgca atccacatct 60
   gatgacacca aaaacgaaca cgagaaaacc ttgccattca agccgaaaat cgtttggtta 120
   aacgtgggtt ttattctggt tcttcacgtt atggccttct atgggatatt tcttcttcca 180
   cagctgaaat tctggacgat gatttggacg atagcttgtc actttgccgg cggctatggc 240
   gtgacaatag gtgcgcaccg actgtgggct cacaggacct tcaaggcaaa atggccgctt 300
   cgcctcgtga ttatgctaat gaacagcatg gctgcgcaga acgacatctt cgagtggtca 360
   cgtgatcatc gcgtgcatca caagtactcg gagaccgatg ctgaccccca taatgcaaag 420
   cgcgggttct ttttctctca cgtgggctgg ctgatgcaga gaaaacaccc ggatgttata 480
   cggaaaggga aaggaattga cctgagcgac ttgtacgccg acagcgtggt catgttccaa 540
   aggaggcatt acaagaaaat ctccatgttg atgtgtgtgc ttatccctac gctggtaccc 600
   agtctctggg gcgagtctct gtggaacgcc tacttcacat cattcgcact ccgttacgtc 660
   atcaccctca acgtcacgtg gtgtgtcaac agcatcgcgc acatgtgggg cgacaagcct 720
   tatgacgtca ccattaaccc agcagagaac ctcttcgtta ccttggcaac gtcaggcgag 780
   ggttaccata actaccatca cacttttccc caggactacg cggctagcga gtttggtact 840
   cgcctcaaca tgagtacgag gcttattgat atgtgggccg cgatgggact agtcagcgat 900
   cgaaaggtgg ttcccaagga gacgattcgt aagaggatga tgcggactgg cgcgctaagg 960
   agcttgggag aaaactgtga aaaggactaa 990
<210> 27
   <211> 329
   <212> PRT
   <213> Nematostella vectensis
<400> 27
<210> 28
   <211> 1380
   <212> DNA
   <213> Pythium irregulare
<400> 28
   atggtggacc tcaagcctgg agtgaagcgc ctggtgagct ggaaggagat ccgcgagcac      60
   gcgacgcccg cgaccgcgtg gatcgtgatt caccacaagg tctacgacat ctccaagtgg     120
   gactcgcacc cgggtggctc cgtgatgctc acgcaggccg gcgaggacgc cacggacgcc     180
   ttcgcggtct tccacccgtc ctcggcgctc aagctgctcg agcagttcta cgtcggcgac     240
   gtggacgaaa cctccaaggc cgagatcgag ggggagccgg cgagcgacga ggagcgcgcg     300
   cgccgcgagc gcatcaacga gttcatcgcg tcctaccgtc gtctgcgcgt caaggtcaag     360
   ggcatggggc tctacgacgc cagcgcgctc tactacgcgt ggaagctcgt gagcacgttc     420
   ggcatcgcgg tgctctcgat ggcgatctgc ttcttcttca acagtttcgc catgtacatg     480
   gtcgccggcg tgattatggg gctcttctac cagcagtccg gatggctggc gcacgacttc     540
   ttgcacaacc aggtgtgcga gaaccgcacg ctcggcaacc ttatcggctg cctcgtgggc     600
   aacgcctggc agggcttcag cgtgcagtgg tggaagaaca agcacaacct gcaccacgcg     660
   gtgccgaacc tgcacagcgc caaggacgag ggcttcatcg gcgacccgga catcgacacc     720
   atgccgctgc tggcgtggtc taaggagatg gcgcgcaagg cgttcgagtc ggcgcacggc     780
   ccgttcttca tccgcaacca ggcgttccta tacttcccgc tgctgctgct cgcgcgcctg     840
   agctggctcg cgcagtcgtt cttctacgtg ttcaccgagt tctcgttcgg catcttcgac     900
   aaggtcgagt tcgacggacc ggagaaggcg ggtctgatcg tgcactacat ctggcagctc     960
   gcgatcccgt acttctgcaa catgagcctg tttgagggcg tggcatactt cctcatgggc    1020
   caggcgtcct gcggcttgct cctggcgctg gtgttcagta ttggccacaa cggcatgtcg    1080
   gtgtacgagc gcgaaaccaa gccggacttc tggcagctgc aggtgaccac gacgcgcaac    1140
   atccgcgcgt cggtattcat ggactggttc accggtggct tgaactacca gatcgaccat    1200
   cacctgttcc cgctcgtgcc gcgccacaac ttgccaaagg tcaacgtgct catcaagtcg    1260
   ctatgcaagg agttcgacat cccgttccac gagaccggct tctgggaggg catctacgag    1320
   gtcgtggacc acctggcgga catcagcaag gaattcatca ccgagttccc agcgatgtaa    1380
<210> 29
   <211> 1320
   <212> DNA
   <213> Thraustochytrium ssp.
<400> 29
   atgggcaagg gcagcgaggg ccgcagcgcg gcgcgcgaga tgacggccga ggcgaacggc      60
   gacaagcgga aaacgattct gatcgagggc gtcctgtacg acgcgacgaa ctttaagcac     120
   ccgggcggtt cgatcatcaa cttcttgacc gagggcgagg ccggcgtgga cgcgacgcag     180
   gcgtaccgcg agtttcatca gcggtccggc aaggccgaca agtacctcaa gtcgctgccg     240
   aagctggatg cgtccaaggt ggagtcgcgg ttctcggcca aagagcaggc gcggcgcgac     300
   gccatgacgc gcgactacgc ggcctttcgc gaggagctcg tcgccgaggg gtactttgac     360
   ccgtcgatcc cgcacatgat ttaccgcgtc gtggagatcg tggcgctctt cgcgctctcg     420
   ttctggctca tgtccaaggc ctcgcccacc tcgctcgtgc tgggcgtggt gatgaacggc     480
   attgcgcagg gccgctgcgg ctgggtcatg cacgagatgg gccacgggtc gttcacgggc     540
   gtcatctggc tcgacgaccg gatgtgcgag ttcttctacg gcgtcggctg cggcatgagc     600
   gggcactact ggaagaacca gcacagcaag caccacgccg cgcccaaccg cctcgagcac     660
   gatgtcgatc tcaacacgct gcccctggtc gcctttaacg agcgcgtcgt gcgcaaggtc     720
   aagccgggat cgctgctggc gctctggctg cgcgtgcagg cgtacctctt tgcgcccgtc     780
   tcgtgcctgc tcatcggcct tggctggacg ctctacctgc acccgcgcta catgctgcgc     840
   accaagcggc acatggagtt cgtctggatc ttcgcgcgct acattggctg gttctcgctc     900
   atgggcgctc tcggctactc gccgggcacc tcggtcggga tgtacctgtg ctcgttcggc     960
   ctcggctgca tttacatttt cctgcagttc gccgtcagcc acacgcacct gccggtgacc    1020
   aacccggagg accagctgca ctggctcgag tacgcggccg accacacggt gaacattagc    1080
   accaagtcct ggctcgtcac gtggtggatg tcgaacctga actttcagat cgagcaccac    1140
   ctcttcccca cggcgccgca gttccgcttc aaggaaatca gtcctcgcgt cgaggccctc    1200
   ttcaagcgcc acaacctccc gtactacgac ctgccctaca cgagcgcggt ctcgaccacc    1260
   tttgccaatc tttattccgt cggccactcg gtcggcgccg acaccaagaa gcaggactga    1320
<210> 30
   <211> 1086
   <212> DNA
   <213> Phytophtora infestans
<400> 30
   atggcgacga aggaggcgta tgtgttcccc actctgacgg agatcaagcg gtcgctacct      60
   aaagactgtt tcgaggcttc ggtgcctctg tcgctctact acaccgtgcg ttgtctggtg     120
   atcgcggtgg ctctaacctt cggtctcaac tacgctcgcg ctctgcccga ggtcgagagc     180
   ttctgggctc tggacgccgc actctgcacg ggctacatct tgctgcaggg catcgtgttc     240
   tggggcttct tcacggtggg ccacgatgcc ggccacggcg ccttctcgcg ctaccacctg     300
   cttaacttcg tggtgggcac tttcatgcac tcgctcatcc tcacgccctt cgagtcgtgg     360
   aagctcacgc accgtcacca ccacaagaac acgggcaaca ttgaccgtga cgaggtcttc     420
   tacccgcaac gcaaggccga cgaccacccg ctgtctcgca acctgattct ggcgctcggg     480
   gcagcgtggc tcgcctattt ggtcgagggc ttccctcctc gtaaggtcaa ccacttcaac     540
   ccgttcgagc ctctgttcgt gcgtcaggtg tcagctgtgg taatctctct tctcgcccac     600
   ttcttcgtgg ccggactctc catctatctg agcctccagc tgggccttaa gacgatggca     660
   atctactact atggacctgt ttttgtgttc ggcagcatgc tggtcattac caccttccta     720
   caccacaatg atgaggagac cccatggtac gccgactcgg agtggacgta cgtcaagggc     780
   aacctctcgt ccgtggaccg atcgtacggc gcgctcattg acaacctgag ccacaacatc     840
   ggcacgcacc agatccacca ccttttccct atcattccgc actacaaact caagaaagcc     900
   actgcggcct tccaccaggc tttccctgag ctcgtgcgca agagcgacga gccaattatc     960
   aaggctttct tccgggttgg acgtctctac gcaaactacg gcgttgtgga ccaggaggcg    1020
   aagctcttca cgctaaagga agccaaggcg gcgaccgagg cggcggccaa gaccaagtcc    1080
   acgtaa                                                               1086
<210> 31
   <211> 873
   <212> DNA
   <213> Physcomitrella patens
<400> 31
   atggaggtcg tggagagatt ctacggtgag ttggatggga aggtctcgca gggcgtgaat    60
   gcattgctgg gtagttttgg ggtggagttg acggatacgc ccactaccaa aggcttgccc   120
   ctcgttgaca gtcccacacc catcgtcctc ggtgtttctg tatacttgac tattgtcatt   180
   ggagggcttt tgtggataaa ggccagggat ctgaaaccgc gcgcctcgga gccatttttg   240
   ctccaagctt tggtgcttgt gcacaacctg ttctgttttg cgctcagtct gtatatgtgc   300
   gtgggcatcg cttatcaggc tattacctgg cggtactctc tctggggcaa tgcatacaat   360
   cctaaacata aagagatggc gattctggta tacttgttct acatgtctaa gtacgtggaa   420
   ttcatggata ccgttatcat gatactgaag cgcagcacca ggcaaataag cttcctccac   480
   gtttatcatc attcttcaat ttccctcatt tggtgggcta ttgctcatca cgctcctggc   540
   ggtgaagcat attggtctgc ggctctgaac tcaggagtgc atgttctcat gtatgcgtat   600
   tacttcttgg ctgcctgcct tcgaagtagc ccaaagttaa aaaataagta ccttttttgg    660
   ggcaggtact tgacacaatt ccaaatgttc cagtttatgc tgaacttagt gcaggcttac   720
   tacgacatga aaacgaatgc gccatatcca caatggctga tcaagatttt gttctactac   780
   atgatctcgt tgctgtttct tttcggcaat ttttacgtac aaaaatacat caaaccctct   840
   gacggaaagc aaaagggagc taaaactgag tga                                 873
<210> 32
   <211> 903
   <212> DNA
   <213> Ostreococcus tauri
<400> 32
   atgagcgcct ccggtgcgct gctgcccgcg atcgcgttcg ccgcgtacgc gtacgcgacg    60
   tacgcctacg cctttgagtg gtcgcacgcg aatggcatcg acaacgtcga cgcgcgcgag   120
   tggatcggtg cgctgtcgtt gaggctcccg gcgatcgcga cgacgatgta cctgttgttc   180
   tgcctggtcg gaccgaggtt gatggcgaag cgcgaggcgt tcgacccgaa ggggttcatg   240
   ctggcgtaca atgcgtatca gacggcgttc aacgtcgtcg tgctcgggat gttcgcgcga   300
   gagatctcgg ggctggggca gcccgtgtgg gggtcaacca tgccgtggag cgatagaaaa   360
   tcgtttaaga tcctcctcgg ggtgtggttg cactacaaca acaaatattt ggagctattg   420
   gacactgtgt tcatggttgc gcgcaagaag acgaagcagt tgagcttctt gcacgtttat 480
   catcacgccc tgttgatctg ggcgtggtgg ttggtgtgtc acttgatggc cacgaacgat 540
   tgtatcgatg cctacttcgg cgcggcgtgc aactcgttca ttcacatcgt gatgtactcg 600
   tattatctca tgtcggcgct cggcattcga tgcccgtgga agcgatacat cacccaggct 660
   caaatgctcc aattcgtcat tgtcttcgcg cacgccgtgt tcgtgctgcg tcagaagcac 720
   tgcccggtca cccttccttg ggcgcaaatg ttcgtcatga cgaacatgct cgtgctcttc 780
   gggaacttct acctcaaggc gtactcgaac aagtcgcgcg gcgacggcgc gagttccgtg 840
   aaaccagccg agaccacgcg cgcgcccagc gtgcgacgca cgcgatctcg aaaaattgac 900
   taa 903
<210> 33
   <211> 1560
   <212> DNA
   <213> Thraustochytrium ssp.
<400> 33
   atgacggtcg gctacgacga ggagatcccg ttcgagcagg tccgcgcgca caacaagccg 60
   gatgacgcct ggtgcgcgat ccacgggcac gtgtacgatg tgaccaagtt cgcgagcgtg 120
   cacccgggcg gcgacattat cctgctggcc gcaggcaagg aggccaccgt gctgtacgag 180
   acttaccatg tgcggggcgt ctcggacgcg gtgctgcgca agtaccgcat cggcaagctg 240
   ccggacggcc aaggcggcgc gaacgagaag gaaaagcgga cgctctcggg cctctcgtcg 300
   gcctcgtact acacgtggaa cagcgacttt tacagggtaa tgcgcgagcg cgtcgtggct 360
   cggctcaagg agcgcggcaa ggcccgccgc ggaggctacg agctctggat caaggcgttc 420
   ctgctgctcg tcggcttctg gagctcgctg tactggatgt gcacgctgga cccctcgttc 480
   ggggccatcc tggccgccat gtcgctgggc gtctttgccg cctttgtggg cacgtgcatc 540
   cagcacgacg gcaaccacgg cgcctttgcc cagtcgcgat gggtcaacaa ggttgccggg 600
   tggacgctcg acatgatcgg cgccagcggc atgacgtggg agttccagca cgtcctgggc 660
   caccatccgt acacgaacct gatcgaggag gagaacggcc tgcaaaaggt gagcggcaag 720
   aagatggaca ccaagctggc cgaccaggag agcgatccgg acgtcttttc cacgtacccg 780
   atgatgcgcc tgcacccgtg gcaccagaag cgctggtacc accgtttcca gcacatttac 840
   ggccccttca tctttggctt catgaccatc aacaaggtgg tcacgcagga cgtcggtgtg 900
   gtgctccgca agcggctctt ccagattgac gccgagtgcc ggtacgcgag cccaatgtac 960
   gtggcgcgtt tctggatcat gaaggcgctc acggtgctct acatggtggc cctgccgtgc 1020
   tacatgcagg gcccgtggca cggcctcaag ctgttcgcga tcgcgcactt tacgtgcggc 1080
   gaggtgctcg caaccatgtt cattgtgaac cacatcatcg agggcgtctc gtacgcttcc 1140
   aaggacgcgg tcaagggcac gatggcgccg ccgaagacga tgcacggcgt gacgcccatg 1200
   aacaacacgc gcaaggaggt ggaggcggag gcgtccaagt ctggcgccgt ggtcaagtca 1260
   gtcccgctcg acgactgggc cgccgtccag tgccagacct cggtgaactg gagcgtcggc 1320
   tcgtggttct ggaatcactt ttccggcggc ctcaaccacc agattgagca ccacctgttc 1380
   cccgggctca gccacgagac gtactaccac atccaggacg tcgttcagtc cacctgcgcc 1440
   gagtacggcg tcccgtacca gcacgagcct tcgctctgga ccgcgtactg gaagatgctc 1500
   gagcacctcc gtcagctcgg caatgaggag acccacgagt cctggcagcg cgctgcctga 1560

<210> 34
   <211> 20
   <212> DNA
   <213> Artificial
<220>
   <223> Primer
<400> 34
   atggcttcta cggctgtgcc 20
<210> 35
   <211> 19
   <212> DNA
   <213> Artificial
<220>
   <223> Primer
<400> 35
   ttaggcggcg ggagggtcg 19
<210> 36
   <211> 20
   <212> DNA
   <213> Artificial
<220>
   <223> Primer
<400> 36
   atggctactc gacagcgtac 20
<210> 37
   <211> 22
   <212> DNA
   <213> Artificial
<220>
   <223> Primer
<400> 37
   ctactccttg gcccatcgca tg 22
<210> 38
   <211> 20
   <212> DNA
   <213> Artificial
<220>
   <223> Primer
<400> 38
   atggctacta ccacgacggt 20
<210> 39
   <211> 23
   <212> DNA
   <213> Artificial
<220>
   <223> Primer
<400> 39
   tcattgcgcc cagtgcagag ctc 23
<210> 40
   <211> 21
   <212> DNA
   <213> Artificial
<220>
   <223> Primer
<400> 40
   atgacggtgg cttcccaggt g 21
<210> 41
   <211> 21
   <212> DNA
   <213> Artificial
<220>
   <223> Primer
<400> 41
   ttacctgtcc ttgaaccaaa g 21
<210> 42
   <211> 21
   <212> DNA
   <213> Artificial
<220>
   <223> Primer
<400> 42
   atgagcagaa cagtcacatt a 21
<210> 43
   <211> 20
   <212> DNA
   <213> Artificial
<220>
   <223> Primer
<400> 43
   tcacgcgctc ttaacatgcg 20
<210> 44
   <211> 20
   <212> DNA
   <213> Artificial
<220>
   <223> Primer
<400> 44
   atgagcacca ccgccctctc 20
<210> 45
   <211> 19
   <212> DNA
   <213> Artificial
<220>
   <223> Primer
<400> 45
   ctattcggaa tcatcctca 19
<210> 46
   <211> 20
   <212> DNA
   <213> Artificial
<220>
   <223> Primer
<400> 46
   atgtcagctg ccacatcaga 20
<210> 47
   <211> 20
   <212> DNA
   <213> Artificial
<220>
   <223> Primer

<400> 47
   ttaatgatcc caccacaaaa 20
<210> 48
   <211> 20
   <212> DNA
   <213> Artificial
<220>
   <223> Primer
<400> 48
   atgtcggata acactgaatc 20
<210> 49
   <211> 20
   <212> DNA
   <213> Artificial
<220>
   <223> Primer
<400> 49
   ttaattcttg aggaaacgaa 20
<210> 50
   <211> 20
   <212> DNA
   <213> Artificial
<220>
   <223> Primer
<400> 50
   atgccgccgt gtcacgcaac 20
<210> 51
   <211> 21
   <212> DNA
   <213> Artificial
<220>
   <223> Primer
<400> 51
   ttagtccttt tcacagtttt c 21
<210> 52
   <211> 31
   <212> DNA
   <213> Artificial
<220>
   <223> Primer
<400> 52
   gcggccgcgc catggtggac ctcaagcctg g 31
<210> 53
   <211> 27
   <212> DNA
   <213> Artificial
<220>
   <223> Primer
<400> 53
   gcggccgtta catcgctggg aactcgg 27
<210> 54
   <211> 31
   <212> DNA
   <213> Artificial
<220>
   <223> Primer
<400> 54
   gcggccgcgc catgggcaag ggcagcgagg g 31
<210> 55
   <211> 32
   <212> DNA
   <213> Artificial
<220>
   <223> Primer
<400> 55
   gcggccgcgc ctcagtcctg cttcttggtg tc 32
<210> 56
   <211> 31
   <212> DNA
   <213> Artificial
<220>
   <223> Primer
<400> 56
   gcggccgcgc catggcgacg aaggaggcgt a 31
<210> 57
   <211> 31
   <212> DNA
   <213> Artificial
<220>
   <223> Primer
<400> 57
   gcggccgcgt tacgtggact tggtcttggc c 31
<210> 58
   <211> 32
   <212> DNA
   <213> Artificial
<220>
   <223> Primer
<400> 58
   gcggccgcgc catggaggtc gtggagagat tc 32
<210> 59
   <211> 28
   <212> DNA
   <213> Artificial
<220>
   <223> Primer
<400> 59
   gcggccgcgt cactcagttt tagctccc 28
<210> 60
   <211> 31
   <212> DNA
   <213> Artificial
<220>
   <223> Primer

<400> 60
   gcggccgcgc catggcttct acggctgtgc c 31
<210> 61
   <211> 29
   <212> DNA
   <213> Artificial
<220>
   <223> Primer
<400> 61
   gcggccgcgt taggcggcgg gagggtcga 29
<210> 62
   <211> 31
   <212> DNA
   <213> Artificial
<220>
   <223> Primer
<400> 62
   gcggccgcgc catggctact cgacagcgta c 31
<210> 63
   <211> 31
   <212> DNA
   <213> Artificial
<220>
   <223> Primer
<400> 63
   gcggccgcgc tactccttgg cccatcgcat g 31
<210> 64
   <211> 32
   <212> DNA
   <213> Artificial
<220>
   <223> Primer
<400> 64
   gcggccgcgc catggctact accacgacgg tc 32
<210> 65
   <211> 29
   <212> DNA
   <213> Artificial
<220>
   <223> Primer
<400> 65
   gcggccgcgt cattgcgccc agtgcagag 29
<210> 66
   <211> 32
   <212> DNA
   <213> Artificial
<220>
   <223> Primer
<400> 66
   gcggccgcgc catgacggtg gcttcccagg tg 32
<210> 67
   <211> 30
   <212> DNA
   <213> Artificial
<220>
   <223> Primer
<400> 67
   gcggccgcgt tacctgtcct tgaaccaaag 30
<210> 68
   <211> 32
   <212> DNA
   <213> Artificial
<220>
   <223> Primer
<400> 68
   gcggccgcgc catgagcaga acagtcacat ta 32
<210> 69
   <211> 29
   <212> DNA
   <213> Artificial
<220>
   <223> Primer
<400> 69
   gcggccgcgt cacgcgctct taacatgcg 29
<210> 70
   <211> 31
   <212> DNA
   <213> Artificial
<220>
   <223> Primer
<400> 70
   gcggccgcgc catgagcacc accgccctct c 31
<210> 71
   <211> 30
   <212> DNA
   <213> Artificial
<220>
   <223> Primer
<400> 71
   gcggccgcgc tattcggaat catcctcaac 30
<210> 72
   <211> 30
   <212> DNA
   <213> Artificial
<220>
   <223> Primer
<400> 72
   gcggccgcgc catgtcagct gccacatcag 30
<210> 73
   <211> 29
   <212> DNA
   <213> Artificial
<220>
   <223> Primer
<400> 73
   gcggccgcgt taatgatccc accacaaaa 29
<210> 74
   <211> 31
   <212> DNA
   <213> Artificial
<220>
   <223> Primer
<400> 74
   gcggccgcgc catgtcggat aacactgaat c 31
<210> 75
   <211> 29
   <212> DNA
   <213> Artificial
<220>
   <223> Primer
<400> 75
   gcggccgcgt taattcttga ggaaacgaa 29

<210> 76
   <211> 31
   <212> DNA
   <213> Artificial
<220>
   <223> Primer
<400> 76
   gcggccgcgc catgccgccg tgtcacgcaa c 31
<210> 77
   <211> 30
   <212> DNA
   <213> Artificial
<220>
   <223> Primer
<400> 77
   gcggccgcgt tagtcctttt cacagttttc 30
<210> 78
   <211> 32
   <212> DNA
   <213> Artificial
<220>
   <223> Primer
<400> 78
   gcggccgcgc catgagcgcc tccggtgcgc tg 32
<210> 79
   <211> 23
   <212> DNA
   <213> Artificial
<220>
   <223> Primer
<400> 79
   gcggccgcgt tagtcaattt ttc 23
<210> 80
   <211> 32
   <212> DNA
   <213> Artificial
<220>
   <223> Primer
<400> 80
   gcggccgcgc catgacggtc ggctacgacg ag 32
<210> 81
   <211> 29
   <212> DNA
   <213> Artificial
<220>
   <223> Primer
<400> 81
   gcggccgcgt caggcagcgc gctgccagg 29
<210> 82
   <211> 60
   <212> DNA
   <213> Artificial
<220>
   <223> Sequencing Primer
<400> 82
   gtcgacccgc ggactagtgg gccctctaga cccgggggat ccggatctgc tggctatgaa 60
<210> 83
   <211> 9
   <212> PRT
   <213> Artificial
<220>
   <223> Desaturase Motif 1
<220>
   <221> misc_feature
   <222> (2)..(3)
   <223> Xaa can be any naturally occurring amino acid
<220>
   <221> misc_feature
   <222> (6)..(6)
   <223> Xaa can be any naturally occurring amino acid
<220>
   <221> misc_feature
   <222> (8)..(8)
   <223> Xaa can be any naturally occurring amino acid
<400> 83
   Leu Xaa Xaa Ile Thr Xaa Leu Xaa His
   1               5
<210> 84
   <211> 8
   <212> PRT
   <213> Artificial
<220>
   <223> Desaturase Motif 2
<220>
<221> misc_feature
<222> (3)..(4)
<223> Xaa can be any naturally occurring amino acid
<220>
   <221> misc_feature
   <222> (6)..(6)
   <223> Xaa can be any naturally occurring amino acid
<400> 84
   Gly Ala Xaa Xaa Thr Xaa Asp Arg
   1               5
<210> 85
   <211> 5
   <212> PRT
   <213> Artificial
<220>
   <223> Desaturase Motif 3
<220>
   <221> misc_feature
   <222> (3)..(3)
   <223> Xaa can be any naturally occurring amino acid
<400> 85
   His Val Xaa His His
   1               5
<210> 86
   <211> 29
   <212> DNA
   <213> Artificial
<220>
   <223> Primer

<400> 86
   gcggccgcgc catggctgtt aaaacggac 29
<210> 87
   <211> 29
   <212> DNA
   <213> Artificial
<220>
   <223> Primer
<400> 87
   gcggccgcgc tatttttcaa cctcaatac 29
<210> 88
   <211> 18
   <212> DNA
   <213> Artificial
<220>
   <223> Primer
<400> 88
   atggctgtta aaacggac 18
<210> 89
   <211> 20
   <212> DNA
   <213> Artificial
<220>
   <223> Primer
<400> 89
   ctatttttca acctcaatac 20
<210> 90
   <211> 2336
   <212> DNA
   <213> Laccaria bicolor
<400> 90
   ttttttcttt ctgtgggtcg tgtctgggat ttgggcgaag gctgggcaaa cttacgagtt 60
   cataaatagc tcaaatacaa ttggcagttg gaatgccaag aaaatgatcc ccaggcgaaa 120
   aaaaaaaaaa aaagactcgg caaactccgt ccaaccccca gaaaatgtag acgctaaaat 180
   ccaatcgccg ctgtttacgt ctcttcgctc ttgccctctt aagtatacca caaggttccc 240
   tcgccggtac ctacgttctc tcctttcaat ggctgttaaa acggacaaaa acggcgtcac 300
   cgtcgtcgag caaggggtct acaacgttcc cgacatccct atcaaggatt tactcgacgc 360
   tattccgtta gtcctctttg ttcttcatct cgctggcact aagttgtcca accaattagc 420
   aaacactgct tccagcgttc tgctttgagg tcgtccgtct acatgtgcgt tcttttcctt 480
   tccagacgtg caacgcacgt ggctcattgc tcgttccttt tccagtttct gggatatctt 540
   cgttattggg tgcatctaca agactgcaag ttacctggac acgttcatca atcccgccac 600
   tatatccctt ccccatcctt acctttatcc ttttgctcga ttcgctctct ggtcgttgta 660
   cggtttctgg gttggcctct ttgcgacagg tctctgggtt gttgcccacg aatgtggtca 720
   ccaggctttc tcagagtcaa aaaccatcaa caatacagtt ggctgggtcc ttcactcggc 780
   gtaggtcgat cggcaattaa ttttcgtgtc actgctaacg tggcatgtga tttcagctta 840
   ggggttccat accaagcttg gcgtatcacc cacgcaaagc atcacgcctc taccggccat 900
   atgactcagg atcaggtctt cgtgccctct acccgctcag atgttggcct ccctagcctc 960
   aacgtcgcaa aagaagaccg attgggttca cgtgtatcgg aggaagtgaa aagggaattc 1020
   aaagaagcat taggcgattc gccgattggc gcaatcattg gttcggcaac ctacttggtc 1080
   agttcgcgtc tggtttcgcg cactaactgt gtctgacgtg ttcttcactt ttaaaagctc 1140
   ggaggatggc ccgcatatat ccttaccaac gcttctggcc agcgcagata ccccaaaggc 1200
   accagtcgta cgtatcatca gcgacctgtt ggagggactg gttctcaatc ttgtcttaca 1260
   gactttaatc ccaaggctgt gatgttctca ccccatcatt actcccagat cattgtttct 1320
   aacatcggag tcgcactctg gttgggcatc attggtacat ggatatacta caagggcttt 1380
   tcggaggtct tccgccttta cctcgtccct tacctttggt acattctcat caatcaaagg 1440
   aagaatatca gttgattgac attttttaac agggccaatc actggcttgt gttgatcacc 1500
   ttcctccaac ataccgaccc tctcctcccc cattaccgtg cccctgagtt cacattccct 1560
   cgtggtgctt tggccactct ggatcgcagc cttcttggag actgtggctc tatcatggcg 1620
   tggatgggcg cacacgccac acatggtatt tctgagacgc atatccttca tcacgtttcg 1680
   agcaagattc ctcattataa tgcgtaagtg ccagagttct accagttgtt ctatgccata 1740
   atgttcttct tgtcagttgg gaagcgagtg cggccctcaa gaagaaactt gctagcgcag 1800
   gtatcccgat gcaaggtggc cctggtggct ggcgtgaagt ttaccgcgtc tacagggaat 1860
   gcaaggtttg tctcatgctt ccagtgttgg acctcttcta acgtatttga tagtttgttg 1920
   aggatgaggg tgatgttgtc ttcttcaaaa actccgaagg catcgccaag atgaggccgg 1980
   tcctccctga gtcgtccgca agtgactctg gtattgaggt tgaaaaatag atagagagtt 2040
   agatttaacc atctagattc acctgccgtc gcctgcgctg taacatagag tggttggatt 2100
   tgtcgcaaac ccctaatgag atttttcttg gatgatatct cgatgaatcc gaacgactag 2160
   acaagaaacc tgctccacta tgggcatgct tgcaaattca tttattttga aatgatttat 2220
   cttacaagaa cgcagattaa atcctactct ctttgacagt ttccttccaa atttctgcaa 2280
   attcttcgat gttccgtcca tttcctaagc tgagagcctc gaatgggatc atattg 2336
<210> 91
   <211> 1323
   <212> DNA
   <213> Laccaria bicolor
<400> 91
   atggctgtta aaacggacaa aaacggcgtc accgtcgtcg agcaaggggt ctacaacgtt      60
   cccgacatcc ctatcaagga tttactcgac gctattccca aacactgctt ccagcgttct     120
   gctttgaggt cgtccgtcta cattttctgg gatatcttcg ttattgggtg catctacaag     180
   actgcaagtt acctggacac gttcatcaat cccgccacta tatcccttcc ccatccttac     240
   ctttatcctt ttgctcgatt cgctctctgg tcgttgtacg gtttctgggt tggcctcttt     300
   gcgacaggtc tctgggttgt tgcccacgaa tgtggtcacc aggctttctc agagtcaaaa     360
   accatcaaca atacagttgg ctgggtcctt cactcggcct taggggttcc ataccaagct     420
   tggcgtatca cccacgcaaa gcatcacgcc tctaccggcc atatgactca ggatcaggtc     480
   ttcgtgccct ctacccgctc agatgttggc ctccctagcc tcaacgtcgc aaaagaagac     540
   cgattgggtt cacgtgtatc ggaggaagtg aaaagggaat tcaaagaagc attaggcgat     600
   tcgccgattg gcgcaatcat tggttcggca acctacttgc tcggaggatg gcccgcatat     660
   atccttacca acgcttctgg ccagcgcaga taccccaaag gcaccagtca ctttaatccc     720
   aaggctgtga tgttctcacc ccatcattac tcccagatca ttgtttctaa catcggagtc     780
   gcactctggt tgggcatcat tggtacatgg atatactaca agggcttttc ggaggtcttc     840
   cgcctttacc tcgtccctta cctttgggcc aatcactggc ttgtgttgat caccttcctc     900
   caacataccg accctctcct cccccattac cgtgcccctg agttcacatt ccctcgtggt     960
   gctttggcca ctctggatcg cagccttctt ggagactgtg gctctatcat ggcgtggatg    1020
   ggcgcacacg ccacacacgg tatttctgag acgcacatcc ttcaccacgt ttcgagcaag    1080
   attcctcact ataatgcttg ggaagcgagt gcggccctca agaagaaact tgctagcgca    1140
   ggtatcccga tgcaaggtgg ccctggtggc tggcgtgaag tttaccgcgt ctacagggaa    1200
   tgcaagtttg ttgaggatga gggtgatgtt gtcttcttca aaaactccga aggcatcgcc    1260
   aagatgaggc cggtcctccc tgagtcgtcc gcaagtgact ctggtattga ggttgaaaaa    1320
   tag                                                                  1323
<210> 92
   <211> 440
   <212> PRT
   <213> Laccaria bicolor
<400> 92
   Met Ala Val Lys Thr Asp Lys Asn Gly Val Thr Val Val Glu Gln Gly
   1               5                   10                  15
   Val Tyr Asn Val Pro Asp Ile Pro Ile Lys Asp Leu Leu Asp Ala Ile
               20                  25                  30
   Pro Lys His Cys Phe Gln Arg Ser Ala Leu Arg Ser Ser Val Tyr Ile
           35                  40                  45
   Phe Trp Asp Ile Phe Val Ile Gly Cys Ile Tyr Lys Thr Ala Ser Tyr
       50                  55                  60
   Leu Asp Thr Phe Ile Asn Pro Ala Thr Ile Ser Leu Pro His Pro Tyr
   65                  70                  75                  80
   Leu Tyr Pro Phe Ala Arg Phe Ala Leu Trp Ser Leu Tyr Gly Phe Trp
                   85                  90                  95
   Val Gly Leu Phe Ala Thr Gly Leu Trp Val Val Ala His Glu Cys Gly
               100                 105                 110
   His Gln Ala Phe Ser Glu Ser Lys Thr Ile Asn Asn Thr Val Gly Trp
           115                 120                 125
   Val Leu His Ser Ala Leu Gly Val Pro Tyr Gln Ala Trp Arg Ile Thr
   130 135 140
   His Ala Lys His His Ala Ser Thr Gly His Met Thr Gln Asp Gln Val
   145 150 155 160
   Phe Val Pro Ser Thr Arg Ser Asp Val Gly Leu Pro Ser Leu Asn Val
   165 170 175
   Ala Lys Glu Asp Arg Leu Gly Ser Arg Val Ser Glu Glu Val Lys Arg
   180 185 190
   Glu Phe Lys Glu Ala Leu Gly Asp Ser Pro Ile Gly Ala Ile Ile Gly
   195 200 205
   Ser Ala Thr Tyr Leu Leu Gly Gly Trp Pro Ala Tyr Ile Leu Thr Asn
   210 215 220
   Ala Ser Gly Gln Arg Arg Tyr Pro Lys Gly Thr Ser His Phe Asn Pro
   225 230 235 240
   Lys Ala Val Met Phe Ser Pro His His Tyr Ser Gln Ile Ile Val Ser
   245 250 255
   Asn Ile Gly Val Ala Leu Trp Leu Gly Ile Ile Gly Thr Trp Ile Tyr
   260 265 270
   Tyr Lys Gly Phe Ser Glu Val Phe Arg Leu Tyr Leu Val Pro Tyr Leu
   275 280 285
   Trp Ala Asn His Trp Leu Val Leu Ile Thr Phe Leu Gln His Thr Asp
   290 295 300
   Pro Leu Leu Pro His Tyr Arg Ala Pro Glu Phe Thr Phe Pro Arg Gly
   305 310 315 320
   Ala Leu Ala Thr Leu Asp Arg Ser Leu Leu Gly Asp Cys Gly Ser Ile
   325 330 335
   Met Ala Trp Met Gly Ala His Ala Thr His Gly Ile Ser Glu Thr His
   340 345 350
   Ile Leu His His Val Ser Ser Lys Ile Pro His Tyr Asn Ala Trp Glu
   355 360 365
   Ala Ser Ala Ala Leu Lys Lys Lys Leu Ala Ser Ala Gly Ile Pro Met
   370 375 380
   Gln Gly Gly Pro Gly Gly Trp Arg Glu Val Tyr Arg Val Tyr Arg Glu
   385 390 395 400
   Cys Lys Phe Val Glu Asp Glu Gly Asp Val Val Phe Phe Lys Asn Ser
   405 410 415
   Glu Gly Ile Ala Lys Met Arg Pro Val Leu Pro Glu Ser Ser Ala Ser
   420 425 430
   Asp Ser Gly Ile Glu Val Glu Lys
   435 440
<210> 93
   <211> 19
   <212> DNA
   <213> Artificial
<220>
   <223> Primer
<400> 93
   atgaactgtg taactgagg 19
<210> 94
   <211> 21
   <212> DNA
   <213> Artificial
<220>
   <223> Primer
<400> 94
   tatttgtaat aaacctctaa c 21
<210> 95
   <211> 20
   <212> DNA
   <213> Artificial
<220>
   <223> Primer
<400> 95
   atggaaacca aatcaggaag 20
<210> 96
   <211> 18
   <212> DNA
   <213> Artificial
<220>
   <223> Primer
<400> 96
   ttatgtataa atatgtac 18
<210> 97
   <211> 21
   <212> DNA
   <213> Artificial

<220>
   <223> Primer
<400> 97
   atgaatgaag ccaataacca c 21
<210> 98
   <211> 22
   <212> DNA
   <213> Artificial
<220>
   <223> Primer
<400> 98
   ttatttatag taatgaattt tg 22
<210> 99
   <211> 19
   <212> DNA
   <213> Artificial
<220>
   <223> Primer
<400> 99
   atgcaatcaa acacagttc 19
<210> 100
   <211> 22
   <212> DNA
   <213> Artificial
<220>
   <223> Primer
<400> 100
   ttagtttgat cgcggatgtt tg 22
<210> 101
   <211> 22
   <212> DNA
   <213> Artificial
<220>
   <223> Primer
<400> 101
   atgtttctcg ccggaagtga tg 22
<210> 102
   <211> 20
   <212> DNA
   <213> Artificial
<220>
   <223> Primer
<400> 102
   tcaggctcct agatctttcc 20
<210> 103
   <211> 30
   <212> DNA
   <213> Artificial
<220>
   <223> Primer
<400> 103
   gcggccgcgc catgaactgt gtaactgagg 30
<210> 104
   <211> 30
   <212> DNA
   <213> Artificial
<220>
   <223> Primer
<400> 104
   gcggccgcgt atttgtaata aacctctaac 30
<210> 105
   <211> 31
   <212> DNA
   <213> Artificial
<220>
   <223> Primer
<400> 105
   gcggccgcgc catggaaacc aaatcaggaa g 31
<210> 106
   <211> 27
   <212> DNA
   <213> Artificial
<220>
   <223> Primer
<400> 106
   gcggccgcgt tatgtataaa tatgtac 27
<210> 107
   <211> 32
   <212> DNA
   <213> artificial
<220>
   <223> Primer

<400> 107
   gcggccgcgc catgaatgaa gccaataacc ac 32
<210> 108
   <211> 31
   <212> DNA
   <213> Artificial
<220>
   <223> Primer
<400> 108
   gcggccgcgt tatttatagt aatgaatttt g 31
<210> 109
   <211> 30
   <212> DNA
   <213> Artificial
<220>
   <223> Primer
<400> 109
   gcggccgcgc catgcaatca aacacagttc 30
<210> 110
   <211> 31
   <212> DNA
   <213> Artificial
<220>
   <223> Primer
<400> 110
   gcggccgcgt tagtttgatc gcggatgttt g 31
<210> 111
   <211> 33
   <212> DNA
   <213> Artificial
<220>
   <223> Primer
<400> 111
   gcggccgcgc catgtttctc gccggaagtg atg 33
<210> 112
   <211> 29
   <212> DNA
   <213> Artificial
<220>
   <223> Primer
<400> 112
   gcggccgcgt caggctccta gatctttcc 29
<210> 113
   <211> 7
   <212> PRT
   <213> Artificial
<220>
   <223> Desaturase motif 4
<220>
   <221> misc_feature
   <222> (2)..(3)
   <223> Xaa can be any naturally occurring amino acid
<220>
   <221> misc_feature
   <222> (6)..(7)
   <223> Xaa can be any naturally occurring amino acid
<400> 113
   Thr Xaa Xaa Gln His Xaa Xaa
   1               5
<210> 114
   <211> 7
   <212> PRT
   <213> Artificial
<220>
   <223> Desaturase motif 5
<220>
   <221> Variant
   <222> (2)..(2)
   <223> Xaa in position 2 is Tyr or Phe
<220>
   <221> variant
   <222> (3)..(3)
   <223> Xaa in position 3 is Leu or Met
<220>
   <221> Variant
   <222> (6)..(6)
   <223> Xaa in position 6 is Thr or Ser or Gln
<220>
   <221> Variant
   <222> (7)..(7)
   <223> Xaa in position 7 is Asp or His or Asn
<400> 114
   Thr Xaa Xaa Gln His Xaa Xaa
   1               5
<210> 115
   <211> 7
   <212> PRT
   <213> Artificial
<220>
   <223> Desaturase motif 6
<220>
   <221> Variant
   <222> (2)..(2)
   <223> Xaa in position 2 is Tyr or Phe
<220>
   <221> Variant
   <222> (6)..(6)
   <223> Xaa in position 6 is Thr or Ser
<220>
   <221> Variant
   <222> (7)..(7)
   <223> Xaa in position 7 is Asp or His
<400> 115
   Thr Xaa Leu Gln His Xaa Xaa
   1               5
<210> 116
   <211> 9
   <212> PRT
   <213> Artificial
<220>
   <223> Desaturase motif 7
<220>
   <221> misc_feature
   <222> (2)..(3)
   <223> Xaa can be any naturally occurring amino acid
<220>
   <221> misc_feature
   <222> (6)..(6)
   <223> Xaa can be any naturally occurring amino acid
<220>
   <221> Variant
   <222> (7)..(7)
   <223> Xaa in position 7 is Gly or no amino acid

<220>
   <221> Variant
   <222> (8)..(8)
   <223> Xaa in position 8 can be any naturally occurring amino acid or no
   amino acid
<220>
   <221> misc_feature
   <222> (9)..(9)
   <223> Xaa can be any naturally occurring amino acid
<400> 116
   Thr Xaa Xaa His His Xaa Xaa Xaa Xaa
   1               5
<210> 117
   <211> 9
   <212> PRT
   <213> Artificial
<220>
   <223> Desaturase motif 8
<220>
   <221> Variant
   <222> (2)..(2)
   <223> Xaa in position 3 is Tyr or Phe
<220>
   <221> Variant
   <222> (3)..(3)
   <223> Xaa in position 3 is Leu or Met
<220>
   <221> Variant
   <222> (6)..(6)
   <223> Xaa in position 6 is His or Thr or ser or val
<220>
   <221> Variant
   <222> (7)..(7)
   <223> Xaa in position 7 is Gly or no amino acid
<220>
   <221> Variant
   <222> (8)..(8)
   <223> Xaa in position 8 is His or Tyr or no amino acid
<220>
   <221> Variant
   <222> (9)..(9)
   <223> Xaa in position 9 is His or Asp or Glu or Gly
<400> 117
   Thr Xaa Xaa His His Xaa Xaa Xaa Xaa
   1               5
<210> 118
   <211> 7
   <212> PRT
   <213> Artificial
<220>
   <223> Desaturase motif 9
<220>
   <221> variant
   <222> (2)..(2)
   <223> Xaa in position 2 is Tyr or Phe
<220>
   <221> Variant
   <222> (3)..(3)
   <223> Xaa in position 3 is Leu or Met
<220>
   <221> variant
   <222> (6)..(6)
   <223> Xaa in position 6 is His or Thr or Ser
<220>
   <221> variant
   <222> (7)..(7)
   <223> Xaa in position 7 is His or Asp or Glu
<400> 118
   Thr Xaa Xaa His His Xaa Xaa
   1               5
<210> 119
   <211> 9
   <212> PRT
   <213> Artificial
<220>
   <223> Desaturase motif 10
<220>
   <221> variant
   <222> (2)..(2)
   <223> Xaa in position 2 is Tyr or Phe
<220>
   <221> Variant
   <222> (3)..(3)
   <223> Xaa in position 3 is Leu or Met
<220>
   <221> Variant
   <222> (6)..(6)
   <223> Xaa in position 6 is His or Thr or Ser or Val
<220>
   <221> Variant
   <222> (8)..(8)
   <223> Xaa in position 8 is His or Tyr
<220>
   <221> Variant
   <222> (9)..(9)
   <223> Xaa in position 9 is His or Asp or Glu or Gly
<400> 119
   Thr Xaa Xaa His His Xaa Gly Xaa Xaa
   1               5
<210> 120
   <211> 7
   <212> PRT
   <213> Artificial
<220>
   <223> Desaturase motif 11
<220>
   <221> misc_feature
   <222> (2)..(3)
   <223> Xaa can be any naturally occurring amino acid
<220>
   <221> misc_feature
   <222> (7)..(7)
   <223> Xaa can be any naturally occurring amino acid
<400> 120
   Thr Xaa Xaa His His Asn Xaa
   1               5
<210> 121
   <211> 7
   <212> PRT
   <213> Artificial
<220>
   <223> Desaturase motif 12
<220>
   <221> Variant
   <222> (2)..(2)
   <223> Xaa in position 2 is Tyr or Phe
<220>
   <221> Variant
   <222> (3)..(3)
   <223> Xaa in position 3 is Leu or Met
<220>
   <221> Variant
   <222> (7)..(7)
   <223> Xaa in position 7 is His or Asp or Glu or Gly
<400> 121
   Thr Xaa Xaa His His Asn Xaa
   1               5

<210> 122
   <211> 1975
   <212> DNA
   <213> Helobdella robusta
<400> 122
   attacaaaac tcttcttaaa agagccgttg atatgataca aaagttttca tacatatttt    60
   tcctctgtta atttataaac agttgttaat tgacgttcaa taatagttac tataaaaaga   120
   attaattttg aaacaatcct ataaaaatct gcaaacttac ccaagccctc tgttaaatga   180
   tgttcccact gagcgatcta atgaactgtg taactgaggt taactccata atagcaagcc   240
   taataaaagc agcgttcctg tattcaaacc cgcaaactaa aatccgagga ataaacctgg   300
   acacccaact accaaaaaat ctgccatcag tcattgagat caagcgagtg ataccaagcc   360
   actgttttgt gccgagcacc tgccgatctc ttttgtacgc cctgaaggat gtggtgcaga   420
   tattatttgc ctgggtgctg ttgtggtacc tcctacccct gaccaattgg attgcgttga   480
   aggttttgat gattttcgtc tactggggta ttcaggggac ttttttcatg ggcctgtttg   540
   tgatggggca tgattgcggg catgggtcgt tctccaagta tcgtctgctt aatgatgtcg   600
   tagggaccat cagccacgct ttcctgttcg tcccttacta ccagtggaag ctcacccatc   660
   agaaccacca caaattcacc gggaatatgg acaaggatga ggtgagtttg cagtgttgct   720
   gacacgattg ttgtgaacaa tttcttcatt tttttttctt gcgttatttg atgtgacgta   780
   gttgaagatt ggtttcatta aaaaacatga cgcacaggcg aaataatatg tttgaagtat   840
   aaaaatgatt attataacca tttataattg tttataatta ttataaatat tataattatt   900
   gataatttcc aggtcttcta cccagcccga gcttcgcaga agccgagcat caacagcgtc   960
   ctccctggct ttggctacgg aatcggttgg ttcacgtacc tcttcattgg ctactttcca  1020
   cgaagggtca gccatttcaa tttgtttgac gagatgttca ggggccacga agttgcttgc  1080
   accctctcgt tgctcactta cgggatgaac gggactctct gctactggtt ttatttgagt  1140
   tacggattca agattctctt cgttttctac ctagcaccgc ttttcattta tgggagttat  1200
   atggttatcg ttaccttttt gcaccacagt gaagtcaata ttccttggta tgctgatcaa  1260
   aagttagtcg ctctcttcag tcattcattt caatttgttc aaattattca cattttaaaa  1320
   attttaagaa ttatttaaaa agcaatttca aagaaaaaat ttttttacat taaacaacaa  1380
   ctgaaactta aaatcaactt caactacaca tacttactca ctaaaaaaac accacaaaac  1440
   cgccacgaaa ccaccaaaaa tccacccacc acaaaattaa aaaaaaaatc ttgcccagct  1500
   ggaactacgt aaaaggtcaa ctctcaacca ttgaccgtaa ctacgggctg gtccaccacg  1560
   caatccactg cataggcacc caccaaatgc accacatgtt caccaaaata ccccactacc  1620
   acctcgagga ggccaccaga cattttagga gtgctttccc ggagttagtg aaatcctgcg  1680
   acgagcccat actttcgtca ttcgtacgca tgttcaagaa gtacaatcag cagcaggtgg  1740
   tcgctgataa cgcgttagag gtttattaca aataaataat aataaatatt attattatat  1800
   tatttattta ttactattat tattattatc gttattatta ctattattat taatactatc  1860
   attattgtta ttattattat tattttaata attttggctg cgggcattta cgattacaga  1920
   aagctttaca tcttcttttc agacttattg tgatatataa atgaaattat tactt       1975
<210> 123
   <211> 1128
   <212> DNA
   <213> Helobdella robusta
<400> 123
   atgaactgtg taactgaggt taactccata atagcaagcc taataaaagc agcgttcctg      60
   tattcaaacc cgcaaactaa aatccgagga ataaacctgg acacccaact accaaaaaat     120
   ctgccatcag tcattgagat caagcgagtg ataccaagcc actgttttgt gccgagcacc     180
   tgccgatctc ttttgtacgc cctgaaggat gtggtgcaga tattatttgc ctgggtgctg     240
   ttgtggtacc tcctacccct gaccaattgg attgcgttga aggttttgat gattttcgtc     300
   tactggggta ttcaggggac ttttttcatg ggcctgtttg tgatggggca tgattgcggg     360
   catgggtcgt tctccaagta tcgtctgctt aatgatgtcg tagggaccat cagccacgct     420
   ttcctgttcg tcccttacta ccagtggaag ctcacccatc agaaccacca caaattcacc     480
   gggaatatgg acaaggatga ggtcttctac ccagcccgag cttcgcagaa gccgagcatc     540
   aacagcgtcc tccctggctt tggctacgga atcggttggt tcacgtacct cttcattggc     600
   tactttccac gaagggtcag ccatttcaat ttgtttgacg agatgttcag gggccacgaa     660
   gttgcttgca ccctctcgtt gctcacttac gggatgaacg ggactctctg ctactggttt     720
   tatttgagtt acggattcaa gattctcttc gttttctacc tagcaccgct tttcatttat     780
   gggagttata tggttatcgt tacctttttg caccacagtg aagtcaatat tccttggtat      840
   gctgatcaaa actggaacta cgtaaaaggt caactctcaa ccattgaccg taactacggg     900
   ctggtccacc acgcaatcca ctgcataggc acccaccaaa tgcaccacat gttcaccaaa     960
   ataccccact accacctcga ggaggccacc agacatttta ggagtgcttt cccggagtta    1020
   gtgaaatcct gcgacgagcc catactttcg tcattcgtac gcatgttcaa gaagtacaat    1080
   cagcagcagg tggtcgctga taacgcgtta gaggtttatt acaaataa                 1128
<210> 124
   <211> 375
   <212> PRT
   <213> Helobdella robusta
<400> 124
   Met Asn Cys Val Thr Glu Val Asn Ser Ile Ile Ala Ser Leu Ile Lys
   1               5                   10                  15
   Ala Ala Phe Leu Tyr Ser Asn Pro Gln Thr Lys Ile Arg Gly Ile Asn
               20                  25                  30
   Leu Asp Thr Gln Leu Pro Lys Asn Leu Pro Ser Val Ile Glu Ile Lys
           35                  40                  45
   Arg Val Ile Pro Ser His Cys Phe Val Pro Ser Thr Cys Arg Ser Leu
       50                  55                  60
   Leu Tyr Ala Leu Lys Asp Val Val Gln Ile Leu Phe Ala Trp Val Leu
   65                  70                  75                  80
   Leu Trp Tyr Leu Leu Pro Leu Thr Asn Trp Ile Ala Leu Lys Val Leu
                   85                  90                  95
   Met Ile Phe Val Tyr Trp Gly Ile Gln Gly Thr Phe Phe Met Gly Leu
               100                 105                 110
   Phe Val Met Gly His Asp Cys Gly His Gly Ser Phe Ser Lys Tyr Arg
           115                 120                 125
   Leu Leu Asn Asp Val Val Gly Thr Ile Ser His Ala Phe Leu Phe Val
       130                 135                 140
   Pro Tyr Tyr Gln Trp Lys Leu Thr His Gln Asn His His Lys Phe Thr
   145                 150                 155                 160
   Gly Asn Met Asp Lys Asp Glu Val Phe Tyr Pro Ala Arg Ala Ser Gln
                   165                 170                 175
   Lys Pro Ser Ile Asn Ser Val Leu Pro Gly Phe Gly Tyr Gly Ile Gly
               180                 185                 190
   Trp Phe Thr Tyr Leu Phe Ile Gly Tyr Phe Pro Arg Arg Val Ser His
           195                 200                 205
   Phe Asn Leu Phe Asp Glu Met Phe Arg Gly His Glu Val Ala Cys Thr
       210                 215                 220
   Leu Ser Leu Leu Thr Tyr Gly Met Asn Gly Thr Leu Cys Tyr Trp Phe
   225                 230                 235                 240
   Tyr Leu Ser Tyr Gly Phe Lys Ile Leu Phe Val Phe Tyr Leu Ala Pro
                   245                 250                 255
   Leu Phe Ile Tyr Gly Ser Tyr Met Val Ile Val Thr Phe Leu His His
               260                 265                 270
   Ser Glu Val Asn Ile Pro Trp Tyr Ala Asp Gln Asn Trp Asn Tyr Val
           275                 280                 285
   Lys Gly Gln Leu Ser Thr Ile Asp Arg Asn Tyr Gly Leu Val His His
       290                 295                 300
   Ala Ile His Cys Ile Gly Thr His Gln Met His His Met Phe Thr Lys
   305                 310                 315                 320
   Ile Pro His Tyr His Leu Glu Glu Ala Thr Arg His Phe Arg Ser Ala
                   325                 330                 335
   Phe Pro Glu Leu Val Lys Ser Cys Asp Glu Pro Ile Leu Ser Ser Phe
               340                 345                 350
   Val Arg Met Phe Lys Lys Tyr Asn Gln Gln Gln Val Val Ala Asp Asn
           355                 360                 365
   Ala Leu Glu Val Tyr Tyr Lys
       370                 375
<210> 125
   <211> 1390
   <212> DNA
   <213> Lottia gigantea
<400> 125
   taatccattg ttattttttc taatttcaga atatggaaac caaatcagga agtcgtttga      60
   agtcattttg gttatttttg gccaaaatag ccttggtaac attagtacaa gaagaatcaa     120
   ccaaaacaga ggaacaagaa aatacagtca cggctgtttc acaagagact gatgagttat     180
   ccaagaccca tctgccaaat gaacttccaa ccataataga cattaaaaga gcaattccta     240
   gtcactgctt tcagtccaat gttatcacct caatgtacca tgcgatgaag gacgtcgtat     300
   tcgtcatagg actttacatt ttgggagagc ttctgttgac gtatcttcct ttctgggcag     360
   ttctagtcac agcaccagtg ttttggtttg ctcagggaac aatgtttaca gcactattcg     420
   tgattggtca tgattgcgga catggttcgt tctcgaaata cgatctgttg aacgacactg     480
   tgggtactgt tatgcattcg tttctgatga cgccctacta cggatggaag gtgtcccata     540
   agaaccacca caaaaacact ggaaacattg acaaagatga ggttttctac ccagtaagga     600
   aaagtttgaa aactcccggc agagcacttc caggctttgg acttggattg ggctattttg     660
   gatatcttgt tttcggctat aacccacgac cagttagaca tttcaatcct ttcgagaaaa     720
   tcttttccaa acatgtcctc ggatgcatcc tatctctagt gacccttacg gtttggagtt     780
   cctacgtgta ccagtattac atgatctttg ggttcttccg attattctac cattatattg     840
   tgcctttatt catctttgct accaacactg ttcttataac atttctgcac cacaccgaag     900
   aggaaattcc ttggtactcc gatagcaagt gggacaacgt tcgtggacag ttaagctccg     960
   ttgatcgtca ctatggaatc gtccacgata ttttgcacaa catcaccacc catcagatac    1020
   atcacttatt cccaaaggtt cctcattacc atcttgaaga agcaactagt cattttagag    1080
   cagcatttcc agaactagtc aatatccgca cggatcgaac atttccagcg ttctaccgaa    1140
   tgttcaagaa atacgtcaaa cagtgtgtca ttccggacaa tactcatgta catatttata    1200
   cataatttta tcaaatgttc cacctactgc caaatgtatt ggatgtaaaa tttaagtaga    1260
   gctatttgca atcgtgtaac atttttcgta aaattggttg atcatacgaa gctgcatatc    1320
   gttcttccat tgaaataaca cacacgaacg taaaatgctg aaatattacc ccaaagcttt    1380
   caaatcacat                                                           1390
<210> 126
   <211> 1101
   <212> DNA
   <213> Lottia gigantea
<400> 126
   atggaaacca aatcaggaag tcgtttgaag tcattttggt tatttttggc caaaatagcc      60
   ttgactgatg agttatccaa gacccatctg ccaaatgaac ttccaaccat aatagacatt     120
   aaaagagcaa ttcctagtca ctgctttcag tccaatgtta tcacctcaat gtaccatgcg     180
   atgaaggacg tcgtattcgt cataggactt tacattttgg gagagcttct gttgacgtat     240
   cttcctttct gggcagttct agtcacagca ccagtgtttt ggtttgctca gggaacaatg     300
   tttacagcac tattcgtgat tggtcatgat tgcggacatg gttcgttctc gaaatacgat     360
   ctgttgaacg acactgtggg tactgttatg cattcgtttc tgatgacgcc ctactacgga     420
   tggaaggtgt cccataagaa ccaccacaaa aacactggaa acattgacaa agatgaggtt     480
   ttctacccag taaggaaaag tttgaaaact cccggcagag cacttccagg ctttggactt     540
   ggattgggct attttggata tcttgttttc ggctataacc cacgaccagt tagacatttc     600
   aatcctttcg agaaaatctt ttccaaacat gtcctcggat gcatcctatc tctagtgacc     660
   cttacggttt ggagttccta cgtgtaccag tattacatga tctttgggtt cttccgatta     720
   ttctaccatt atattgtgcc tttattcatc tttgctacca acactgttct tataacattt     780
   ctgcaccaca ccgaagagga aattccttgg tactccgata gcaagtggga caacgttcgt     840
   ggacagttaa gctccgttga tcgtcactat ggaatcgtcc acgatatttt gcacaacatc     900
   accacccatc agatacatca cttattccca aaggttcctc attaccatct tgaagaagca     960
   actagtcatt ttagagcagc atttccagaa ctagtcaata tccgcacgga tcgaacattt    1020
   ccagcgttct accgaatgtt caagaaatac gtcaaacagt gtgtcattcc ggacaatact    1080
   catgtacata tttatacata a                                              1101
<210> 127
   <211> 366
   <212> PRT
   <213> Lottia gigantea
<400> 127
   Met Glu Thr Lys Ser Gly Ser Arg Leu Lys Ser Phe Trp Leu Phe Leu
   1               5                   10                  15
   Ala Lys Ile Ala Leu Thr Asp Glu Leu Ser Lys Thr His Leu Pro Asn
               20                  25                  30
   Glu Leu Pro Thr Ile Ile Asp Ile Lys Arg Ala Ile Pro Ser His Cys
           35                  40                  45
   Phe Gln Ser Asn Val Ile Thr Ser Met Tyr His Ala Met Lys Asp Val
       50                  55                  60
   Val Phe Val Ile Gly Leu Tyr Ile Leu Gly Glu Leu Leu Leu Thr Tyr
   65                  70                  75                  80
   Leu Pro Phe Trp Ala Val Leu Val Thr Ala Pro Val Phe Trp Phe Ala
                   85                  90                  95
   Gln Gly Thr Met Phe Thr Ala Leu Phe Val Ile Gly His Asp Cys Gly
               100                 105                 110
   His Gly Ser Phe Ser Lys Tyr Asp Leu Leu Asn Asp Thr Val Gly Thr
           115                 120                 125
   Val Met His Ser Phe Leu Met Thr Pro Tyr Tyr Gly Trp Lys Val Ser
       130                 135                 140
   His Lys Asn His His Lys Asn Thr Gly Asn Ile Asp Lys Asp Glu Val
   145                 150                 155                 160
   Phe Tyr Pro Val Arg Lys Ser Leu Lys Thr Pro Gly Arg Ala Leu Pro
                   165                 170                 175
   Gly Phe Gly Leu Gly Leu Gly Tyr Phe Gly Tyr Leu Val Phe Gly Tyr
               180                 185                 190
   Asn Pro Arg Pro Val Arg His Phe Asn Pro Phe Glu Lys Ile Phe Ser
           195                 200                 205
   Lys His Val Leu Gly Cys Ile Leu Ser Leu Val Thr Leu Thr Val Trp
       210                 215                 220
   Ser Ser Tyr Val Tyr Gln Tyr Tyr Met Ile Phe Gly Phe Phe Arg Leu
   225                 230                 235                 240
   Phe Tyr His Tyr Ile Val Pro Leu Phe Ile Phe Ala Thr Asn Thr Val
                   245                 250                 255
   Leu Ile Thr Phe Leu His His Thr Glu Glu Glu Ile Pro Trp Tyr Ser
               260                 265                 270
   Asp Ser Lys Trp Asp Asn Val Arg Gly Gln Leu Ser Ser Val Asp Arg
           275                 280                 285
   His Tyr Gly Ile Val His Asp Ile Leu His Asn Ile Thr Thr His Gln
       290                 295                 300
   Ile His His Leu Phe Pro Lys Val Pro His Tyr His Leu Glu Glu Ala
   305                 310                 315                 320
   Thr Ser His Phe Arg Ala Ala Phe Pro Glu Leu Val Asn Ile Arg Thr
   325 330 335
   Asp Arg Thr Phe Pro Ala Phe Tyr Arg Met Phe Lys Lys Tyr Val Lys
   340 345 350
   Gln Cys Val Ile Pro Asp Asn Thr His Val His Ile Tyr Thr
   355 360 365
<210> 128
   <211> 1513
   <212> DNA
   <213> Lottia gigantea

<400> 128
   caaagaaaag cggcagtact cttgtccata ttcgtcgtca ataaccaaaa gtttctgttt 60
   ttaattaatc caaacaacga attgcacaaa tggccattga aagaaatgtc aaagtaaatg 120
   gctcaatgga tatcttacag accagtgaag atattggaaa tggtctgtct actgcaaaga 180
   atgggcatat aagtaatgat atgaatgaag ccaataacca cgtgttcgac acaacgactg 240
   gcgaggaaca acatctatcc aaacgaaacg gttcttccaa cggtacaagc aagaacgggg 300
   ttgccaacaa aatacccagt atttcagaaa tcaaggcagc tatccccaac cattgtttta 360
   aatcaactat caaacagtct atgtattacg ttttcaaaga cattatcctt ataatagcgt 420
   tatactgttt tggacattgg gtcttacagt gcaattccat aatactcaag acggcactgt 480
   cgccagtata ttggttctta caaggtacgc tgttattcgc ggtatttgta ttgggacatg 540
   attgtaatca tggctcgttt tctagctatg cattattgaa cgattgcctt ggtactgttc 600
   tacattcatt tgtaatgacg ccgtattacc cctggaaaat ttcgcatcgt catcaccaca 660
   acaatactgg taatatggac aaagatgaga tattttatcc cattaggaag aaagacaaca 720
   ataagaactc ttttgcatta ttttttggtc ttggattcgg ctggttggcg tatctgtggc 780
   gcggattcgg accacggcaa atgaatcatt ggataccacg acatgcgatc ttcgctaaac 840
   atgtcgtagg gtgtatattg tctataggcg ttgtgggtat atgggtcggt atattaggat 900
   attatgtgca actgatgggt atggtgagtt tggtatacca ttatatgata ccggtcttta 960
   tatgtggatg ttatatcgtc atggtaacgt ttctacacca tagtgatatc aaccttccat 1020
   ggtacagcga cgacaactgg gacagtgtta aaggcaaact aagctcagtc gacagagact 1080
   atgggatttt tcacgacgtt atccatacta taggaacgca ccaagttcac catttgtttc 1140
   ccataattcc tcattataat ctacgcgaag caacgacgca cttccggaag gcattcccgc 1200
   aattagtgca tatcaacgat aaaagcattt ttccagcatt tactgaaatg ttctttaaat 1260
   attccagtca gtgtatagta gaaaatgatg ccaaaattca ttactataaa taattaaata 1320
   tggcattaag tatatggagt agggcataaa aaatattaaa acagaacctg aaacatgttg 1380
   ttactcagag taacatgata tttatttctt ttcattcttg tttcatattt attcttttaa 1440
   acgatcgtat atttttagca tctatttgca atattttcaa attcaaaaat ggctttttgt 1500
   gcaacataat cta 1513
<210> 129
   <211> 1113
   <212> DNA
   <213> Lottia gigantea
<400> 129
   atgaatgaag ccaataacca cgtgttcgac acaacgactg gcgaggaaca acatctatcc     60
   aaacgaaacg gttcttccaa cggtacaagc aagaacgggg ttgccaacaa aatacccagt    120
   atttcagaaa tcaaggcagc tatccccaac cattgtttta aatcaactat caaacagtct    180
   atgtattacg ttttcaaaga cattatcctt ataatagcgt tatactgttt tggacattgg    240
   gtcttacagt gcaattccat aatactcaag acggcactgt cgccagtata ttggttctta    300
   caaggtacgc tgttattcgc ggtatttgta ttgggacatg attgtaatca tggctcgttt    360
   tctagctatg cattattgaa cgattgcctt ggtactgttc tacattcatt tgtaatgacg    420
   ccgtattacc cctggaaaat ttcgcatcgt catcaccaca acaatactgg taatatggac    480
   aaagatgaga tattttatcc cattaggaag aaagacaaca ataagaactc ttttgcatta    540
   ttttttggtc ttggattcgg ctggttggcg tatctgtggc gcggattcgg accacggcaa    600
   atgaatcatt ggataccacg acatgcgatc ttcgctaaac atgtcgtagg gtgtatattg    660
   tctataggcg ttgtgggtat atgggtcggt atattaggat attatgtgca actgatgggt    720
   atggtgagtt tggtatacca ttatatgata ccggtcttta tatgtggatg ttatatcgtc    780
   atggtaacgt ttctacacca tagtgatatc aaccttccat ggtacagcga cgacaactgg    840
   gacagtgtta aaggcaaact aagctcagtc gacagagact atgggatttt tcacgacgtt    900
   atccatacta taggaacgca ccaagttcac catttgtttc ccataattcc tcattataat    960
   ctacgcgaag caacgacgca cttccggaag gcattcccgc aattagtgca tatcaacgat   1020
   aaaagcattt ttccagcatt tactgaaatg ttctttaaat attccagtca gtgtatagta   1080
   gaaaatgatg ccaaaattca ttactataaa taa                                1113
<210> 130
   <211> 370
   <212> PRT
   <213> Lottia gigantea
<400> 130
   Met Asn Glu Ala Asn Asn His Val Phe Asp Thr Thr Thr Gly Glu Glu
   1               5                   10                  15
   Gln His Leu Ser Lys Arg Asn Gly Ser Ser Asn Gly Thr Ser Lys Asn
               20                  25                  30
   Gly Val Ala Asn Lys Ile Pro Ser Ile Ser Glu Ile Lys Ala Ala Ile
           35                  40                  45
   Pro Asn His Cys Phe Lys Ser Thr Ile Lys Gln Ser Met Tyr Tyr Val
       50                  55                  60
   Phe Lys Asp Ile Ile Leu Ile Ile Ala Leu Tyr Cys Phe Gly His Trp
   65                  70                  75                  80
   Val Leu Gln Cys Asn Ser Ile Ile Leu Lys Thr Ala Leu Ser Pro Val
                   85                  90                  95
   Tyr Trp Phe Leu Gln Gly Thr Leu Leu Phe Ala Val Phe Val Leu Gly
               100                 105                 110
   His Asp Cys Asn His Gly Ser Phe Ser Ser Tyr Ala Leu Leu Asn Asp
           115                 120                 125
   Cys Leu Gly Thr Val Leu His Ser Phe Val Met Thr Pro Tyr Tyr Pro
       130                 135                 140
   Trp Lys Ile Ser His Arg His His His Asn Asn Thr Gly Asn Met Asp
   145                 150                 155                 160
   Lys Asp Glu Ile Phe Tyr Pro Ile Arg Lys Lys Asp Asn Asn Lys Asn
                   165                 170                 175
   Ser Phe Ala Leu Phe Phe Gly Leu Gly Phe Gly Trp Leu Ala Tyr Leu
               180                 185                 190
   Trp Arg Gly Phe Gly Pro Arg Gln Met Asn His Trp Ile Pro Arg His
           195                 200                 205
   Ala Ile Phe Ala Lys His Val Val Gly Cys Ile Leu Ser Ile Gly Val
       210                 215                 220
   Val Gly Ile Trp Val Gly Ile Leu Gly Tyr Tyr Val Gln Leu Met Gly
   225                 230                 235                 240
   Met Val Ser Leu Val Tyr His Tyr Met Ile Pro Val Phe Ile Cys Gly
                   245                 250                 255
   Cys Tyr Ile Val Met Val Thr Phe Leu His His Ser Asp Ile Asn Leu
               260                 265                 270
   Pro Trp Tyr Ser Asp Asp Asn Trp Asp Ser Val Lys Gly Lys Leu Ser
           275                 280                 285
   Ser Val Asp Arg Asp Tyr Gly Ile Phe His Asp Val Ile His Thr Ile
       290                 295                 300
   Gly Thr His Gln Val His His Leu Phe Pro Ile Ile Pro His Tyr Asn
   305                 310                 315                 320
   Leu Arg Glu Ala Thr Thr His Phe Arg Lys Ala Phe Pro Gln Leu Val
                   325                 330                 335
   His Ile Asn Asp Lys Ser Ile Phe Pro Ala Phe Thr Glu Met Phe Phe
               340                 345                 350
   Lys Tyr Ser Ser Gln Cys Ile Val Glu Asn Asp Ala Lys Ile His Tyr
           355                 360                 365
   Tyr Lys
       370
<210> 131
   <211> 3060
   <212> DNA
   <213> Microcoleus chthonoplastes
<400> 131
<210> 132
   <211> 1077
   <212> DNA
   <213> Microcoleus chthonoplastes
<400> 132
   atgcaatcaa acacagttct caagcaggca acgattacag cctcatccac tcctaaagaa     60
   gagttaccct ttactctcca agacttaaaa gcggcaattc ccgcctactg ttttgaacct    120
   tctgtttgga agtctctcag ttatttcttt ctagacattg gcataatcgc tggattttat    180
   gccatcgcct ataaactgga ttcctggtta tttttcccca tcttttgggt actgcaagga    240
   actatgtttt gggcgttgtt tgtggtgggt catgactgcg gtcatggctc tttctctaag    300
   attaaatggc tgaataacct gattggacat ctgtctcaca ctcccatcct ggttcccttc    360
   cacgggtggc gcatcagtca cagaactcat catgccaata cgggtagcct ggaaaccgat    420
   gaaagttggt atccggtatc tcagactaag tataagcaga tgcggtggta tgaaaagcta    480
   tttcgctata atttaccatt gctagcttat cccctctatc tatttaagcg atcgcctaat    540
   cgcgagggtt cccattttct gcccaatagt ccattatttc gtccctcgga aaaatgggat    600
   attgtcacca gttccgttct ctgggtgtta atggttgcct ttttaggggg actgacgttt    660
   cagttcggtt ggctatttct ggtgaagtat tacctggttc cttatgttgt atttgtcatg    720
   tggctagatt tggtcacatt cctgcatcat actgaaccag atattccctg gtatcgggga    780
   gatgattggt atttcctgaa aggggcatta tcaacgattg atcgggatta tggctttatt    840
   aatcccattc atcatgatat cgggactcat gtggcgcatc atattttctt gaatatgccc    900
   cactatcact tgaagacggc aactgaggca cttaaacccg tgttaggcga gtattatcgc    960
   tgttccaatg agccgatttg gaagagtttt atgcgttctt actgggcttg tcatttcgtg   1020
   aaggatcaag gatctcccgt ttactatgaa tctcccaaac atccgcgatc aaactaa      1077
<210> 133
   <211> 358
   <212> PRT
   <213> Microcoleus chthonoplastes

<400> 133
   Met Gln Ser Asn Thr Val Leu Lys Gln Ala Thr Ile Thr Ala Ser Ser
   1               5                   10                  15
   Thr Pro Lys Glu Glu Leu Pro Phe Thr Leu Gln Asp Leu Lys Ala Ala
               20                  25                  30
   Ile Pro Ala Tyr Cys Phe Glu Pro Ser Val Trp Lys Ser Leu Ser Tyr
           35                  40                  45
   Phe Phe Leu Asp Ile Gly Ile Ile Ala Gly Phe Tyr Ala Ile Ala Tyr
       50                  55                  60
   Lys Leu Asp Ser Trp Leu Phe Phe Pro Ile Phe Trp Val Leu Gln Gly
   65                  70                  75                  80
   Thr Met Phe Trp Ala Leu Phe Val Val Gly His Asp Cys Gly His Gly
                   85                  90                  95
   Ser Phe Ser Lys Ile Lys Trp Leu Asn Asn Leu Ile Gly His Leu Ser
               100                 105                 110
   His Thr Pro Ile Leu Val Pro Phe His Gly Trp Arg Ile Ser His Arg
           115                 120                 125
   Thr His His Ala Asn Thr Gly Ser Leu Glu Thr Asp Glu Ser Trp Tyr
       130                 135                 140
   Pro Val Ser Gln Thr Lys Tyr Lys Gln Met Arg Trp Tyr Glu Lys Leu
   145 150 155 160
   Phe Arg Tyr Asn Leu Pro Leu Leu Ala Tyr Pro Leu Tyr Leu Phe Lys
   165 170 175
   Arg Ser Pro Asn Arg Glu Gly Ser His Phe Leu Pro Asn Ser Pro Leu
   180 185 190
   Phe Arg Pro Ser Glu Lys Trp Asp Ile Val Thr Ser Ser Val Leu Trp
   195 200 205
   Val Leu Met Val Ala Phe Leu Gly Gly Leu Thr Phe Gln Phe Gly Trp
   210 215 220
   Leu Phe Leu Val Lys Tyr Tyr Leu Val Pro Tyr Val Val Phe Val Met
   225 230 235 240
   Trp Leu Asp Leu Val Thr Phe Leu His His Thr Glu Pro Asp Ile Pro
   245 250 255
   Trp Tyr Arg Gly Asp Asp Trp Tyr Phe Leu Lys Gly Ala Leu Ser Thr
   260 265 270
   Ile Asp Arg Asp Tyr Gly Phe Ile Asn Pro Ile His His Asp Ile Gly
   275 280 285
   Thr His Val Ala His His Ile Phe Leu Asn Met Pro His Tyr His Leu
   290 295 300
   Lys Thr Ala Thr Glu Ala Leu Lys Pro Val Leu Gly Glu Tyr Tyr Arg
   305 310 315 320
   Cys Ser Asn Glu Pro Ile Trp Lys Ser Phe Met Arg Ser Tyr Trp Ala
   325 330 335
   Cys His Phe Val Lys Asp Gln Gly Ser Pro Val Tyr Tyr Glu Ser Pro
   340 345 350
   Lys His Pro Arg Ser Asn
   355
<210> 134
   <211> 10047
   <212> DNA
   <213> Mycosphaerella fijiensis
<400> 134
   ctatcacatc tcgcggctcg acggatacat tatgtgttga cctgatgtag cagacgcatt     60
   tcttcgacaa gtcatcgccg ctgaactttc tcgaccaagt tggtctgcgc tgaggtgtgg    120
   tcatcttgtc tgttggagtt cctgtgtggt ttgtatggat acgtcgcagc aggctattaa    180
   atgtcatctc gctcaggaca atgtgttgtg gcccccacga ctcgggcggg cggcctctgc    240
   taaatatgtg tttccagatc acagccacct acccagcgga gatgctgtgg aaccttgcgt 300
   cacctgtgag gcccagccag accacacacc ggtcgcatcg agtgatgctc gtgcactgca 360
   gtcccgctct cttcgtgacg caggcgcttt ggaggcagtg cttgtgcgcg ctcccctctg 420
   cccgcagcct tcgagccata cgcaccggcc tcgcttccat catcgcctcc agccgctcgg 480
   ttagcctgcc gccagctcac cagttggctg caatgactgt caccgcgacc tgcttatttg 540
   gagtacgtcg gctgtggaca tcgctaagga catcatgctg tggtattatc accatgaagg 600
   cgcacgtccg gttgtgtttg tagttgcagc cctccgacat gcatcacaag gcgcagtaca 660
   tcgccagagg agaggctgga gatgttgcac gcagattgac cattccacag gagcctgagt 720
   tctcgtatta aactttgccg taagttctct cttcactatc gaactaacat tatagcctct 780
   ataatggcct cacagtcgga tgttcttgtg tcaggcgagc agtcagcagt tgtcggtagc 840
   gccgacagtg gtatagtcat acctagaacc ttgcctaaat gcggccggcg atacaattcc 900
   gacacttcag ccaggagtcc atggcgaaga aaaattattc tatcactagg tacggaaaac 960
   ctcgatcgac atcatatttc caggatcaac cacttgactg acagacttcg cagacggtgg 1020
   aggaattcga ggctatgcgt cgctgaaaat tctggaacgt ctcatgcaga aggtcgagcg 1080
   gatggagcag agcaatgaga ctagatacca cgctgcctac ggacagatca cgagcagcgc 1140
   cgcatatccg tggaatagca cacaagtacc cgcaggcctt tcagtctcca atcccgatgt 1200
   ccctcgccag tctttcgacg cgggctttcg tcctcatcat tactttgatt acttcttcgg 1260
   caccagtaca ggagggtgag tactatagca acctgacgaa gttatcggct tctcatgtgc 1320
   ttcttgcaac gcatcgtccc gtggctttta gctgatttca ctcctgcaga ttgagctcca 1380
   ttatgctcgg ccgccttcag atgtcagttt ctaccgcgct taagcaatac gacgtggttg 1440
   gaaacagcgt atttgcccgc ggacggccgt atgtcactgc cttgggcagt attatggtgc 1500
   caaagtacag aagtcgcgac atggagcaag ctttgtccaa agtgttgcag aacggcctag 1560
   tagggaagac gctgacgagg ccggcggatg agctcgcact tcagaatgag aatgagggtg 1620
   cggcacgaac gtaagtcagc ctccacctcg tgaagccttt cgctcggcct gcatatgtat 1680
   actctgtcgc cgtgtcgggc tgttcggttg aaggtctcga cttccaagta tcgtcaacta 1740
   atgatcacag cgttgtcgtc tcccatggac catcaaccct tggcggtgtc tcgatgcgat 1800
   atctctttcg gagctacgaa catccaaaac cgtccactcg gcttaccgat ggccaagaag 1860
   aagatcactt gaatccaggc ccgttacctc caactacgat catagaagcg gcgagagcca 1920
   caagtgctgc gccgacctat ttctctgaga aggaagtcgg aggtcttctc tacatggatg 1980
   gcgccgttga tgccaacaat cccgtgcaac tcgcatataa tgaggtcacg cagatgcatc 2040
   actctcagca acctcgcttg atcatctcaa taggcactgg agaagcagca gaatggcagc 2100
   cgcacagtgc ttggcgggtg ctcaagaccg tcaagcgcac cgcagaccac ttaagcgact 2160
   ttgctcgaaa tctcacagat acggagggcc cgcacaaaga gctcaagaag acgatcagac 2220
   atctgcccaa gagtaaacag ccgctatact ttcgattcaa tattccgagc tcttctggta 2280
   tttgcgatat tggtctcgac gagtggcagc caagaaaagc cagccaccgg gcatcggccg   2340
   ggcacgaaac caagtcaaag atgacagacg ctgtcaacga gtactgtaag actatcgatg   2400
   accaactaga agagtgtgcc agaattctag tcgaggttcg aagggagaga gcagaaaccg   2460
   aacgctggga gagattcgct ttgcataggt cctactcctg ccccgaagca gattgcgagg   2520
   cgcctagttt ccctggcaga caagacctac gacagcatct cttcgactca cacggcatcg   2580
   tgcgccacgt ttggtgccgg ggcgagttgc caagttatca gaaacatcaa tttgcttgca   2640
   tttggcacga ttgtggcgat tatactatca caacctttga tcactcgcaa tctggcatct   2700
   gcgatcatct agatgcacgt acgagatcta tcgtggaaag ttatatgaag agtgagcacg   2760
   gcgtacctga tgcgaaggtc atgacgagtg aggattttga agaattactt gatcggggtc   2820
   gagacctaga cttgctgaag aaggcgagga cgatgaagac ttgaggtttt cgtcattttt   2880
   gcgcacctgg aaattcgagt attaccctgg accatcacca ccctctcgtc tctggatctc   2940
   tcacatccga atccggaatc ttcacaaaaa tatgcttcca gtacacgaca agaatatccg   3000
   ccagcagccc cgcgaagctc atcttcctcc gtctagtccc aggccaccca tccggcagtc   3060
   tctcttccgt ataaaagacc gtaatatcct tgaccgcaat cctctcgcca aacgtctgca   3120
   acaggataca cgtctgcgac caggtcgtac catacaatct caaccccaaa ctccgcaacc   3180
   ctttataccc aaaagctact ttacactctt tatcgcgcct aacacgagtt ctcgcaagac   3240
   tctcacgcgt aattccaccg cctggagcag catcttcgag aaaggcttga aacatcgcgg   3300
   gctggaaagt gaaattatcg ccttgccgat catctagtct ggtgaaagag gcatcgtgct   3360
   cgatcacgtt gtgtttgcgt agatcgagta gtgtgaaagt tccgtctgag tggaggaggg   3420
   gtttcacgat gctggataag acgtccgcga gcagaggtgt gcagtcggca gagactagca   3480
   atgcggcttt gaggtgcgat tgtgttatgc cggtgccgtc tcgagggcta tcgtggtcaa   3540
   caattctggc tggtctacga agtttccatt tatcaccgta ggcggcactt acagataccc   3600
   atgattagaa agactgttca aaaacggaca tggagaccgc gtgctcggat cgccgcggat   3660
   ataggtatgg tctgcttctc gaatttcttg gatttccttt ttgggcggag atttgaagaa   3720
   agctattagc ggctggaaca gacttgatac ttgaatccca cacattttgg gtcggacatc   3780
   gctagggata ttctagatgg gctttgggat ggcaatgatc ttgtgtcgag aggtctgaca   3840
   tatttgactg aaatggtctg actacgccca ttctgtcagc tagaccagta gtgacagatg   3900
   ccatgtgcac tcagctggtg gtaggagcag ccacagccag caggtttcgg ttttaccgtc   3960
   gcctgcatag atgttgaata ctgaatattc agtatttaga gggtatattc agtgaatatg   4020
   aatataactt ttcgtatatt caggtgaata gttagtattc agtcttccgc ggaggtggat   4080
   agatagatta atagctacct ctacgtcgtt atcgctgtct attaatacta ttgttataga   4140
   tactattatt atagatacta ttgttataga tactgcttct atagatacta taataataat   4200
   actatcgata tattactaca aggcgcctac tatatagtac tatatatcta ataagtcttc   4260
   tatcttatct ttattaatag tactgttatc tattactagt aatgtaatac tagccttcta   4320
   ctattgttta gaaggctcta ttatacgcac tgtcttacta tctagttagc ttcggtccta   4380
   tagaatagta cgccttgcta tactaaaggt acgctcgctc ttagcggata tagcgaaggt 4440
   agataagacg tcgatagtaa gctacgataa taaaggctac gcctatcgct aagctagtta 4500
   taactactat tgtaatagac ctattcggac gtcgttacta ctgctatctt agataacagg 4560
   caataggcac ggagcggcat agacgaagct attaaactcg tccttaaccg tatcgataac 4620
   ctccgcggct aattactacg actcgaagct agtaaggtct ataaccgcta tagtagtagt 4680
   tagttttgct aaaggctcgt acctgtcctt atattcttac tataatgccc tcgctcttac 4740
   tattgctagc tctaactagt tagtgttctt tactctagtc tagcgcttct aaagctaagt 4800
   aagtcgcttt aaagggtata gtaagagagc cgctacgtac gtaccggatt cgtcgataag 4860
   ttagtagtac ttactaaaca ggtgccaaga gacaataata gacttcgtaa tagcaggtct 4920
   atacttatat agctcgagag acttctcgta atagttaacg aggtattcta tagcttctta 4980
   gaagctatct agtatatagt tatagctcta taggcgtatt ataacctcct taaatagata 5040
   taggaagttg cgagcgtgtt taattactat ctagtcggct atagttagct ctacgtctta 5100
   tagtaaaggc tctcgtacga atagaaggtc taatgttagc ttagctttaa tagcctcgtc 5160
   tataagcaag agctagctat tctgccgagt cttactaggt gtctttagta ccgctgctat 5220
   agtagcggct ctagctaaga acttaaatat agcctatata atatcgtctt agaggcggag 5280
   taacaggtta tatagcttct gtatagtctc ttgcttagta aacgaggctg tatcgatatt 5340
   aagctatata gagacggaga cattatcgtt actagtagca gcggcattat aagcagctat 5400
   ctgctactta ataaaggcac gctcgttatt ctttgccttc tcgaaggcta tcttagccga 5460
   ctcttaagac tccgagaata taaaggctta tatagcgagg ttaataatat atctaatata 5520
   gcgaagccgg cgtatcttct cgtactacta agtaatacct tcggagcgta gaaagcgctc 5580
   aatcttacgt attaacgtat tattagacgt aacgttatcg cttgtaatcc aaccgatatt 5640
   gttaacaata ttaaactgct taaaggcctt aataagaagc tgtataagct cggctctagt 5700
   atagcgtata ataacgatag gaaggtcgag aagtacttat tatagcttac tatctttgtc 5760
   gacgaattgg cagttaatag cttaatagta cttatagcta tactacgact tctaggtgtc 5820
   tatatagagg tgaatatagc taatagctat agagaggtat ttcactactt aatagcgtat 5880
   ataatagaag aggcggaaga ctcgtaagta gagcgaagta tataactata ggaagactcg 5940
   taaagcctat agattaacgg ctaatataat gttatagaaa ctaggtgaaa ctagcttgtt 6000
   aaaggctata ctctactcgg aggcgaaggc agcgagagcg ttacgatatt gctatgtttt 6060
   agctacgtta cgtaggatta atagtacgtg ttcctctcga gaagatgcct tagtaagtac 6120
   ttaaatagct atactatcgc gaatatcggc ttgcttctat ctcggctcct tgcctttgta 6180
   tttactagta tataagctat gccttcttat taaataatag tcggtattgc ttatcgtact 6240
   atactataga cctaagtcta gctcgaaggc tgttttatag caatcctcga atatatagta 6300
   ctataccttg tatctatagt tatctatata ctctgcctcc tacggataag gctctcttat 6360
   acatgctagt attttaggat ctaccgcttt ctgtcttgtc ttcttgcttc taggtaccga 6420
   taatagcttt agtacttata gcaaagaagg tgtcgattat tgtatagatt tagtcgacta 6480
   cgtattaata gaaggtatct cgactagtat cggatttagg accgcttcga ggtcgctatc 6540
   gcttttatag ctagcgctat cgctacgctt acgttttcga tataggtcgt tttcccgtta 6600
   cgaagcctag ctatttagca aattttcttg tactttgtat attaatttat atatagtgcg 6660
   gaagttaact atagcggtgt tagtagtcga ggtataatta ttagtataga tttgaaaaat 6720
   ttaagtttac tagaaatata cggccggaaa ggcccgatcg cggaccgctc tcggttatac 6780
   tatccggata cgattattag gctacttaag ccttatataa cgctatagga ccctagataa 6840
   gcagtaatag tatataaagc aactaatacc ttctatattc tatcttcttt atttataaca 6900
   ctactactaa tatactcctt tactatcgct attgccgcta aagcgttacg agcgcgctac 6960
   gtagcgagct cttcctttag cttatattcc ttaccgctat atagcatata taggttataa 7020
   ctatacgggt ccgcgaatct aaagtgcctc gctttctctt ctattctacg aatacggtcg 7080
   agatatacct cgcaacgata gtctctcgga tatactagca catagttgct actcttccga 7140
   atatactcgt cgaaagcctc taccttcttt agataaatat cgagcttagc attcttcgca 7200
   attatcctat attctatagc cttatatccc ttcctaatct cctagcgttg ttctagtagc 7260
   cgccctatta ccttatacta agtatctatc tcggctcgct attccttaag ctactctatc 7320
   gccttcgacg tcttcttcgg attcttataa agcctcttcg taataggctc tttaattagt 7380
   aacggtttac gctcgatagt aggctcggtc ttaataatag gcttagttat tagcggcggc 7440
   aatattagcg gcggcaatat tactagcgac gatattagct taggtttaga ctccgagtct 7500
   aacagctaat tacgagttag cactactact agaataacta gtattattac ctatctacct 7560
   cgattacttc tacgcgcttt atacctatta ttatataaag aaagagccca aaataccgta 7620
   aaattgaaaa tctatatagg atctatatac gccgacgtat agcctgccta aatataaata 7680
   tacctactat ttagtctacc tatatttaag gttcatttaa ttaaatataa atatatattc 7740
   aaactgaata ttcacaattc aacatctata gtcgcctgat cggtttggct tttgagatga 7800
   ccacagcttc gctgtcactg gcttgaggac tatgcaaagt gcctatttgg gcatggcgtg 7860
   ggagtgctgg aggtgagctc tttgtggtgt tcttgttctt ggatctggat ctgctcacgt 7920
   ggtcccggac ctgaaacaga ctgctggctg actattagca cacgcagcga gatgtttgac 7980
   attcgtttct tgtcagtgtc gccttcctga tctcctatga ctatgacaga tcgactcttc 8040
   agctctgggc acgttctcag ttagacgacc tatatagtcc tcacaagtgt aaccggtctc 8100
   ttggcagtgt agccacagat tttcggtgta ggttcggttt caaaaccgaa cccaacccaa 8160
   ccaaaccaat gggtggttta cttagaagaa gcgatttaga tatcaaatcg cggtatacgt 8220
   gctcgccaac aaaggctctt cggaattata cggcttccct gccagccgac caaaaggctt 8280
   tcggaattcc tacgtctagc atagaactcc aatgcatagc ctacctaaca tgaagggttg 8340
   actccggagt ccgaacggct caagaccggc tcaatgtgtg catttacact ttggaagcat 8400
   tggagcacca cagacacaaa aagataagag caccgtctat ccctatctcg aacaatgttc 8460
   caatcttcga agtcatgttt ctcgccggaa gtgatgaggt cggagaaatt tccggactgt 8520
   cgagtattcc aaccaggaca acgaccagca agatttcgtt gcagcggctt cgaaaagcga 8580
   tcccagaggc atgttttcgt ccaagcgtgg tgagatctgc tgcacacgtt cttgcagatg 8640
   ttctcctggc tgtaggtctt gcattcgcag ctctcgaact cgtcccgcaa atcggcagtc 8700
   gagcacttcg tgttgctctg tggatgcttt acgggtatgt ccaaggcttg gtgttcactg 8760
   gaatctggat tcttgcacac gagtgcggac attacgcatt atttccgggc agaaaggtca 8820
   atgatgcgat cgggtttttc ctgcatagtg ctcttcttgt tccatatttc tcttgggtga 8880
   gttcaacgag tccaagactc aggttcttga tggggtgtct gctgataccg aactctacaa 8940
   cctggtagaa gtacacgcat gctcgtcatc atcgatacac aaaccatatt gagaaagata 9000
   cggcgtatgt gccttccagg gaagaggaga taacatggtc cagacgaatt tctgaagttc 9060
   ttcatcagac ggaggacgct cctttgcatt ctgccattct gcttgtctgc catcagcttt 9120
   tcggctggca ggcgtacatc gcatgctatg caagtggtgg acctggctcg ttggtgagag 9180
   cgtcgagggg caagacgttt gatcgatgcc atttgaatcc agcagcgtgc atctttacgg 9240
   cgtcagaagc ggcattcgta gggttgtcca cgctggggtt gttgggaatg ctgttgctcc 9300
   tcgccatggc agctagcaag atcggttggg ccaatgtggc actcctctac ggagttccat 9360
   atgcctgggt caacaattgg ctggtcgcga tcacatatct tcatcacaca catcgagaca 9420
   ttcagcactt cgccagctcg ggctggacgt acatgaatgg tgtgctgtcc acaattgatc 9480
   ggcctttcgg catcgtggga cgatatgtgt tccacggcat aatcgacttc catgtcgtcc 9540
   atcatctctt tccgtaagtg ctcgctcata catctgggcc ggtcccaact tctggcggca 9600
   actctaagca gtcatacagc cagataccat tctaccatgc ggaagaagcg acagaagcag 9660
   tcaagaagga attgggtgag cgctactgct tcgacgacac gcctttctgg cttgctcttt 9720
   ggcggacgtt ccgggaatgt caggtcgtgg tgccatcgaa gcatgcatcc gatgtgcttg 9780
   tctggaaaga tctaggagcc tgagcctgag agtaagaaag tgagaagagc caagccaagc 9840
   caagcgcaca tggcagcagt ccttgccatt cctcgccgtc gcaaatactg caaactcacc 9900
   accttacatt catgactctg acatcgcagg cgcgcattct cgtcaccaac tgcaaatcta 9960
   tacttccagc ctcgctgttg cactcatctt cctcgccgta tcaagaccca gctttcgagt 10020
   caaatgtttc cccagcctaa cgcatgg 10047
<210> 135
   <211> 1191
   <212> DNA
   <213> Mycosphaerella fijiensis
<400> 135
   atgtttctcg ccggaagtga tgaggtcgga gaaatttccg gactgtcgag tattccaacc     60
   aggacaacga ccagcaagat ttcgttgcag cggcttcgaa aagcgatccc agaggcatgt    120
   tttcgtccaa gcgtggtgag atctgctgca cacgttcttg cagatgttct cctggctgta    180
   ggtcttgcat tcgcagctct cgaactcgtc ccgcaaatcg gcagtcgagc acttcgtgtt    240
   gctctgtgga tgctttacgg gtatgtccaa ggcttggtgt tcactggaat ctggattctt    300
   gcacacgagt gcggacatta cgcattattt ccgggcagaa aggtcaatga tgcgatcggg    360
   tttttcctgc atagtgctct tcttgttcca tatttctctt ggaagtacac gcatgctcgt    420
   catcatcgat acacaaacca tattgagaaa gatacggcgt atgtgccttc cagggaagag    480
   gagataacat ggtccagacg aatttctgaa gttcttcatc agacggagga cgctcctttg    540
   cattctgcca ttctgcttgt ctgccatcag cttttcggct ggcaggcgta catcgcatgc    600
   tatgcaagtg gtggacctgg ctcgttggtg agagcgtcga ggggcaagac gtttgatcga    660
   tgccatttga atccagcagc gtgcatcttt acggcgtcag aagcggcatt cgtagggttg    720
   tccacgctgg ggttgttggg aatgctgttg ctcctcgcca tggcagctag caagatcggt    780
   tgggccaatg tggcactcct ctacggagtt ccatatgcct gggtcaacaa ttggctggtc    840
   gcgatcacat atcttcatca cacacatcga gacattcagc acttcgccag ctcgggctgg    900
   acgtacatga atggtgtgct gtccacaatt gatcggcctt tcggcatcgt gggacgatat    960
   gtgttccacg gcataatcga cttccatgtc gtccatcatc tctttcccca gataccattc   1020
   taccatgcgg aagaagcgac agaagcagtc aagaaggaat tgggtgagcg ctactgcttc   1080
   gacgacacgc ctttctggct tgctctttgg cggacgttcc gggaatgtca ggtcgtggtg   1140
   ccatcgaagc atgcatccga tgtgcttgtc tggaaagatc taggagcctg a            1191
<210> 136
   <211> 396
   <212> PRT
   <213> Mycosphaerella fijiensis

<400> 136
   Met Phe Leu Ala Gly Ser Asp Glu Val Gly Glu Ile Ser Gly Leu Ser
   1               5                   10                  15
   Ser Ile Pro Thr Arg Thr Thr Thr Ser Lys Ile Ser Leu Gln Arg Leu
               20                  25                  30
   Arg Lys Ala Ile Pro Glu Ala Cys Phe Arg Pro Ser Val Val Arg Ser
           35                  40                  45
   Ala Ala His Val Leu Ala Asp Val Leu Leu Ala Val Gly Leu Ala Phe
       50                  55                  60
   Ala Ala Leu Glu Leu Val Pro Gln Ile Gly Ser Arg Ala Leu Arg Val
   65                  70                  75                  80
   Ala Leu Trp Met Leu Tyr Gly Tyr Val Gln Gly Leu Val Phe Thr Gly
                   85                  90                  95
   Ile Trp Ile Leu Ala His Glu Cys Gly His Tyr Ala Leu Phe Pro Gly
               100                 105                 110
   Arg Lys Val Asn Asp Ala Ile Gly Phe Phe Leu His Ser Ala Leu Leu
           115                 120                 125
   Val Pro Tyr Phe Ser Trp Lys Tyr Thr His Ala Arg His His Arg Tyr
       130                 135                 140
   Thr Asn His Ile Glu Lys Asp Thr Ala Tyr Val Pro Ser Arg Glu Glu
   145                 150                 155                 160
   Glu Ile Thr Trp Ser Arg Arg Ile Ser Glu Val Leu His Gln Thr Glu
                   165                 170                 175
   Asp Ala Pro Leu His Ser Ala Ile Leu Leu Val Cys His Gln Leu Phe
               180                 185                 190
   Gly Trp Gln Ala Tyr Ile Ala Cys Tyr Ala Ser Gly Gly Pro Gly Ser
           195                 200                 205
   Leu Val Arg Ala Ser Arg Gly Lys Thr Phe Asp Arg Cys His Leu Asn
       210                 215                 220
   Pro Ala Ala Cys Ile Phe Thr Ala Ser Glu Ala Ala Phe Val Gly Leu
   225                 230                 235                 240
   Ser Thr Leu Gly Leu Leu Gly Met Leu Leu Leu Leu Ala Met Ala Ala
                   245                 250                 255
   Ser Lys Ile Gly Trp Ala Asn Val Ala Leu Leu Tyr Gly Val Pro Tyr
               260                 265                 270
   Ala Trp Val Asn Asn Trp Leu Val Ala Ile Thr Tyr Leu His His Thr
           275                 280                 285
   His Arg Asp Ile Gln His Phe Ala Ser Ser Gly Trp Thr Tyr Met Asn
       290                 295                 300
   Gly Val Leu Ser Thr Ile Asp Arg Pro Phe Gly Ile Val Gly Arg Tyr
   305                 310                 315                 320
   Val Phe His Gly Ile Ile Asp Phe His Val Val His His Leu Phe Pro
                   325                 330                 335
   Gln Ile Pro Phe Tyr His Ala Glu Glu Ala Thr Glu Ala Val Lys Lys
               340                 345                 350
   Glu Leu Gly Glu Arg Tyr Cys Phe Asp Asp Thr Pro Phe Trp Leu Ala
           355                 360                 365
   Leu Trp Arg Thr Phe Arg Glu Cys Gln Val Val Val Pro Ser Lys His
       370                 375                 380
   Ala Ser Asp Val Leu Val Trp Lys Asp Leu Gly Ala
   385                 390                 395
<210> 137
   <211> 28346
   <212> DNA
   <213> Artificial
<220>
   <223> Binäres T-Plasmid LJB765 (synthetisch)
<400> 137
   gacatacaaa tggacgaacg gataaacctt ttcacgccct tttaaatatc cgattattct 60
   aataaacgct cttttctctt aggtttaccc gccaatatat cctgtcaaac actgatagtt 120
   taaactgaag gcgggaaacg acaatcagat ctagtaggaa acagctatga ccatgattac 180
   gccaagcttg catgcctgca ggtcgactct agactagtgg atccgatatc gattacgcca 240
   agctatcaac tttgtataga aaagttgcca tgattacgcc aagcttcccg ggctgcagca 300
   aatttacaca ttgccactaa acgtctaaac ccttgtaatt tgtttttgtt ttactatgtg 360
   tgttatgtat ttgatttgcg ataaattttt atatttggta ctaaatttat aacacctttt 420
   atgctaacgt ttgccaacac ttagcaattt gcaagttgat taattgattc taaattattt 480
   ttgtcttcta aatacatata ctaatcaact ggaaatgtaa atatttgcta atatttctac 540
   tataggagaa ttaaagtgag tgaatatggt accacaaggt ttggagattt aattgttgca 600
   atgctgcatg gatggcatat acaccaaaca ttcaataatt cttgaggata ataatggtac 660
   cacacaagat ttgaggtgca tgaacgtcac gtggacaaaa ggtttagtaa tttttcaaga 720
   caacaatgtt accacacaca agttttgagg tgcatgcatg gatgccctgt ggaaagttta 780
   aaaatatttt ggaaatgatt tgcatggaag ccatgtgtaa aaccatgaca tccacttgga 840
   ggatgcaata atgaagaaaa ctacaaattt acatgcaact agttatgcat gtagtctata 900
   taatgaggat tttgcaatac tttcattcat acacactcac taagttttac acgattataa 960
   tttcttcata gccagtacca tggaagttgt tgagaggttc tacggagagt tggatggaaa 1020
   ggtttcccaa ggagtgaacg ctttgttggg atctttcgga gttgagttga ctgatacccc 1080
   aactactaag ggattgccac tcgttgattc tccaactcca attgtgttgg gagtgtctgt 1140
   ttacttgacc atcgtgatcg gaggattgct ttggatcaag gctagagatc tcaagccaag 1200
   agcttctgag ccattcttgt tgcaagcttt ggtgttggtg cacaacttgt tctgcttcgc 1260
   tttgtctctt tacatgtgcg tgggtatcgc ttaccaagct atcacctgga gatattcctt 1320
   gtggggaaac gcttataacc caaagcacaa ggagatggct atcctcgttt acctcttcta 1380
   catgtccaag tacgtggagt tcatggatac cgtgatcatg atcctcaaga gatccaccag 1440
   acagatttct ttcctccacg tgtaccacca ttcttctatc tcccttatct ggtgggctat 1500
   tgctcatcat gctccaggag gagaggctta ttggagtgct gctctcaact ctggagtgca 1560
   tgtgttgatg tacgcttact acttcttggc tgcttgcttg agatcttccc caaagctcaa 1620
   gaacaagtac ctcttctggg gaagatacct cacccaattc cagatgttcc agttcatgct 1680
   caacttggtg caagcttact acgatatgaa aaccaacgct ccatatccac aatggctcat 1740
   caagatcctc ttctactaca tgatctccct cttgttcctc ttcggaaact tctacgtgca 1800
   aaagtacatc aagccatccg atggaaagca aaagggagct aagaccgagt gattaattaa 1860
   ggccgcagat ctccaccgcg gtggcggccg ctctagcccg atatctcgac aagctcgagt 1920
   ttctccataa taatgtgtga gtagttccca gataagggaa ttagggttcc tatagggttt 1980
   cgctcatgtg ttgagcatat aagaaaccct tagtatgtat ttgtatttgt aaaatacttc 2040
   tatcaataaa atttctaatt cctaaaacca aaatccagta ctaaaatcca gatcccccga 2100
   attaattcgg cgttaattca ggatatcggg cggatcgatc ctctagcata tgctcgagtt 2160
   accatttctt tttcctgcat ctcaatagta tatagggtat caaatagtga ttatccaaac 2220
   ttaaataagt tagaggaaac accaagatat gccatatact ctcatatttg acactatgat 2280
   tcaaagttgc acttgcataa aacttattaa ttcaatagta aaaccaaact tgtgcgtgat 2340
   acagttaaaa tgactaaact actaattaag gtccctccca ttagtaaata agttattttc 2400
   ttagaaaaag aaaataataa aaagaatgac gagtctatct aaatcatatt aacaagtaat 2460
   acatattgat tcattcgatg gaggaggcca ataattgtag taaacaagca gtgccgaggt 2520
   taatatatgc tcaagacagt aaataatcta aatgaattaa gacagtgatt tgcaaagagt 2580
   agatgcagag aagagaacta aagatttgct gctacacgta tataagaata gcaacagata 2640
   ttcattctgt ctctttgtgg aatatggata tctactaatc atcatctatc tgtgaagaat 2700
   aaaagaagcg gccacaagcg cagcgtcgca catatgatgt gtatcaaatt aggactccat 2760
   agccatgcat gctgaagaat gtcacacacg ttctgtcaca cgtgttactc tctcactgtt 2820
   ctcctcttcc tataaatcac cgcgccacag cttctccact tcaccacttc accacttcac 2880
   tcacaatcct tcattagttg tttactatca cagtcacaga tctagagcgg ccgcggcgcg 2940
   ccggggccca aaatggtgga cctcaagcct ggagtgaagc gcctggtgag ctggaaggag 3000
   atccgcgagc acgcgacgcc cgcgaccgcg tggatcgtga ttcaccacaa ggtctacgac 3060
   atctccaagt gggactcgca cccgggtggc tccgtgatgc tcacgcaggc cggcgaggac 3120
   gccacggacg ccttcgcggt cttccacccg tcctcggcgc tcaagctgct cgagcagttc 3180
   tacgtcggcg acgtggacga aacctccaag gccgagatcg agggggagcc ggcgagcgac 3240
   gaggagcgcg cgcgccgcga gcgcatcaac gagttcatcg cgtcctaccg ccgtctgcgc 3300
   gtcaaggtca agggcatggg gctctacgac gccagcgcgc tctactacgc gtggaagctc 3360
   gtgagcacgt tcggcatcgc ggtgctctcg atggcgatct gcttcttctt caacagtttc 3420
   gccatgtaca tggtcgccgg cgtgattatg gggctcttct accagcagtc cggatggctg 3480
   gcgcacgact tcttgcacaa ccaggtgtgc gagaaccgca cgctcggcaa ccttatcggc 3540
   tgcctcgtgg gcaacgcctg gcagggcttc agcatgcagt ggtggaagaa caagcacaac 3600
   ctgcaccacg cggtgccgaa cctgcacagc gccaaggacg agggcttcat cggcgacccg 3660
   gacatcgaca ccatgccgct gctggcgtgg tctaaggaga tggcgcgcaa ggcgttcgag 3720
   tcggcgcacg gcccgttctt catccgcaac caggcgttcc tatacttccc gctgctgctg 3780
   ctcgcgcgcc tgagctggct cgcgcagtcg ttcttctacg tgttcaccga gttctcgttc 3840
   ggcatcttcg acaaggtcga gttcgacgga ccggagaagg cgggtctgat cgtgcactac 3900
   atctggcagc tcgcgatccc gtacttctgc aacatgagcc tgtttgaggg cgtggcatac 3960
   ttcctcatgg gccaggcgtc ctgcggcttg ctcctggcgc tggtgttcag tattggccac 4020
   aacggcatgt cggtgtacga gcgcgaaacc aagccggact tctggcagct gcaggtgacc 4080
   acgacgcgca acatccgcgc gtcggtattc atggactggt tcaccggtgg cttgaactac 4140
   cagatcgacc atcacctgtt cccgctcgtg ccgcgccaca acttgccaaa ggtcaacgtg 4200
   ctcatcaagt cgctatgcaa ggagttcgac atcccgttcc acgagaccgg cttctgggag 4260
   ggcatctacg aggtcgtgga ccacctggcg gacatcagca aggaatttat caccgagttc 4320
   ccagcgatgt aagttaactt aattaaggcc gcagatatca gatctggtcg accgatcctg 4380
   caatagaatg ttgaggtgac cactttctgt aataaaataa ttataaaata aatttagaat 4440
   tgctgtagtc aagaacatca gttctaaaat attaataaag ttatggcctt ttgacatatg 4500
   tgtttcgata aaaaaatcaa aataaattga gatttattcg aaatacaatg aaagtttgca 4560
   gatatgagat atgtttctac aaaataataa cttaaaactc aactatatgc taatgttttt 4620
   cttggtgtgt ttcatagaaa attgtatccg tttcttagaa aatgctcgta aggatcggca 4680
   tgcaagctga tccccgggta cccaagtttg tacaaaaaag caggctgcga tcgctaagga 4740
   tgacctaccc attcttgaga caaatgttac attttagtat cagagtaaaa tgtgtaccta 4800
   taactcaaat tcgattgaca tgtatccatt caacataaaa ttaaaccagc ctgcacctgc 4860
   atccacattt caagtatttt caaaccgttc ggctcctatc caccgggtgt aacaagacgg 4920
   attccgaatt tggaagattt tgactcaaat tcccaattta tattgaccgt gactaaatca 4980
   actttaactt ctataattct gattaagctc ccaatttata ttcccaacgg cactacctcc 5040
   aaaatttata gactctcatc cccttttaaa ccaacttagt aaacgttttt tttttaattt 5100
   tatgaagtta agtttttacc ttgtttttaa aaagaatcgt tcataagatg ccatgccaga 5160
   acattagcta cacgttacac atagcatgca gccgcggaga attgtttttc ttcgccactt 5220
   gtcactccct tcaaacacct aagagcttct ctctcacagc acacacatac aatcacatgc 5280
   gtgcatgcat tattacacgt gatcgccatg caaatctcct ttatagccta taaattaact 5340
   catcggcttc actctttact caaaccaaaa ctcatcaata caaacaagat taaaaacaac 5400
   catggctaca aaggaggctt acgttttccc aactctcacc gagatcaaga gatctctccc 5460
   aaaggattgc ttcgaggctt ctgtgccttt gtctctctac tacactgtga gatgcttggt 5520
   tattgctgtg gctttgacct tcggattgaa ctacgctaga gctttgccag aggttgagtc 5580
   tttctgggct ttggatgctg ctttgtgcac tggatatatc ctcctccagg gaattgtgtt 5640
   ctggggattc ttcactgttg gacacgatgc tggacacgga gctttctcta gataccacct 5700
   cttgaacttc gttgtgggaa ccttcatgca ctctctcatc ttgaccccat tcgagtcttg 5760
   gaagttgacc cacagacacc accacaagaa caccggaaac atcgatagag atgaggtgtt 5820
   ctacccacag agaaaggctg atgatcaccc attgtccagg aacttgatct tggctttggg 5880
   agctgcttgg cttgcttatt tggtggaggg attcccacca agaaaggtga accacttcaa 5940
   cccattcgag ccactttttg tgagacaagt gtccgctgtg gttatctctt tgctcgctca 6000
   cttcttcgtt gctggactct ctatctactt gtctctccag ttgggactta agaccatggc 6060
   tatctactac tacggaccag ttttcgtgtt cggatctatg ttggtgatta ccaccttctt 6120
   gcaccacaac gatgaggaga ctccatggta tgctgattct gagtggactt acgtgaaggg 6180
   aaacttgtcc tctgtggata gatcttacgg tgctctcatc gataacctct cccacaacat 6240
   cggaactcac cagatccacc acctcttccc aattatccca cactacaagc tcaagaaggc 6300
   tactgctgct ttccaccaag ctttcccaga gcttgtgaga aagtccgatg agccaatcat 6360
   caaggctttc ttcagagtgg gaaggttgta tgctaactac ggagtggttg atcaagaggc 6420
   taagctcttc actttgaagg aggctaaggc tgctactgaa gctgctgcta agaccaagtc 6480
   tacctgagtt cgagtattat ggcattggga aaactgtttt tcttgtacca tttgttgtgc 6540
   ttgtaattta ctgtgttttt tattcggttt tcgctatcga actgtgaaat ggaaatggat 6600
   ggagaagagt taatgaatga tatggtcctt ttgttcattc tcaaattaat attatttgtt 6660
   ttttctctta tttgttgtgt gttgaatttg aaattataag agatatgcaa acattttgtt 6720
   ttgagtaaaa atgtgtcaaa tcgtggcctc taatgaccga agttaatatg aggagtaaaa 6780
   cacttgtagt tgtaccatta tgcttattca ctaggcaaca aatatatttt cagacctaga 6840
   aaagctgcaa atgttactga atacaagtat gtcctcttgt gttttagaca tttatgaact 6900
   ttcctttatg taattttcca gaatccttgt cagattctaa tcattgcttt ataattatag 6960
   ttatactcat ggatttgtag ttgagtatga aaatattttt taatgcattt tatgacttgc 7020
   caattgattg acaacatgca tcaatattta aattcgacgg cccggactgt atccaacttc 7080
   tgatctttga atctctctgt tccaacatgt tctgaaggag ttctaagact tttcagaaag 7140
   cttgtaacat gctttgtaga ctttctttga attactcttg caaactctga ttgaacctac 7200
   gtgaaaactg ctccagaagt tctaaccaaa ttccgtcttg ggaaggccca aaatttattg 7260
   agtacttcag tttcatggac gtgtcttcaa agatttataa cttgaaatcc catcattttt 7320
   aagagaagtt ctgttccgca atgtcttaga tctcattgaa atctacaact cttgtgtcag 7380
   aagttcttcc agaatcaact tgcatcatgg tgaaaatctg gccagaagtt ctgaacttgt 7440
   catatttctt aacagttaga aaaatttcta agtgtttaga attttgactt ttccaaagca 7500
   aacttgactt ttgactttct taataaaaca aacttcatat tctaacatgt cttgatgaaa 7560
   tgtgattctt gaaatttgat gttgatgcaa aagtcaaagt ttgacttttc agtgtgcaat 7620
   tgaccatttt gctcttgtgc caattccaaa cctaaattga tgtatcagtg ctgcaaactt 7680
   gatgtcatgg aagatcttat gagaaaattc ttgaagactg agaggaaaaa ttttgtagta 7740
   caacacaaag aatcctgttt ttcatagtcg gactagacac attaacataa aacaccactt 7800
   cattcgaaga gtgattgaag aaggaaatgt gcagttacct ttctgcagtt cataagagca 7860
   acttacagac acttttacta aaatactaca aagaggaaga ttttaacaac ttagagaagt 7920
   aatgggagtt aaagagcaac acattaaggg ggagtgttaa aattaatgtg ttgtaaccac 7980
   cactaccttt agtaagtatt ataagaaaat tgtaatcatc acattataat tattgtcctt 8040
   atttaaaatt atgataaagt tgtatcatta agattgagaa aaccaaatag tcctcgtctt 8100
   gatttttgaa ttattgtttt ctatgttact tttcttcaag cctatataaa aactttgtaa 8160
   tgctaaattg tatgctggaa aaaaatgtgt aatgaattca atagaaatta tggtatttca 8220
   aagtccaaaa tccatcaata gaaatttagt acaaaacgta actcaaaaat attctcttat 8280
   tttaaatttt acaacaatat aaaaatattc tcttatttta aattttacaa taatataatt 8340
   tatcacctgt cacctttaga ataccaccaa caatattaat acttagatat tttattctta 8400
   ataattttga gatctctcaa tatatctgat atttatttta tatttgtgtc atattttctt 8460
   atgttttaga gttaaccctt atatcttggt caaactagta attcaatata tgagtttgtg 8520
   aaggacacat tgacatcttg aaacattggt tttaaccttg ttggaatgtt aaaggtaata 8580
   aaacattcag aattatgacc atctattaat atacttcctt tgtcttttaa aaaagtgtgc 8640
   atgaaaatgc tctatggtaa gctagagtgt cttgctggcc tgtgtatatc aattccattt 8700
   ccagatggta gaaactgcca ctacgaataa ttagtcataa gacacgtatg ttaacacacg 8760
   tccccttgca tgttttttgc catatattcc gtctctttct ttttcttcac gtataaaaca 8820
   atgaactaat taatagagcg atcaagctga accatggatg cttataacgc tgctatggat 8880
   aagattggag ctgctatcat cgattggagt gatccagatg gaaagttcag agctgatagg 8940
   gaggattggt ggttgtgcga tttcagatcc gctatcacca ttgctctcat ctacatcgct 9000
   ttcgtgatct tgggatctgc tgtgatgcaa tctctcccag ctatggaccc ataccctatc 9060
   aagttcctct acaacgtgtc tcaaatcttc ctctgcgctt acatgactgt tgaggctgga 9120
   ttcctcgctt ataggaacgg atacaccgtt atgccatgca accacttcaa cgtgaacgat 9180
   ccaccagttg ctaacttgct ctggctcttc tacatctcca aagtgtggga tttctgggat 9240
   accatcttca ttgtgctcgg aaagaagtgg agacaactct ctttcttgca cgtgtaccac 9300
   cacaccacca tcttcctctt ctactggttg aacgctaacg tgctctacga tggagatatc 9360
   ttcttgacca tcctcctcaa cggattcatt cacaccgtga tgtacaccta ctacttcatc 9420
   tgcatgcaca ccaaggattc taagaccgga aagtctttgc caatctggtg gaagtcatct 9480
   ttgaccgctt tccaactctt gcaattcacc atcatgatgt cccaagctac ctacttggtt 9540
   ttccacggat gcgataaggt ttccctcaga atcaccatcg tgtacttcgt gtacattctc 9600
   tcccttttct tcctcttcgc tcagttcttc gtgcaatcct acatggctcc aaagaagaag 9660
   aagtccgctt gacctgcaga tagactatac tatgttttag cctgcctgct ggctagctac 9720
   tatgttatgt tatgttgtaa aataaacacc tgctaaggta tatctatcta tattttagca 9780
   tggctttctc aataaattgt ctttccttat cgtttactat cttataccta ataatgaaat 9840
   aataatatca catatgagga acggggcagg tttaggcata tatatacgag tgtagggcgg 9900
   agtggggggg cccctgtgca tatgtcaata tcgtaaatta taatcattgt atttattggt 9960
   aattttgtca tttatctgag ggatacgcaa gtccaaacat cttaaaaata cgttcgaata 10020
   catcaatctt tttctctatt ttggttagac gccatattta tcgaatacag aagcgctaac 10080
   tgaaaaacac agcttatgtt gacgacatca taataacata tgatcgcgca actattaaat 10140
   attttgaata taaaatttta aaatcttgtt aggtgtccaa agaattggaa aaacgataag 10200
   agacctgggc aggcggaaac atcaaaagcg ttaaacgata aatcgtatat gacaccacag 10260
   aagtcatgca tgataacaca acttcagtga caaaaataca atttaaaccc atgacatata 10320
   attggtcttg cgatacaaac tatgcatctc ttttgtttat acatttaata cattatcatc 10380
   aacggttctt atcagcccaa tctttccatg tattttagcg ggtaatttga actcctatca 10440
   aaagaaccat gcactcaagc acgtgtccaa acacttaaga cacgtatcaa ttgtgaactt 10500
   ttgagttctc tctccctctc atcacctccc cttccttata ccatgtgtgt tgagaccgag 10560
   aacaacgatg gaatccctac tgtggagatc gctttcgatg gagagagaga aagagctgag 10620
   gctaacgtga agttgtctgc tgagaagatg gaacctgctg ctttggctaa gaccttcgct 10680
   agaagatacg tggttatcga gggagttgag tacgatgtga ccgatttcaa acaccctgga 10740
   ggaaccgtga ttttctacgc tctctctaac actggagctg atgctactga ggctttcaag 10800
   gagttccacc acagatctag aaaggctagg aaggctttgg ctgctttgcc ttctagacct 10860
   gctaagaccg ctaaagtgga tgatgctgag atgctccagg atttcgctaa gtggagaaag 10920
   gagttggaga gggacggatt cttcaagcct tctcctgctc acgttgctta cagattcgct 10980
   gagttggctg ctatgtacgc tttgggaacc tacttgatgt acgctagata cgttgtgtcc 11040
   tctgtgttgg tttacgcttg cttcttcgga gctagatgtg gatgggttca acacgaggga 11100
   ggacactctt ctttgaccgg aaacatctgg tgggataaga gaatccaagc tttcactgct 11160
   ggattcggat tggctggatc tggagatatg tggaactcca tgcacaacaa gcaccacgct 11220
   actcctcaaa aagtgaggca cgatatggat ttggatacca ctcctgctgt tgctttcttc 11280
   aacaccgctg tggaggataa tagacctagg ggattctcta agtactggct cagattgcaa 11340
   gcttggacct tcattcctgt gacttctgga ttggtgttgc tcttctggat gttcttcctc 11400
   cacccttcta aggctttgaa gggaggaaag tacgaggagc ttgtgtggat gttggctgct 11460
   cacgtgatta gaacctggac cattaaggct gttactggat tcaccgctat gcaatcctac 11520
   ggactcttct tggctacttc ttgggtttcc ggatgctact tgttcgctca cttctctact 11580
   tctcacaccc acttggatgt tgttcctgct gatgagcact tgtcttgggt taggtacgct 11640
   gtggatcaca ccattgatat cgatccttct cagggatggg ttaactggtt gatgggatac 11700
   ttgaactgcc aagtgattca ccacctcttc ccttctatgc ctcaattcag acaacctgag 11760
   gtgtccagaa gattcgttgc tttcgctaag aagtggaacc tcaactacaa ggtgatgact 11820
   tatgctggag cttggaaggc tactttggga aacctcgata atgtgggaaa gcactactac 11880
   gtgcacggac aacactctgg aaagaccgct tgagccggag gagtaaaaga gaaggccaac 11940
   gtacgtcaag agtgtgtttg gtttgttgtt agtggttaga actttggttc tgacctctct 12000
   tgtaactttt tttttccata gtcttgttta tttgttaatg tacttttgca ttgtttttgt 12060
   tgttgccatg tttaaagttg tattgtgttt gtataatgtt ttgttttgtt taaatctttg 12120
   tagatggtaa atgagaaata agtaaatatt tccgagagac tgttgagtga cgacgttact 12180
   tcttgtcctg caggacccag ctttcttgta caaagtggcc atgattacgc caagcttccc 12240
   gggctgcagc aaatttacac attgccacta aacgtctaaa cccttgtaat ttgtttttgt 12300
   tttactatgt gtgttatgta tttgatttgc gataaatttt tatatttggt actaaattta 12360
   taacaccttt tatgctaacg tttgccaaca cttagcaatt tgcaagttga ttaattgatt 12420
   ctaaattatt tttgtcttct aaatacatat actaatcaac tggaaatgta aatatttgct 12480
   aatatttcta ctataggaga attaaagtga gtgaatatgg taccacaagg tttggagatt 12540
   taattgttgc aatgctgcat ggatggcata tacaccaaac attcaataat tcttgaggat 12600
   aataatggta ccacacaaga tttgaggtgc atgaacgtca cgtggacaaa aggtttagta 12660
   atttttcaag acaacaatgt taccacacac aagttttgag gtgcatgcat ggatgccctg 12720
   tggaaagttt aaaaatattt tggaaatgat ttgcatggaa gccatgtgta aaaccatgac 12780
   atccacttgg aggatgcaat aatgaagaaa actacaaatt tacatgcaac tagttatgca 12840
   tgtagtctat ataatgagga ttttgcaata ctttcattca tacacactca ctaagtttta 12900
   cacgattata atttcttcat agccagtggc gcgccaccat ggcgactaga caaagaactg 12960
   ctactactgt ggtggttgag gatttgccaa aggttacctt ggaggctaag tctgagccag 13020
   ttttcccaga tatcaagacc atcaaggatg ctattccagc tcattgcttc caaccatctc 13080
   tcgtgacttc cttctactac gtgttcaggg atttcgctat ggtttctgct cttgtgtggg 13140
   ctgctttgac ttacatccca tccatcccag atcaaacctt gagagtggct gcttggatgg 13200
   tttacggatt cgtgcaagga cttttctgta ccggagtgtg gattttggga catgagtgtg 13260
   gacatggagc tttctccctt catggaaagg ttaacaacgt gaccggatgg ttcttgcatt 13320
   ctttcctcct cgtgccatac ttctcttgga agtactctca tcacagacac catagattca 13380
   ccggacacat ggatttggat atggctttcg tgccaaagac tgagccaaag ccatctaagt 13440
   ctctcatgat cgctggaatt gatgttgctg agttggttga ggatactcca gctgctcaaa 13500
   tggtgaagct catcttccat caactcttcg gatggcaggc ttacttgttc ttcaacgctt 13560
   cttccggaaa gggatctaag caatgggagc caaagactgg attgtccaag tggttcagag 13620
   tgtctcattt cgagccaacc tctgctgttt tcagaccaaa cgaggctatc ttcatcctca 13680
   tctcagatat cggattggct ttgatgggaa ccgctctcta tttcgcttct aagcaagttg 13740
   gagtgtccac tatcttgttc ttgtacctcg tgccttattt gtgggttcac cattggttgg 13800
   tggctattac ttacttgcat caccaccata ctgagttgcc acattacact gctgagggat 13860
   ggacttatgt gaagggagct ttggctactg ttgataggga gttcggattc atcggaaagc 13920
   acctcttcca cggaattatt gagaagcacg tggtgcatca tttgttccca aagatcccat 13980
   tctacaaggc tgatgaggct actgaggcta ttaagccagt gatcggagat cattactgcc 14040
   acgatgatag atctttcctc ggacaactct ggactatttt cggaaccctc aagtacgttg 14100
   agcatgatcc agctagacca ggtgctatga ggtggaacaa ggattgatta attaaggccg 14160
   cagatctcca ccgcggtggc ggccgctcta gcccgatatc tcgacaagct cgagtttctc 14220
   cataataatg tgtgagtagt tcccagataa gggaattagg gttcctatag ggtttcgctc 14280
   atgtgttgag catataagaa acccttagta tgtatttgta tttgtaaaat acttctatca 14340
   ataaaatttc taattcctaa aaccaaaatc cagtactaaa atccagatcc cccgaattaa 14400
   ttcggcgtta attcaggata tcgggcggat ccccgggtac ccaactttat tatacatagt 14460
   tgataattca ctggccgatt tggtttaaac gaattcgccc ttctttagca gatatttggt 14520
   gtctaaatgt ttattttgtg atatgttcat gtttgaaatg gtggtttcga aaccagggac 14580
   aacgttggga tctgataggg tgtcaaagag tattatggat tgggacaatt tcggtcatga 14640
   gttgcaaatt caagtatatc gttcgattat gaaaattttc gaagaatatc ccatttgaga 14700
   gagtctttac ctcattaatg tttttagatt atgaaatttt atcatagttc atcgtagtct 14760
   ttttggtgta aaggctgtaa aaagaaattg ttcacttttg ttttcgttta tgtgaaggct 14820
   gtaaaagatt gtaaaagact attttggtgt tttggataaa atgatagttt ttatagattc 14880
   ttttgctttt agaagaaata catttgaaat tttttccatg ttgagtataa aataccgaaa 14940
   tcgattgaag atcatagaaa tattttaact gaaaacaaat ttataactga ttcaattctc 15000
   tccattttta tacctattta accgtaatcg attctaatag atgatcgatt ttttatataa 15060
   tcctaattaa ccaacggcat gtattggata attaaccgat caactctcac ccctaataga 15120
   atcagtattt tccttcgacg ttaattgatc ctacactatg taggtcatat ccatcgtttt 15180
   aatttttggc caccattcaa ttctgtcttg cctttaggga tgtgaatatg aacggccaag 15240
   gtaagagaat aaaaataatc caaattaaag caagagaggc caagtaagat aatccaaatg 15300
   tacacttgtc attgccaaaa ttagtaaaat actcggcata ttgtattccc acacattatt 15360
   aaaataccgt atatgtattg gctgcatttg catgaataat actacgtgta agcccaaaag 15420
   aacccacgtg tagcccatgc aaagttaaca ctcacgaccc cattcctcag tctccactat 15480
   ataaacccac catccccaat ctcaccaaac ccaccacaca actcacaact cactctcaca 15540
   ccttctagag gatctgatat ctgcggccgc ggcgcgccac catgggaaaa ggatctgagg 15600
   gaagatctgc tgctagagag atgactgctg aggctaacgg agataagaga aagaccatcc 15660
   tcattgaggg agtgttgtac gatgctacca acttcaaaca cccaggaggt tccattatta 15720
   acttcctcac cgagggagaa gctggagttg atgctaccca agcttacaga gagttccatc 15780
   agagatccgg aaaggctgat aagtacctca agtccctccc aaagttggat gcttctaagg 15840
   tggagtctag gttctctgct aaggagcagg ctagaaggga cgctatgacc agggattacg 15900
   ctgctttcag agaggagttg gttgctgagg gatacttcga tccatctatc ccacacatga 15960
   tctacagagt ggtggagatt gtggctttgt tcgctttgtc tttctggttg atgtctaagg 16020
   cttctccaac ctctttggtt ttgggagtgg tgatgaacgg aatcgctcaa ggaagatgcg 16080
   gatgggttat gcatgagatg ggacacggat ctttcactgg agttatctgg ctcgatgata 16140
   ggatgtgcga gttcttctac ggagttggat gtggaatgtc tggacactac tggaagaacc 16200
   agcattctaa gcaccatgct gctccaaaca gattggagca cgatgtggat ttgaacacct 16260
   tgccactcgt tgctttcaac gagagagttg tgaggaaggt taagccagga tctttgttgg 16320
   ctttgtggct cagagttcag gcttatttgt tcgctccagt gtcttgcttg ttgatcggat 16380
   tgggatggac cttgtacttg cacccaagat atatgctcag gaccaagaga catatggagt 16440
   ttgtgtggat cttcgctaga tatatcggat ggttctcctt gatgggagct ttgggatatt 16500
   ctcctggaac ttctgtggga atgtacctct gctctttcgg acttggatgc atctacatct 16560
   tcctccaatt cgctgtgtct catacccatt tgccagttac caacccagag gatcaattgc 16620
   attggcttga gtacgctgct gatcataccg tgaacatctc taccaagtct tggttagtta 16680
   cctggtggat gtctaacctc aacttccaaa tcgagcatca tttgttccca accgctccac 16740
   aattcaggtt caaggagatc tctccaagag ttgaggctct cttcaagaga cataacctcc 16800
   cttactacga tttgccatac acctctgctg tttctactac cttcgctaac ctctactctg 16860
   ttggacattc tgttggagct gataccaaga agcaggattg attaattaag gccgcctcga 16920
   gcatgcatct agagggcccg ctagcgttaa ccctgcttta atgagatatg cgagacgcct 16980
   atgatcgcat gatatttgct ttcaattctg ttgtgcacgt tgtaaaaaac ctgagcatgt 17040
   gtagctcaga tccttaccgc cggtttcggt tcattctaat gaatatatca cccgttacta 17100
   tcgtattttt atgaataata ttctccgttc aatttactga ttgtccgtcc acgggcagga 17160
   catagggact actacaagca tagtatgctt cagacaaaga gctaggaaag aactcttgat 17220
   ggaggttaag agaaaaaagt gctagagggg catagtaatc aaacttgtca aaaccgtcat 17280
   catgatgagg gatgacataa tataaaaagt tgactaaggt cttggtagta ctctttgatt 17340
   agtattatat attggtgaga acatgagtca agaggagaca agaaaccgag gaaccatagt 17400
   ttagcaacaa gatggaagtt gcaaagttga gctagccgct cgattagtta catctcctaa 17460
   gcagtactac aaggaatggt ctctatactt tcatgtttag cacatggtag tgcggattga 17520
   caagttagaa acagtgctta ggagacaaag agtcagtaaa ggtattgaaa gagtgaagtt 17580
   gatgctcgac aggtcaggag aagtccctcc gccagatggt gactaccaag gggttggtat 17640
   cagctgagac ccaaataaga ttcttcggtt gaaccagtgg ttcgaccgag actcttaggg 17700
   tgggatttca ctgtaagatt tgtgcatttt gttgaatata aattgacaat tttttttatt 17760
   taattataga ttatttagaa tgaattacat atttagtttc taacaaggat agcaatggat 17820
   gggtatgggt acaggttaaa catatctatt acccacccat ctagtcgtcg ggttttacac 17880
   gtacccaccc gtttacataa accagaccgg aattttaaac cgtacccgtc cgttagcggg 17940
   tttcagattt acccgtttaa tcgggtaaaa cctgattact aaatatatat tttttatttg 18000
   ataaacaaaa caaaaatgtt aatattttca tattggatgc aattttaaga aacacatatt 18060
   cataaatttc catatttgta ggaaaataaa aagaaaaata tattcaagaa cacaaatttc 18120
   accgacatga cttttattac agagttggaa ttagatctaa caattgaaaa attaaaatta 18180
   agatagaata tgttgaggaa catgacatag tataatgctg ggttacccgt cgggtaggta 18240
   tcgaggcgga tactactaaa tccatcccac tcgctatccg ataatcactg gtttcgggta 18300
   tacccattcc cgtcaacagg cctttttaac cggataattt caacttatag tgaatgaatt 18360
   ttgaataaat agttagaata ccaaaatcct ggattgcatt tgcaatcaaa ttttgtgaac 18420
   cgttaaattt tgcatgtact tgggatagat ataatagaac cgaattttca ttagtttaat 18480
   ttataactta ctttgttcaa agaaaaaaaa tatctatcca atttacttat aataaaaaat 18540
   aatctatcca agttacttat tataatcaac ttgtaaaaag gtaagaatac aaatgtggta 18600
   gcgtacgtgt gattatatgt gacgaaatgt tatatctaac aaaagtccaa attcccatgg 18660
   taaaaaaaat caaaatgcat ggcaggctgt ttgtaacctt ggaataagat gttggccaat 18720
   tctggagccg ccacgtacgc aagactcagg gccacgttct cttcatgcaa ggatagtaga 18780
   acaccactcc acccacctcc tatattagac ctttgcccaa ccctccccaa ctttcccatc 18840
   ccatccacaa agaaaccgac atttttatca taaatcggcg cgccgttaac gctagcgcca 18900
   tgggtgcagg cggtcgaatg caagatccca ccaacggtgg caacaaaacc gagcccgaac 18960
   caatccaacg ggtcccacat gaaaaacccc cattcacagt tggagacatc aagaaagcga 19020
   tcccacctca ttgtttcaac cgatcggtaa ttcgttcatt ttcatacgtc ttttacgacc 19080
   tcacaatcgc gtcaatcttg tactacattg ccaacaatta catctctacc ctccctagcc 19140
   cgctcgccta cgtggcatgg cccgtttact gggccgtcca agggtgcgtc ttaaccgggg 19200
   tgtgggtcat agcccacgaa tgtggccatc atgcttttag cgaccaccaa tggctcgatg 19260
   acaccgtggg tctcgtcttg cactcgttcc tactcgtgcc ctacttttcg tggaaatata 19320
   gccaccgtag gcaccactcg aacacgggtt cgatcgagca cgatgaggtt ttcgtcccga 19380
   agttgaaatc gggcgtccgg tcaaccgccc ggtacctaaa caacccaccg ggccgaatct 19440
   tgaccctact cgtaacccta accctcggtt ggcctctata cctcacgttc aacgtttcgg 19500
   gccgttacta cgaccggttc gcgtgccatt tcgacccgaa tagcccgatc tactcgaagc 19560
   gcgaacgggc tcaaatcttc atatccgacg ccgggatctt agccgtagtc ttcgtactct 19620
   tccgactcgc aatgaccaaa gggctcacgt gggtcctaac catgtacggt ggcccgttac 19680
   tcgtggtcaa cggtttccta gtcttgatca cattcctaca acacactcac ccttcgctcc 19740
   cgcactatga ctcaaccgaa tgggattggt tacgtggggc cctcaccaca atcgaccgtg 19800
   attacgggat cctaaacaaa gtgttccata acataaccga cactcacgtg gcccaccatt 19860
   tgttctctac aatgcctcat taccatgcaa tggaagccac gaaggtgatc aaaccgattt 19920
   tgggcgatta ttatcagttt gacgggacct cgatttttaa ggcgatgtat cgggaaacaa 19980
   aggagtgcat ttatgttgat aaggatgagg aggtgaaaga tggtgtttat tggtatcgta 20040
   ataagattta agttaacgct agcttaatta aggccgcaga tatcagatct ggtcgaccta 20100
   gaggatcccc ggccgcaaag ataataacaa aagcctacta tataacgtac atgcaagtat 20160
   tgtatgatat taatgttttt acgtacgtgt aaacaaaaat aattacgttt gtaacgtatg 20220
   gtgatgatgt ggtgcactag gtgtaggcct tgtattaata aaaagaagtt tgttctatat 20280
   agagtggttt agtacgacga tttatttact agtcggattg gaatagagaa ccgaattctt 20340
   caatccttgc ttttgatcaa gaattgaaac cgaatcaaat gtaaaagttg atatatttga 20400
   aaaacgtatt gagcttatga aaatgctaat actctcatct gtatggaaaa gtgactttaa 20460
   aaccgaactt aaaagtgaca aaaggggaat atcgcatcaa accgaatgaa accgatagaa 20520
   gggcgaattc gcggccgcta aattcaattc gccctatagt gagtcgtatt acaattcact 20580
   ggccgtcgtt ttacaacgtc gtgactggga aaaccctggc gttacccaac ttaatcgcct 20640
   tgcagcacat ccccctttcg ccagctggcg taatagcgaa gaggcccgca ccgatcgccc 20700
   ttcccaacag ttgcaaatga tatcgcccgg gctcgaggta ccatcgttca aacatttggc 20760
   aataaagttt cttaagattg aatcctgttg ccggtcttgc gatgattatc atataatttc 20820
   tgttgaatta cgttaagcat gtaataatta acatgtaatg catgacgtta tttatgagat 20880
   gggtttttat gattagagtc ccgcaattat acatttaata cgcgatagaa aacaaaatat 20940
   agcgcgcaaa ctaggataaa ttatcgcgcg cggtgtcatc tatgttacta gatcgggcat 21000
   taccctgtta tccctagagc tcgaattcac tggccgtcgt tttacaacga ctcaggatcc 21060
   tgtcaaacac tgatagttta aactgaaggc gggaaacgac aatctgatca tgagcggaga 21120
   attaagggag tcacgttatg acccccgccg atgacgcggg acaagccgtt ttacgtttgg 21180
   aactgacaga accgcaacgt tgaaggagcc actcagccgc gggtttctgg agtttaatga 21240
   gctaagcaca tacgtcagaa accattattg cgcgttcaaa agtcgcctaa ggtcactatc 21300
   agctagcaaa tatttcttgt caaaaatgct ccactgacgt tccataaatt cccctcggta 21360
   tccaattaga gtctcatatt cactctcaat ccaaataatc tgcaccggat ctggatcgtt 21420
   tcgcatgatt gaacaagatg gattgcacgc aggttctccg gccgcttggg tggagaggct 21480
   attcggctat gactgggcac aacagacaat cggctgctct gatgccgccg tgttccggct 21540
   gtcagcgcag gggcgcccgg ttctttttgt caagaccgac ctgtccggtg ccctgaatga 21600
   actgcaggac gaggcagcgc ggctatcgtg gctggccacg acgggcgttc cttgcgcagc 21660
   tgtgctcgac gttgtcactg aagcgggaag ggactggctg ctattgggcg aagtgccggg 21720
   gcaggatctc ctgtcatctc accttgctcc tgccgagaaa gtatccatca tggctgatgc 21780
   aatgcggcgg ctgcatacgc ttgatccggc tacctgccca ttcgaccacc aagcgaaaca 21840
   tcgcatcgag cgagcacgta ctcggatgga agccggtctt gtcgatcagg atgatctgga 21900
   cgaagagcat caggggctcg cgccagccga actgttcgcc aggctcaagg cgcgcatgcc 21960
   cgacggcgag gatctcgtcg tgacccatgg cgatgcctgc ttgccgaata tcatggtgga 22020
   aaatggccgc ttttctggat tcatcgactg tggccggctg ggtgtggcgg accgctatca 22080
   ggacatagcg ttggctaccc gtgatattgc tgaagagctt ggcggcgaat gggctgaccg 22140
   cttcctcgtg ctttacggta tcgccgctcc cgattcgcag cgcatcgcct tctatcgcct 22200
   tcttgacgag ttcttctgag ctagggataa cagggtaatt gagcgggacc caagctctag 22260
   atcttgctgc gttcggatat tttcgtggag ttcccgccac agacccggat gatccccgat 22320
   cgttcaaaca tttggcaata aagtttctta agattgaatc ctgttgccgg tcttgcgatg 22380
   attatcatat aatttctgtt gaattacgtt aagcatgtaa taattaacat gtaatgcatg 22440
   acgttattta tgagatgggt ttttatgatt agagtcccgc aattatacat ttaatacgcg 22500
   atagaaaaca aaatatagcg cgcaaactag gataaattat cgcgcgcggt gtcatctatg 22560
   ttactagatc gggcctcctg tcaagctctg cttggtaata attgtcatta gattgttttt 22620
   atgcatagat gcactcgaaa tcagccaatt ttagacaagt atcaaacgga tgttaattca 22680
   gtacattaaa gacgtccgca atgtgttatt aagttgtcta agcgtcaatt tgtttacacc 22740
   acaatatatc ctgccaccag ccagccaaca gctccccgac cggcagctcg gcacaaaatc 22800
   accacgcgtt accaccacgc cggccggccg catggtgttg accgtgttcg ccggcattgc 22860
   cgagttcgag cgttccctaa tcatcgaccg cacccggagc gggcgcgagg ccgccaaggc 22920
   ccgaggcgtg aagtttggcc cccgccctac cctcaccccg gcacagatcg cgcacgcccg 22980
   cgagctgatc gaccaggaag gccgcaccgt gaaagaggcg gctgcactgc ttggcgtgca 23040
   tcgctcgacc ctgtaccgcg cacttgagcg cagcgaggaa gtgacgccca ccgaggccag 23100
   gcggcgcggt gccttccgtg aggacgcatt gaccgaggcc gacgccctgg cggccgccga 23160
   gaatgaacgc caagaggaac aagcatgaaa ccgcaccagg acggccagga cgaaccgttt 23220
   ttcattaccg aagagatcga ggcggagatg atcgcggccg ggtacgtgtt cgagccgccc 23280
   gcgcacgtct caaccgtgcg gctgcatgaa atcctggccg gtttgtctga tgccaagctg 23340
   gcggcctggc cggccagctt ggccgctgaa gaaaccgagc gccgccgtct aaaaaggtga 23400
   tgtgtatttg agtaaaacag cttgcgtcat gcggtcgctg cgtatatgat gcgatgagta 23460
   aataaacaaa tacgcaaggg gaacgcatga aggttatcgc tgtacttaac cagaaaggcg 23520
   ggtcaggcaa gacgaccatc gcaacccatc tagcccgcgc cctgcaactc gccggggccg 23580
   atgttctgtt agtcgattcc gatccccagg gcagtgcccg cgattgggcg gccgtgcggg 23640
   aagatcaacc gctaaccgtt gtcggcatcg accgcccgac gattgaccgc gacgtgaagg 23700
   ccatcggccg gcgcgacttc gtagtgatcg acggagcgcc ccaggcggcg gacttggctg 23760
   tgtccgcgat caaggcagcc gacttcgtgc tgattccggt gcagccaagc ccttacgaca 23820
   tatgggccac cgccgacctg gtggagctgg ttaagcagcg cattgaggtc acggatggaa 23880
   ggctacaagc ggcctttgtc gtgtcgcggg cgatcaaagg cacgcgcatc ggcggtgagg 23940
   ttgccgaggc gctggccggg tacgagctgc ccattcttga gtcccgtatc acgcagcgcg 24000
   tgagctaccc aggcactgcc gccgccggca caaccgttct tgaatcagaa cccgagggcg 24060
   acgctgcccg cgaggtccag gcgctggccg ctgaaattaa atcaaaactc atttgagtta 24120
   atgaggtaaa gagaaaatga gcaaaagcac aaacacgcta agtgccggcc gtccgagcgc 24180
   acgcagcagc aaggctgcaa cgttggccag cctggcagac acgccagcca tgaagcgggt 24240
   caactttcag ttgccggcgg aggatcacac caagctgaag atgtacgcgg tacgccaagg 24300
   caagaccatt accgagctgc tatctgaata catcgcgcag ctaccagagt aaatgagcaa 24360
   atgaataaat gagtagatga attttagcgg ctaaaggagg cggcatggaa aatcaagaac 24420
   aaccaggcac cgacgccgtg gaatgcccca tgtgtggagg aacgggcggt tggccaggcg 24480
   taagcggctg ggttgtctgc cggccctgca atggcactgg aacccccaag cccgaggaat 24540
   cggcgtgagc ggtcgcaaac catccggccc ggtacaaatc ggcgcggcgc tgggtgatga 24600
   cctggtggag aagttgaagg ccgcgcaggc cgcccagcgg caacgcatcg aggcagaagc 24660
   acgccccggt gaatcgtggc aagcggccgc tgatcgaatc cgcaaagaat cccggcaacc 24720
   gccggcagcc ggtgcgccgt cgattaggaa gccgcccaag ggcgacgagc aaccagattt 24780
   tttcgttccg atgctctatg acgtgggcac ccgcgatagt cgcagcatca tggacgtggc 24840
   cgttttccgt ctgtcgaagc gtgaccgacg agctggcgag gtgatccgct acgagcttcc 24900
   agacgggcac gtagaggttt ccgcagggcc ggccggcatg gccagtgtgt gggattacga 24960
   cctggtactg atggcggttt cccatctaac cgaatccatg aaccgatacc gggaagggaa 25020
   gggagacaag cccggccgcg tgttccgtcc acacgttgcg gacgtactca agttctgccg 25080
   gcgagccgat ggcggaaagc agaaagacga cctggtagaa acctgcattc ggttaaacac 25140
   cacgcacgtt gccatgcagc gtacgaagaa ggccaagaac ggccgcctgg tgacggtatc 25200
   cgagggtgaa gccttgatta gccgctacaa gatcgtaaag agcgaaaccg ggcggccgga 25260
   gtacatcgag atcgagctag ctgattggat gtaccgcgag atcacagaag gcaagaaccc 25320
   ggacgtgctg acggttcacc ccgattactt tttgatcgat cccggcatcg gccgttttct 25380
   ctaccgcctg gcacgccgcg ccgcaggcaa ggcagaagcc agatggttgt tcaagacgat 25440
   ctacgaacgc agtggcagcg ccggagagtt caagaagttc tgtttcaccg tgcgcaagct 25500
   gatcgggtca aatgacctgc cggagtacga tttgaaggag gaggcggggc aggctggccc 25560
   gatcctagtc atgcgctacc gcaacctgat cgagggcgaa gcatccgccg gttcctaatg 25620
   tacggagcag atgctagggc aaattgccct agcaggggaa aaaggtcgaa aaggtctctt 25680
   tcctgtggat agcacgtaca ttgggaaccc aaagccgtac attgggaacc ggaacccgta 25740
   cattgggaac ccaaagccgt acattgggaa ccggtcacac atgtaagtga ctgatataaa 25800
   agagaaaaaa ggcgattttt ccgcctaaaa ctctttaaaa cttattaaaa ctcttaaaac 25860
   ccgcctggcc tgtgcataac tgtctggcca gcgcacagcc gaagagctgc aaaaagcgcc 25920
   tacccttcgg tcgctgcgct ccctacgccc cgccgcttcg cgtcggccta tcgcggccgc 25980
   tggccgctca aaaatggctg gcctacggcc aggcaatcta ccagggcgcg gacaagccgc 26040
   gccgtcgcca ctcgaccgcc ggcgcccaca tcaaggcacc ctgcctcgcg cgtttcggtg 26100
   atgacggtga aaacctctga cacatgcagc tcccggagac ggtcacagct tgtctgtaag 26160
   cggatgccgg gagcagacaa gcccgtcagg gcgcgtcagc gggtgttggc gggtgtcggg 26220
   gcgcagccat gacccagtca cgtagcgata gcggagtgta tactggctta actatgcggc 26280
   atcagagcag attgtactga gagtgcacca tatgcggtgt gaaataccgc acagatgcgt 26340
   aaggagaaaa taccgcatca ggcgctcttc cgcttcctcg ctcactgact cgctgcgctc 26400
   ggtcgttcgg ctgcggcgag cggtatcagc tcactcaaag gcggtaatac ggttatccac 26460
   agaatcaggg gataacgcag gaaagaacat gtgagcaaaa ggccagcaaa aggccaggaa 26520
   ccgtaaaaag gccgcgttgc tggcgttttt ccataggctc cgcccccctg acgagcatca 26580
   caaaaatcga cgctcaagtc agaggtggcg aaacccgaca ggactataaa gataccaggc 26640
   gtttccccct ggaagctccc tcgtgcgctc tcctgttccg accctgccgc ttaccggata 26700
   cctgtccgcc tttctccctt cgggaagcgt ggcgctttct catagctcac gctgtaggta 26760
   tctcagttcg gtgtaggtcg ttcgctccaa gctgggctgt gtgcacgaac cccccgttca 26820
   gcccgaccgc tgcgccttat ccggtaacta tcgtcttgag tccaacccgg taagacacga 26880
   cttatcgcca ctggcagcag ccactggtaa caggattagc agagcgaggt atgtaggcgg 26940
   tgctacagag ttcttgaagt ggtggcctaa ctacggctac actagaagga cagtatttgg 27000
   tatctgcgct ctgctgaagc cagttacctt cggaaaaaga gttggtagct cttgatccgg 27060
   caaacaaacc accgctggta gcggtggttt ttttgtttgc aagcagcaga ttacgcgcag 27120
   aaaaaaagga tctcaagaag atcctttgat cttttctacg gggtctgacg ctcagtggaa 27180
   cgaaaactca cgttaaggga ttttggtcat gcatgatata tctcccaatt tgtgtagggc 27240
   ttattatgca cgcttaaaaa taataaaagc agacttgacc tgatagtttg gctgtgagca 27300
   attatgtgct tagtgcatct aacgcttgag ttaagccgcg ccgcgaagcg gcgtcggctt 27360
   gaacgaattt ctagctagac attatttgcc gactaccttg gtgatctcgc ctttcacgta 27420
   gtggacaaat tcttccaact gatctgcgcg cgaggccaag cgatcttctt cttgtccaag 27480
   ataagcctgt ctagcttcaa gtatgacggg ctgatactgg gccggcaggc gctccattgc 27540
   ccagtcggca gcgacatcct tcggcgcgat tttgccggtt actgcgctgt accaaatgcg 27600
   ggacaacgta agcactacat ttcgctcatc gccagcccag tcgggcggcg agttccatag 27660
   cgttaaggtt tcatttagcg cctcaaatag atcctgttca ggaaccggat caaagagttc 27720
   ctccgccgct ggacctacca aggcaacgct atgttctctt gcttttgtca gcaagatagc 27780
   cagatcaatg tcgatcgtgg ctggctcgaa gatacctgca agaatgtcat tgcgctgcca 27840
   ttctccaaat tgcagttcgc gcttagctgg ataacgccac ggaatgatgt cgtcgtgcac 27900
   aacaatggtg acttctacag cgcggagaat ctcgctctct ccaggggaag ccgaagtttc 27960
   caaaaggtcg ttgatcaaag ctcgccgcgt tgtttcatca agccttacgg tcaccgtaac 28020
   cagcaaatca atatcactgt gtggcttcag gccgccatcc actgcggagc cgtacaaatg 28080
   tacggccagc aacgtcggtt cgagatggcg ctcgatgacg ccaactacct ctgatagttg 28140
   agtcgatact tcggcgatca ccgcttcccc catgatgttt aactttgttt tagggcgact 28200
   gccctgctgc gtaacatcgt tgctgctcca taacatcaaa catcgaccca cggcgtaacg 28260
   cgcttgctgc ttggatgccc gaggcataga ctgtacccca aaaaaacagt cataacaagc 28320
   catgaaaacc gccactgcgt tccatg 28346
<210> 138
   <211> 819
   <212> DNA
   <213> Thalassiosira pseudonana

<400> 138
   atggatgctt ataacgctgc tatggataag attggagctg ctatcatcga ttggagtgat 60
   ccagatggaa agttcagagc tgatagggag gattggtggt tgtgcgattt cagatccgct 120
   atcaccattg ctctcatcta catcgctttc gtgatcttgg gatctgctgt gatgcaatct 180
   ctcccagcta tggacccata ccctatcaag ttcctctaca acgtgtctca aatcttcctc 240
   tgcgcttaca tgactgttga ggctggattc ctcgcttata ggaacggata caccgttatg 300
   ccatgcaacc acttcaacgt gaacgatcca ccagttgcta acttgctctg gctcttctac 360
   atctccaaag tgtgggattt ctgggatacc atcttcattg tgctcggaaa gaagtggaga 420
   caactctctt tcttgcacgt gtaccaccac accaccatct tcctcttcta ctggttgaac 480
   gctaacgtgc tctacgatgg agatatcttc ttgaccatcc tcctcaacgg attcattcac 540
   accgtgatgt acacctacta cttcatctgc atgcacacca aggattctaa gaccggaaag 600
   tctttgccaa tctggtggaa gtcatctttg accgctttcc aactcttgca attcaccatc 660
   atgatgtccc aagctaccta cttggttttc cacggatgcg ataaggtttc cctcagaatc 720
   accatcgtgt acttcgtgta cattctctcc cttttcttcc tcttcgctca gttcttcgtg 780
   caatcctaca tggctccaaa gaagaagaag tccgcttga 819
<210> 139
   <211> 1371
   <212> DNA
   <213> Ostreococcus tauri
<400> 139
   atgtgtgttg agaccgagaa caacgatgga atccctactg tggagatcgc tttcgatgga    60
   gagagagaaa gagctgaggc taacgtgaag ttgtctgctg agaagatgga acctgctgct   120
   ttggctaaga ccttcgctag aagatacgtg gttatcgagg gagttgagta cgatgtgacc   180
   gatttcaaac accctggagg aaccgtgatt ttctacgctc tctctaacac tggagctgat   240
   gctactgagg ctttcaagga gttccaccac agatctagaa aggctaggaa ggctttggct   300
   gctttgcctt ctagacctgc taagaccgct aaagtggatg atgctgagat gctccaggat   360
   ttcgctaagt ggagaaagga gttggagagg gacggattct tcaagccttc tcctgctcac   420
   gttgcttaca gattcgctga gttggctgct atgtacgctt tgggaaccta cttgatgtac   480
   gctagatacg ttgtgtcctc tgtgttggtt tacgcttgct tcttcggagc tagatgtgga   540
   tgggttcaac acgagggagg acactcttct ttgaccggaa acatctggtg ggataagaga   600
   atccaagctt tcactgctgg attcggattg gctggatctg gagatatgtg gaactccatg   660
   cacaacaagc accacgctac tcctcaaaaa gtgaggcacg atatggattt ggataccact   720
   cctgctgttg ctttcttcaa caccgctgtg gaggataata gacctagggg attctctaag   780
   tactggctca gattgcaagc ttggaccttc attcctgtga cttctggatt ggtgttgctc   840
   ttctggatgt tcttcctcca cccttctaag gctttgaagg gaggaaagta cgaggagctt   900
   gtgtggatgt tggctgctca cgtgattaga acctggacca ttaaggctgt tactggattc   960
   accgctatgc aatcctacgg actcttcttg gctacttctt gggtttccgg atgctacttg  1020
   ttcgctcact tctctacttc tcacacccac ttggatgttg ttcctgctga tgagcacttg  1080
   tcttgggtta ggtacgctgt ggatcacacc attgatatcg atccttctca gggatgggtt  1140
   aactggttga tgggatactt gaactgccaa gtgattcacc acctcttccc ttctatgcct  1200
   caattcagac aacctgaggt gtccagaaga ttcgttgctt tcgctaagaa gtggaacctc  1260
   aactacaagg tgatgactta tgctggagct tggaaggcta ctttgggaaa cctcgataat  1320
   gtgggaaagc actactacgt gcacggacaa cactctggaa agaccgcttg a           1371
<210> 140
   <211> 1209
   <212> DNA
   <213> Fusarium monoliforme
<400> 140
   atggcgacta gacaaagaac tgctactact gtggtggttg aggatttgcc aaaggttacc 60
   ttggaggcta agtctgagcc agttttccca gatatcaaga ccatcaagga tgctattcca 120
   gctcattgct tccaaccatc tctcgtgact tccttctact acgtgttcag ggatttcgct 180
   atggtttctg ctcttgtgtg ggctgctttg acttacatcc catccatccc agatcaaacc 240
   ttgagagtgg ctgcttggat ggtttacgga ttcgtgcaag gacttttctg taccggagtg 300
   tggattttgg gacatgagtg tggacatgga gctttctccc ttcatggaaa ggttaacaac 360
   gtgaccggat ggttcttgca ttctttcctc ctcgtgccat acttctcttg gaagtactct 420
   catcacagac accatagatt caccggacac atggatttgg atatggcttt cgtgccaaag 480
   actgagccaa agccatctaa gtctctcatg atcgctggaa ttgatgttgc tgagttggtt 540
   gaggatactc cagctgctca aatggtgaag ctcatcttcc atcaactctt cggatggcag 600
   gcttacttgt tcttcaacgc ttcttccgga aagggatcta agcaatggga gccaaagact 660
   ggattgtcca agtggttcag agtgtctcat ttcgagccaa cctctgctgt tttcagacca 720
   aacgaggcta tcttcatcct catctcagat atcggattgg ctttgatggg aaccgctctc 780
   tatttcgctt ctaagcaagt tggagtgtcc actatcttgt tcttgtacct cgtgccttat 840
   ttgtgggttc accattggtt ggtggctatt acttacttgc atcaccacca tactgagttg 900
   ccacattaca ctgctgaggg atggacttat gtgaagggag ctttggctac tgttgatagg 960
   gagttcggat tcatcggaaa gcacctcttc cacggaatta ttgagaagca cgtggtgcat 1020
   catttgttcc caaagatccc attctacaag gctgatgagg ctactgaggc tattaagcca 1080
   gtgatcggag atcattactg ccacgatgat agatctttcc tcggacaact ctggactatt 1140
   ttcggaaccc tcaagtacgt tgagcatgat ccagctagac caggtgctat gaggtggaac 1200
   aaggattga 1209
<210> 141
   <211> 1152
   <212> DNA
   <213> Calendula officinalis
<400> 141
   atgggtgcag gcggtcgaat gcaagatccc accaacggtg gcaacaaaac cgagcccgaa 60
   ccaatccaac gggtcccaca tgaaaaaccc ccattcacag ttggagacat caagaaagcg 120
   atcccacctc attgtttcaa ccgatcggta attcgttcat tttcatacgt cttttacgac    180
   ctcacaatcg cgtcaatctt gtactacatt gccaacaatt acatctctac cctccctagc    240
   ccgctcgcct acgtggcatg gcccgtttac tgggccgtcc aagggtgcgt cttaaccggg    300
   gtgtgggtca tagcccacga atgtggccat catgctttta gcgaccacca atggctcgat    360
   gacaccgtgg gtctcgtctt gcactcgttc ctactcgtgc cctacttttc gtggaaatat    420
   agccaccgta ggcaccactc gaacacgggt tcgatcgagc acgatgaggt tttcgtcccg    480
   aagttgaaat cgggcgtccg gtcaaccgcc cggtacctaa acaacccacc gggccgaatc    540
   ttgaccctac tcgtaaccct aaccctcggt tggcctctat acctcacgtt caacgtttcg    600
   ggccgttact acgaccggtt cgcgtgccat ttcgacccga atagcccgat ctactcgaag    660
   cgcgaacggg ctcaaatctt catatccgac gccgggatct tagccgtagt cttcgtactc    720
   ttccgactcg caatgaccaa agggctcacg tgggtcctaa ccatgtacgg tggcccgtta    780
   ctcgtggtca acggtttcct agtcttgatc acattcctac aacacactca cccttcgctc    840
   ccgcactatg actcaaccga atgggattgg ttacgtgggg ccctcaccac aatcgaccgt    900
   gattacggga tcctaaacaa agtgttccat aacataaccg acactcacgt ggcccaccat    960
   ttgttctcta caatgcctca ttaccatgca atggaagcca cgaaggtgat caaaccgatt   1020
   ttgggcgatt attatcagtt tgacgggacc tcgattttta aggcgatgta tcgggaaaca   1080
   aaggagtgca tttatgttga taaggatgag gaggtgaaag atggtgttta ttggtatcgt   1140
   aataagattt aa                                                       1152
<210> 142
   <211> 3201
   <212> DNA
   <213> Physcomitrella patens
<400> 142
   tccagaagag gctgcccgtt gcgctggggc tcaggcggac ccggtgggcc agagctgggg      60
   ggggggggga gccacgtttg ttttgggaca tgagcgacgc agcagaaggg gcaagaccgc      120
   agagggaggg agtgagagag agggaagaag aagatctgct gctgcattgt taccatcgcc      180
   gctttcatca cgtcgtgaca gaacgcagat atctaaatac atcgatttat ttttttatta      240
   attaatttat tcaccacatg gccagcgtcc ataggtcacg tcgagaaaga cacagaccgt      300
   acgtcaggtt tccacttttg gtaggcagac ggtgcggggc tgtcataata tttgtcatgc      360
   caatacttgg agtaacttac aaacctcatg aaaatatgac atcgaatgcc cgattctgac      420
   caagacccgc ccctccccct ccctgtcccc tcctccccac tcggcgtaaa tatgcagttc      480
   ctgtctgttg ggtcgtctct ctatcattgc cttaggtagg agacaggcgc tgcgagtcca      540
   ggaggttgac ccggggcgga gccaggagag ctcttggggc gaatagcgat gtcgtccaga      600
   aggggcatga tctcgatgga tcaagccagt aagattgctg acaatccttc tgcggtatcg      660
   ctacgcagcg cggaggacga tggcaagctg gctgcaatct ccgtgaagca tcagcataat      720
   agggatgatg aggagttcga tgcgtcgacg cggccgccgt tcagtcttgg tcagatacgg      780
   gccgccattc ccaagctctg ctgggaaaga agcattgtga ggtcgttcag ctacgtcggc 840
   agggatgtta ccattgtagt ggcgctggcg tgtgtgacgg cgtatcttga tagctggttt 900
   ctgtggccgt tctattggat tgcacagggg acgatgttct gggcgatctt cgttcttggc 960
   cacgactgcg ggcatggcag cttctccaac agtaaaagat tgaacaacct agttggccac 1020
   atcatgcact cgttcattct tgtgccgtat catgggtggc gcatcagcca taggacgcat 1080
   cacgccaatc acgggcatgt cgaaaacgat gagtcctggt acccgatgac agaggcggtg 1140
   ttcaagacac tgaatttctg ggctaaatgt gggcgtctca agtttccaac tactctgttt 1200
   gcctatccag tgtacttgtg gacgaggagt ccagggaagg atggttccca tttcaatccc 1260
   tccagcgact tatttgatca gagtgagtgg agcgaggtga cgatgtcgac cgcgtgctgg 1320
   ttggcaatgg gggctatctt aacggcagca gtgtcacagt tcgggttcct ctggatgctt 1380
   aagctctact tcgtcccgta tatcattaat gtgatgtggt tggatgccgt gacatacctt 1440
   caccaccatg ggtacgataa aaagattccc tggtaccgag gtaaggagtg gaactatttg 1500
   cggggaggac tgtccaccgt cgatcgtgac tacggttttt tcaacaagat ccaccacgac 1560
   attggcacgc acgtcgtgca tcacctgttt cctcagatcc cgcattatca tttatgtgag 1620
   gcaaccgcgg ctgtcaagtc tttgcttgga aatttctacc gagaaccaga gaagtcaggt 1680
   cccattcctc ttcatctcat ccctgtcatt atgaagagtt tctctgagga tcacttcgtg 1740
   gccgacgagg gtgacattgt cttttaccaa aaagatccaa aacagtgcaa ttgcagataa 1800
   atctcttcca ggttacttgc tctcttcccc ttccattgta gcataagtcg cggattagga 1860
   agctgtggat gatacactcg actcactgtc taaggaagct ggattttgac gcccctccct 1920
   gtctagtcgt cattggcagc cattgatgat cgagcgatta agtaggaagg aaccatccac 1980
   agacagatag aaatgcaaca ggaaaacaag tagagatcgg ctctcattct ccagtaagag 2040
   aggcgcgtga gcacgcacag gggatcatct ggtgtccaag acggtgtgac gtggatctat 2100
   tcgctatttt tgttcattgc gaaagaattt cttcacttgc atcgtgtttt catcgagctt 2160
   ttggaaatgg cattgcttat tttttcgctg caaatatcca aacattagca gctgtttttg 2220
   ttggtgcaca tgagccatcg ggagtgtgta tctttctcct ctgctggctt gcagttgcag 2280
   cttggttcgt gaacttttaa agtcgatgat taggtttctg cttgatattt gcagctgtgt 2340
   tgtgatcgat tgttccgagt ggaatatgga tttgagtatt catgacctgt ttaggaaatc 2400
   accaattttt attcccgatt actgcaatcg gtcctttctt tataaatcat atctttgaca 2460
   ttgtgacata gtcattgatc ttctaatcat ctctcataca atccccgtca accagagcag 2520
   gcttttcaag gtgtctgaga aggatcgtga acatgggcgt gcctgcctac catatgatcc 2580
   gccacaaaca tgtcttgccc atttgatgct atttgagatt ctctcatact ttgattcata 2640
   actacatatt tttaattgtt atatagagtt gtgaccctcc cccactttgg agtaaagata 2700
   ttctcatcta tttagtcatt tttatttgcc tctcttaact tttatcaaaa aatcacttaa 2760
   gtgattttaa tttgttttct tctactcttt ggttttgtat agtcacatac tttcttatta 2820
   aaagccgtga ttaatccttg tacatatctc aagtttcttt tatttatcca ttacaattca 2880
   tgtggagggt aatctttgaa aatatacaag tattgaattc ccttcatatt tgtatgtttt 2940
   ttttattaaa tattacttct acttgatcca taatttgatt ccacacctct ttttaaggtg 3000
   aaatctttgc cttctttttg tagaagccac tcacatcttc tacaaatttc ttcctaagaa 3060
   cacttacata aaaaatcgaa tgcctcatca atctcttcaa tatgtctaag atatatatat 3120
   tcttgtataa tatgtttgca acttcataag gacctgccta ctattagatc catttgaaag 3180
   tgagatgtat taggcaagtg a 3201
<210> 143
   <211> 1185
   <212> DNA
   <213> Physcomitrella patens
<400> 143
   atggatcaag ccagtaagat tgctgacaat ccttctgcgg tatcgctacg cagcgcggag 60
   gacgatggca agctggctgc aatctccgtg aagcatcagc ataataggga tgatgaggag 120
   ttcgatgcgt cgacgcggcc gccgttcagt cttggtcaga tacgggccgc cattcccaag 180
   ctctgctggg aaagaagcat tgtgaggtcg ttcagctacg tcggcaggga tgttaccatt 240
   gtagtggcgc tggcgtgtgt gacggcgtat cttgatagct ggtttctgtg gccgttctat 300
   tggattgcac aggggacgat gttctgggcg atcttcgttc ttggccacga ctgcgggcat 360
   ggcagcttct ccaacagtaa aagattgaac aacctagttg gccacatcat gcactcgttc 420
   attcttgtgc cgtatcatgg gtggcgcatc agccatagga cgcatcacgc caatcacggg 480
   catgtcgaaa acgatgagtc ctggtacccg atgacagagg cggtgttcaa gacactgaat 540
   ttctgggcta aatgtgggcg tctcaagttt ccaactactc tgtttgccta tccagtgtac 600
   ttgtggacga ggagtccagg gaaggatggt tcccatttca atccctccag cgacttattt 660
   gatcagagtg agtggagcga ggtgacgatg tcgaccgcgt gctggttggc aatgggggct 720
   atcttaacgg cagcagtgtc acagttcggg ttcctctgga tgcttaagct ctacttcgtc 780
   ccgtatatca ttaatgtgat gtggttggat gccgtgacat accttcacca ccatgggtac 840
   gataaaaaga ttccctggta ccgaggtaag gagtggaact atttgcgggg aggactgtcc 900
   accgtcgatc gtgactacgg ttttttcaac aagatccacc acgacattgg cacgcacgtc 960
   gtgcatcacc tgtttcctca gatcccgcat tatcatttat gtgaggcaac cgcggctgtc 1020
   aagtctttgc ttggaaattt ctaccgagaa ccagagaagt caggtcccat tcctcttcat 1080
   ctcatccctg tcattatgaa gagtttctct gaggatcact tcgtggccga cgagggtgac 1140
   attgtctttt accaaaaaga tccaaaacag tgcaattgca gataa 1185
<210> 144
   <211> 394
   <212> PRT
   <213> Physcomitrella patens

<400> 144
   Met Asp Gln Ala Ser Lys Ile Ala Asp Asn Pro Ser Ala Val Ser Leu
   1               5                   10                  15
   Arg Ser Ala Glu Asp Asp Gly Lys Leu Ala Ala Ile Ser Val Lys His
               20                  25                  30
   Gln His Asn Arg Asp Asp Glu Glu Phe Asp Ala Ser Thr Arg Pro Pro
           35                  40                  45
   Phe Ser Leu Gly Gln Ile Arg Ala Ala Ile Pro Lys Leu Cys Trp Glu
       50                  55                  60
   Arg Ser Ile Val Arg Ser Phe Ser Tyr Val Gly Arg Asp Val Thr Ile
   65                  70                  75                  80
   Val Val Ala Leu Ala Cys Val Thr Ala Tyr Leu Asp Ser Trp Phe Leu
                   85                  90                  95
   Trp Pro Phe Tyr Trp Ile Ala Gln Gly Thr Met Phe Trp Ala Ile Phe
               100                 105                 110
   Val Leu Gly His Asp Cys Gly His Gly Ser Phe Ser Asn Ser Lys Arg
           115                 120                 125
   Leu Asn Asn Leu Val Gly His Ile Met His Ser Phe Ile Leu Val Pro
       130                 135                 140
   Tyr His Gly Trp Arg Ile Ser His Arg Thr His His Ala Asn His Gly
   145                 150                 155                 160
   His Val Glu Asn Asp Glu Ser Trp Tyr Pro Met Thr Glu Ala Val Phe
                   165                 170                 175
   Lys Thr Leu Asn Phe Trp Ala Lys Cys Gly Arg Leu Lys Phe Pro Thr
               180                 185                 190
   Thr Leu Phe Ala Tyr Pro Val Tyr Leu Trp Thr Arg Ser Pro Gly Lys
           195                 200                 205
   Asp Gly Ser His Phe Asn Pro Ser Ser Asp Leu Phe Asp Gln Ser Glu
       210                 215                 220
   Trp Ser Glu Val Thr Met Ser Thr Ala Cys Trp Leu Ala Met Gly Ala
   225                 230                 235                 240
   Ile Leu Thr Ala Ala Val Ser Gln Phe Gly Phe Leu Trp Met Leu Lys
                   245                 250                 255
   Leu Tyr Phe Val Pro Tyr Ile Ile Asn Val Met Trp Leu Asp Ala Val
               260                 265                 270
   Thr Tyr Leu His His His Gly Tyr Asp Lys Lys Ile Pro Trp Tyr Arg
           275                 280                 285
   Gly Lys Glu Trp Asn Tyr Leu Arg Gly Gly Leu Ser Thr Val Asp Arg
   290 295 300
   Asp Tyr Gly Phe Phe Asn Lys Ile His His Asp Ile Gly Thr His Val
   305 310 315 320
   Val His His Leu Phe Pro Gln Ile Pro His Tyr His Leu Cys Glu Ala
   325 330 335
   Thr Ala Ala Val Lys Ser Leu Leu Gly Asn Phe Tyr Arg Glu Pro Glu
   340 345 350
   Lys Ser Gly Pro Ile Pro Leu His Leu Ile Pro Val Ile Met Lys Ser
   355 360 365
   Phe Ser Glu Asp His Phe Val Ala Asp Glu Gly Asp Ile Val Phe Tyr
   370 375 380
   Gln Lys Asp Pro Lys Gln Cys Asn Cys Arg
   385 390
<210> 145
   <211> 1543
   <212> DNA
   <213> Physcomitrella patens
<400> 145
   gcgctgcgag gtccagcacg tagagacgga ccgtgtctgt tcagtgtgtg gcaaaggcga 60
   attcagagtg gttggaagaa gggggtggat caagctgctg ctgtgatcga tcgcgggagt 120
   cagcgatggc ttcgcggaga ggcatgatct cgttggatca ggccaagtcc atgatcgaag 180
   agctgccgcc agtggtgtcg atgcggagcg cggaggatga tgacaaaggc ggcggcggcg 240
   gcggcggcgg cggcgtcgtc tctgtggaag ccaaaggtca ggaggaagcg tgggacgcgt 300
   cgaagaagcc ccccttcacc ctcggccaga tccgagccgc cattccaaag cactgctggg 360
   agcgcagcat tccacgctcg ttgcgatacg tcgcaaacga catcgccatt gtggtggcgc 420
   tcgcggtggt tgctgcgtac gtcgatagtt ggttcttgtg gccgttttac tggttcgtcc 480
   aagggacaat gttctggtcg ctcttcgtcc tcggccacga ttgcggacac ggtagcttct 540
   ccccaagcag gcgattgaac aacttcgtcg gccacatcgt tcactcgttt atcctggtcc 600
   cttaccatgg ctggcgcatc agccaccgga cgcatcacgc taaccacggc cacgtcgaga 660
   acgacgagtc gtggtatccc atgacggagg cgctgtacag gactctcaac atctttgaga 720
   aactgggtcg gttgcagttt ccattcccac tcttagcgta tcctttctac ctgtggacga 780
   ggagcccagg caagaatgga acccattaca gcccttcgag cgacctgttt gattcgagcg 840
   agtggaagga agtgatcacc tcaaccgctt gctggttcgc tatggtggcg atcctggccg 900
   acgtcgtctt tcataaaggt ttcctctgga cgtttaagct ctatttcata ccttacatcg 960
   tcaatgtcgt gtggctggac ttcgtcacat accttcatca ccatggctac gagaagaaaa 1020
   ttccatggta tcgtgggcaa gagtggaact acatgcgcgg ggggttgtcc accatcgacc 1080
   gcgactacgg gatcttcaac aaaatccatc acgacatcgg cacgcacgtt gtgcaccatt 1140
   tgttccctca gattccgcat taccatttgg tcgaggcaac cgcggcggtg aagcctttgc 1200
   ttgggaacta ctacagggag ccgaaaaggt caggtctgat tccactgcat ctcctcccca 1260
   ttctcgtgaa gagcttcaac gaggatcatt atgtggccga cgagggcgac attgtctttt 1320
   atcagaacga tgtaaagcag tgattcgtca cctcttcttg gccacagaaa aagaaaaaaa 1380
   gaaaaaaaat catggctata gcgcggcccg tttgctctca acctttctag catgcactct 1440
   cgcgttggaa atgatccatc aatcggcgat gcatcgtgca aggagggtca gactccgacg 1500
   ggacgttgat tgaagacctc cttcaaagga tttagcgtct tat 1543
<210> 146
   <211> 1143
   <212> DNA
   <213> Physcomitrella patens
<400> 146
   atgcggagcg cggaggatga tgacaaaggc ggcggcggcg gcggcggcgg cggcgtcgtc      60
   tctgtggaag ccaaaggtca ggaggaagcg tgggacgcgt cgaagaagcc ccccttcacc     120
   ctcggccaga tccgagccgc cattccaaag cactgctggg agcgcagcat tccacgctcg     180
   ttgcgatacg tcgcaaacga catcgccatt gtggtggcgc tcgcggtggt tgctgcgtac     240
   gtcgatagtt ggttcttgtg gccgttttac tggttcgtcc aagggacaat gttctggtcg     300
   ctcttcgtcc tcggccacga ttgcggacac ggtagcttct ccccaagcag gcgattgaac     360
   aacttcgtcg gccacatcgt tcactcgttt atcctggtcc cttaccatgg ctggcgcatc     420
   agccaccgga cgcatcacgc taaccacggc cacgtcgaga acgacgagtc gtggtatccc     480
   atgacggagg cgctgtacag gactctcaac atctttgaga aactgggtcg gttgcagttt     540
   ccattcccac tcttagcgta tcctttctac ctgtggacga ggagcccagg caagaatgga     600
   acccattaca gcccttcgag cgacctgttt gattcgagcg agtggaagga agtgatcacc     660
   tcaaccgctt gctggttcgc tatggtggcg atcctggccg acgtcgtctt tcataaaggt     720
   ttcctctgga cgtttaagct ctatttcata ccttacatcg tcaatgtcgt gtggctggac     780
   ttcgtcacat accttcatca ccatggctac gagaagaaaa ttccatggta tcgtgggcaa     840
   gagtggaact acatgcgcgg ggggttgtcc accatcgacc gcgactacgg gatcttcaac     900
   aaaatccatc acgacatcgg cacgcacgtt gtgcaccatt tgttccctca gattccgcat     960
   taccatttgg tcgaggcaac cgcggcggtg aagcctttgc ttgggaacta ctacagggag    1020
   ccgaaaaggt caggtctgat tccactgcat ctcctcccca ttctcgtgaa gagcttcaac    1080
   gaggatcatt atgtggccga cgagggcgac attgtctttt atcagaacga tgtaaagcag    1140
   tga                                                                   1143
<210> 147
   <211> 380
   <212> PRT
   <213> Physcomitrella patens
<400> 147
   Met Arg Ser Ala Glu Asp Asp Asp Lys Gly Gly Gly Gly Gly Gly Gly
   1               5                   10                  15
   Gly Gly Val Val Ser Val Glu Ala Lys Gly Gln Glu Glu Ala Trp Asp
               20                  25                  30
   Ala Ser Lys Lys Pro Pro Phe Thr Leu Gly Gln Ile Arg Ala Ala Ile
           35                  40                  45
   Pro Lys His Cys Trp Glu Arg Ser Ile Pro Arg Ser Leu Arg Tyr Val
       50                  55                  60
   Ala Asn Asp Ile Ala Ile Val Val Ala Leu Ala Val Val Ala Ala Tyr
   65                  70                  75                  80
   Val Asp Ser Trp Phe Leu Trp Pro Phe Tyr Trp Phe Val Gln Gly Thr
                   85                  90                  95
   Met Phe Trp Ser Leu Phe Val Leu Gly His Asp Cys Gly His Gly Ser
               100                 105                 110
   Phe Ser Pro Ser Arg Arg Leu Asn Asn Phe Val Gly His Ile Val His
           115                 120                 125
   Ser Phe Ile Leu Val Pro Tyr His Gly Trp Arg Ile Ser His Arg Thr
       130                 135                 140
   His His Ala Asn His Gly His Val Glu Asn Asp Glu Ser Trp Tyr Pro
   145                 150                 155                 160
   Met Thr Glu Ala Leu Tyr Arg Thr Leu Asn Ile Phe Glu Lys Leu Gly
                   165                 170                 175
   Arg Leu Gln Phe Pro Phe Pro Leu Leu Ala Tyr Pro Phe Tyr Leu Trp
               180                 185                 190
   Thr Arg Ser Pro Gly Lys Asn Gly Thr His Tyr Ser Pro Ser Ser Asp
           195                 200                 205
   Leu Phe Asp Ser Ser Glu Trp Lys Glu Val Ile Thr Ser Thr Ala Cys
       210                 215                 220
   Trp Phe Ala Met Val Ala Ile Leu Ala Asp Val Val Phe His Lys Gly
   225                 230                 235                 240
   Phe Leu Trp Thr Phe Lys Leu Tyr Phe Ile Pro Tyr Ile Val Asn Val
                   245                 250                 255
   Val Trp Leu Asp Phe Val Thr Tyr Leu His His His Gly Tyr Glu Lys
   260 265 270
   Lys Ile Pro Trp Tyr Arg Gly Gln Glu Trp Asn Tyr Met Arg Gly Gly
   275 280 285
   Leu Ser Thr Ile Asp Arg Asp Tyr Gly Ile Phe Asn Lys Ile His His
   290 295 300
   Asp Ile Gly Thr His Val Val His His Leu Phe Pro Gln Ile Pro His
   305 310 315 320
   Tyr His Leu Val Glu Ala Thr Ala Ala Val Lys Pro Leu Leu Gly Asn
   325 330 335
   Tyr Tyr Arg Glu Pro Lys Arg Ser Gly Leu Ile Pro Leu His Leu Leu
   340 345 350
   Pro Ile Leu Val Lys Ser Phe Asn Glu Asp His Tyr Val Ala Asp Glu
   355 360 365
   Gly Asp Ile Val Phe Tyr Gln Asn Asp Val Lys Gln
   370 375 380
<210> 148
   <211> 1495
   <212> DNA
   <213> Physcomitrella patens

<400> 148
   ttctttccaa taagtgtctc gggaagggga gagtgagtga gagagagaga ctccagtgga     60
   gagtcgcggc ggagctcttg tggactggag cgatgtcgtc tagaaggggc ttgatatcgc    120
   tggatcagcc ccgaaagatt ggtcacaatc ttcccgtggt gtcgttggat agcattgaga    180
   atgtcaggaa cgtgggtggg atgtccgtga agcatgagat taataggaag gatgaagaat    240
   tcgatgcgtc tagaaagccg cccttcagcc ttggccagtt acgagccgcc attcccaagc    300
   actgctggga gcggagtctg tcgagatctt tcagctatgt agtcagagat gttgccattg    360
   tagtgacgct agcgtatgtg gcggtatact tcgatagctg gtttctgtgg ccagtgtatt    420
   ggttggcaca ggggacaatg ttttgggcga tcttcgttct gggtcacgac tgtgggcatg    480
   gcagcttttc aagcaacaag agcctgaaca acctggtcgg acacatcctg cactcgttca    540
   ttctggtgcc atatcacggg tggcgcatca gtcacaggac acatcatgcc aaccatggcc    600
   acgtcaagaa cgacgagtcc tggtacccga tgacagaggg gctgtttatc acactggatt    660
   tctggggcaa atttggacgg ctgaactttc cggctacgct gtttgcgtat cccgcgtacc    720
   tctggtggag aactcctggc aagtcgggtt cccatttcaa tccgtccagc gatttgtttg    780
   acccgagtga gtggaaggag gtggcaacgt cgaccacatg ctggttgaca atggttgcca    840
   tctcgatgac ttcggtgtcg caagtcgggt ttctttttgt gttcaagctt tacttcgtcc    900
   cctacatcgt caacgtgatg tggttggacg ctgtgaccta ccttcaccac cacgggtatg    960
   agaaacaaat cccctggtac cgaggtcagg aatggagtta cctgcggggt ggactgtcca   1020
   ccatcgatcg cgactacggt ttgtttaatg agatccacca cgacattggc acacacgtcg   1080
   tgcatcacct cttccctcag atcccgcatt atcatttgcg tgaagctaca gcagccgtaa   1140
   ggcccttgtt aaaaagttac taccgcgagc cccaaaagtc gggtcttttt cctcttcacc   1200
   tcatctctgt catgatgaag agtttctccg agcatcactt cgtggctgat gagtgcgata   1260
   ttgtcttcta tcagaagagc acaaatgcta agtgaggttc tcgagcctag agaaatttcg   1320
   ctaatgtttt ttgcttccac cctacaaatg tggatctttt atcaggtggg tcagtaatct   1380
   tgtattctac attagaatcg gaatgggagc ctgtaactgg tattattgac caattctgga   1440
   gttaaaagac gtcatttttg gattattctc cacatgtggc tctgtacaat aataa        1495
<210> 149
   <211> 1095
   <212> DNA
   <213> Physcomitrella patens
<400> 149
   atgtccgtga agcatgagat taataggaag gatgaagaat tcgatgcgtc tagaaagccg     60
   cccttcagcc ttggccagtt acgagccgcc attcccaagc actgctggga gcggagtctg    120
   tcgagatctt tcagctatgt agtcagagat gttgccattg tagtgacgct agcgtatgtg    180
   gcggtatact tcgatagctg gtttctgtgg ccagtgtatt ggttggcaca ggggacaatg    240
   ttttgggcga tcttcgttct gggtcacgac tgtgggcatg gcagcttttc aagcaacaag    300
   agcctgaaca acctggtcgg acacatcctg cactcgttca ttctggtgcc atatcacggg    360
   tggcgcatca gtcacaggac acatcatgcc aaccatggcc acgtcaagaa cgacgagtcc    420
   tggtacccga tgacagaggg gctgtttatc acactggatt tctggggcaa atttggacgg    480
   ctgaactttc cggctacgct gtttgcgtat cccgcgtacc tctggtggag aactcctggc    540
   aagtcgggtt cccatttcaa tccgtccagc gatttgtttg acccgagtga gtggaaggag    600
   gtggcaacgt cgaccacatg ctggttgaca atggttgcca tctcgatgac ttcggtgtcg    660
   caagtcgggt ttctttttgt gttcaagctt tacttcgtcc cctacatcgt caacgtgatg    720
   tggttggacg ctgtgaccta ccttcaccac cacgggtatg agaaacaaat cccctggtac    780
   cgaggtcagg aatggagtta cctgcggggt ggactgtcca ccatcgatcg cgactacggt    840
   ttgtttaatg agatccacca cgacattggc acacacgtcg tgcatcacct cttccctcag    900
   atcccgcatt atcatttgcg tgaagctaca gcagccgtaa ggcccttgtt aaaaagttac    960
   taccgcgagc cccaaaagtc gggtcttttt cctcttcacc tcatctctgt catgatgaag   1020
   agtttctccg agcatcactt cgtggctgat gagtgcgata ttgtcttcta tcagaagagc   1080
   acaaatgcta agtga                                                     1095
<210> 150
   <211> 364
   <212> PRT
   <213> Physcomitrella patens
<400> 150
   Met Ser Val Lys His Glu Ile Asn Arg Lys Asp Glu Glu Phe Asp Ala
   1               5                   10                  15
   Ser Arg Lys Pro Pro Phe Ser Leu Gly Gln Leu Arg Ala Ala Ile Pro
               20                  25                  30
   Lys His Cys Trp Glu Arg Ser Leu Ser Arg Ser Phe Ser Tyr Val Val
           35                  40                  45
   Arg Asp Val Ala Ile Val Val Thr Leu Ala Tyr Val Ala Val Tyr Phe
       50                  55                  60
   Asp Ser Trp Phe Leu Trp Pro Val Tyr Trp Leu Ala Gln Gly Thr Met
   65                  70                  75                  80
   Phe Trp Ala Ile Phe Val Leu Gly His Asp Cys Gly His Gly Ser Phe
                   85                  90                  95
   Ser Ser Asn Lys Ser Leu Asn Asn Leu Val Gly His Ile Leu His Ser
               100                 105                 110
   Phe Ile Leu Val Pro Tyr His Gly Trp Arg Ile Ser His Arg Thr His
           115                 120                 125
   His Ala Asn His Gly His Val Lys Asn Asp Glu Ser Trp Tyr Pro Met
       130                 135                 140
   Thr Glu Gly Leu Phe Ile Thr Leu Asp Phe Trp Gly Lys Phe Gly Arg
   145                 150                 155                 160
   Leu Asn Phe Pro Ala Thr Leu Phe Ala Tyr Pro Ala Tyr Leu Trp Trp
                   165                 170                 175
   Arg Thr Pro Gly Lys Ser Gly Ser His Phe Asn Pro Ser Ser Asp Leu
               180                 185                 190
   Phe Asp Pro Ser Glu Trp Lys Glu Val Ala Thr Ser Thr Thr Cys Trp
           195                 200                 205
   Leu Thr Met Val Ala Ile Ser Met Thr Ser Val Ser Gln Val Gly Phe
       210                 215                 220
   Leu Phe Val Phe Lys Leu Tyr Phe Val Pro Tyr Ile Val Asn Val Met
   225                 230                 235                 240
   Trp Leu Asp Ala Val Thr Tyr Leu His His His Gly Tyr Glu Lys Gln
                   245                 250                 255
   Ile Pro Trp Tyr Arg Gly Gln Glu Trp Ser Tyr Leu Arg Gly Gly Leu
               260                 265                 270
   Ser Thr Ile Asp Arg Asp Tyr Gly Leu Phe Asn Glu Ile His His Asp
           275                 280                 285
   Ile Gly Thr His Val Val His His Leu Phe Pro Gln Ile Pro His Tyr
       290                 295                 300
   His Leu Arg Glu Ala Thr Ala Ala Val Arg Pro Leu Leu Lys Ser Tyr
   305                 310                 315                 320
   Tyr Arg Glu Pro Gln Lys Ser Gly Leu Phe Pro Leu His Leu Ile Ser
                   325                 330                 335
   Val Met Met Lys Ser Phe Ser Glu His His Phe Val Ala Asp Glu Cys
               340                 345                 350
   Asp Ile Val Phe Tyr Gln Lys Ser Thr Asn Ala Lys
           355                 360
<210> 151
   <211> 2468
   <212> DNA
   <213> Postia placenta
<400> 151
   cgcgctgggc tcctagcagc tccgtgtgat agatcattcg aagtcaggcg cgcttgtcgc    60
   accagaaccc ggggggtgtc actgtcccag acaagctgtt ggaccgtggg cggcacgata   120
   aaacgatctt taatcggaga gttctcttcc gccccctctt gagaagtcca ctccgcgctt   180
   cttgctatcc accacccgca atggctacca cggctgattc tgcaggccca gcacggactc   240
   agtcggcgtc cacgtactca gaggaccagc tccctgaatt cacacctatg ccatggtacg   300
   tcaagattct cacaacttca tgaacagctc tcatgcgctg gcacaggact ctgagtgaga   360
   ttcgcgctgc tatccctgca catctccacg cgcgccagac gtggaagggg atgctctacc   420
   tcttgcgtga catcctaatg gcagccattg tatggaagct cgcgctctac atcgatcctt   480
   cgttcaagag tgagactgcc gtgcgaacgt tgacgccggt aggagcggaa gccgcacgct   540
   ggggcgcctg gcttgtttag tatgtgatct tactagtgta gtcaggcgct gcgactcaga   600
   gcaacctaag ttggtggttc cagggtctga tcttcacagg gatctgggtg atcgggcacg   660
   aggtgggtac ttgacagtac gcaaccttac ttgacccctt cgcttatatc agtcttagtg   720
   cggtcacggc gcattctcgt ccaacaagcg tatttgcgat atcattggat ttgtgagtag   780
   cacgtcgaag tcaagcgggg ttcacgtcgg tggctgatat ctacattgaa tagattacgc   840
   acacgttcct gtggacaccc tacttcagct ggcagatctc tcatcatcgt catcacagca   900
   atcatgcgtc catggagcgc gacgaggtat atgtgcccaa gacacgcgcc gacctgggca   960
   tccctaagga gagtgagggt gcaatcgact atgaggatta cctcggcgat acaccaatct  1020
   acacgttgtt tgcgcttatc cgacaacagg ttttggcctt cccggcatat cttcgtaagt  1080
   ttgcttcttc gtattaacaa tggttagatt cacagcaggc tgttagtatt caacgtctca  1140
   gggcagaaga actacccgaa atggaccaat cacttcgatc gtacgtaagc gccgtgtcga 1200
   ggcatgaccc acgtcctaat ctcgttcaca gccaattcta ttcttttcac gaagaggcag 1260
   cgcaatgccg tcatcatgtc gaatatcggc atcgctacta tggtctacat tgtgctcgag 1320
   gccagtcgca cctggggcgc gagtgagatc atgaagtact atggcatccc atggctgtcg 1380
   gtcactcact ggtaaattat atctgtcgtg acgtttatat gatgaggtta atcaggcgac 1440
   taggttcatc atgatcactt accttcacca cacggacgtc gaactccctc actaccgcaa 1500
   cggacagtgg aacttccagc ggggcgctgc ggcgactgta gatcgtaatt tcctcggctg 1560
   gcaagggcgc ttcttcctgc acgatgtcgc ccacttccac gtcattcacc actttttccc 1620
   tatgatgccc ttctgtgagt gtcttgcttt gaaatgcggt gcgcctgtca ggctgacgat 1680
   gtgcggcaga ccacgggcct gaggcaacga aatatctcaa ggagttcatt ggcgaccact 1740
   atcggtattc tgataagccc gtgttcaagg cgctctggga cacgtacaac aattgccaat 1800
   ttgtcgaaga tgagggtgcg tcatctcctg aatttggtaa agccggtgct gatcctgcgc 1860
   ccgtataggt gacattctct tctaccgtga ccgcaagggc caggcgcgcg tacgccccgc 1920
   tgagcccttc agggaatcca ctatatcagc gagcgtgtct cgttgatctt caaaccaccg 1980
   tatacagttc agaagtgtat catcgccgga ttgccctggc gcccttgctt ctacactgtt 2040
   catctatcgt tcacgctgtc ttaccacgct atgttctgtg tttcggtagc ctgtcggcat 2100
   tacttgttcc aacactgatg tctccttaga acccctctcc ccccttgtaa ccgcagcatg 2160
   atgttgtatg ccttgtaatc gaggcatctt ttgtactggt atgggtcaga taatcagtgt 2220
   tgttgtacga ccatcaccgt aatgccattt tcacgattcc atgatctcac ttctctattc 2280
   agtctcctag actatctgcg tctcatatca tgatgtaatc tgtcgctact ggtaatgcat 2340
   cgagacgagg tcccatcact atggttattc aaaaggaaga gatcggtgta caatctggcg 2400
   atgtcacagt atgtgtttgc aagcgtgaag gagggctgaa aatgcagctg ctctcattgg 2460
   cgacatct 2468
<210> 152
   <211> 1284
   <212> DNA
   <213> Postia placenta
<400> 152
   atggctacca cggctgattc tgcaggccca gcacggactc agtcggcgtc cacgtactca      60
   gaggaccagc tccctgaatt cacacctatg ccatggactc tgagtgagat tcgcgctgct     120
   atccctgcac atctccacgc gcgccagacg tggaagggga tgctctacct cttgcgtgac     180
   atcctaatgg cagccattgt atggaagctc gcgctctaca tcgatccttc gttcaagagt     240
   gagactgccg tgcgaacgtt gacgccggta ggagcggaag ccgcacgctg gggcgcctgg     300
   cttgtttatt ggtggttcca gggtctgatc ttcacaggga tctgggtgat cgggcacgag     360
   tgcggtcacg gcgcattctc gtccaacaag cgtatttgcg atatcattgg atttattacg     420
   cacacgttcc tgtggacacc ctacttcagc tggcagatct ctcatcatcg tcatcacagc     480
   aatcatgcgt ccatggagcg cgacgaggta tatgtgccca agacacgcgc cgacctgggc     540
   atccctaagg agagtgaggg tgcaatcgac tatgaggatt acctcggcga tacaccaatc     600
   tacacgttgt ttgcgcttat ccgacaacag gttttggcct tcccggcata tcttctattc     660
   aacgtctcag ggcagaagaa ctacccgaaa tggaccaatc acttcgatcc caattctatt     720
   cttttcacga agaggcagcg caatgccgtc atcatgtcga atatcggcat cgctactatg     780
   gtctacattg tgctcgaggc cagtcgcacc tggggcgcga gtgagatcat gaagtactat     840
   ggcatcccat ggctgtcggt cactcactgg ttcatcatga tcacttacct tcaccacacg     900
   gacgtcgaac tccctcacta ccgcaacgga cagtggaact tccagcgggg cgctgcggcg     960
   actgtagatc gtaatttcct cggctggcaa gggcgcttct tcctgcacga tgtcgcccac    1020
   ttccacgtca ttcaccactt tttccctatg atgcccttct accacgggcc tgaggcaacg    1080
   aaatatctca aggagttcat tggcgaccac tatcggtatt ctgataagcc cgtgttcaag    1140
   gcgctctggg acacgtacaa caattgccaa tttgtcgaag atgagggtga cattctcttc    1200
   taccgtgacc gcaagggcca ggcgcgcgta cgccccgctg agcccttcag ggaatccact    1260
   atatcagcga gcgtgtctcg ttga                                           1284
<210> 153
   <211> 427
   <212> PRT
   <213> Postia placenta

<400> 153
   Met Ala Thr Thr Ala Asp Ser Ala Gly Pro Ala Arg Thr Gln Ser Ala
   1               5                   10                  15
   Ser Thr Tyr Ser Glu Asp Gln Leu Pro Glu Phe Thr Pro Met Pro Trp
               20                  25                  30
   Thr Leu Ser Glu Ile Arg Ala Ala Ile Pro Ala His Leu His Ala Arg
           35                  40                  45
   Gln Thr Trp Lys Gly Met Leu Tyr Leu Leu Arg Asp Ile Leu Met Ala
       50                  55                  60
   Ala Ile Val Trp Lys Leu Ala Leu Tyr Ile Asp Pro Ser Phe Lys Ser
   65                  70                  75                  80
   Glu Thr Ala Val Arg Thr Leu Thr Pro Val Gly Ala Glu Ala Ala Arg
                   85                  90                  95
   Trp Gly Ala Trp Leu Val Tyr Trp Trp Phe Gln Gly Leu Ile Phe Thr
               100                 105                 110
   Gly Ile Trp Val Ile Gly His Glu Cys Gly His Gly Ala Phe Ser Ser
           115                 120                 125
   Asn Lys Arg Ile Cys Asp Ile Ile Gly Phe Ile Thr His Thr Phe Leu
       130                 135                 140
   Trp Thr Pro Tyr Phe Ser Trp Gln Ile Ser His His Arg His His Ser
   145                 150                 155                 160
   Asn His Ala Ser Met Glu Arg Asp Glu Val Tyr Val Pro Lys Thr Arg
                   165                 170                 175
   Ala Asp Leu Gly Ile Pro Lys Glu Ser Glu Gly Ala Ile Asp Tyr Glu
               180                 185                 190
   Asp Tyr Leu Gly Asp Thr Pro Ile Tyr Thr Leu Phe Ala Leu Ile Arg
           195                 200                 205
   Gln Gln Val Leu Ala Phe Pro Ala Tyr Leu Leu Phe Asn Val Ser Gly
       210                 215                 220
   Gln Lys Asn Tyr Pro Lys Trp Thr Asn His Phe Asp Pro Asn Ser Ile
   225                 230                 235                 240
   Leu Phe Thr Lys Arg Gln Arg Asn Ala Val Ile Met Ser Asn Ile Gly
                   245                 250                 255
   Ile Ala Thr Met Val Tyr Ile Val Leu Glu Ala Ser Arg Thr Trp Gly
               260                 265                 270
   Ala Ser Glu Ile Met Lys Tyr Tyr Gly Ile Pro Trp Leu Ser Val Thr
           275                 280                 285
   His Trp Phe Ile Met Ile Thr Tyr Leu His His Thr Asp Val Glu Leu
       290                 295                 300
   Pro His Tyr Arg Asn Gly Gln Trp Asn Phe Gln Arg Gly Ala Ala Ala
   305                 310                 315                 320
   Thr Val Asp Arg Asn Phe Leu Gly Trp Gln Gly Arg Phe Phe Leu His
                   325                 330                 335
   Asp Val Ala His Phe His Val Ile His His Phe Phe Pro Met Met Pro
               340                 345                 350
   Phe Tyr His Gly Pro Glu Ala Thr Lys Tyr Leu Lys Glu Phe Ile Gly
           355                 360                 365
   Asp His Tyr Arg Tyr Ser Asp Lys Pro Val Phe Lys Ala Leu Trp Asp
       370                 375                 380
   Thr Tyr Asn Asn Cys Gln Phe Val Glu Asp Glu Gly Asp Ile Leu Phe
   385                 390                 395                 400
   Tyr Arg Asp Arg Lys Gly Gln Ala Arg Val Arg Pro Ala Glu Pro Phe
   405 410 415
   Arg Glu Ser Thr Ile Ser Ala Ser Val Ser Arg
   420 425
<210> 154
   <211> 4066
   <212> DNA
   <213> Selaginella moellendorffii
<400> 154
   cgcaaaatat ggggattttc ccccgttcct cgcagtcact ctccttgcct tcaaagcttt     60
   gctacaagac gccgagatca tcgctaagtt ttcgatcgcc caagcaagcg tgtgtcatcg    120
   ctggccttgg ctcttccttt tctccactct tggctcttaa aagaaggctc aagcccggct    180
   cgagctcgcc atccattcca atggtcgctg ttccactccg aactgtcact gctcctcaaa    240
   gcgatgtggt cggtgacttc tccaaggagt ttgatcctgc tgctccacca ccattttctc    300
   tgggagatat cagagctgcc attccaaagc attgctggga gaagaatgtg tggagatcga    360
   ttagctatgt cgtgagggac atagcggtgg ttctcggact ggctgctggt gctgcttact    420
   tgaataattg gctcgtctgg ccactctact gggctgccca agggacgatg ttctgggctc    480
   tctttgtgct cggccatgac tggtaagttt ttcttctccc aaaaacttgt ttttgctttc    540
   tcgttcttac gcgtctttga tcagtggaca cgggagtttt tccaataaca agaagcttaa    600
   ccatgtcttc ggccacattc tccattcttc catcctggtt ccataccacg ggtggtacgt    660
   atgcttttcc atttcttgtt tggcttagag cttaaacttt gtggaaatcc aggaggataa    720
   gccaccggac gcatcaccag aatcacggcc atgtcgagaa cgacgagtcc tggcatccag    780
   taagtcgcct taagctcttc caagtgaaaa ccatttcgtt tttccttatc ttacactgtt    840
   cttcatgtag atgaccgaga agctctacaa agacgcagat atctttactc gcttcggccg    900
   gttccagctt ccatgggtca tgtttgctta tccgctttat ctggtattac tagaagttcc    960
   attttgcttt gcctcgtttc taatctttct cacgaccagg cgacgaggag tccgggtaaa   1020
   actggctctc acttccatcc ggacagctct ctcttcgtcc cgagcgagag aaacgacgtg   1080
   ataacgtcga cagtgtgctg gtcggcgatg ttggcgttgc tggctgccat gaccgctgct   1140
   gttggccccg tttggatgtt caagctttac gtcgtacctt acttggtaag ttttccagag   1200
   ttctacgcaa tcgcttgatg cttgtgaccg cagatcaata ttgtttggct ggatgctgtg   1260
   acttacatgc atcaccacgg gcacgaagtc aaggttccgt ggtacagagg aaaggtactg   1320
   tgcaagcttt ttgttttttt tcaaggctct ttatgctgga actctaggag tggaactaca   1380
   tgagaggggg actttccacc atagacagag actacggagt gatcaacaac atccaccacg   1440
   atatcggcac tcacgtcgtg catcatttgt tccctcaaat tcctcactac aatttggtgg   1500
   aagctgtgag ttcatcttag cgcttttttg cttcggaacg agctaatcgt ggttcttttt   1560
   tctttttttc tttttctttt tttctttttt tttttgtttt cttggtgtag accaaagcga   1620
   taaagcctgt gctgggaaaa tactacaggg aaccagaaaa atctggtctg cttcccttgc   1680
   acttgtacaa aattcttaag tcgagcctcg aggaggacca ttttgtgccg aatgaaggcg 1740
   acattgtctt ctaccagcgg gactccattt aaagaggaac aatgaaaaaa tcattttttg 1800
   gcgaaaatgc gaagccaaca aaaaagaaaa cacgcaaaaa gcattctttt ctccagaaat 1860
   gtcatataga gtttgatctt tataaagagt cccattcgaa agattcgttt gatcaggagg 1920
   agcctaccta taaatataaa agaatgccag tggagagatg cgatacgggt tccctagaag 1980
   cttttataca gcgctctttc tttcttttct tggcggcaat ccaggtggcg gctcttgcga 2040
   tccgatcgat cgccattaga tcgattccat cgatcgatcc atggcggacg acggcgccaa 2100
   ggggtttgat ccatcagcgc cgccgccgtt caccatcgcc gaggtccgcg ccgccatccc 2160
   tagccactgc tggaacaaga gcctgggcag atctttgagc ttcctggcca gggatctggc 2220
   gggcgtcgcg atcttggcct gcgttgccgg atatttcgat cactggctcg tctggcccgc 2280
   gtattggatc gcgcaaggca cgctcttctg ggcgctcttc gtcatcggcc acgactggta 2340
   agcaaaagct tccatttcca tcgactcgca ttggatttca ttgcacggca gtggtgagaa 2400
   ttttgtctct ttggtttgct cttggatttc tggtgccctg cattgcgatt ctttcacctg 2460
   aatacctcga agttcccttt ctcttggatt tcgcgctcga tacttatttt ttcttttact 2520
   ttctgtgtac gtttgcgtgc gtgctttttt attttttgtt tttttatttc ttgcgttttt 2580
   tgtgacttag aacatgtctc tagtttgtct tctacttccc ttgagcaact tgcgtgaaag 2640
   ttactctatg aattcttttt ctatgcagtg gacacggaag cttttctgac aacaaggacg 2700
   tgaacacagt cgtaggacat tttgtccact cgtttatttt ggtcccgtat catgggtggt 2760
   gagtgatcag attctcttgt cctgtttttg tcctccccca tgactgattc cccggcctcg 2820
   caggagaatc agccacagaa ctcatcatca aaatcacggt cacgcagaca atgacgagtc 2880
   ctggtcacct gtaagccgtt ctgagcttgc aaattttcaa aattttcatg gttttttcag 2940
   atgaccgaga gcatgtataa gtcgtctcct ccactgaagc ttgttgcacg gttccaattg 3000
   ccgtggccat tgttggtatt cccgttctat ctcgtaagga tttgtttatt ctccattgtt 3060
   cgagcttgac accttcttgt gcttgtaggt gaaaagaact cctggcaagg atggttctca 3120
   tttccatcca gagagcccct tgttcaagcc cagtgagagg ggtgatgttt tgacgtcaac 3180
   cgcgtgttgg acggtcatgt tagccatcct gatcgccttg acgataagaa tcgggccact 3240
   ctggatgttg aaactctact ttgttcctta cgtggtaagt cctgtacgct cttgagtatg 3300
   ccatccagtg tagaactcta gcgtttttaa cttttcgttt ctttatcatt ttcagatcca 3360
   gataatgtgg attgctggtg ttacttacat gcaccaccat ggccactacc agaaagtacc 3420
   atggtatcgt gggaaggtac aagtattttc ctgttctgag gattcatttc tctcaaacga 3480
   gctcttttac atctcaggaa tggaattaca tccgaggcgg cctatcaact atagaccgag 3540
   actatggatt gttcaataac attcaccacg acattggaac gcatgtcgtg catcatttgt 3600
   tcccgcagat tccacactac catttgatcg aagccgtaag tttctctgag tggtatcaag 3660
   ggtcatcgat taacgttctt ttcaaatttc ctgtgtagac taaagccata aagccgattc 3720
   tcgggaaata ctacagggac cctgagacgt ccggaccagt gccgatccat ttgttcaagg 3780
   ttctaagcaa gagcctcaag gaagaccagt tcgtcgccga cgacggcgat gttgtcttct   3840
   accagagcga tccaggcttg aagtaagtct gtttttttgt ctctggaaga ccaaagtttt   3900
   ggagagtttt aataatccag ccggcgatgc taaggtaacg ataaaagcac ctcttttact   3960
   gaccattttt tagtgttctt catcttagta ctcggttttg agaaaaaaaa aatccattcc   4020
   atcgaagttt tcacgagctt gcaactcgaa ttttcacgaa tgttgc                  4066
<210> 155
   <211> 1125
   <212> DNA
   <213> Selaginella moellendorffii
<400> 155
   atggtcgctg ttccactccg aactgtcact gctcctcaaa gcgatgtggt cggtgacttc     60
   tccaaggagt ttgatcctgc tgctccacca ccattttctc tgggagatat cagagctgcc    120
   attccaaagc attgctggga gaagaatgtg tggagatcga ttagctatgt cgtgagggac    180
   atagcggtgg ttctcggact ggctgctggt gctgcttact tgaataattg gctcgtctgg    240
   ccactctact gggctgccca agggacgatg ttctgggctc tctttgtgct cggccatgac    300
   tgtggacacg ggagtttttc caataacaag aagcttaacc atgtcttcgg ccacattctc    360
   cattcttcca tcctggttcc ataccacggg tggaggataa gccaccggac gcatcaccag    420
   aatcacggcc atgtcgagaa cgacgagtcc tggcatccaa tgaccgagaa gctctacaaa    480
   gacgcagata tctttactcg cttcggccgg ttccagcttc catgggtcat gtttgcttat    540
   ccgctttatc tggcgacgag gagtccgggt aaaactggct ctcacttcca tccggacagc    600
   tctctcttcg tcccgagcga gagaaacgac gtgataacgt cgacagtgtg ctggtcggcg    660
   atgttggcgt tgctggctgc catgaccgct gctgttggcc ccgtttggat gttcaagctt    720
   tacgtcgtac cttacttgat caatattgtt tggctggatg ctgtgactta catgcatcac    780
   cacgggcacg aagtcaaggt tccgtggtac agaggaaagg aatggaatta catccgaggc    840
   ggcctatcaa ctatagaccg agactatgga ttgttcaata acattcacca cgacattgga    900
   acgcatgtcg tgcatcattt gttcccgcag attccacact accatttgat cgaagccact    960
   aaagccataa agccgattct cgggaaatac tacagggacc ctgagacgtc cggaccagtg   1020
   ccgatccatt tgttcaaggt tctaagcaag agcctcaagg aagaccagtt cgtcgccgac   1080
   gacggcgatg ttgtcttcta ccagagcgat ccaggcttga agtaa                   1125
<210> 156
   <211> 374
   <212> PRT
   <213> Selaginella moellendorffii
<400> 156
   Met Val Ala Val Pro Leu Arg Thr Val Thr Ala Pro Gln Ser Asp Val
   1               5                   10                  15
   Val Gly Asp Phe Ser Lys Glu Phe Asp Pro Ala Ala Pro Pro Pro Phe
               20                  25                  30
   Ser Leu Gly Asp Ile Arg Ala Ala Ile Pro Lys His Cys Trp Glu Lys
           35                  40                  45
   Asn Val Trp Arg Ser Ile Ser Tyr Val Val Arg Asp Ile Ala Val Val
       50                  55                  60
   Leu Gly Leu Ala Ala Gly Ala Ala Tyr Leu Asn Asn Trp Leu Val Trp
   65                  70                  75                  80
   Pro Leu Tyr Trp Ala Ala Gln Gly Thr Met Phe Trp Ala Leu Phe Val
                   85                  90                  95
   Leu Gly His Asp Cys Gly His Gly Ser Phe Ser Asn Asn Lys Lys Leu
               100                 105                 110
   Asn His Val Phe Gly His Ile Leu His Ser Ser Ile Leu Val Pro Tyr
           115                 120                 125
   His Gly Trp Arg Ile Ser His Arg Thr His His Gln Asn His Gly His
       130                 135                 140
   Val Glu Asn Asp Glu Ser Trp His Pro Met Thr Glu Lys Leu Tyr Lys
   145                 150                 155                 160
   Asp Ala Asp Ile Phe Thr Arg Phe Gly Arg Phe Gln Leu Pro Trp Val
                   165                 170                 175
   Met Phe Ala Tyr Pro Leu Tyr Leu Ala Thr Arg Ser Pro Gly Lys Thr
               180                 185                 190
   Gly Ser His Phe His Pro Asp Ser Ser Leu Phe Val Pro Ser Glu Arg
           195                 200                 205
   Asn Asp Val Ile Thr Ser Thr Val Cys Trp Ser Ala Met Leu Ala Leu
       210                 215                 220
   Leu Ala Ala Met Thr Ala Ala Val Gly Pro Val Trp Met Phe Lys Leu
   225                 230                 235                 240
   Tyr Val Val Pro Tyr Leu Ile Asn Ile Val Trp Leu Asp Ala Val Thr
                   245                 250                 255
   Tyr Met His His His Gly His Glu Val Lys Val Pro Trp Tyr Arg Gly
               260                 265                 270
   Lys Glu Trp Asn Tyr Ile Arg Gly Gly Leu Ser Thr Ile Asp Arg Asp
           275                 280                 285
   Tyr Gly Leu Phe Asn Asn Ile His His Asp Ile Gly Thr His Val Val
       290                 295                 300
   His His Leu Phe Pro Gln Ile Pro His Tyr His Leu Ile Glu Ala Thr
   305                 310                 315                 320
   Lys Ala Ile Lys Pro Ile Leu Gly Lys Tyr Tyr Arg Asp Pro Glu Thr
                   325                 330                 335
   Ser Gly Pro Val Pro Ile His Leu Phe Lys Val Leu Ser Lys Ser Leu
               340                 345                 350
   Lys Glu Asp Gln Phe Val Ala Asp Asp Gly Asp Val Val Phe Tyr Gln
           355                 360                 365
   Ser Asp Pro Gly Leu Lys
       370
<210> 157
   <211> 1209
   <212> DNA
   <213> Microdochium nivale

<400> 157
   atgattgcga ccacccagac caagacggcc gtcacggacc gcgggacgac ccggatgaga      60
   ggctcgtcaa cagcccagga gttcccagac atccagacca tccgcgatgc tatccccaag     120
   cactgctttg agccctcaac agtccgttcc ttgagctatg tcgcccgcga tgtaaccatg     180
   gcctcggccc tcatctgggc ggccgtccgc ttcatccccc agatcgagga ctcggtcctg     240
   cgcttctcgg cctggatggt ctacggcctt gtccagggca tggtctgcac cggcgtctgg     300
   atcctcgccc acgagtgcgg ccacggcgcc ttcagcaagc accagaccct caacgacttt     360
   gtcggctggg tcctgcactc gagtctcggc gtcccctact tctcatggaa gttctcacac     420
   caccgccacc accgcttcac tggcaacatg gagaaggaca tggtctttgt ccccgccgtc     480
   aagaccgagg agccccctaa gcgccgcctc gcctccttct acctcgaccc tgagatcctc     540
   gaggacgccc ccattgtcag cctcatccag ctcattgccc accagctcgc cggctggcag     600
   atgtacatgc tcttcaacgt ctcatcgggc aaggacagca agcagcgcaa ccagtctggc     660
   tggctgcgcg tcagccactt tgagcccacc agcgccgtct tccgccctag cgaggcctgg     720
   tacatcttcc tcgccgacgt cggccttgct ctcaccggcg ccgccatcta ctacggctcc     780
   acccttgtcg gctggcccac catgttcttt gtctactttg tcccctacat gtggtggaac     840
   cactggctcg ttgccatcac ctacctccac cacacccacc cggaagtcca ccactacgag     900
   gcggacagct ggacctacgt caagggcgcc ctcgccaccg tcgaccgcga ctttggctgg     960
   attgacaagc acctcttcca cggcatcatt ggcttccacg tcatccacca catctttgcc    1020
   aagatcccct tctactacgc cgaggaggcc accgcggcca tccagcccgt cattggcaac    1080
   cactaccacc gtgctcccgg ctccttcctc ggcgacctct ggctcacttt caccaagtgc    1140
   cgcttcgtcg agaaggaccc cgagcaccct ggcgccatgc gctgggtcgc accccgcaag    1200
   gacctttag                                                            1209
<210> 158
   <211> 402
   <212> PRT
   <213> Microdochium nivale
<400> 158
   Met Ile Ala Thr Thr Gln Thr Lys Thr Ala Val Thr Asp Arg Gly Thr
   1 5 10 15
   Thr Arg Met Arg Gly Ser Ser Thr Ala Gln Glu Phe Pro Asp Ile Gln
   20 25 30
   Thr Ile Arg Asp Ala Ile Pro Lys His Cys Phe Glu Pro Ser Thr Val
   35 40 45
   Arg Ser Leu Ser Tyr Val Ala Arg Asp Val Thr Met Ala Ser Ala Leu
   50 55 60
   Ile Trp Ala Ala Val Arg Phe Ile Pro Gln Ile Glu Asp Ser Val Leu
   65 70 75 80
   Arg Phe Ser Ala Trp Met Val Tyr Gly Leu Val Gln Gly Met Val Cys
   85 90 95
   Thr Gly Val Trp Ile Leu Ala His Glu Cys Gly His Gly Ala Phe Ser
   100 105 110
   Lys His Gln Thr Leu Asn Asp Phe Val Gly Trp Val Leu His Ser Ser
   115 120 125
   Leu Gly Val Pro Tyr Phe Ser Trp Lys Phe Ser His His Arg His His
   130 135 140
   Arg Phe Thr Gly Asn Met Glu Lys Asp Met Val Phe Val Pro Ala Val
   145 150 155 160
   Lys Thr Glu Glu Pro Pro Lys Arg Arg Leu Ala Ser Phe Tyr Leu Asp
   165 170 175
   Pro Glu Ile Leu Glu Asp Ala Pro Ile Val Ser Leu Ile Gln Leu Ile
   180 185 190
   Ala His Gln Leu Ala Gly Trp Gln Met Tyr Met Leu Phe Asn Val Ser
   195 200 205
   Ser Gly Lys Asp Ser Lys Gln Arg Asn Gln Ser Gly Trp Leu Arg Val
   210 215 220
   Ser His Phe Glu Pro Thr Ser Ala Val Phe Arg Pro Ser Glu Ala Trp
   225 230 235 240
   Tyr Ile Phe Leu Ala Asp Val Gly Leu Ala Leu Thr Gly Ala Ala Ile
   245 250 255
   Tyr Tyr Gly Ser Thr Leu Val Gly Trp Pro Thr Met Phe Phe Val Tyr
   260 265 270
   Phe Val Pro Tyr Met Trp Trp Asn His Trp Leu Val Ala Ile Thr Tyr
   275 280 285
   Leu His His Thr His Pro Glu Val His His Tyr Glu Ala Asp Ser Trp
   290 295 300
   Thr Tyr Val Lys Gly Ala Leu Ala Thr Val Asp Arg Asp Phe Gly Trp
   305 310 315 320
   Ile Asp Lys His Leu Phe His Gly Ile Ile Gly Phe His Val Ile His
   325 330 335
   His Ile Phe Ala Lys Ile Pro Phe Tyr Tyr Ala Glu Glu Ala Thr Ala
   340 345 350
   Ala Ile Gln Pro Val Ile Gly Asn His Tyr His Arg Ala Pro Gly Ser
   355 360 365
   Phe Leu Gly Asp Leu Trp Leu Thr Phe Thr Lys Cys Arg Phe Val Glu
   370 375 380
   Lys Asp Pro Glu His Pro Gly Ala Met Arg Trp Val Ala Pro Arg Lys
   385 390 395 400
   Asp Leu
<210> 159
   <211> 22
   <212> DNA
   <213> Artificial
<220>
   <223> Primer
<400> 159
   atggatcaag ccagtaagat tg 22
<210> 160
   <211> 23
   <212> DNA
   <213> Artificial
<220>
   <223> Primer
<400> 160
   ttatctgcaa ttgcactgtt ttg 23
<210> 161
   <211> 22
   <212> DNA
   <213> Artificial
<220>
   <223> Primer
<400> 161
   atgcggagcg cggaggatga tg 22
<210> 162
   <211> 21
   <212> DNA
   <213> Artificial
<220>
   <223> Primer
<400> 162
   tcactgcttt acatcgttct g 21
<210> 163
   <211> 20
   <212> DNA
   <213> Artificial
<220>
   <223> Primer
<400> 163
   atgtccgtga agcatgagat 20
<210> 164
   <211> 22
   <212> DNA
   <213> Artificial

<220>
   <223> Primer
<400> 164
   tcacttagca tttgtgctct tc 22
<210> 165
   <211> 22
   <212> DNA
   <213> Artificial
<220>
   <223> Primer
<400> 165
   atggctacca cggctgattc tg 22
<210> 166
   <211> 20
   <212> DNA
   <213> Artificial
<220>
   <223> Primer
<400> 166
   tcaacgagac acgctcgctg 20
<210> 167
   <211> 20
   <212> DNA
   <213> Artificial
<220>
   <223> Primer
<400> 167
   atggtcgctg ttccactccg 20
<210> 168
   <211> 22
   <212> DNA
   <213> Artificial
<220>
   <223> Primer
<400> 168
   ttacttcaag cctggatcgc tc 22
<210> 169
   <211> 21
   <212> DNA
   <213> Artificial
<220>
   <223> Primer
<400> 169
   atgattgcga ccacccagac c 21
<210> 170
   <211> 20
   <212> DNA
   <213> Artificial
<220>
   <223> Primer
<400> 170
   ctaaaggtcc ttgcggggtg 20
<210> 171
   <211> 33
   <212> DNA
   <213> Artificial
<220>
   <223> Primer
<400> 171
   gcggccgcgc catggatcaa gccagtaaga ttg 33
<210> 172
   <211> 32
   <212> DNA
   <213> Artificial
<220>
   <223> Primer
<400> 172
   gcggccgcgt tatctgcaat tgcactgttt tg 32
<210> 173
   <211> 33
   <212> DNA
   <213> Artificial
<220>
   <223> Primer
<400> 173
   gcggccgcgc catgcggagc gcggaggatg atg 33
<210> 174
   <211> 30
   <212> DNA
   <213> Artificial
<220>
   <223> Primer
<400> 174
   gcggccgcgt cactgcttta catcgttctg 30
<210> 175
   <211> 31
   <212> DNA
   <213> Artificial

<220>
   <223> Primer
<400> 175
   gcggccgcgc catgtccgtg aagcatgaga t 31
<210> 176
   <211> 31
   <212> DNA
   <213> Artificial
<220>
   <223> Primer
<400> 176
   gcggccgcgt cacttagcat ttgtgctctt c 31
<210> 177
   <211> 33
   <212> DNA
   <213> Artificial
<220>
   <223> Primer
<400> 177
   gcggccgcgc catggctacc acggctgatt ctg 33
<210> 178
   <211> 29
   <212> DNA
   <213> Artificial
<220>
   <223> Primer
<400> 178
   gcggccgcgt caacgagaca cgctcgctg 29
<210> 179
   <211> 31
   <212> DNA
   <213> Artificial
<220>
   <223> Primer
<400> 179
   gcggccgcgc catggtcgct gttccactcc g 31
<210> 180
   <211> 31
   <212> DNA
   <213> Artificial
<220>
   <223> Primer
<400> 180
   gcggccgcgt tacttcaagc ctggatcgct c 31
<210> 181
   <211> 32
   <212> DNA
   <213> Artificial
<220>
   <223> Primer
<400> 181
   gcggccgcgc catgattgcg accacccaga cc 32
<210> 182
   <211> 29
   <212> DNA
   <213> Artificial
<220>
   <223> Primer
<400> 182
   gcggccgcgc taaaggtcct tgcggggtg 29
<210> 183
   <211> 16093
   <212> DNA
   <213> Artificial
<220>
   <223> Plasmid
<400> 183
   aaaagttgcc atgattacgc caagcttggc cactaaggcc aatttaaatc tactaggccg 60
   gccaaagtag gcgcctacta ccggtaattc ccgggattag cggccgctag tctgtgcgca 120
   cttgtatcct gcaggtcaat cgtttaaaca ctgtacggac cgtggcctaa taggccggta 180
   cccaagtttg tacaaaaaag caggctccat gattacgcca agcttggcca ctaaggccaa 240
   tttaaatcta ctaggccggc catcgacggc ccggactgta tccaacttct gatctttgaa 300
   tctctctgtt ccaacatgtt ctgaaggagt tctaagactt ttcagaaagc ttgtaacatg 360
   ctttgtagac tttctttgaa ttactcttgc aaactctgat tgaacctacg tgaaaactgc 420
   tccagaagtt ctaaccaaat tccgtcttgg gaaggcccaa aatttattga gtacttcagt 480
   ttcatggacg tgtcttcaaa gatttataac ttgaaatccc atcattttta agagaagttc 540
   tgttccgcaa tgtcttagat ctcattgaaa tctacaactc ttgtgtcaga agttcttcca 600
   gaatcaactt gcatcatggt gaaaatctgg ccagaagttc tgaacttgtc atatttctta 660
   acagttagaa aaatttctaa gtgtttagaa ttttgacttt tccaaagcaa acttgacttt 720
   tgactttctt aataaaacaa acttcatatt ctaacatgtc ttgatgaaat gtgattcttg 780
   aaatttgatg ttgatgcaaa agtcaaagtt tgacttttca gtgtgcaatt gaccattttg 840
   ctcttgtgcc aattccaaac ctaaattgat gtatcagtgc tgcaaacttg atgtcatgga 900
   agatcttatg agaaaattct tgaagactga gaggaaaaat tttgtagtac aacacaaaga 960
   atcctgtttt tcatagtcgg actagacaca ttaacataaa acaccacttc attcgaagag 1020
   tgattgaaga aggaaatgtg cagttacctt tctgcagttc ataagagcaa cttacagaca 1080
   cttttactaa aatactacaa agaggaagat tttaacaact tagagaagta atgggagtta 1140
   aagagcaaca cattaagggg gagtgttaaa attaatgtgt tgtaaccacc actaccttta 1200
   gtaagtatta taagaaaatt gtaatcatca cattataatt attgtcctta tttaaaatta 1260
   tgataaagtt gtatcattaa gattgagaaa accaaatagt cctcgtcttg atttttgaat 1320
   tattgttttc tatgttactt ttcttcaagc ctatataaaa actttgtaat gctaaattgt 1380
   atgctggaaa aaaatgtgta atgaattgaa tagaaattat ggtatttcaa agtccaaaat 1440
   ccatcaatag aaatttagta caaaacgtaa ctcaaaaata ttctcttatt ttaaatttta 1500
   caacaatata aaaatattct cttattttaa attttacaat aatataattt atcacctgtc 1560
   acctttagaa taccaccaac aatattaata cttagatatt ttattcttaa taattttgag 1620
   atctctcaat atatctgata tttattttat atttgtgtca tattttctta tgttttagag 1680
   ttaaccctta tatcttggtc aaactagtaa ttcaatatat gagtttgtga aggacacatt 1740
   gacatcttga aacattggtt ttaaccttgt tggaatgtta aaggtaataa aacattcaga 1800
   attatgacca tctattaata tacttccttt gtcttttaaa aaagtgtgca tgaaaatgct 1860
   ctatggtaag ctagagtgtc ttgctggcct gtgtatatca attccatttc cagatggtag 1920
   aaactgccac tacgaataat tagtcataag acacgtatgt taacacacgt ccccttgcat 1980
   gttttttgcc atatattccg tctctttctt tttcttcacg tataaaacaa tgaactaatt 2040
   aatagagcga tcaagctgaa ccgcgccacc atgagcagaa ctgttacttt agaggctgct 2100
   cctgctacta ctttgagcgt ggagaagaaa acccctacca tccgtcaaat aaggaacgct 2160
   atccctgagc attgcttcaa acctaccgct tggaaatcat ctgctcacgc tatcttcgat 2220
   tgctcagtag ctgctcttat tgctttcgct gcttacaaga ccatccctct cgttgaatac 2280
   tggcctgcta gatgggcttt gtgggctctt tacggttaca tagagggatt agtgttcact 2340
   ggaatctgga tcgttgctca tgagtgtgga catggtggac tttacacctc aaactgggct 2400
   aacgatatag ttggttacac cttacatact tctcttttag tgccttactt cccttggaag 2460
   tacactcatg ctagacacca cagatacacc ggacacatgg aaaaggatac cgctttcgtt 2520
   cctcatagag ctggtgaaaa gtcaatagga agcaagatag ctgaggttat cggacatgct 2580
   gaggatgctc cactttacat gttcggagga ttagttatgc atcaactttt aggatggcag 2640
   gcttaccttc tcttctatgt gagcgctggt gctagatcta ctcctaaggc tctcgaggga 2700
   tcatcttggg ctggatctca cttcgatcct atggctaacc tttggacccc ttctcaaaga 2760
   cctttcgtgt tcttgagcac tgttggatta ggagctgtta tgttcgctct ctatcagctt 2820
   tcaggtgtta tcggagtagc taacaccttc cttctctacg gacttcctta cctctgggta 2880
   aacaactggc tagtggctat cacttacctt catcacaccc accctgattc tcatcattac 2940
   gaggcttcta gatggacttt cttagatggt gctctaacta ccgtggatag gcctttcgga 3000
   ttcatcggaa gaaaggtgtt ccacggaatc atagatttcc acgttgttca ccaccttttc 3060
   ccttcaatgc ctttctatca tgctgaggaa gctactaagg ctatgagacc tgtgttagga 3120
   gattactacc gtagagatga cactcctttc tggatagctt tatggaaaac cttctcttca 3180
   tgccaagctg ttcagcctaa agagggtgaa gagggagttc ttgagtggga gaccaagaaa 3240
   acccacgtga agagcgcttg atgattaatt aaggccgcct cgaccgtacc ccctgcagat 3300
   agactatact atgttttagc ctgcctgctg gctagctact atgttatgtt atgttgtaaa 3360
   ataaacacct gctaaggtat atctatctat attttagcat ggctttctca ataaattgtc 3420
   tttccttatc gtttactatc ttatacctaa taatgaaata ataatatcac atatgaggaa 3480
   cggggcaggt ttaggcatat atatacgagt gtagggcgga gtggggggcg cctactaccg 3540
   gtaattcccg ggattagcgg ccgctagtct gtgcgcactt gtatcctgca ggtcaatcgt 3600
   ttaaacactg tacggaccgt ggcctaatag gccggtacca cccagctttc ttgtacaaag 3660
   tggccatgat tacgccaagc tctccaccgc ggtggcggcc gctctagccc aagctttaag 3720
   gatgacctac ccattcttga gacaaatgtt acattttagt atcagagtaa aatgtgtacc 3780
   tataactcaa attcgattga catgtatcca ttcaacataa aattaaacca gcctgcacct 3840
   gcatccacat ttcaagtatt ttcaaaccgt tcggctccta tccaccgggt gtaacaagac 3900
   ggattccgaa tttggaagat tttgactcaa attcccaatt tatattgacc gtgactaaat 3960
   caactttaac ttctataatt ctgattaagc tcccaattta tattcccaac ggcactacct 4020
   ccaaaattta tagactctca tcccctttta aaccaactta gtaaacgttt tttttttaat 4080
   tttatgaagt taagttttta ccttgttttt aaaaagaatc gttcataaga tgccatgcca 4140
   gaacattagc tacacgttac acatagcatg cagccgcgga gaattgtttt tcttcgccac 4200
   ttgtcactcc cttcaaacac ctaagagctt ctctctcaca gcacacacat acaatcacat 4260
   gcgtgcatgc attattacac gtgatcgcca tgcaaatctc ctttatagcc tataaattaa 4320
   ctcatcggct tcactcttta ctcaaaccaa aactcatcaa tacaaacaag attaaaaaca 4380
   taaggcgcgc caattgacta gtaggcctat cgattaggag aataacaatg gtgcgctcct 4440
   ccaagaacgt catcaaggag ttcatgcgct tcaaggtgcg catggagggc accgtgaacg 4500
   gccacgagtt cgagatcgag ggcgagggcg agggccgccc ctacgagggc cacaacaccg 4560
   tgaagctgaa ggtgaccaag ggcggccccc tgcccttcgc ctgggacatc ctgtcccccc 4620
   agttccagta cggctccaag gtgtacgtga agcaccccgc cgacatcccc gactacaaga 4680
   agctgtcctt ccccgagggc ttcaagtggg agcgcgtgat gaacttcgag gacggcggcg 4740
   tggtgaccgt gacccaggac tcctccctgc aggacggctg cttcatctac aaggtgaagt 4800
   tcatcggcgt gaacttcccc tccgacggcc ccgtaatgca gaagaagacc atgggctggg 4860
   aggcctccac cgagcgcctg tacccccgcg acggcgtgct gaagggcgag atccacaagg 4920
   ccctgaagct gaaggacggc ggccactacc tggtggagtt caagtccatc tacatggcca 4980
   agaagcccgt gcagctgccc ggctactact acgtggactc caagctggac atcacctccc 5040
   acaacgagga ctacaccatc gtggagcagt acgagcgcac cgagggccgc caccacctgt 5100
   tcctgctcga gtctagaggt accggttgtt aacgttagcc ggctacgtat actccggaat 5160
   attaataggc ctaggatgca tatggcggcc gcctgcagct ggcgccatcg attaattaag 5220
   gccgcctcga gcatgcatct agagggcccg ctagcgttaa ccctgcttta atgagatatg 5280
   cgagacgcct atgatcgcat gatatttgct ttcaattctg ttgtgcacgt tgtaaaaaac 5340
   ctgagcatgt gtagctcaga tccttaccgc cggtttcggt tcattctaat gaatatatca 5400
   cccgttacta tcgtattttt atgaataata ttctccgttc aatttactga ttgtccgtcg 5460
   agcatatgct agaggatccc cgggtaccca actttattat acatagttga taattcactg 5520
   gccggatgta ccgaattcgc ggccgcaagc ttgtacacta gtacgcgtca attggcgatc 5580
   gcggatctga gatgaaaccg gtgattatca gaacctttta tggtctttgt atgcatatgg 5640
   taaaaaaact tagtttgcaa tttcctgttt gttttggtaa tttgagtttc ttttagttgt 5700
   tgatctgcct gctttttggt ttacgtcaga ctactactgc tgttgttgtt tggtttcctt 5760
   tctttcattt tataaataaa taatccggtt cggtttactc cttgtgactg gctcagtttg 5820
   gttattgcga aatgcgaatg gtaaattgag taattgaaat tcgttattag ggttctaagc 5880
   tgttttaaca gtcactgggt taatatctct cgaatcttgc atggaaaatg ctcttaccat 5940
   tggtttttaa ttgaaatgtg ctcatatggg ccgtggtttc caaattaaat aaaactacga 6000
   tgtcatcgag aagtaaaatc aactgtgtcc acattatcag ttttgtgtat acgatgaaat 6060
   agggtaattc aaaatctagc ttgatatgcc ttttggttca ttttaacctt ctgtaaacat 6120
   tttttcagat tttgaacaag taaatccaaa aaaaaaaaaa aaaaatctca actcaacact 6180
   aaattatttt aatgtataaa agatgcttaa aacatttggc ttaaaagaaa gaagctaaaa 6240
   acatagagaa ctcttgtaaa ttgaagtatg aaaatatact gaattgggta ttatatgaat 6300
   ttttctgatt taggattcac atgatccaaa aaggaaatcc agaagcacta atcagacatt 6360
   ggaagtagga atatttcaaa aagttttttt tttttaagta agtgacaaaa gcttttaaaa 6420
   aatagaaaag aaactagtat taaagttgta aatttaataa acaaaagaaa ttttttatat 6480
   tttttcattt ctttttccag catgaggtta tgatggcagg atgtggattt catttttttc 6540
   cttttgatag ccttttaatt gatctattat aattgacgaa aaaatattag ttaattatag 6600
   atatatttta ggtagtatta gcaatttaca cttccaaaag actatgtaag ttgtaaatat 6660
   gatgcgttga tctcttcatc attcaatggt tagtcaaaaa aataaaagct taactagtaa 6720
   actaaagtag tcaaaaattg tactttagtt taaaatatta catgaataat ccaaaacgac 6780
   atttatgtga aacaaaaaca atatagatcc attaccctgt tatccctaga ggggaaaatt 6840
   cgaatccaaa aattacggat atgaatatag gcatatccgt atccgaatta tccgtttgac 6900
   agctagcaac gattgtacaa ttgcttcttt aaaaaaggaa gaaagaaaga aagaaaagaa 6960
   tcaacatcag cgttaacaaa cggccccgtt acggcccaaa cggtcatata gagtaacggc 7020
   gttaagcgtt gaaagactcc tatcgaaata cgtaaccgca aacgtgtcat agtcagatcc 7080
   cctcttcctt caccgcctca aacacaaaaa taatcttcta cagcctatat atacaacccc 7140
   cccttctatc tctcctttct cacaattcat catctttctt tctctacccc caattttaag 7200
   aaatcctctc ttctcctctt cattttcaag gtaaatctct ctctctctct ctctctctgt 7260
   tattccttgt tttaattagg tatgtattat tgctagtttg ttaatctgct tatcttatgt 7320
   atgccttatg tgaatatctt tatcttgttc atctcatccg tttagaagct ataaatttgt 7380
   tgatttgact gtgtatctac acgtggttat gtttatatct aatcagatat gaatttcttc 7440
   atattgttgc gtttgtgtgt accaatccga aatcgttgat ttttttcatt taatcgtgta 7500
   gctaattgta cgtatacata tggatctacg tatcaattgt tcatctgttt gtgtttgtat 7560
   gtatacagat ctgaaaacat cacttctctc atctgattgt gttgttacat acatagatat 7620
   agatctgtta tatcattttt tttattaatt gtgtatatat atatgtgcat agatctggat 7680
   tacatgattg tgattattta catgattttg ttatttacgt atgtatatat gtagatctgg 7740
   actttttgga gttgttgact tgattgtatt tgtgtgtgta tatgtgtgtt ctgatcttga 7800
   tatgttatgt atgtgcagct gaaccatggc ggcggcaaca acaacaacaa caacatcttc 7860
   ttcgatctcc ttctccacca aaccatctcc ttcctcctcc aaatcaccat taccaatctc 7920
   cagattctcc ctcccattct ccctaaaccc caacaaatca tcctcctcct cccgccgccg 7980
   cggtatcaaa tccagctctc cctcctccat ctccgccgtg ctcaacacaa ccaccaatgt 8040
   cacaaccact ccctctccaa ccaaacctac caaacccgaa acattcatct cccgattcgc 8100
   tccagatcaa ccccgcaaag gcgctgatat cctcgtcgaa gctttagaac gtcaaggcgt 8160
   agaaaccgta ttcgcttacc ctggaggtac atcaatggag attcaccaag ccttaacccg 8220
   ctcttcctca atccgtaacg tccttcctcg tcacgaacaa ggaggtgtat tcgcagcaga 8280
   aggatacgct cgatcctcag gtaaaccagg tatctgtata gccacttcag gtcccggagc 8340
   tacaaatctc gttagcggat tagccgatgc gttgttagat agtgttcctc ttgtagcaat 8400
   cacaggacaa gtccctcgtc gtatgattgg tacagatgcg tttcaagaga ctccgattgt 8460
   tgaggtaacg cgttcgatta cgaagcataa ctatcttgtg atggatgttg aagatatccc 8520
   taggattatt gaggaagctt tctttttagc tacttctggt agacctggac ctgttttggt 8580
   tgatgttcct aaagatattc aacaacagct tgcgattcct aattgggaac aggctatgag 8640
   attacctggt tatatgtcta ggatgcctaa acctccggaa gattctcatt tggagcagat 8700
   tgttaggttg atttctgagt ctaagaagcc tgtgttgtat gttggtggtg gttgtttgaa 8760
   ttctagcgat gaattgggta ggtttgttga gcttacgggg atccctgttg cgagtacgtt 8820
   gatggggctg ggatcttatc cttgtgatga tgagttgtcg ttacatatgc ttggaatgca 8880
   tgggactgtg tatgcaaatt acgctgtgga gcatagtgat ttgttgttgg cgtttggggt 8940
   aaggtttgat gatcgtgtca cgggtaagct tgaggctttt gctagtaggg ctaagattgt 9000
   tcatattgat attgactcgg ctgagattgg gaagaataag actcctcatg tgtctgtgtg 9060
   tggtgatgtt aagctggctt tgcaagggat gaataaggtt cttgagaacc gagcggagga 9120
   gcttaagctt gattttggag tttggaggaa tgagttgaac gtacagaaac agaagtttcc 9180
   gttgagcttt aagacgtttg gggaagctat tcctccacag tatgcgatta aggtccttga 9240
   tgagttgact gatggaaaag ccataataag tactggtgtc gggcaacatc aaatgtgggc 9300
   ggcgcagttc tacaattaca agaaaccaag gcagtggcta tcatcaggag gccttggagc 9360
   tatgggattt ggacttcctg ctgcgattgg agcgtctgtt gctaaccctg atgcgatagt 9420
   tgtggatatt gacggagatg gaagctttat aatgaatgtg caagagctag ccactattcg 9480
   tgtagagaat cttccagtga aggtactttt attaaacaac cagcatcttg gcatggttat 9540
   gcaatgggaa gatcggttct acaaagctaa ccgagctcac acatttctcg gggatccggc 9600
   tcaggaggac gagatattcc cgaacatgtt gctgtttgca gcagcttgcg ggattccagc 9660
   ggcgagggtg acaaagaaag cagatctccg agaagctatt cagacaatgc tggatacacc 9720
   aggaccttac ctgttggatg tgatttgtcc gcaccaagaa catgtgttgc cgatgatccc 9780
   gaatggtggc actttcaacg atgtcataac ggaaggagat ggccggatta aatactgata 9840
   gggataacag ggtaatctcg acgagatgaa accggtgatt atcagaacct tttatggtct 9900
   ttgtatgcat atggtaaaaa aacttagttt gcaatttcct gtttgttttg gtaatttgag 9960
   tttcttttag ttgttgatct gcctgctttt tggtttacgt cagactacta ctgctgttgt 10020
   tgtttggttt cctttctttc attttataaa taaataatcc ggttcggttt actccttgtg 10080
   actggctcag tttggttatt gcgaaatgcg aatggtaaat tgagtaattg aaattcgtta 10140
   ttagggttct aagctgtttt aacagtcact gggttaatat ctctcgaatc ttgcatggaa 10200
   aatgctctta ccattggttt ttaattgaaa tgtgctcata tgggccgtgg tttccaaatt 10260
   aaataaaact acgatgtcat cgagaagtaa aatcaactgt gtccacatta tcagttttgt 10320
   gtatacgatg aaatagggta attcaaaatc tagcttgata tgccttttgg ttcattttaa 10380
   ccttctgtaa acattttttc agattttgaa caagtaaatc caaaaaaaaa aaaaaaaaat 10440
   ctcaactcaa cactaaatta ttttaatgta taaaagatgc ttaaaacatt tggcttaaaa 10500
   gaaagaagct aaaaacatag agaactcttg taaattgaag tatgaaaata tactgaattg 10560
   ggtattatat gaatttttct gatttaggat tcacatgatc caaaaaggaa atccagaagc 10620
   actaatcaga cattggaagt aggaatattt caaaaagttt ttttttttta agtaagtgac 10680
   aaaagctttt aaaaaataga aaagaaacta gtattaaagt tgtaaattta ataaacaaaa 10740
   gaaatttttt atattttttc atttcttttt ccagcatgag gttatgatgg caggatgtgg 10800
   atttcatttt tttccttttg atagcctttt aattgatcta ttataattga cgaaaaaata 10860
   ttagttaatt atagatatat tttaggtagt attagcaatt tacacttcca aaagactatg 10920
   taagttgtaa atatgatgcg ttgatctctt catcattcaa tggttagtca aaaaaataaa 10980
   agcttaacta gtaaactaaa gtagtcaaaa attgtacttt agtttaaaat attacatgaa 11040
   taatccaaaa cgacatttat gtgaaacaaa aacaatatgt cgaggcgatc gcagtactta 11100
   atcagtgatc agtaactaaa ttcagtacat taaagacgtc cgcaatgtgt tattaagttg 11160
   tctaagcgtc aatttgttta caccacaata tatcctgcca ccagccagcc aacagctccc 11220
   cgaccggcag ctcggcacaa aatcactgat catctaaaaa ggtgatgtgt atttgagtaa 11280
   aacagcttgc gtcatgcggt cgctgcgtat atgatgcgat gagtaaataa acaaatacgc 11340
   aaggggaacg catgaaggtt atcgctgtac ttaaccagaa aggcgggtca ggcaagacga 11400
   ccatcgcaac ccatctagcc cgcgccctgc aactcgccgg ggccgatgtt ctgttagtcg 11460
   attccgatcc ccagggcagt gcccgcgatt gggcggccgt gcgggaagat caaccgctaa 11520
   ccgttgtcgg catcgaccgc ccgacgattg accgcgacgt gaaggccatc ggccggcgcg 11580
   acttcgtagt gatcgacgga gcgccccagg cggcggactt ggctgtgtcc gcgatcaagg 11640
   cagccgactt cgtgctgatt ccggtgcagc caagccctta cgacatttgg gccaccgccg 11700
   acctggtgga gctggttaag cagcgcattg aggtcacgga tggaaggcta caagcggcct 11760
   ttgtcgtgtc gcgggcgatc aaaggcacgc gcatcggcgg tgaggttgcc gaggcgctgg 11820
   ccgggtacga gctgcccatt cttgagtccc gtatcacgca gcgcgtgagc tacccaggca 11880
   ctgccgccgc cggcacaacc gttcttgaat cagaacccga gggcgacgct gcccgcgagg 11940
   tccaggcgct ggccgctgaa attaaatcaa aactcatttg agttaatgag gtaaagagaa 12000
   aatgagcaaa agcacaaaca cgctaagtgc cggccgtccg agcgcacgca gcagcaaggc 12060
   tgcaacgttg gccagcctgg cagacacgcc agccatgaag cgggtcaact ttcagttgcc 12120
   ggcggaggat cacaccaagc tgaagatgta cgcggtacgc caaggcaaga ccattaccga 12180
   gctgctatct gaatacatcg cgcagctacc agagtaaatg agcaaatgaa taaatgagta 12240
   gatgaatttt agcggctaaa ggaggcggca tggaaaatca agaacaacca ggcaccgacg 12300
   ccgtggaatg ccccatgtgt ggaggaacgg gcggttggcc aggcgtaagc ggctgggttg 12360
   tctgccggcc ctgcaatggc actggaaccc ccaagcccga ggaatcggcg tgagcggtcg 12420
   caaaccatcc ggcccggtac aaatcggcgc ggcgctgggt gatgacctgg tggagaagtt 12480
   gaaggccgcg caggccgccc agcggcaacg catcgaggca gaagcacgcc ccggtgaatc 12540
   gtggcaaggg gccgctgatc gaatccgcaa agaatcccgg caaccgccgg cagccggtgc 12600
   gccgtcgatt aggaagccgc ccaagggcga cgagcaacca gattttttcg ttccgatgct 12660
   ctatgacgtg ggcacccgcg atagtcgcag catcatggac gtggccgttt tccgtctgtc 12720
   gaagcgtgac cgacgagctg gcgaggtgat ccgctacgag cttccagacg ggcacgtaga 12780
   ggtttccgca ggccccgccg gcatggccag tgtgtgggat tacgacctgg tactgatggc 12840
   ggtttcccat ctaaccgaat ccatgaaccg ataccgggaa gggaagggag acaagcccgg 12900
   ccgcgtgttc cgtccacacg ttgcggacgt actcaagttc tgccggcgag ccgatggcgg 12960
   aaagcagaaa gacgacctgg tagaaacctg cattcggtta aacaccacgc acgttgccat 13020
   gcagcgtacc aagaaggcca agaacggccg cctggtgacg gtatccgagg gtgaagcctt 13080
   gattagccgc tacaagatcg taaagagcga aaccgggcgg ccggagtaca tcgagatcga 13140
   gcttgctgat tggatgtacc gcgagatcac agaaggcaag aacccggacg tgctgacggt 13200
   tcaccccgat tactttttga tcgaccccgg catcggccgt tttctctacc gcctggcacg 13260
   ccgcgccgca ggcaaggcag aagccagatg gttgttcaag acgatctacg aacgcagtgg 13320
   cagcgccgga gagttcaaga agttctgttt caccgtgcgc aagctgatcg ggtcaaatga 13380
   cctgccggag tacgatttga aggaggaggc ggggcaggct ggcccgatcc tagtcatgcg 13440
   ctaccgcaac ctgatcgagg gcgaagcatc cgccggttcc taatgtacgg agcagatgct 13500
   agggcaaatt gccctagcag gggaaaaagg tcgaaaaggt ctctttcctg tggatagcac 13560
   gtacattggg aacccaaagc cgtacattgg gaaccggaac ccgtacattg ggaacccaaa 13620
   gccgtacatt gggaaccggt cacacatgta agtgactgat ataaaagaga aaaaaggcga 13680
   tttttccgcc taaaactctt taaaacttat taaaactctt aaaacccgcc tggcctgtgc 13740
   ataactgtct ggccagcgca cagccgaaga gctgcaaaaa gcgcctaccc ttcggtcgct 13800
   gcgctcccta cgccccgccg cttcgcgtcg gcctatcgcg gcctatgcgg tgtgaaatac 13860
   cgcacagatg cgtaaggaga aaataccgca tcaggcgctc ttccgcttcc tcgctcactg 13920
   actcgctgcg ctcggtcgtt cggctgcggc gagcggtatc agctcactca aaggcggtaa 13980
   tacggttatc cacagaatca ggggataacg caggaaagaa catgtgagca aaaggccagc 14040
   aaaaggccag gaaccgtaaa aaggccgcgt tgctggcgtt tttccatagg ctccgccccc 14100
   ctgacgagca tcacaaaaat cgacgctcaa gtcagaggtg gcgaaacccg acaggactat 14160
   aaagatacca ggcgtttccc cctggaagct ccctcgtgcg ctctcctgtt ccgaccctgc 14220
   cgcttaccgg atacctgtcc gcctttctcc cttcgggaag cgtggcgctt tctcatagct 14280
   cacgctgtag gtatctcagt tcggtgtagg tcgttcgctc caagctgggc tgtgtgcacg 14340
   aaccccccgt tcagcccgac cgctgcgcct tatccggtaa ctatcgtctt gagtccaacc 14400
   cggtaagaca cgacttatcg ccactggcag cagccactgg taacaggatt agcagagcga 14460
   ggtatgtagg cggtgctaca gagttcttga agtggtggcc taactacggc tacactagaa 14520
   ggacagtatt tggtatctgc gctctgctga agccagttac cttcggaaaa agagttggta 14580
   gctcttgatc cggcaaacaa accaccgctg gtagcggtgg tttttttgtt tgcaagcagc 14640
   agattacgcg cagaaaaaaa ggatctcaag aagatccttt gatcttttct acggggtcct 14700
   tcaactcatc gatagtttgg ctgtgagcaa ttatgtgctt agtgcatcta acgcttgagt 14760
   taagccgcgc cgcgaagcgg cgtcggcttg aacgaatttc tagctagaca ttatttgcca 14820
   acgaccttcg tgatctcgcc cttgacatag tggacaaatt cttcgagctg gtcggcccgg 14880
   gacgcgagac ggtcttcttc ttggcccaga taggcttggc gcgcttcgag gatcacgggc 14940
   tggtattgcg ccggaaggcg ctccatcgcc cagtcggcgg cgacatcctt cggcgcgatc 15000
   ttgccggtaa ccgccgagta ccaaatccgg ctcagcgtaa ggaccacatt gcgctcatcg 15060
   cccgcccaat ccggcgggga gttccacagg gtcagcgtct cgttcagtgc ttcgaacaga 15120
   tcctgttccg gcaccgggtc gaaaagttcc tcggccgcgg ggccgacgag ggccacgcta 15180
   tgctcccggg ccttggtgag caggatcgcc agatcaatgt cgatggtggc cggttcaaag 15240
   atacccgcca gaatatcatt acgctgccat tcgccgaact ggagttcgcg tttggccgga 15300
   tagcgccagg ggatgatgtc atcgtgcacc acaatcgtca cctcaaccgc gcgcaggatt 15360
   tcgctctcgc cgggggaggc ggacgtttcc agaaggtcgt tgataagcgc gcggcgcgtg 15420
   gtctcgtcga gacggacggt aacggtgaca agcaggtcga tgtccgaatg gggcttaagg 15480
   ccgccgtcaa cggcgctacc atacagatgc acggcgagga gggtcggttc gaggtggcgc 15540
   tcgatgacac ccacgacttc cgacagctgg gtggacacct cggcgatgac cgcttcaccc 15600
   atgatgttta actttgtttt agggcgactg ccctgctgcg taacatcgtt gctgctccat 15660
   aacatcaaac atcgacccac ggcgtaacgc gcttgctgct tggatgcccg aggcatagac 15720
   tgtaccccaa aaaaacagtc ataacaagcc atgaaaaccg ccactgcgtt ccatgaatat 15780
   tcaaacaaac acatacagcg cgacttatca tggatattga catacaaatg gacgaacgga 15840
   taaacctttt cacgcccttt taaatatccg attattctaa taaacgctct tttctcttag 15900
   gtttacccgc caatatatcc tgtcaaacac tgatagttta aactgaaggc gggaaacgac 15960
   aatctgatca ctgattagta actaaggcct ttaattaatc tagaggcgcg ccgggccccc 16020
   tgcagggagc tcggccggcc aatttaaatt gatatcggta catcgattac gccaagctat 16080
   caactttgta tag 16093

<210> 184
   <211> 16097
   <212> DNA
   <213> Artificial
<220>
   <223> Plasmid
<400> 184
   aaaagttgcc atgattacgc caagcttggc cactaaggcc aatttaaatc tactaggccg 60
   gccaaagtag gcgcctacta ccggtaattc ccgggattag cggccgctag tctgtgcgca 120
   cttgtatcct gcaggtcaat cgtttaaaca ctgtacggac cgtggcctaa taggccggta 180
   cccaagtttg tacaaaaaag caggctccat gattacgcca agcttggcca ctaaggccaa 240
   tttaaatcta ctaggccggc catcgacggc ccggactgta tccaacttct gatctttgaa    300
   tctctctgtt ccaacatgtt ctgaaggagt tctaagactt ttcagaaagc ttgtaacatg    360
   ctttgtagac tttctttgaa ttactcttgc aaactctgat tgaacctacg tgaaaactgc    420
   tccagaagtt ctaaccaaat tccgtcttgg gaaggcccaa aatttattga gtacttcagt    480
   ttcatggacg tgtcttcaaa gatttataac ttgaaatccc atcattttta agagaagttc    540
   tgttccgcaa tgtcttagat ctcattgaaa tctacaactc ttgtgtcaga agttcttcca    600
   gaatcaactt gcatcatggt gaaaatctgg ccagaagttc tgaacttgtc atatttctta    660
   acagttagaa aaatttctaa gtgtttagaa ttttgacttt tccaaagcaa acttgacttt    720
   tgactttctt aataaaacaa acttcatatt ctaacatgtc ttgatgaaat gtgattcttg    780
   aaatttgatg ttgatgcaaa agtcaaagtt tgacttttca gtgtgcaatt gaccattttg    840
   ctcttgtgcc aattccaaac ctaaattgat gtatcagtgc tgcaaacttg atgtcatgga    900
   agatcttatg agaaaattct tgaagactga gaggaaaaat tttgtagtac aacacaaaga    960
   atcctgtttt tcatagtcgg actagacaca ttaacataaa acaccacttc attcgaagag   1020
   tgattgaaga aggaaatgtg cagttacctt tctgcagttc ataagagcaa cttacagaca   1080
   cttttactaa aatactacaa agaggaagat tttaacaact tagagaagta atgggagtta   1140
   aagagcaaca cattaagggg gagtgttaaa attaatgtgt tgtaaccacc actaccttta   1200
   gtaagtatta taagaaaatt gtaatcatca cattataatt attgtcctta tttaaaatta   1260
   tgataaagtt gtatcattaa gattgagaaa accaaatagt cctcgtcttg atttttgaat   1320
   tattgttttc tatgttactt ttcttcaagc ctatataaaa actttgtaat gctaaattgt   1380
   atgctggaaa aaaatgtgta atgaattgaa tagaaattat ggtatttcaa agtccaaaat   1440
   ccatcaatag aaatttagta caaaacgtaa ctcaaaaata ttctcttatt ttaaatttta   1500
   caacaatata aaaatattct cttattttaa attttacaat aatataattt atcacctgtc   1560
   acctttagaa taccaccaac aatattaata cttagatatt ttattcttaa taattttgag   1620
   atctctcaat atatctgata tttattttat atttgtgtca tattttctta tgttttagag   1680
   ttaaccctta tatcttggtc aaactagtaa ttcaatatat gagtttgtga aggacacatt   1740
   gacatcttga aacattggtt ttaaccttgt tggaatgtta aaggtaataa aacattcaga   1800
   attatgacca tctattaata tacttccttt gtcttttaaa aaagtgtgca tgaaaatgct   1860
   ctatggtaag ctagagtgtc ttgctggcct gtgtatatca attccatttc cagatggtag   1920
   aaactgccac tacgaataat tagtcataag acacgtatgt taacacacgt ccccttgcat   1980
   gttttttgcc atatattccg tctctttctt tttcttcacg tataaaacaa tgaactaatt   2040
   aatagagcga tcaagctgaa ccatggctac tagacaaaga acttccacta ctgtggttgt   2100
   ggagaagcca tctgctaagg ttacattgga gccacaacaa caaccacagt tcccagatat   2160
   caagaccatc aaggatgcta ttccagctca ctgcttccaa ccatccctct tcacttcctt   2220
   ctactacgtg ttcagggatt tcgctatggt tgctactctt gtgtgggctg ctttgactta   2280
   cattccagct atcccagatc aaagactcag agtggctgct tggatggttt acggattcgt   2340
   gcaaggattg gtgtgtactg gagtgtggat tttgggacac gagtgtggac acggagcttt 2400
   ctctacccac ggaaagttga acaacgttgt gggatggttc ttgcactctt tcctcctcgt 2460
   gccatacttc tcttggaagt actcacacca cagacatcac agattcaccg gacacatgga 2520
   tttggatatg gctttcgtgc cagctactca acctaagaag cactctatcc tcgctggaat 2580
   cgatttgaac gagttgttcg aggatactcc aatcgctcaa ctcatcagga ttgtgttcca 2640
   ccaactcttc ggatggcaag tgtacttgct cttcaacgct tctgctggaa agggatctaa 2700
   gcaatgggag ccaactggat tggctaagtg gttcagagtg tctcacttcg agccaacctc 2760
   tgctgttttc agaccatccg aggctatctt catcttcatc tccgatttgg gattggctat 2820
   taccttcacc gctctctact tcgcttctaa ggctgttgga acttctaccg tgttgttctt 2880
   gtacgctgtg ccttatttct gggtgcacca ctggttggtt gctatcactt acttgcacca 2940
   ccaccacact gaggtgccac actacactaa cgagggatgg acttacgtta agggagcttt 3000
   ggctactgtt gatagggagt tcggattcat cggaaagcac ctcttccacg gaattattga 3060
   gaagcacgtg gttcaccact tgttcccaag aatcccattc tacaaggctg atgaggctac 3120
   tgaggctatt aagccattgc tcggagatct ctactaccac gatgagagga acttcatcgg 3180
   acaactctgg tctgttttcg gttctctcaa gtacgttgag cacgatccaa ctaaccaggg 3240
   agctatgaga tgggctaagg agtgatgatt aattaaggcc gcctcgaccg taccccctgc 3300
   agatagacta tactatgttt tagcctgcct gctggctagc tactatgtta tgttatgttg 3360
   taaaataaac acctgctaag gtatatctat ctatatttta gcatggcttt ctcaataaat 3420
   tgtctttcct tatcgtttac tatcttatac ctaataatga aataataata tcacatatga 3480
   ggaacggggc aggtttaggc atatatatac gagtgtaggg cggagtgggg ggcgcctact 3540
   accggtaatt cccgggatta gcggccgcta gtctgtgcgc acttgtatcc tgcaggtcaa 3600
   tcgtttaaac actgtacgga ccgtggccta ataggccggt accacccagc tttcttgtac 3660
   aaagtggcca tgattacgcc aagctctcca ccgcggtggc ggccgctcta gcccaagctt 3720
   taaggatgac ctacccattc ttgagacaaa tgttacattt tagtatcaga gtaaaatgtg 3780
   tacctataac tcaaattcga ttgacatgta tccattcaac ataaaattaa accagcctgc 3840
   acctgcatcc acatttcaag tattttcaaa ccgttcggct cctatccacc gggtgtaaca 3900
   agacggattc cgaatttgga agattttgac tcaaattccc aatttatatt gaccgtgact 3960
   aaatcaactt taacttctat aattctgatt aagctcccaa tttatattcc caacggcact 4020
   acctccaaaa tttatagact ctcatcccct tttaaaccaa cttagtaaac gttttttttt 4080
   taattttatg aagttaagtt tttaccttgt ttttaaaaag aatcgttcat aagatgccat 4140
   gccagaacat tagctacacg ttacacatag catgcagccg cggagaattg tttttcttcg 4200
   ccacttgtca ctcccttcaa acacctaaga gcttctctct cacagcacac acatacaatc 4260
   acatgcgtgc atgcattatt acacgtgatc gccatgcaaa tctcctttat agcctataaa 4320
   ttaactcatc ggcttcactc tttactcaaa ccaaaactca tcaatacaaa caagattaaa 4380
   aacataaggc gcgccaattg actagtaggc ctatcgatta ggagaataac aatggtgcgc 4440
   tcctccaaga acgtcatcaa ggagttcatg cgcttcaagg tgcgcatgga gggcaccgtg 4500
   aacggccacg agttcgagat cgagggcgag ggcgagggcc gcccctacga gggccacaac 4560
   accgtgaagc tgaaggtgac caagggcggc cccctgccct tcgcctggga catcctgtcc 4620
   ccccagttcc agtacggctc caaggtgtac gtgaagcacc ccgccgacat ccccgactac 4680
   aagaagctgt ccttccccga gggcttcaag tgggagcgcg tgatgaactt cgaggacggc 4740
   ggcgtggtga ccgtgaccca ggactcctcc ctgcaggacg gctgcttcat ctacaaggtg 4800
   aagttcatcg gcgtgaactt cccctccgac ggccccgtaa tgcagaagaa gaccatgggc 4860
   tgggaggcct ccaccgagcg cctgtacccc cgcgacggcg tgctgaaggg cgagatccac 4920
   aaggccctga agctgaagga cggcggccac tacctggtgg agttcaagtc catctacatg 4980
   gccaagaagc ccgtgcagct gcccggctac tactacgtgg actccaagct ggacatcacc 5040
   tcccacaacg aggactacac catcgtggag cagtacgagc gcaccgaggg ccgccaccac 5100
   ctgttcctgc tcgagtctag aggtaccggt tgttaacgtt agccggctac gtatactccg 5160
   gaatattaat aggcctagga tgcatatggc ggccgcctgc agctggcgcc atcgattaat 5220
   taaggccgcc tcgagcatgc atctagaggg cccgctagcg ttaaccctgc tttaatgaga 5280
   tatgcgagac gcctatgatc gcatgatatt tgctttcaat tctgttgtgc acgttgtaaa 5340
   aaacctgagc atgtgtagct cagatcctta ccgccggttt cggttcattc taatgaatat 5400
   atcacccgtt actatcgtat ttttatgaat aatattctcc gttcaattta ctgattgtcc 5460
   gtcgagcata tgctagagga tccccgggta cccaacttta ttatacatag ttgataattc 5520
   actggccgga tgtaccgaat tcgcggccgc aagcttgtac actagtacgc gtcaattggc 5580
   gatcgcggat ctgagatgaa accggtgatt atcagaacct tttatggtct ttgtatgcat 5640
   atggtaaaaa aacttagttt gcaatttcct gtttgttttg gtaatttgag tttcttttag 5700
   ttgttgatct gcctgctttt tggtttacgt cagactacta ctgctgttgt tgtttggttt 5760
   cctttctttc attttataaa taaataatcc ggttcggttt actccttgtg actggctcag 5820
   tttggttatt gcgaaatgcg aatggtaaat tgagtaattg aaattcgtta ttagggttct 5880
   aagctgtttt aacagtcact gggttaatat ctctcgaatc ttgcatggaa aatgctctta 5940
   ccattggttt ttaattgaaa tgtgctcata tgggccgtgg tttccaaatt aaataaaact 6000
   acgatgtcat cgagaagtaa aatcaactgt gtccacatta tcagttttgt gtatacgatg 6060
   aaatagggta attcaaaatc tagcttgata tgccttttgg ttcattttaa ccttctgtaa 6120
   acattttttc agattttgaa caagtaaatc caaaaaaaaa aaaaaaaaat ctcaactcaa 6180
   cactaaatta ttttaatgta taaaagatgc ttaaaacatt tggcttaaaa gaaagaagct 6240
   aaaaacatag agaactcttg taaattgaag tatgaaaata tactgaattg ggtattatat 6300
   gaatttttct gatttaggat tcacatgatc caaaaaggaa atccagaagc actaatcaga 6360
   cattggaagt aggaatattt caaaaagttt ttttttttta agtaagtgac aaaagctttt 6420
   aaaaaataga aaagaaacta gtattaaagt tgtaaattta ataaacaaaa gaaatttttt 6480
   atattttttc atttcttttt ccagcatgag gttatgatgg caggatgtgg atttcatttt 6540
   tttccttttg atagcctttt aattgatcta ttataattga cgaaaaaata ttagttaatt 6600
   atagatatat tttaggtagt attagcaatt tacacttcca aaagactatg taagttgtaa 6660
   atatgatgcg ttgatctctt catcattcaa tggttagtca aaaaaataaa agcttaacta 6720
   gtaaactaaa gtagtcaaaa attgtacttt agtttaaaat attacatgaa taatccaaaa 6780
   cgacatttat gtgaaacaaa aacaatatag atccattacc ctgttatccc tagaggggaa 6840
   aattcgaatc caaaaattac ggatatgaat ataggcatat ccgtatccga attatccgtt 6900
   tgacagctag caacgattgt acaattgctt ctttaaaaaa ggaagaaaga aagaaagaaa 6960
   agaatcaaca tcagcgttaa caaacggccc cgttacggcc caaacggtca tatagagtaa 7020
   cggcgttaag cgttgaaaga ctcctatcga aatacgtaac cgcaaacgtg tcatagtcag 7080
   atcccctctt ccttcaccgc ctcaaacaca aaaataatct tctacagcct atatatacaa 7140
   cccccccttc tatctctcct ttctcacaat tcatcatctt tctttctcta cccccaattt 7200
   taagaaatcc tctcttctcc tcttcatttt caaggtaaat ctctctctct ctctctctct 7260
   ctgttattcc ttgttttaat taggtatgta ttattgctag tttgttaatc tgcttatctt 7320
   atgtatgcct tatgtgaata tctttatctt gttcatctca tccgtttaga agctataaat 7380
   ttgttgattt gactgtgtat ctacacgtgg ttatgtttat atctaatcag atatgaattt 7440
   cttcatattg ttgcgtttgt gtgtaccaat ccgaaatcgt tgattttttt catttaatcg 7500
   tgtagctaat tgtacgtata catatggatc tacgtatcaa ttgttcatct gtttgtgttt 7560
   gtatgtatac agatctgaaa acatcacttc tctcatctga ttgtgttgtt acatacatag 7620
   atatagatct gttatatcat tttttttatt aattgtgtat atatatatgt gcatagatct 7680
   ggattacatg attgtgatta tttacatgat tttgttattt acgtatgtat atatgtagat 7740
   ctggactttt tggagttgtt gacttgattg tatttgtgtg tgtatatgtg tgttctgatc 7800
   ttgatatgtt atgtatgtgc agctgaacca tggcggcggc aacaacaaca acaacaacat 7860
   cttcttcgat ctccttctcc accaaaccat ctccttcctc ctccaaatca ccattaccaa 7920
   tctccagatt ctccctccca ttctccctaa accccaacaa atcatcctcc tcctcccgcc 7980
   gccgcggtat caaatccagc tctccctcct ccatctccgc cgtgctcaac acaaccacca 8040
   atgtcacaac cactccctct ccaaccaaac ctaccaaacc cgaaacattc atctcccgat 8100
   tcgctccaga tcaaccccgc aaaggcgctg atatcctcgt cgaagcttta gaacgtcaag 8160
   gcgtagaaac cgtattcgct taccctggag gtacatcaat ggagattcac caagccttaa 8220
   cccgctcttc ctcaatccgt aacgtccttc ctcgtcacga acaaggaggt gtattcgcag 8280
   cagaaggata cgctcgatcc tcaggtaaac caggtatctg tatagccact tcaggtcccg 8340
   gagctacaaa tctcgttagc ggattagccg atgcgttgtt agatagtgtt cctcttgtag 8400
   caatcacagg acaagtccct cgtcgtatga ttggtacaga tgcgtttcaa gagactccga 8460
   ttgttgaggt aacgcgttcg attacgaagc ataactatct tgtgatggat gttgaagata 8520
   tccctaggat tattgaggaa gctttctttt tagctacttc tggtagacct ggacctgttt 8580
   tggttgatgt tcctaaagat attcaacaac agcttgcgat tcctaattgg gaacaggcta 8640
   tgagattacc tggttatatg tctaggatgc ctaaacctcc ggaagattct catttggagc 8700
   agattgttag gttgatttct gagtctaaga agcctgtgtt gtatgttggt ggtggttgtt 8760
   tgaattctag cgatgaattg ggtaggtttg ttgagcttac ggggatccct gttgcgagta 8820
   cgttgatggg gctgggatct tatccttgtg atgatgagtt gtcgttacat atgcttggaa 8880
   tgcatgggac tgtgtatgca aattacgctg tggagcatag tgatttgttg ttggcgtttg 8940
   gggtaaggtt tgatgatcgt gtcacgggta agcttgaggc ttttgctagt agggctaaga 9000
   ttgttcatat tgatattgac tcggctgaga ttgggaagaa taagactcct catgtgtctg 9060
   tgtgtggtga tgttaagctg gctttgcaag ggatgaataa ggttcttgag aaccgagcgg 9120
   aggagcttaa gcttgatttt ggagtttgga ggaatgagtt gaacgtacag aaacagaagt 9180
   ttccgttgag ctttaagacg tttggggaag ctattcctcc acagtatgcg attaaggtcc 9240
   ttgatgagtt gactgatgga aaagccataa taagtactgg tgtcgggcaa catcaaatgt 9300
   gggcggcgca gttctacaat tacaagaaac caaggcagtg gctatcatca ggaggccttg 9360
   gagctatggg atttggactt cctgctgcga ttggagcgtc tgttgctaac cctgatgcga 9420
   tagttgtgga tattgacgga gatggaagct ttataatgaa tgtgcaagag ctagccacta 9480
   ttcgtgtaga gaatcttcca gtgaaggtac ttttattaaa caaccagcat cttggcatgg 9540
   ttatgcaatg ggaagatcgg ttctacaaag ctaaccgagc tcacacattt ctcggggatc 9600
   cggctcagga ggacgagata ttcccgaaca tgttgctgtt tgcagcagct tgcgggattc 9660
   cagcggcgag ggtgacaaag aaagcagatc tccgagaagc tattcagaca atgctggata 9720
   caccaggacc ttacctgttg gatgtgattt gtccgcacca agaacatgtg ttgccgatga 9780
   tcccgaatgg tggcactttc aacgatgtca taacggaagg agatggccgg attaaatact 9840
   gatagggata acagggtaat ctcgacgaga tgaaaccggt gattatcaga accttttatg 9900
   gtctttgtat gcatatggta aaaaaactta gtttgcaatt tcctgtttgt tttggtaatt 9960
   tgagtttctt ttagttgttg atctgcctgc tttttggttt acgtcagact actactgctg 10020
   ttgttgtttg gtttcctttc tttcatttta taaataaata atccggttcg gtttactcct 10080
   tgtgactggc tcagtttggt tattgcgaaa tgcgaatggt aaattgagta attgaaattc 10140
   gttattaggg ttctaagctg ttttaacagt cactgggtta atatctctcg aatcttgcat 10200
   ggaaaatgct cttaccattg gtttttaatt gaaatgtgct catatgggcc gtggtttcca 10260
   aattaaataa aactacgatg tcatcgagaa gtaaaatcaa ctgtgtccac attatcagtt 10320
   ttgtgtatac gatgaaatag ggtaattcaa aatctagctt gatatgcctt ttggttcatt 10380
   ttaaccttct gtaaacattt tttcagattt tgaacaagta aatccaaaaa aaaaaaaaaa 10440
   aaatctcaac tcaacactaa attattttaa tgtataaaag atgcttaaaa catttggctt 10500
   aaaagaaaga agctaaaaac atagagaact cttgtaaatt gaagtatgaa aatatactga 10560
   attgggtatt atatgaattt ttctgattta ggattcacat gatccaaaaa ggaaatccag 10620
   aagcactaat cagacattgg aagtaggaat atttcaaaaa gttttttttt tttaagtaag 10680
   tgacaaaagc ttttaaaaaa tagaaaagaa actagtatta aagttgtaaa tttaataaac 10740
   aaaagaaatt ttttatattt tttcatttct ttttccagca tgaggttatg atggcaggat 10800
   gtggatttca tttttttcct tttgatagcc ttttaattga tctattataa ttgacgaaaa 10860
   aatattagtt aattatagat atattttagg tagtattagc aatttacact tccaaaagac 10920
   tatgtaagtt gtaaatatga tgcgttgatc tcttcatcat tcaatggtta gtcaaaaaaa 10980
   taaaagctta actagtaaac taaagtagtc aaaaattgta ctttagttta aaatattaca 11040
   tgaataatcc aaaacgacat ttatgtgaaa caaaaacaat atgtcgaggc gatcgcagta 11100
   cttaatcagt gatcagtaac taaattcagt acattaaaga cgtccgcaat gtgttattaa 11160
   gttgtctaag cgtcaatttg tttacaccac aatatatcct gccaccagcc agccaacagc 11220
   tccccgaccg gcagctcggc acaaaatcac tgatcatcta aaaaggtgat gtgtatttga 11280
   gtaaaacagc ttgcgtcatg cggtcgctgc gtatatgatg cgatgagtaa ataaacaaat 11340
   acgcaagggg aacgcatgaa ggttatcgct gtacttaacc agaaaggcgg gtcaggcaag 11400
   acgaccatcg caacccatct agcccgcgcc ctgcaactcg ccggggccga tgttctgtta 11460
   gtcgattccg atccccaggg cagtgcccgc gattgggcgg ccgtgcggga agatcaaccg 11520
   ctaaccgttg tcggcatcga ccgcccgacg attgaccgcg acgtgaaggc catcggccgg 11580
   cgcgacttcg tagtgatcga cggagcgccc caggcggcgg acttggctgt gtccgcgatc 11640
   aaggcagccg acttcgtgct gattccggtg cagccaagcc cttacgacat ttgggccacc 11700
   gccgacctgg tggagctggt taagcagcgc attgaggtca cggatggaag gctacaagcg 11760
   gcctttgtcg tgtcgcgggc gatcaaaggc acgcgcatcg gcggtgaggt tgccgaggcg 11820
   ctggccgggt acgagctgcc cattcttgag tcccgtatca cgcagcgcgt gagctaccca 11880
   ggcactgccg ccgccggcac aaccgttctt gaatcagaac ccgagggcga cgctgcccgc 11940
   gaggtccagg cgctggccgc tgaaattaaa tcaaaactca tttgagttaa tgaggtaaag 12000
   agaaaatgag caaaagcaca aacacgctaa gtgccggccg tccgagcgca cgcagcagca 12060
   aggctgcaac gttggccagc ctggcagaca cgccagccat gaagcgggtc aactttcagt 12120
   tgccggcgga ggatcacacc aagctgaaga tgtacgcggt acgccaaggc aagaccatta 12180
   ccgagctgct atctgaatac atcgcgcagc taccagagta aatgagcaaa tgaataaatg 12240
   agtagatgaa ttttagcggc taaaggaggc ggcatggaaa atcaagaaca accaggcacc 12300
   gacgccgtgg aatgccccat gtgtggagga acgggcggtt ggccaggcgt aagcggctgg 12360
   gttgtctgcc ggccctgcaa tggcactgga acccccaagc ccgaggaatc ggcgtgagcg 12420
   gtcgcaaacc atccggcccg gtacaaatcg gcgcggcgct gggtgatgac ctggtggaga 12480
   agttgaaggc cgcgcaggcc gcccagcggc aacgcatcga ggcagaagca cgccccggtg 12540
   aatcgtggca aggggccgct gatcgaatcc gcaaagaatc ccggcaaccg ccggcagccg 12600
   gtgcgccgtc gattaggaag ccgcccaagg gcgacgagca accagatttt ttcgttccga 12660
   tgctctatga cgtgggcacc cgcgatagtc gcagcatcat ggacgtggcc gttttccgtc 12720
   tgtcgaagcg tgaccgacga gctggcgagg tgatccgcta cgagcttcca gacgggcacg 12780
   tagaggtttc cgcaggcccc gccggcatgg ccagtgtgtg ggattacgac ctggtactga 12840
   tggcggtttc ccatctaacc gaatccatga accgataccg ggaagggaag ggagacaagc 12900
   ccggccgcgt gttccgtcca cacgttgcgg acgtactcaa gttctgccgg cgagccgatg 12960
   gcggaaagca gaaagacgac ctggtagaaa cctgcattcg gttaaacacc acgcacgttg 13020
   ccatgcagcg taccaagaag gccaagaacg gccgcctggt gacggtatcc gagggtgaag 13080
   ccttgattag ccgctacaag atcgtaaaga gcgaaaccgg gcggccggag tacatcgaga 13140
   tcgagcttgc tgattggatg taccgcgaga tcacagaagg caagaacccg gacgtgctga 13200
   cggttcaccc cgattacttt ttgatcgacc ccggcatcgg ccgttttctc taccgcctgg 13260
   cacgccgcgc cgcaggcaag gcagaagcca gatggttgtt caagacgatc tacgaacgca 13320
   gtggcagcgc cggagagttc aagaagttct gtttcaccgt gcgcaagctg atcgggtcaa 13380
   atgacctgcc ggagtacgat ttgaaggagg aggcggggca ggctggcccg atcctagtca 13440
   tgcgctaccg caacctgatc gagggcgaag catccgccgg ttcctaatgt acggagcaga 13500
   tgctagggca aattgcccta gcaggggaaa aaggtcgaaa aggtctcttt cctgtggata 13560
   gcacgtacat tgggaaccca aagccgtaca ttgggaaccg gaacccgtac attgggaacc 13620
   caaagccgta cattgggaac cggtcacaca tgtaagtgac tgatataaaa gagaaaaaag 13680
   gcgatttttc cgcctaaaac tctttaaaac ttattaaaac tcttaaaacc cgcctggcct 13740
   gtgcataact gtctggccag cgcacagccg aagagctgca aaaagcgcct acccttcggt 13800
   cgctgcgctc cctacgcccc gccgcttcgc gtcggcctat cgcggcctat gcggtgtgaa 13860
   ataccgcaca gatgcgtaag gagaaaatac cgcatcaggc gctcttccgc ttcctcgctc 13920
   actgactcgc tgcgctcggt cgttcggctg cggcgagcgg tatcagctca ctcaaaggcg 13980
   gtaatacggt tatccacaga atcaggggat aacgcaggaa agaacatgtg agcaaaaggc 14040
   cagcaaaagg ccaggaaccg taaaaaggcc gcgttgctgg cgtttttcca taggctccgc 14100
   ccccctgacg agcatcacaa aaatcgacgc tcaagtcaga ggtggcgaaa cccgacagga 14160
   ctataaagat accaggcgtt tccccctgga agctccctcg tgcgctctcc tgttccgacc 14220
   ctgccgctta ccggatacct gtccgccttt ctcccttcgg gaagcgtggc gctttctcat 14280
   agctcacgct gtaggtatct cagttcggtg taggtcgttc gctccaagct gggctgtgtg 14340
   cacgaacccc ccgttcagcc cgaccgctgc gccttatccg gtaactatcg tcttgagtcc 14400
   aacccggtaa gacacgactt atcgccactg gcagcagcca ctggtaacag gattagcaga 14460
   gcgaggtatg taggcggtgc tacagagttc ttgaagtggt ggcctaacta cggctacact 14520
   agaaggacag tatttggtat ctgcgctctg ctgaagccag ttaccttcgg aaaaagagtt 14580
   ggtagctctt gatccggcaa acaaaccacc gctggtagcg gtggtttttt tgtttgcaag 14640
   cagcagatta cgcgcagaaa aaaaggatct caagaagatc ctttgatctt ttctacgggg 14700
   tccttcaact catcgatagt ttggctgtga gcaattatgt gcttagtgca tctaacgctt 14760
   gagttaagcc gcgccgcgaa gcggcgtcgg cttgaacgaa tttctagcta gacattattt  14820
   gccaacgacc ttcgtgatct cgcccttgac atagtggaca aattcttcga gctggtcggc  14880
   ccgggacgcg agacggtctt cttcttggcc cagataggct tggcgcgctt cgaggatcac  14940
   gggctggtat tgcgccggaa ggcgctccat cgcccagtcg gcggcgacat ccttcggcgc  15000
   gatcttgccg gtaaccgccg agtaccaaat ccggctcagc gtaaggacca cattgcgctc  15060
   atcgcccgcc caatccggcg gggagttcca cagggtcagc gtctcgttca gtgcttcgaa  15120
   cagatcctgt tccggcaccg ggtcgaaaag ttcctcggcc gcggggccga cgagggccac  15180
   gctatgctcc cgggccttgg tgagcaggat cgccagatca atgtcgatgg tggccggttc  15240
   aaagataccc gccagaatat cattacgctg ccattcgccg aactggagtt cgcgtttggc  15300
   cggatagcgc caggggatga tgtcatcgtg caccacaatc gtcacctcaa ccgcgcgcag  15360
   gatttcgctc tcgccggggg aggcggacgt ttccagaagg tcgttgataa gcgcgcggcg  15420
   cgtggtctcg tcgagacgga cggtaacggt gacaagcagg tcgatgtccg aatggggctt  15480
   aaggccgccg tcaacggcgc taccatacag atgcacggcg aggagggtcg gttcgaggtg  15540
   gcgctcgatg acacccacga cttccgacag ctgggtggac acctcggcga tgaccgcttc  15600
   acccatgatg tttaactttg ttttagggcg actgccctgc tgcgtaacat cgttgctgct  15660
   ccataacatc aaacatcgac ccacggcgta acgcgcttgc tgcttggatg cccgaggcat  15720
   agactgtacc ccaaaaaaac agtcataaca agccatgaaa accgccactg cgttccatga  15780
   atattcaaac aaacacatac agcgcgactt atcatggata ttgacataca aatggacgaa  15840
   cggataaacc ttttcacgcc cttttaaata tccgattatt ctaataaacg ctcttttctc  15900
   ttaggtttac ccgccaatat atcctgtcaa acactgatag tttaaactga aggcgggaaa  15960
   cgacaatctg atcactgatt agtaactaag gcctttaatt aatctagagg cgcgccgggc  16020
   cccctgcagg gagctcggcc ggccaattta aattgatatc ggtacatcga ttacgccaag  16080
   ctatcaactt tgtatag                                                 16097

## Beschreibung

Die vorliegende Erfindung betrifft Polynukleotide aus Helobdella robusta, Laccaria bicolor, Lottia gigantea, Microcoleus chthonoplastes, Monosiga brevicollis, Mycosphaerella fijiensis, Mycospaerella graminicola, Naegleria gruberi, Nectria haematococca, Nematostella vectensis, Phycomyces blakesleeanus, Physcomitrella patens, Postia placenta, Selaginella moellendorffii, Microdochium nivale und Trichoderma resii, die Desaturasen kodieren und zur rekombinanten Herstellung von mehrfach ungesättigten Fettsäuren eingesetzt werden können. Weiterhin betrifft die Erfindung Vektoren, Wirtszellen und transgene nicht-humane Organismen, die die erfindungsgemäßen Polynukleotide enthalten, sowie die von den Polynukleotiden kodierten Polypeptide. Die Erfindung betrifft zudem Antikörper gegen die erfindungsgemäßen Polypeptide. Schließlich betrifft die Erfindung noch Herstellungsverfahren für die mehrfach ungesättigten Fettsäuren und für Öl-, Lipid- und Fettsäurezusammensetzungen und deren Verwendung als Arzneimittel, Kosmetik, Nahrungsmittel, Futtermittel, vorzugsweise Fischfutter oder Nahrungsergänzungsmittel.

Fettsäuren und Triacylglyceride haben eine Vielzahl von Anwendungen in der Lebensmittelindustrie, der Tierernährung, der Kosmetik und im Pharmabereich. Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfach ungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar.

Mehrfach ungesättigte langkettige ω-3-Fettsäuren wie Eicosapentaensäure (= EPA, C20:5^{Δ5,8,11,14,17}) oder Docosahexaensäure (= DHA, C22:6^{Δ4,7,10,13,16,19}) sind wichtige Komponenten der menschlichen Ernährung aufgrund ihrer verschiedenen Rollen in der Gesundheit, die Aspekte wie die Entwicklung des kindlichen Gehirns, die Funktionalität des Auges, die Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, C22:6^{Δ4,7,10,13,16,19}) oder Eisosapentaensäure (= EPA, C20:5^{Δ5,8,11,14,17}) der Babynahrung zur Erhöhung des Nährwertes zugesetzt. Der ungesättigten Fettsäure DHA wird dabei ein positiver Effekt in Bezug auf die Entwicklung und Aufrechterhaltung von Gehirnfunktionen zugeschrieben.
Im folgenden werden mehrfach ungesättigte Fettsäuren als PUFA, PUFAs, LCPUFA oder LCPUFAs bezeichnet (poly unsaturated fatty acids, PUFA, mehrfach ungesättigte Fettsäuren; long chain poly unsaturated fatty acids, LCPUFA, langkettige mehrfach ungesättigte Fettsäuren).

Hauptsächlich werden die verschiedenen Fettsäuren und Triglyceride aus Mikroorganismen wie Mortierella oder Schizochytrium oder aus ÖI-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, C20:4^{Δ5,8,11,14}), Dihomo-γ-linolensäure (C20:3^{Δ8,11,14}) oder Docosapentaensäure (DPA, C22:5^{Δ7,10,13,16,19}) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch, oder Algen.

Je nach Anwendungszweck werden Öle mit gesättigten oder ungesättigten Fettsäuren bevorzugt. So werden z.B. in der humanen Ernährung Lipide mit ungesättigten Fettsäuren, speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche, speziell chronisch entzündliche Prozesse im Rahmen immunologischer Erkrankungen wie rheumatoider Arthritis lassen sich durch ω-3-Fettsäuren positiv beeinflussen. Sie werden deshalb speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.
ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, sowie den Thromoxanen und Leukotrienen, die sich von der Arachidonsäure und der Eicosapentaensäure ableiten. Eicosanoide (sog. PG₂-Serie), die aus ω-6-Fettsäuren gebildet werden, fördern in der Regel Entzündungsreaktionen, während Eicosanoide (sog. PG₃-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase und in WO 94/11516 eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben. Deren Anwendung zur Produktion in transgenen Organismen wird beispielsweise in WO98/46763 WO98/46764 oder WO98/46765 beschrieben. Dabei wird auch die Expression verschiedener Desaturasen und die Bildung polyungesättigter Fettsäuren beschrieben und beansprucht; siehe z.B. WO99/64616 oder WO98/46776. Bezüglich der Effektivität der Expression von Desaturasen und ihrem Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus w-3- und ω-6-Fettsäuren erhalten.

Besonders geeignete Mikroorganismen zur Herstellung von PUFAs sind Mikroorganismen, beispielsweise Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moose wie Physcomitrella, bevorzugt Physcomitrella patens, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Zudem fallen diese in der Regel je nach verwendetem Mikroorganismus als Fettsäuregemische aus beispielsweise EPA, DPA und ARA an.

Für die Synthese von Arachidonsäure, Eicosapentaensäure (EPA) und Docosahexaensäure (DHA) werden verschiedene Synthesewege diskutiert. So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Eine alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des in Zank et al. und in Sakuradani et al. beschriebenen Weges über Δ6-Desaturase, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein weiterer Elongationsschritt auf C₂₄, eine weitere Δ6-Desaturierung und abschliessend eine β-Oxidation auf die C₂₂-Kettenlänge. Für die Herstellung in Pflanzen und Mikroorganismen ist der sogenannte Sprecher-Syntheseweg allerdings nicht geeignet, da die Regulationsmechanismen bisher nicht aufgeklärt werden konnten.

Die mehrfach ungesättigten Fettsäuren können entsprechend ihrem Desaturierungsmuster in zwei große Klassen, in w-6- oder ω-3-Fettsäuren, eingeteilt werden, die metabolisch und funktionell unterschiedliche Aktivitäten haben. Als Ausgangsprodukt für den ω-6-Stoffwechselweg fungiert die Fettsäure Linolsäure (18:2^{Δ9,12}), während der w-3-Weg über Linolensäure (18:3^{Δ9,12,15}) abläuft. Linolensäure wird dabei durch Aktivität einer Δ15-Desaturase gebildet (Tocher et al. 1998, Prog. Lipid Res. 37, 73-117 ; Domergue et al. 2002, Eur. J. Biochem. 269, 4105-4113). Säugetiere und damit auch der Mensch verfügen über keine entsprechende Desaturaseaktivität (Δ-12- und Δ15-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen mehrfach ungesättigten Fettsäuren Arachidonsäure (= ARA, 20:4^{Δ5,8,11,14}), eine ω-6-Fettsäure, und die beiden ω-3-Fettsäuren Eicosapentaen- (= EPA, 20:5^{Δ5,8,11,14,17}) und Docosahexaensäure (DHA, 22:6^{Δ4,7,10,13,17,19}) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthritis (Cleland und James 2000, J. Rheumatol. 27, 2305-2307).

Höhere Pflanzen enthalten mehrfach ungesättigte Fettsäuren wie Linolsäure (C18:2) und Linolensäure (C18:3). Dagegen kommen ARA, EPA und DHA im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen (bevorzugt in Ölsaaten wie Raps, Lein, Sonnenblume und Soja) LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Ein möglicher Weg führt über gentechnische Methoden, in denen Gene kodierend für Enzyme der Biosynthese von LCPUFAs in Ölsaaten eingeführt und exprimiert werden. Dies sind Gene, die beispielsweise für Δ-6-Desaturasen, Δ-6-Elongasen, Δ-5-Desaturasen oder Δ-4-Desaturasen codieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert werden (Zank, T.K. et al. Plant Journal 31:255-268, 2002, Beaudoin et al. Biochem Soc Trans 28 :661-663, 2000).

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) beschrieben. Diese Pflanzen produzieren allerdings LCPUFAs in Mengen, die für eine Aufarbeitung der in den Pflanzen enthaltenen Öle noch weiter optimiert werden müssen.

Um eine Anreicherung der Nahrung und des Futters mit diesen mehrfach ungesättigten Fettsäuren zu ermöglichen, besteht daher ein großer Bedarf an Mitteln und Maßnahmen für eine einfache, kostengünstige Herstellung dieser mehrfach ungesättigten Fettsäuren speziell in eukaryontischen Systemen.

Die der vorliegenden Erfindung zugrunde liegende Aufgabe ist die Bereitstellung von solchen Mitteln und Maßnahmen. Die Aufgabe wird durch die Ausführungsformen gelöst, die in den Patentansprüchen und im Folgenden beschrieben werden.

Die vorliegende Erfindung betrifft somit ein Polynukleotid umfassend eine Nukleinsäuresequenz, die ausgewählt ist aus der Gruppe bestehend aus:
(a) Nukleinsäuresequenz wie in einer der SEQ ID NR: 1, 2, gezeigt;
(b) Nukleinsäuresequenz, die ein Polypeptid kodiert, das eine Aminosäuresequenz wie in einer der SEQ ID NR: 3, gezeigt aufweist;
(c) Nukleinsäuresequenz, die mindestens 95% identisch zu einer der Nukleinsäuresequenzen aus (a) oder (b) ist und ein Polypeptid mit einer Desaturase Aktivität kodiert; und
(d) Nukleinsäuresequenz für ein Fragment einer Nukleinsäure aus (a), (b) oder (c), wobei das Fragment ein Polypeptid mit einer Desaturase Aktivität kodiert.

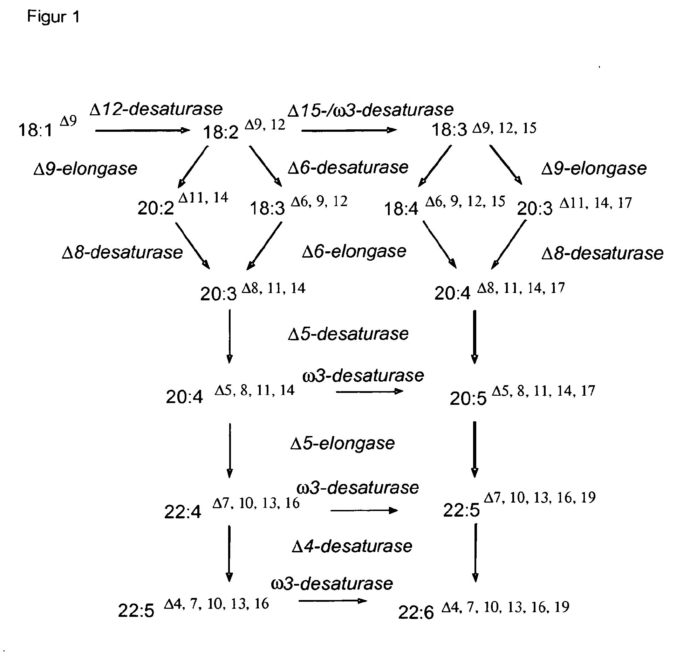

Die ω6-Fettsäure Klasse geht von der ω6-Fettsäure Linolsäure (18:2Δ9,12) aus, wohingegen die ω3-Fettsäure Klasse von der ω3-Fettsäure Linolensäure (18:3Δ9,12,15) ausgeht; siehe Figur 1. Diese beiden Fettsäuren sind die Substrate für die Synthese langkettiger ω6- bzw. ω3-PUFAs. Die Erhöhung des Gehaltes an diesen Fettsäuren entsprechend der eingebrachten Gene führt zu einer Erhöhung des Gehaltes an langkettigen PUFAs.

Die vorliegende Erfindung stellt Polynukleotidsequenzen zur Verfügung, die zu einer Erhöhung der Substrate 18:2Δ9,12 führen. Es wurden Polynukleotidsequenzen identifiziert, die für Enzyme mit Δ12-Desaturase Aktivität kodieren.

Der Begriff "Polynukleotid" betrifft erfindungsgemäß Polynukleotide, die Nukleinsäuresequenzen umfassen, die Polypeptide mit Desaturase Aktivität kodieren. Die Desaturase Aktivitäten werden vorzugsweise für die Biosynthese von Lipiden oder Fettsäuren benötigt. Besonders bevorzugt handelt es sich um die folgenden Desaturase Aktivitäten: Δ-12-Desaturase- Aktivität. Die Desaturasen sind bevorzugt an der Synthese mehrfach ungesättigter Fettsäuren (PUFAs) und besonders bevorzugt an der Synthese lankettiger PUFAs (LCPUFAs) beteiligt. Geeignete Nachweissysteme für diese Desaturase Aktivitäten sind in den Beispielen oder in WO2005/083053 beschrieben. Besonders bevorzugt weisen die erfindungsgemäßen Desaturasen Aktivitäten, Substratspezifitäten und/oder Umsetzungsraten auf, die denen der jeweiligen homologen Desaturase Enzyme aus Pythium irregulare, Ostreococcus tauri, Phytophtora sojae oder Phytophtora infestans vergleichbar sind. Beschrieben werden spezifischen Polynukleotide, d.h. die Polynukleotide mit einer Nukleinsäuresequenz gemäß SEQ ID NR: 1, 2, 4, 5, 7, 8, 10, 11, 13, 14, 16, 17, 19, 20, 22, 23, 25, 26, 90, 91, 122, 123, 125, 126, 128, 129, 131, 132, 134, 135, 142, 143, 145, 146, 148, 149, 151, 152, 154, 155 oder 157 die aus Nectria haematococca, Trichoderma resii, Monosiga brevicollis, Mycosphaerella fijiensis, Mycospaerella graminicola (Septoria tritici), Naegleria gruberi, Phycomyces blakesleeanus, Nematostella vectensis, Helobdella robusta, Lottia gigantea, Microcoleus chthonoplastes, Laccaria bicolor, Physcomitrella patens, Postia placenta, Selaginella moellendorffii oder Microdochium nivale gewonnen werden.
Insbesondere stammen die Nukleinsäuresequenzen gemäß SEQ ID NR: 1, 2, 4 und 5 aus Nectria haematococca, die Nukleinsäuresequenzen gemäß SEQ ID NR: 7 und 8 aus Trichoderma resii, die Nukleinsäuresequenzen gemäß SEQ ID NR: 10 und 11 aus Monosiga brevicollis, die Nukleinsäuresequenzen gemäß SEQ ID NR: 13, 14, 16 und 17 aus Mycospaerella graminicola (Septoria tritici), die Nukleinsäuresequenzen gemäß SEQ ID NR: 19 und 20 aus Naegleria gruberi, die Nukleinsäuresequenzen gemäß SEQ ID NR: 22 und 23 aus Phycomyces blakesleeanus, die Nukleinsäuresequenzen gemäß SEQ ID NR: 25 und 26 aus Nematostella vectensis, die Nukleinsäuresequenzen gemäß SEQ ID NR: 90 und 91 aus Laccaria bicolor, die Nukleinsäuresequenzen gemäß SEQ ID NR: 134 und 135 aus Mycosphaerella fijiensis, die Nukleinsäuresequenzen gemäß SEQ ID NR: 122 und 123 aus Helobdella robusta, die Nukleinsäuresequenzen gemäß SEQ ID NR: 125, 126, 128 und 129 aus Lottia gigantea, die Nukleinsäuresequenzen gemäß SEQ ID NR: 131 und 132 aus Microcoleus chthonoplastes, die Nukleinsäuresequenzen gemäß SEQ ID NR: 142, 143, 145, 146, 148, 149 aus Physcomitrella patens, die Nukleinsäuresequenzen gemäß SEQ ID NR: 151, 152 aus Postia placenta, die Nukleinsäuresequenzen gemäß SEQ ID NR: 154, 155 aus Selaginella moellendorffii und die Nukleinsäuresequenz gemäß SEQ ID NR: 157 aus Microdochium nivale. Bei den SEQ ID NR: 1, 4, 7, 10, 13, 16, 19, 22, 25, 90, 122, 125, 128, 131, 134, 142, 145, 148, 151 und 154 handelt es sich um genomische Sequenzen, wohingegen die SEQ ID NR: 2, 5, 8, 11, 14, 17, 20, 23, 26, 91, 123, 126, 129, 132, 135, 143, 146, 149, 152, 155 und 157 kodierende Sequenzen (cds) darstellen. Die SEQ ID NR: 3, 6, 9,12, 15, 18, 21, 24, 27, 92, 124, 127, 130, 133,136, 144, 147, 150, 153, 156 und 158 zeigen die zugehörigen Aminosäuresequenzen.

Insbesondere sind also erfindungsgemäße Polynukleotide:
Polynukleotide, die ein Polypeptid mit Δ-12-Desaturase Aktivität kodieren und umfassen (i) eine Nukleinsäuresequenz, wie in SEQ ID NR: 1 oder 2 gezeigt, (ii) eine Nukleinsäuresequenz, die für ein Polypeptid kodiert wie in SEQ ID NR: 3 gezeigt, (iii) eine Nukleinsäuresequenz, die mindestens 95% identisch zu einer der Nukleinsäuresequenzen aus (i) oder (ii) ist, oder (iv) eine Nukleinsäuresequenz für ein Fragment einer Nukleinsäure aus (i), (ii) oder (iii), wobei das Fragment ein Polypeptid mit einer Δ-12-Desaturase Aktivität kodiert.

Der Begriff "delta-12-Desaturase (oder Δ-12-Desaturase oder d-12 Desaturase oder d12-Des oder d12Des)" oder "delta-12-Desaturase (oder Δ-12-Desaturase oder d-12 Desaturase oder d12-Des oder d12Des) Aktivität" wie hier gebraucht bezeichnet ein Enzym mit der enzymatischen Funktion zur Dehydrogenierung von C18 Fettsäuren, die am C-Atom 9-10 bereits dehydrogeniert sind. Dabei werden die C-Atome C12 und C13 um je ein Wasserstoffatom dehydrogeniert, wobei eine Doppelbindung zwischen diesen beiden C-Atomen entsteht.

Der Begriff "delta-15-Desaturase (oder Δ-15-Desaturase oder d-15 Desaturase oder d15-Des oder d15Des)" oder "delta-15-Desaturase (oder Δ-15-Desaturase oder d-15 Desaturase oder d15-Des oder d15Des) Aktivität" wie hier gebraucht bezeichnet ein Enzym mit der enzymatischen Funktion zur Dehydrogenierung von C18- und/oder C20-Fettsäuren, die an den C-Atomen 6-7, 8-9, 9-10, 12-13 und/oder 13-14 dehydrogeniert sind. Dabei werden die C-Atome C15-16 und/oder C17-18 um je ein Wasserstoffatom dehydrogeniert, wobei eine Doppelbindung zwischen den beiden C-Atomen entsteht.

Der Begriff "delta-12- und delta-15-Desaturase (oder Δ-12- und Δ-15-Desaturase oder in oben angeführter Schreibweise)" oder "delta-12- und delta-15-Desaturase (oder Δ-12- und Δ-15-Desaturase oder in oben angeführter Schreibweise) Aktivität" wie hier gebraucht bezeichnet ein Enzym mit der enzymatischen Funktion zur Dehydrogenierung von C18- und/oder C20-Fettsäuren, die an den C-Atomen 6-7, 8-9, 9-10 und/oder 13-14 dehydrogeniert sind. Dabei werden die C-Atome C12-13 und C15-16 und/oder C17-18 um je ein Wasserstoffatom dehydrogeniert, wobei eine Doppelbindung zwischen den beiden C-Atomen entsteht.

Der Begriff "omega 3-Desaturase (oder ω3-Desaturase oder ω3-Des oder ω3Des oder omega3 Des oder o3Des)" oder "omega 3-Desaturase (oder ω3-Desaturase oder ω3-Des oder ω3Des oder omega3 Des oder o3Des) Aktivität" wie hier gebraucht bezeichnet ein Enzym mit der enzymatischen Funktion zur Dehydrogenierung von C18-, C20- und/oder C22-Fettsäuren, die an den C-Atomen 4-5, 5-6, 6-7, 8-9, 9-10, 13-14 und/oder 16-17 dehydrogeniert sind. Dabei werden die C-Atome C15-16 und/oder C17-18 und/oder C19-20 um je ein Wasserstoffatom dehydrogeniert, wobei eine Doppelbindung zwischen den beiden C-Atomen entsteht.

Die erfindungsgemäßen Polynukleotidsequenzen bzw. Polypeptidsequenzen stammen hierbei bevorzugt aus den oben angeführten Organismen.

Es versteht sich, dass die zuvor genannten spezifischen Sequenzen angesichts des degenerierten genetischen Codes auch verändert werden können, wobei von den veränderten Polynukleotiden immer noch Polypeptide mit einer Aminosäuresequenz gemäß einer der SEQ ID NR: 3, 6, 9, 12, 15, 18, 21, 24, 27, 92, 124, 127, 130, 133, 136, 144, 147, 150, 153, 156, oder 158 kodiert werden, die die zuvor genannten Desaturase Aktivitäten aufweisen.

Der Begriff "Polynukleotid" umfasst auch Varianten der zuvor genannten spezifischen Polynukleotide. Bei diesen kann es sich um homologe, orthologe oder paraloge Sequenzen handeln. Solche Varianten umfassen Nukleinsäuresequenzen, die mindestens einen Basenaustausch, eine Basenaddition oder eine Basendeletion aufweisen, wobei die Varianten immer noch ein Polypeptid mit der zuvor genannten biologischen Aktivität der jeweiligen Ausgangssequenz kodieren sollen. Varianten umfassen Polynukleotide, die mit den zuvor genannten Polynukleotiden hybridisieren können, bevorzugt unter stringenten Bedingungen. Besonders bevorzugte stringente Bedingungen sind dem Fachmann bekannt und lassen sich in Current Protocols in Molecular Biology, John Wiley & Sons, N. Y. (1989), 6.3.1-6.3.6., finden. Ein bevorzugtes Beispiel für stringente Hybridisierungsbedingungen sind Hybridisierungen in 6 x Natriumchlorid/Natriumcitrat (sodium chloride/sodiumcitrate = SSC) bei etwa 45°C, vorzugsweise bei 50°C, 55°C, 60°C, und am meisten bevorzugt bei 62°C, gefolgt von einem oder mehreren Waschschritten in 0,1 x SSC, 0,1 % SDS bei 50 bis 65°C, bevorzugt 55 bis 65°C, noch mehr bevorzugt bei 60 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können. Alternativ können Varianten der erfindungsgemäßen spezifischen Polynukleotide auch durch Polymerasekettenreaktion (PCR) basierte Verfahren bereitgestellt werden. Hierzu können zunächst Primer von konservierten Sequenzen (z.B. Sequenzen, die funktionelle Domänen im Polypeptid kodieren) abgeleitet werden. Konserviere Sequenzen können durch Sequenzvergleiche mit Polynukleotiden, die Polypeptide ähnlicher Aktivität kodieren, ermittelt werden. Als Matrize kann DNA oder cDNA aus Bakterien, Pilzen, Pflanzen oder Tieren vedrwendet werden. DNA Fragmente, die durch die PCR erhalten wurde, können zum Screening von entsprechenden genomischen oder cDNA Bibliotheken verwendet werden um, - falls erforderlich - den kompletten offenen Leserahmen des Polynukleotids zu isolieren und durch Sequenzierung zu ermitteln. Bevorzugte Varianten umfassen Polynukleotide, die eine Nukleinsäuresequenz umfassen, die mindestens 95%, mindestens 96%, mindestens 97%, mindestens 98% oder mindestens 99% (oder zu einem anderen hierin genannten Prozentsatz) identisch ist mit einer der zuvor genannten spezifischen Nukleinsäuresequenzen und ein Polypeptid mit der jeweiligen biologischen Aktivität kodiert. Ebenso bevorzugt umfasst sind Polynukleotide, die Nukleinsäuresequenzen umfassen, die ein Polypeptid mit einer Aminosäuresequenz kodieren, die mindestens 95%, mindestens 96%, mindestens 97%, mindestens 98% oder mindestens 99% (oder zu einem anderen hierin genannten Prozentsatz) identisch ist mit einer der zuvor genannten spezifischen Aminosäuresequenzen und wobei das Polypeptid die jeweilige biologische Aktivität der Ausgangssequenz aufweist.

Der Prozentsatz identischer Nukleotide oder Aminosäuren bezieht sich vorzugsweise auf einen Sequenzabschnitt von mindestens 50% der zu vergleichenden Sequenzen und besonders bevorzugt über die gesamte Länge der zu vergleichenden Sequenzen. Eine Vielzahl von Programmen, die Algorithmen für solche Vergleiche implementieren ist im Stand der Technik beschrieben und kommerziell erhältlich. Insbesondere sei auf die Algorithmen von Needleman und Wunsch oder Smith und Waterman verwiesen, die besonders zuverlässige Ergebnisse liefern. Diese Algorithmen können vorzugsweise durch die folgenden Programme implementiert werden: PileUp (J. Mol. Evolution., 25, 351-360, 1987, Higgins et al., CABIOS, 5 1989: 151-153), Gap und BestFit (Needleman and Wunsch (J. Mol. Biol. 48; 443-453 (1970)) und Smith und Waterman (Adv. Appl. Math. 2; 482-489 (1981))), als Teil der GCG software [Genetics Computer Group, 575 Science Drive, Madison, Wisconsin, USA 53711 (1991)]. Besonders bevorzugt wird der Prozentsatz (%) der Sequenzidentität im Rahmen der vorliegenden Erfindung mit dem GAP Programm über die gesamte Sequenz mit folgenden festgelegten Größen bestimmt: Gap Weight: 50, Length Weight: 3, Average Match: 10.000 und Average Mismatch: 0.000.

Ein Polynukleotid, das lediglich ein Fragment der zuvor genannten Nukleinsäuresequenzen umfasst, ist auch ein erfindungsgemäßes Polynukleotid. Das Fragment soll hierbei ein Polypeptid kodieren, das die biologische Aktivität der Ausgangssequenz bzw. des davon kodierten Polypeptids aufweist. Polypeptide, die von solchen Polynukleotiden kodiert werden, umfassen oder bestehen daher aus Domänen der zuvor genannten spezifischen Polypeptide (Ausgangspolypeptide), die die biologische Aktivität vermitteln. Ein Fragment im Sinne der Erfindung umfasst vorzugsweise mindestens 50, mindestens 100, mindestens 250 oder mindestens 500 aufeinanderfolgende Nukleotide der zuvor genannten spezifischen Sequenzen oder kodiert eine Aminosäuresequenz umfassend mindestens 20, mindestens 30, mindestens 50, mindestens 80, mindestens 100 oder mindestens 150 aufeinanderfolgende Aminosäuren einer der zuvor genannten spezifischen Aminosäuresequenzen und vermittelt biologische Aktivität, vorzugsweise Desaturase Aktivität wie zuvor beschrieben.

Die Polynukleotid-Varianten weisen vorzugsweise mindestens 10%, mindestens 20%, mindestens 30%, mindestens 40%, mindestens 50%, mindestens 60%, mindestens 70%, mindestens 80% oder mindestens 90% der jeweiligen biologischen Aktivität des Polypeptids, das von der Ausgangssequenz kodiert wird, auf. D.h. die Polypeptide, die von den erfindungsgemäßen Polynukleotiden kodiert werden, können am Stoffwechsel von zum Aufbau von Fettsäuren, Fettsäureestern wie Diacylglyceride und/oder Triacylglyceride in einem Organismus, bevorzugt in einer Pflanze oder Pflanzenzelle, notwendigen Verbindungen oder am Transport von Molekülen über Membranen teilnehmen, wobei C₁₈-, C₂₀- oder C₂₂-Kohlenstoffketten im Fettsäuremolekül mit Doppelbindungen an mindestens zwei, vorteilhaft drei, vier, fünf oder sechs Stellen gemeint sind.

Die Polynukleotide umfassen entweder die zuvor genannten spezifischen Nukleinsäuresequenzen oder bestehen daraus. D.h. die erfindungsgemäßen Polynukleotide können grundsätzlich auch noch weitere Nukleotide umfassen. Dabei kann es sich vorzugsweise um 3' oder 5' untranslatierte Bereiche der genomischen Nukleinsäuresequenz handeln. Diese bestehen vorzugsweise aus mindestens 100, 200 oder 500 Nukleotiden am 5' Terminus und mindestens 20, 50 oder 100 Nukleotiden am 3' Terminus des kodierenden Bereichs. Weitere Polynukleotide, die zusätzliche Nukleinsäuresequenzen umfassen, sind solche, die für Fusionsproteine kodieren. Solche Fusionsproteine können zusätzlich zu den zuvor genannten Polypeptiden weitere Polypeptide bzw. Polypeptidanteile kodieren. Bei dem zusätzlichen Polypeptid bzw. Polypeptidanteil kann es sich um weitere Enzyme der Lipid- oder Fettsäure Biosynthese handeln. Auch denkbar sind Polypeptide, die als Marker für die Expression dienen können (Grün-, Gelb-, Rot-, Blau-fluoreszierende Proteine, alkalische Phosphatase u.a.) bzw. sogenannte "tags" als Marker oder Hilfe für die Aufreinigung (z.B. FLAG-tags, 6-Histidin-tags, MYC-tags u.a.).

Polynukleotid-Varianten können von verschiedenen natürlichen oder künstlichen Quellen isoliert werden. Beispielsweise können sie künstlich durch in vitro oder in vivo Mutagenese erzeugt werden. Homologe oder Orthologe der spezifischen Sequenzen können aus den verschiedensten Tieren, Pflanzen oder Mikroorganismen gewonnen werden. Vorzugsweise werden sie aus Algen gewonnen. Bevorzugt sind Algen wie Isochrysis, Euglena oder Crypthecodinium, Algen/Diatomeen wie Thalassiosira, Phaeodactylum oder Thraustochytrium, Pythium, Moose wie Physcomitrella, bevorzugt Physcomitrella patens oder Ceratodon, ganz besonders bevorzugt sind die Algen der Gattungen Euglena oder die Diatomeen aus der Klasse der Oomycota wie die Gattungen Pythium oder Phytophtora oder Pilze wie Postia placenta oder Microdochium nivale oder aus der Abteilung der Zygomycota aus den Gattungen Rhizopus. Die Polynukleotide können ebenfalls aus Pflanzen, bevorzugt aus der Familie der Selaginellaceae, wie Selaginella moellendorffii, oder aus höheren Pflanzen gewonnen werden, wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Rhizopus, Mucor oder Mortierella, Bakterien wie Shewanella, Cyanobacterien wie Synechococcus, Hefen oder Tieren wie Nematoden z.B. Caenorhabditis, Mollusken, Insekten oder Fische. Die Polynukleotid-Varianten stammen ebenfalls bevorzugt aus einem Tier aus der Ordnung der Vertebraten. Besonders bevorzugt stammen die Polynukleotide aus der Klasse der Vertebrata; Euteleostomi, Actinopterygii; Neopterygii; Teleostei; Euteleostei, Protacanthopterygii, Salmoniformes; Salmonidae bzw. Oncorhynchus und, ganz besonders bevorzugt, aus der Ordnung der Salmoniformes wie der Familie der Salmonidae wie der Gattung Salmo beispielsweise aus den Gattungen und Arten Oncorhynchus mykiss, Trutta trutta oder Salmo trutta fario. Die erfindungsgemäßen Polynukleotide können hierbei mittels molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lassen sich Polynukleotide oder Fragmente davon, durch Polymerasekettenreaktion (PCR) isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis der in den SEQ ID Nummern (SEQ ID NR:) dargestellten Polynukleotid- und Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Die erfindungsgemäßen Polynukleotide können entweder als isolierte Polynukleotide (d.h. isoliert aus ihrem natürlichen Ursprung, z.B. dem genomischen Locus) oder in genetisch veränderter Form bereitgestellt werden (d.h. die Polynukleotide können auch an ihrem natürlichen genetischen Locus vorliegen, müssen dann aber genetisch verändert werden). Ein isoliertes Polynukleotid umfasst vorzugsweise weniger als 5 kb, 4 kb, 3 kb, 2 kb, 1 kb, 0,5 kb oder 0,1 kb an Nukleinsäuresequenz, die natürlicherweise in seiner Nachbarschaft vorkommt. Das erfindungsgemäße Polynukleotid kann als einzelsträngiges oder doppelsträngiges Nukleinsäuremolekül vorliegen und genomische DNA, cDNA oder RNA sein. Vorzugsweise besteht das erfindungsgemäße Polynukleotid aus RNA oder DNA. Die erfindungsgemäßen Polynukleotide umfassen alle Orientierungen der in den SEQ ID Nummern aufgezeigten Sequenzen, d.h. auch komplementäre Stränge sowie reverse oder revers-komplementäre Orientierungen. Ferner umfasst der Begriff auch chemisch modifizierte Nucleinsäuren, wie die natürlich vorkommenden methylierten DNA Moleküle, oder artifizielle Nucleinsäuren z.B. biotinylierte Nukleinsäuren.

Beschrieben sind auch Oligonukleotide von mindestens 15 bp, bevorzugt mindestens 20 bp, mindestens 25 bp, mindestens 30 bp, mindestens 35 bp, oder mindestens 50 bp, die spezifisch mit einem der zuvor genannten Polynukleotide unter stringenten Bedingungen hybridisieren können. Die Oliguncleotide können aus DNA oder RNA oder beidem bestehen. Solche Oligonukleotide können als Primer für die PCR, als die Expression hemmende, antisense Oligonukleotide, für RNA Interferenz (RNAi) oder für Chimero- oder Genoplastie Ansätze verwendet werden. RNAi Verfahren sind beispielsweise in Fire et al., Nature (1998) 391:806-811; Fire, Trends Genet. 15, 358-363 (1999); Sharp, RNA interference 2001. Genes Dev. 15,485-490 (2001); Hammond et al. Nature Rev. Genet. 2, 1110-1119 (2001); Tuschl, Chem. Biochem. 2, 239-245 (2001); Hamilton et al., Science 286, 950-952 (1999); Hammond et al., Nature 404, 293-296 (2000); Zamore et al., Cell 101, 25-33 (2000); Bernstein et al., Nature 409, 363-366 (2001); Elbashir et al., Genes Dev. 15, 188-200 (2001); WO 01/29058; WO 99/32619; oder Elbashir et al., 2001 Nature 411: 494-498 beschrieben und dienen der Inhibierung der Genexpression durch Abbau der mRNA. Chimero- oder Genoplastie Ansätze dienen der in vivo Modifizierung (z.B. dem Einfügen von Punktmutationen) in Genen an deren endogenen Loci. Entsprechende Verfahren sind offenbart in US5,565,350, US5,756,325, US5,871,984, US5,731,181, US5,795,972, US6,573,046, US6,211,351, US6,586,184, US6,271,360 und US6,479,292.

Vorteilhafterweise hat sich gezeigt, dass die erfindungsgemäßen Polynukleotide zur rekombinanten Herstellung mehrfach ungesättigter Fettsäuren in Wirtszellen und transgenen Organismen besonders effektiv eingesetzt weden können. Insbesondere können die von den erfindungsgemäßen Polynukleotiden kodierten Polypeptide mit Δ-12-Desaturase, Δ15-Desaturase, Δ-12- und Δ-15-Desaturase oder Omega-3-Desaturase Aktivität C₁₈-, C₂₀- und C₂₂-Fettsäuren mit ein, zwei, drei, vier oder fünf Doppelbindungen und bevorzugt mehrfach ungesättigte C₁₈-Fettsäuren mit ein, zwei oder drei Doppelbindungen wie C18:1^{Δ9}, C18:2^{Δ9,12} oder C18:3 ^{Δ6,9,12}, mehrfach ungesättigte C₂₀-Fettsäuren mit drei oder vier Doppelbindungen wie C20:3^{Δ8,11,14}, C20:4^{Δ5,8,11,14} oder C20:4^{Δ8,11,14,17} oder mehrfach ungesättigte C₂₂-Fettsäuren mit vier oder fünf Doppelbindungen wie C22:4^{Δ7,10,13,16} oder C22:5^{Δ7,10,13,16,19} umsetzen. Besonders bevorzugt führen die erfindungsgemäßen Polynukleotid- und Aminosäuresequenzen zu einer Erhöhung der 18:2Δ9,12 beziehungsweise 18:3Δ9,12,15 Fettsäuren. Figur 1 verdeutlicht, wo die erfindungsgemäßen Desaturasen in der Biosynthese langkettiger mehrfach ungesättigter Fettsäuren angreifen bzw. wie sie zur Herstellung dieser Fettsäuren verwendet werden können.

Besonders bevorzugt können in diesem Zusammenhang die Δ-6-Desaturase, kodiert von der Polynukleotidsequenz mit SEQ ID NR: 28 (d6Des(Pir)), die Δ-6-Elongase kodiert von der Polynukleotidsequenz mit SEQ ID NR: 31 (d6Elo(Pp)), die Δ-5-Desaturase kodiert von der Polynukleotidsequenz mit SEQ ID NR: 29 (d5Des(Tc)), die Δ-5-Elongase kodiert von der Polynukleotidsequenz mit SEQ ID NR: 32 (dSElo(Ot)), die Δ-4-Desaturase kodiert von der Polynukleotidsequenz mit SEQ ID NR: 33 (d4Des(Tc)), die Δ-6-Elongase kodiert von der Polynukleotidsequenz mit der SEQ ID NR: 138 (d6Elo(Tp), die Δ-6-Desaturase kodiert von der Polynukleotidsequenz mit der SEQ ID NR: 139 (d6Des(Ot), mit einer oder mehreren der erfindungsgemäßen Desaturasen zur Synthese langkettiger mehrfach ungesättigter Fettsäuren eingesetzt werden; siehe hierzu beispielsweise WO2006/100241. Statt der o.a. Δ-6-Desaturase und der Δ-6-Elongase kann alternativ auch eine Δ-9-Elongase und eine Δ-8-Desaturase eingesetzt werden, wie in WO2004/057001 beschrieben. Je nach Fettsäure, die hergestellt werden soll, können verschiedene Kombinationen der erfindungsgemäßen Polynukleotide mit den zuvor genannten Desaturasen bzw. Elongasen in den nachfolgend beschriebenen Wirtszellen oder transgenen Organismen koexprimiert oder in den erfindungsgemäßen Verfahren verwendet werden. Besonders bevorzugte Kombinationen für die Herstellung von Eicosapentaensäure sind in den Tabellen 5 und 8 und für Docosahexaensäure in Tabelle 6 nachfolgend aufgeführt. Beispielsweise kann eine erfindungsgemäße Δ-12-Desaturase, Δ15-Desaturase, Δ-12- und Δ-15-Desaturase, oder Omega-3-Desaturase, allein oder in geeigneter Kombination (etwa eine Δ-12-Desaturase und eine Δ15-Desaturase), zusammen mit d6Des(Pir) und/oder d6Des(Ot), d6Elo(Pp), d5Des(Tc), und ω3Des(Pi) zur Herstellung von EPA verwendet werden. Gleichermaßen kann eine erfindungsgemäße Δ-12-Desaturase, Δ15-Desaturase, Δ-12- und Δ-15-Desaturase, oder Omega-3-Desaturase, allein oder in geeigneter Kombination, zusammen mit d6Des(Pir) und/oder d6Des(Ot), d6Elo(Pp), d5Des(Tc), ω3Des(Pi), d5Elo(Ot), d4Des(Tc) zur Herstellung von Docosahexaensäure verwendet werden.

Bevorzugt werden die Fettsäuren in Phospholipiden oder CoA-Fettsäureestern desaturiert, vorteilhaft in den CoA-Fettsäureester. Somit ist eine einfache, kostengünstige Herstellung dieser mehrfach ungesättigten Fettsäuren speziell in eukaryontischen Systemen möglich. Die mittels der erfindungsgemäßen Polynukleotide hergestellten ungesättigten Fettsäuren können dann als Öl-, Lipid- und Fettsäurezusammensetzungen formuliert und entsprechend eingesetzt werden.

Die vorliegende Erfindung betrifft weiterhin einen Vektor, der das erfindungsgemäße Polynukleotid umfasst.

Der Begriff "Vektor" bezeichnet ein Nukleinsäuremolekül, das ein anderes Nukleinsäuremolekül, wie die erfindungsgemäßen Polynukleotide, transportieren kann, an welche es gebunden ist. Ein Vektortyp ist ein Plasmid, das für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier auch als Expressionsvektoren bezeichnet. Gewöhnlich haben Expressionsvektoren, die für DNA-Rekombinationstechniken geeignet sind, die Form von Plasmiden. In der vorliegenden Beschreibung können die Begriffe Plasmid und Vektor austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, künstliche Chromosomen, umfassen. Der Begriff umfasst schließlich auch Konstrukte für zielgerichtete, d.h. homologe Rekombination, oder heterologe Insertion von Polynukleotiden.

Vektoren lassen sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakteriumvermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al, Trends in Plant Science (2000) 5, 446-451. Die Vektoren mit den inserierten erfindungsgemäßen Polynukleotiden lassen sich in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen. Mittels der Klonierungsvektoren können die erfindungsgemäßen Polynukleotide in Organismen wie Mikroorganismen oder Pflanzen eingebracht werden und damit zur Pflanzentransformation verwendet werden. Geeignet Vektoren dafür sind veröffentlicht in: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)).

Bevorzugt handelt es sich bei dem Vektor um einen Expressionsvektor. Bei dem erfindungsgemäßen Expressionsvektor liegt das Polynukleotid in operativer (d.h. funktioneller) Verbindung zu einer Expressionskontrollsequenz vor. Die Expressionskontrollsequenz zusammen mit dem Polynukleotid und optional weiteren Sequenzelementen des Vektors wird auch als Expressionskassette bezeichnet. Durch die Expressionskotrollsequenz wird sichergestellt, dass das Polynukleotid nach Transformation oder Transfektion in eine Wirtszelle exprimiert werden kann. Die zu verwendende Expressionskontrollsequenz enthält vorzugsweise cis-regulatorische Elemente wie Promotor und/oder Enhancer Nukleinsäuresequenzen, die von der Transkriptionsmaschinerie der Wirtszellen erkannt werden. Der Begriff umfasst zudem andere Expressionskontrollelemente z.B. Polyadenylierungssignale und RNA stabilisierende Sequenzen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Expressionskontrollsequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann. Die erfindungsgemäßen Polynukleotide können in einer oder mehreren Kopien in der Expressionskassette oder dem erfindungsgemäßen Expressionsvektor vorliegen (z.B. in Form mehrerer Expressionskassetten). Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Weitere Expressionskontrollsequenzen im Sinne der vorliegenden Erfindung sind Translationsterminatoren am 3'-Ende der zu translatierenen Polynukleotide. Verwendet werden kann hier z.B. der OCS1 Terminator. Wie auch für die Promotoren, so sollte hier für jedes zu exprimierende Polynukleotid eine unterschiedliche Terminatorsequenz verwendet werden.

Bevorzugte Expressionskontrollsequenzen bzw. Regulationssequenzen liegen in Promotoren vor, wie dem cos-, tac-, trp-, tet-, trp-tet-, lpp-, lac-, lpp-lac-, laclq-, T7-, T5-, T3-, gal-, trc-, ara-, SP6-, λ-PR- oder λ-PL-Promotor und werden vorteilhafterweise in Gram-negativen Bakterien angewendet. Weitere vorteilhafte Regulationssequenzen liegen beispielsweise in den Gram-positiven Promotoren amy und SPO2, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylprophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2- oder lpt-1-Promotor aus Gerste (WO 95/15389 und WO 95/23230), Hordein-Promotor aus Gerste und andere, in WO 99/16890 beschriebene geeignete Promotoren. Es ist im Prinzip möglich, alle natürlichen Promotoren mit ihren Regulationssequenzen, wie die oben genannten, als Expressionskontrollsequenzen zu verwenden. Es ist ebenfalls möglich, zusätzlich oder alleine synthetische Promotoren zu verwenden, besonders wenn sie eine Samenspezifische Expression vermitteln, wie z.B. beschrieben in WO 99/16890.

Um einen besonders hohen Gehalt an PUFAs vor allem in transgenen Pflanzen zu erzielen, sollten die Polynukleotide der vorliegenden Erfindung bevorzugt samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Im Folgenden sind vorteilhafte bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und Ipt1(Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der verschiedenen Biosynthesegene in die transgene Pflanze über mehrere Generation sicherzustellen, sollte jedes der erfindungsgemäßen Polynukleotide unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt (vorteilhaft in einem Polylinker), und anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle, beispielsweise im Polylinker, hinter den Promotor inseriert. Vorteilhaft hat jede Nukleinsäuresequenz ihren eigenen Promotor und gegebenenfalls ihren eigenen Terminator. Derartige vorteilhafte Konstrukte werden beispielsweise in DE 10102337 oder DE 10102338 offenbart. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Die verwendeten rekombinanten Expressionsvektoren können zur Expression in prokaryotischen oder eukaryotischen Zellen gestaltet sein. Dies ist vorteilhaft, da häufig Zwischenschritte der Vektorkonstruktion der Einfachheit halber in Mikroorganismen durchgeführt werden. Beispielsweise können die Δ-12-Desaturase-, Δ-15-Desaturase-, Δ-12- und Δ-15-Desaturase, (-3-Desaturase, Δ-6-Desaturase-, Δ-6-Elongase-, Δ-9-Elongase-, Δ-8-Desaturase-, Δ-5-Desaturase-, Δ-5-Elongase- und/oder Δ-4-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird. Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gnl) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt. Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCI, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Polynukleotide der vorliegenden Erfindung auch in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Bevorzugte Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38. Eine Pflanzen-Expressionskassette enthält vorzugsweise Expressionskontrollsequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet. Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711). Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco. Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor. Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen). Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der cIpP-Promotor aus Arabidopsis, beschrieben in WO 99/46394.

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Der Expressionsvektor kann, wie oben beschrieben, zusätzlich zu den erfindungsgemäßen Polynukleotiden auch weitere Gene umfassen, die in die Organismen eingebracht werden sollen. Es ist möglich und bevorzugt, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Heterologe Gene oder Polynukleotide stammen aus einem Ursprungsorganismus, der sich von dem Zielorganismus unterscheidet, in den die Gene oder Polynukleotide eingebracht werden sollen. Bei homologen Gene oder Polynukleotide sind Zielorganismus und Ursprungsorganismus gleich. Vorzugsweise umfasst der Vektor daher mindestens ein weiteres Polynukleotid, das ein weiteres Enzym kodiert, das in die Biosynthese von Lipiden oder Fettsäuren eingebunden ist. Das Enzym ist vorzugsweise ausgewählt aus der Gruppe bestehend aus: Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n), Fettsäure-Elongase(n), Δ4-Desaturase(n), Δ5-Desaturase(n), Δ6-Desaturase(n), Δ8-Desaturase(n), Δ9-Desaturase(n), Δ12-Desaturase(n), Δ-15-Desaturase(n), Δ-12- und Δ-15-Desaturasen, ω-3-Desaturase, Δ5-Elongase(n), Δ6-Elongase(n) und Δ9-Elongase(n).
Besonders bevorzugte Gen-Kombinationen sind in den Tabellen 5 und 6 und in den folgenden Beispielen aufgeführt.

Die Erfindung betrifft auch eine Wirtszelle, die das erfindungsgemäße Polynukleotid oder den erfindungsgemäßen Vektor umfasst.

Wirtszellen im Sinne der vorliegenden Erfindung können prinzipiell alle eukaryotischen oder prokaryotischen Zellen sein. Es können primäre Zellen aus Tieren, Pflanzen oder mehrzelligen Mikroorganismen sein, z.B. aus denen, die in der Beschreibung an anderer Stelle genannt sind. Ferner umfasst der Begriff auch Zelllinien, die aus diesen Organismen gewonnen werden können.

Wirtszellen im Sinne der Erfindung können aber auch einzellige Mikroorganismen sein, z.B. Bakterien oder Pilze. Besonders bevorzugte Mikroorganismen sind Pilze ausgewählt aus der Gruppe der Familien Chaetomiaceae, Choanephoraceae, Cryptococcaceae, Cunninghamellaceae, Demetiaceae, Hydnangiaceae (Gattung Laccaria), Moniliaceae, Mortierellaceae, Mucoraceae, Pythiaceae, Sacharomycetaceae, Saprolegniaceae, Schizosacharomycetaceae, Sodariaceae oder Tuberculariaceae. Weitere bevorzugte Mikroorganismen sind ausgewählt aus der Gruppe: Choanephoraceae wie den Gattungen Blakeslea, Choanephora z.B. die Gattungen und Arten Blakeslea trispora, Choanephora cucurbitarum, Choanephora infundibulifera var. cucurbitarum, Hydnangiaceae (z.B. Gattung Laccaria, insbesondere Art Laccaria bicolor), Mortierellaceae wie der Gattung Mortierella z.B. die Gattungen und Arten Mortierella isabellina, Mortierella polycephala , Mortierella ramanniana , Mortierella vinacea, Mortierella zonata, der Familie der Mucorales wie die Gattungen und Arten Rhizopus oryzae, Rhizopus stolonifer, Fusarium graminearium, Pythiaceae wie den Gattungen Phytium, Phytophthora z.B. die Gattungen und Arten Pythium debaryanum, Pythium intermedium, Pythium irregulare, Pythium megalacanthum, Pythium paroecandrum, Pythium sylvaticum, Pythium ultimum, Phytophthora cactorum, Phytophthora cinnamomi, Phytophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica, Phytophthora syringae, Saccharomycetaceae wie den Gattungen Hansenula, Pichia, Saccharomyces, Saccharomycodes, Yarrowia z.B. die Gattungen und Arten Hansenula anomala, Hansenula californica, Hansenula canadensis, Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia alcoholophila, Pichia angusta, Pichia anomala, Pichia bispora, Pichia burtonii, Pichia canadensis, Pichia capsulata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii, Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia pastoris, Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis, Saccharomyces heterogenicus, Saccharomyces hienipiensis, Saccharomyces inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii, Yarrowia lipolytica, Schizosacharomycetaceae wie die Gattungen Schizosaccharomyces z.B die Arten Schizosaccharomyces japonicus var. japonicus, Schizosaccharomyces japonicus var. versatilis, Schizosaccharomyces malidevorans, Schizosaccharomyces octosporus, Schizosaccharomyces pombe var. malidevorans, Schizosaccharomyces pombe var. pombe, Thraustochytriaceae wie die Gattungen Althornia, Aplanochytrium, Japonochytrium, Schizochytrium, Thraustochytrium wie z.B. Schizochytrium aggregatum, Schizochytrium limacinum, Schizochytrium mangrovei, Schizochytrium minutum, Schizochytrium octosporum, Thraustochytrium aggregatum, Thraustochytrium amoeboideum, Thraustochytrium antacticum, Thraustochytrium arudimentale, Thraustochytrium aureum, Thraustochytrium benthicola, Thraustochytrium globosum, Thraustochytrium indicum, Thraustochytrium kerguelense, Thraustochytrium kinnei, Thraustochytrium motivum, Thraustochytrium multirudimentale, Thraustochytrium pachydermum, Thraustochytrium proliferum, Thraustochytrium roseum, Thraustochytrium rossii, Thraustochytrium striatum oder Thraustochytrium visurgense.

Ebenfalls bevorzugt als Mikroorganismen sind Bakterien ausgewählt aus der Gruppe der Familien Bacillaceae, Enterobacteriacae oder Rhizobiaceae. Besonders bevorzugt seien die folgenden Bakterien genannt ausgewählt aus der Gruppe: Bacillaceae wie die Gattung Bacillus z.B die Gattungen und Arten Bacillus acidocaldarius, Bacillus acidoterrestris, Bacillus alcalophilus, Bacillus amyloliquefaciens, Bacillus amylolyticus, Bacillus brevis, Bacillus cereus, Bacillus circulans, Bacillus coagulans, Bacillus sphaericus subsp. fusiformis, Bacillus galactophilus, Bacillus globisporus, Bacillus globisporus subsp. marinus, Bacillus halophilus, Bacillus lentimorbus, Bacillus lentus, Bacillus licheniformis, Bacillus megaterium, Bacillus polymyxa, Bacillus psychrosaccharolyticus, Bacillus pumilus, Bacillus sphaericus, Bacillus subtilis subsp. spizizenii, Bacillus subtilis subsp. subtilis oder Bacillus thuringiensis; Enterobacteriacae wie die Gattungen Citrobacter, Edwardsiella, Enterobacter, Erwinia, Escherichia, Klebsiella, Salmonella oder Serratia z.B die Gattungen und Arten Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia carnegieana, Erwinia carotovora subsp. atroseptica, Erwinia carotovora subsp. betavasculorum, Erwinia carotovora subsp. odorifera, Erwinia carotovora subsp. wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoniae, Klebsiella sp., Klebsiella terrigena, Klebsiella trevisanii, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans oder Serratia rubidaea; Rhizobiaceae wie die Gattungen Agrobacterium, Carbophilus, Chelatobacter, Ensifer, Rhizobium, Sinorhizobium z.B. die Gattungen und Arten Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis, Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium adhaerens, Sinorhizobium arboris, Sinorhizobium fredii, Sinorhizobium kostiense, Sinorhizobium kummerowiae, Sinorhizobium medicae, Sinorhizobium meliloti, Sinorhizobium morelense, Sinorhizobium saheli oder Sinorhizobium xinjiangense.

Nutzbare Wirtszellen sind weiterhin genannt in: Goeddel, Gene Expression Technology: Methods in Enzymology 185, Academic Press, San Diego, CA (1990). Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128. Hierzu gehören Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Polynukleotide oder Vektoren können mit im Stand der Technik bekannten Transformations- bzw. Transfektionsverfahren in die Wirtszelle eingebracht werden wie zuvor beschrieben. Bedingungen und Medien für die Kultivierung der Wirtszellen sind dem Fachmann ebenfalls bekannt.

Bevorzugt umfasst die erfindungsgemäße Wirtszelle zusätzlich mindestens ein weiteres Enzym, das in die Biosynthese von Lipiden oder Fettsäuren eingebunden ist. Bevorzugte Enzyme sind bereits an anderer Stelle in der Beschreibung genannt. Das Enzym kann endogen in der Wirtszelle vorliegen, d.h. die Wirtszelle exprimiert bereits natürlicherweise ein Gen, das für ein entsprechendes Enzym kodiert. Alternativ kann auch ein heterologes Polynukleotid in die Wirtszelle eingebracht werden, das für das Enzym kodiert. Geeignete Verfahren und Maßnahmen für die Expression eines heterologen Polynukleotids sind im Stand der Technik bekannt und in Zusammenhang mit den erfindungsgemäßen Polynukleotiden, Vektoren und Wirtszellen hier beschrieben.

Die Erfindung betrifft auch ein Verfahren zur Herstellung eines Polypeptids mit Desaturase Aktivität umfassend die Schritte:
(a) Exprimieren eines erfindungsgemäßen Polynukleotids wie oben definiert in einer Wirtszelle; und
(b) Gewinnen des Polypeptids, das von dem Polynukleotid in (a) kodiert wird, aus der Wirtszelle.

Das Polypeptid kann hierbei durch alle gängigen Verfahren zur Proteinreinigung gewonnen bzw. isoliert werden. Die Verfahren umfassen beispielsweise Affinitätschromatographie, Molsiebchromatographie, Hochdruck Flüssigkeitschromatographie oder auch Proteinpräzipitation ggf. mit spezifischen Antikörpern. Obwohl dies bevorzugt ist, muss das Verfahren nicht notwendigerweise ein reines Präparat des Polypeptids bereitstellen.

Die Erfindung betrifft somit auch ein Polypeptid, das von dem erfindungsgemäßen Polynukleotid kodiert wird oder das erhätlich ist durch das zuvor genannte erfindungsgemäße Verfahren.

Der Begriff "Polypeptid" bezeichnet sowohl ein im Wesentlichen reines Polypeptid als auch ein Polypeptid Präparat, das noch weitere Komponenten oder Verunreinigungen aufweist. Der Begriff wird auch für Fusionsproteine oder Proteinaggregate verwendet, die das erfindungsgemäße Polypeptid und zusätzlich noch weitere Komponenten umfassen. Der Begriff bezeichnet auch chemisch modifizierte Polypeptide. Chemische Modifikationen beinhalten in diesem Zusammenhang künstliche Modifikationen oder natürlich auftretende Modifikationen, z.B posttranslationale Modifikationen wie Phosphorylierung, Myristylierung, Glykosylierung usw.. Die Begriffe Polypeptid, Peptid oder Protein sind austauschbar und werden entsprechend in der Beschreibung und im Stand der Technik verwendet. Die erfindungsgemäßen Polypeptide haben die zuvor genannten biologischen Aktivitäten, also Desaturase Aktivitäten, und können die Biosynthese von mehrfach ungesättigten Fettsäuren (PUFAs), vorzugsweise den langkettigen PUFAs (LCPUFAs), wie hier beschrieben beeinflussen.

Von der Erfindung umfasst ist auch ein Antikörper, der das erfindungsgemäße Polypeptid spezifisch erkennt.

Antikörper gegen das erfindungsgemäße Polypeptid können mit bekannten Verfahren hergestellt werden, bei denen gereinigtes Polypeptid oder Fragmente davon mit geeigneten Epitopen als Antigen verwendet werden. Geeignete Epitope können mittels bekannter Algorithmen zur Antigenizitätsbestimmung basierend auf den hier bereitgestellten Aminosäuresequenzen der erfindungsgemäßen Polypeptide ermittelt werden. Die entsprechenden Polypeptide oder Fragmente können dann synthetisiert oder rekombinant gewonnen werden. Nach Immunisierung von geeigneten Tieren, vorzugsweise von Säugern z.B. Hasen, Ratten oder Mäusen, können die Antikörper dann aus dem Serum mit bekannten Verfahren gewonnen werden. Alternativ können monoklonale Antikörper oder Antikörperfragmente mit den bekannte Verfahren bereitgestellt werden; siehe z.B. Harlow and Lane "Antibodies, A Laboratory Manual", CSH Press, Cold Spring Harbor, 1988, oder Köhler und Milstein, Nature 256 (1975), 495, und Galfré, Meth. Enzymol. 73 (1981), 3.

Vorzugsweise handelt es sich bei den Antikörpern um monoklonale oder polyklonale Antikörper, "single chain"-Antikörper oder chimäre Antikörper sowie Fragmenten davon wie Fab, Fv oder scFv. Weitere Antikörper im Sinne der Erfindung sind bispezifische Antikörper, synthetische Antikörper oder deren chemisch modifizierte Derivate.

Die erfindungsgemäßen Antikörper erkennen die erfindungsgemäßen Polypeptide spezifisch, d.h. sie kreuzreagieren nicht in signifikantem Ausmaß mit anderen Proteinen. Beispielsweise reagiert ein erfindungsgemäßer Antikörper, der spezifisch eine Δ-12-Desaturase bindet, nicht mit einer Δ-6-Desaturase. Dies kann mit im Stand der Technik bekannten Verfahren getestet werden. Die Antikörper können beispielsweise zu Nachweisreaktionen, zur Immunopräzipitation, zur Immunhistochemie oder zur Proteinreinigung (z.B. Affinitätschromatographie) eingesetzt werden.

Die Erfindung betrifft ferner einen transgenen, nicht-humanen Organismus, der das Polynukleotid, den Vektor oder die Wirtszelle der vorliegenden Erfindung umfasst. Bevorzugt handelt es sich bei dem transgenen, nicht humanen Organismus um ein Tier, eine Pflanze oder einen multizelluläreren Mikroorganismus.

Unter dem Begriff "transgen" ist zu verstehen, dass ein heterologes Polynukleotid, also ein in dem jeweiligen Organismus nicht natürlicherweise vorkommendes Polynukleotid, in den Organismus eingebracht wird. Dies kann entweder durch zufällige Insertion des Polynukleotids oder durch homologe Rekombination erreicht werden. Selbstverständlich kann statt des Polynukleotids auch der erfindungsgemäße Vektor eingebracht werden. Verfahren zum Einbringen von Polynukleotiden oder Vektoren zwecks zufälliger Insertion oder homologer Rekombination sind im Stand der Technik bekannt und auch nachfolgend genauer beschrieben. Wirtszellen, die das Polynukleotid oder den Vektor enthalten, können ebenfalls in einen Organismus eingebracht werden und so einen transgenen Organismus erzeugen. Bei einem solchen Organismus handelt es sich dann aber um einen chimären Organismus bei dem lediglich die Zellen, die sich von den eingebrachten Zellen ableiten, transgen sind, d.h. das heterologe Polynukleotid umfassen.

Bevorzugt sind die transgenen, nicht-humanen Organismen Öl-produzierende Organismen, das heißt solche, die für die Herstellung von Ölen verwendet werden, beispielsweise Pilze wie Rhizopus oder Thraustochytrium, Algen wie Euglena, Nephroselmis, Pseudoscourfielda, Prasinococcus, Scherffelia, Tetraselmis, Mantoniella, Ostreococcus, Crypthecodinium, Phaeodactylum, Diatomeen wie Pytium oder Phytophtora oder Pflanzen.

Als transgene Pflanzen können grundsätzlich alle Pflanzen verwendet werden, d.h. sowohl zweikeimblättrige als auch einkeimblättrige Pflanzen. Vorzugsweise handelt es sich um Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Saflor, Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf. Prinzipiell kommen aber alle Pflanzen in Frage, die in der Lage sind Fettsäuren zu synthetisieren wie alle dikotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae, Prasinophyceae oder Gemüsepflanzen oder Zierpflanzen wie Tagetes in Betracht.

Besonders bevorzugt seien die folgenden Pflanzen genannt ausgewählt aus der Gruppe: Adelotheciaceae wie die Gattungen Physcomitrella z.B. die Gattung und Arten Physcomitrella patens, Anacardiaceae wie die Gattungen Pistacia, Mangifera, Anacardium z.B. die Gattung und Arten Pistacia vera [Pistazie], Mangifer indica [Mango] oder Anacardium occidentale [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblume], Carthamus tinctorius [Färberdistel, safflower], Centaurea cyanus [Kornblume], Cichorium intybus [Wegwarte], Cynara scolymus [Artichoke], Helianthus annus [Sonnenblume], Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta [Salat], Tagetes lucida, Tagetes erecta oder Tagetes tenuifolia [Studentenblume], Apiaceae wie die Gattung Daucus z.B. die Gattung und Art Daucus carota [Karotte], Betulaceae wie die Gattung Corylus z.B. die Gattungen und Arten Corylus avellana oder Corylus colurna [Haselnuss], Boraginaceae wie die Gattung Borago z.B. die Gattung und Art Borago officinalis [Borretsch], Brassicaceae wie die Gattungen Brassica, Camelina, Melanosinapis, Sinapis, Arabadopsis z.B. die Gattungen und Arten Brassica napus, Brassica rapa ssp. [Raps], Sinapis arvensis Brassica juncea, Brassica juncea var. juncea, Brassica juncea var. crispifolia, Brassica juncea var. foliosa, Brassica nigra, Brassica sinapioides, Camelina sativa, Melanosinapis communis [Senf], Brassica oleracea [Futterrübe] oder Arabidopsis thaliana, Bromeliaceae wie die Gattungen Anana, Bromelia (Ananas) z.B. die Gattungen und Arten Anana comosus, Ananas ananas oder Bromelia comosa [Ananas], Caricaceae wie die Gattung Carica wie die Gattung und Art Carica papaya [Papaya], Cannabaceae wie die Gattung Cannabis wie die Gattung und Art Cannabis sative [Hanf], Convolvulaceae wie die Gattungen Ipomea, Convolvulus z.B. die Gattungen und Arten Ipomoea batatus, Ipomoea pandurata, Convolvulus batatas, Convolvulus tiliaceus, Ipomoea fastigiata, Ipomoea tiliacea, Ipomoea triloba oder Convolvulus panduratus [Süßkartoffel, Batate], Chenopodiaceae wie die Gattung Beta wie die Gattungen und Arten Beta vulgaris, Beta vulgaris var. altissima, Beta vulgaris var. Vulgaris, Beta maritima, Beta vulgaris var. perennis, Beta vulgaris var. conditiva oder Beta vulgaris var. esculenta [Zuckerrübe], Crypthecodiniaceae wie die Gattung Crypthecodinium z.B. die Gattung und Art Cryptecodinium cohnii, Cucurbitaceae wie die Gattung Cucubita z.B. die Gattungen und Arten Cucurbita maxima, Cucurbita mixta, Cucurbita pepo oder Cucurbita moschata [Kürbis], Cymbellaceae wie die Gattungen Amphora, Cymbella, Okedenia, Phaeodactylum, Reimeria z.B. die Gattung und Art Phaeodactylum tricornutum, Ditrichaceae wie die Gattungen Ditrichaceae, Astomiopsis, Ceratodon, Chrysoblastella, Ditrichum, Distichium, Eccremidium, Lophidion, Philibertiella, Pleuridium, Saelania, Trichodon, Skottsbergia z.B. die Gattungen und Arten Ceratodon antarcticus, Ceratodon columbiae, Ceratodon heterophyllus, Ceratodon purpurascens, Ceratodon purpureus, Ceratodon purpureus ssp. convolutus, Ceratodon purpureus ssp. stenocarpus, Ceratodon purpureus var. rotundifolius, Ceratodon ratodon, Ceratodon stenocarpus, Chrysoblastella chilensis, Ditrichum ambiguum, Ditrichum brevisetum, Ditrichum crispatissimum, Ditrichum difficile, Ditrichum falcifolium, Ditrichum flexicaule, Ditrichum giganteum, Ditrichum heteromallum, Ditrichum lineare, Ditrichum lineare, Ditrichum montanum, Ditrichum montanum, Ditrichum pallidum, Ditrichum punctulatum, Ditrichum pusillum, Ditrichum pusillum var. tortile, Ditrichum rhynchostegium, Ditrichum schimperi, Ditrichum tortile, Distichium capillaceum, Distichium hagenii, Distichium inclinatum, Distichium macounii, Eccremidium floridanum, Eccremidium whiteleggei, Lophidion strictus, Pleuridium acuminatum, Pleuridium alternifolium, Pleuridium holdridgei, Pleuridium mexicanum, Pleuridium ravenelii, Pleuridium subulatum, Saelania glaucescens, Trichodon borealis, Trichodon cylindricus oder Trichodon cylindricus var. oblongus, Elaeagnaceae wie die Gattung Elaeagnus z.B. die Gattung und Art Olea europaea [Olive], Ericaceae wie die Gattung Kalmia z.B. die Gattungen und Arten Kalmia latifolia, Kalmia angustifolia, Kalmia microphylla, Kalmia polifolia, Kalmia occidentalis, Cistus chamaerhodendros oder Kalmia lucida [Berglorbeer], Euphorbiaceae wie die Gattungen Manihot, Janipha, Jatropha, Ricinus z.B. die Gattungen und Arten Manihot utilissima, Janipha manihot" Jatropha manihot., Manihot aipil, Manihot dulcis, Manihot manihot, Manihot melanobasis, Manihot esculenta [Manihot] oder Ricinus communis [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten Pisum sativum, Pisum arvense, Pisum humile [Erbse], Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa [Seidenbaum], Medicago sativa, Medicago falcata, Medicago varia [Alfalfa] Glycine max Dolichos soja, Glycine gracilis, Glycine hispida, Phaseolus max, Soja hispida oder Soja max [Sojabohne], Funariaceae wie die Gattungen Aphanorrhegma, Entosthodon, Funaria, Physcomitrella, Physcomitrium z.B. die Gattungen und Arten Aphanorrhegma serratum, Entosthodon attenuatus, Entosthodon bolanderi, Entosthodon bonplandii, Entosthodon californicus, Entosthodon drummondii, Entosthodon jamesonii, Entosthodon leibergii, Entosthodon neoscoticus, Entosthodon rubrisetus, Entosthodon spathulifolius, Entosthodon tucsoni, Funaria americana, Funaria bolanderi, Funaria calcarea, Funaria californica, Funaria calvescens, Funaria convoluta, Funaria flavicans, Funaria groutiana, Funaria hygrometrica, Funaria hygrometrica var. arctica, Funaria hygrometrica var. calvescens, Funaria hygrometrica var. convoluta, Funaria hygrometrica var. muralis, Funaria hygrometrica var. utahensis, Funaria microstoma, Funaria microstoma var. obtusifolia, Funaria muhlenbergii, Funaria orcuttii, Funaria plano-convexa, Funaria polaris, Funaria ravenelii, Funaria rubriseta, Funaria serrata, Funaria sonorae, Funaria sublimbatus, Funaria tucsoni, Physcomitrella californica, Physcomitrella patens, Physcomitrella readeri, Physcomitrium australe, Physcomitrium californicum, Physcomitrium collenchymatum, Physcomitrium coloradense, Physcomitrium cupuliferum, Physcomitrium drummondii, Physcomitrium eurystomum, Physcomitrium flexifolium, Physcomitrium hookeri, Physcomitrium hookeri var. serratum, Physcomitrium immersum, Physcomitrium kellermanii, Physcomitrium megalocarpum, Physcomitrium pyriforme, Physcomitrium pyriforme var. serratum, Physcomitrium rufipes, Physcomitrium sandbergii, Physcomitrium subsphaericum, Physcomitrium washingtoniense, Geraniaceae wie die Gattungen Pelargonium, Cocos, Oleum z.B. die Gattungen und Arten Cocos nucifera, Pelargonium grossularioides oder Oleum cocois [Kokusnuss], Gramineae wie die Gattung Saccharum z.B. die Gattung und Art Saccharum officinarum, Juglandaceae wie die Gattungen Juglans, Wallia z.B. die Gattungen und Arten Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra oder Wallia nigra [Walnuss], Lauraceae Wie die Gattungen Persea, Laurus z.B. die Gattungen und Arten Laurus nobilis [Lorbeer], Persea americana, Persea gratissima oder Persea persea [Avocado], Leguminosae wie die Gattung Arachis z.B. die Gattung und Art Arachis hypogaea [Erdnuss], Linaceae wie die Gattungen Linum, Adenolinum z.B. die Gattungen und Arten Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense oder Linum trigynum [Lein], Lythrarieae wie die Gattung Punica z.B. die Gattung und Art Punica granatum [Granatapfel], Malvaceae wie die Gattung Gossypium z.B. die Gattungen und Arten Gossypium hirsutum, Gossypium arboreum, Gossypium barbadense, Gossypium herbaceum oder Gossypium thurberi [Baumwolle], Marchantiaceae wie die Gattung Marchantia z.B. die Gattungen und Arten Marchantia berteroana, Marchantia foliacea, Marchantia macropora, Musaceae wie die Gattung Musa z.B. die Gattungen und Arten Musa nana, Musa acuminata, Musa paradisiaca, Musa spp. [Banane], Onagraceae wie die Gattungen Camissonia, Oenothera z.B. die Gattungen und Arten Oenothera biennis oder Camissonia brevipes [Nachtkerze], Palmae wie die Gattung Elacis z.B. die Gattung und Art Elaeis guineensis [Ölpalme], Papaveraceae wie die Gattung Papaver z.B. die Gattungen und Arten Papaver orientale, Papaver rhoeas, Papaver dubium [Mohn], Pedaliaceae wie die Gattung Sesamum z.B. die Gattung und Art Sesamum indicum [Sesam], Piperaceae wie die Gattungen Piper, Artanthe, Peperomia, Steffensia z.B. die Gattungen und Arten Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata. [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum [Gerste], Secale cereale [Roggen], Avena sativa, Avena fatua, Avena byzantina, Avena fatua var. sativa, Avena hybrida [Hafer], Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia [Reis], Zea mays [Mais] Triticum aestivum, Triticum durum, Triticum turgidum, Triticum hybernum, Triticum macha, Triticum sativum oder Triticum vulgare [Weizen], Porphyridiaceae wie die Gattungen Chroothece, Flintiella, Petrovanella, Porphyridium, Rhodella, Rhodosorus, Vanhoeffenia z.B. die Gattung und Art Porphyridium cruentum, Proteaceae wie die Gattung Macadamia z.B. die Gattung und Art Macadamia intergrifolia [Macadamia], Prasinophyceae wie die Gattungen Nephroselmis, Prasinococcus, Scherffelia, Tetraselmis, Mantoniella, Ostreococcus z.B. die Gattungen und Arten Nephroselmis olivacea, Prasinococcus capsulatus, Scherffelia dubia, Tetraselmis chui, Tetraselmis suecica, Mantoniella squamata, Ostreococcus tauri, Rubiaceae wie die Gattung Coffea z.B. die Gattungen und Arten Cofea spp., Coffea arabica, Coffea canephora oder Coffea liberica [Kaffee], Scrophulariaceae wie die Gattung Verbascum z.B. die Gattungen und Arten Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum oder Verbascum thapsus [Königskerze], Solanaceae wie die Gattungen Capsicum, Nicotiana, Solanum, Lycopersicon z.B. die Gattungen und Arten Capsicum annuum, Capsicum annuum var. glabriusculum, Capsicum frutescens [Pfeffer], Capsicum annuum [Paprika], Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris [Tabak], Solanum tuberosum [Kartoffel], Solanum melongena [Aubergine] Lycopersicon esculentum, Lycopersicon lycopersicum., Lycopersicon pyriforme, Solanum integrifolium oder Solanum lycopersicum [Tomate], Sterculiaceae wie die Gattung Theobroma z.B. die Gattung und Art Theobroma cacao [Kakao] oder Theaceae wie die Gattung Camellia z.B. die Gattung und Art Camellia sinensis [Tee].

Multizelluläre Mikroorganismen, die als transgene nicht-humane Organismen eingesetzt werden können, sind vorzugsweise Protisten oder Diatomeen ausgewählt aus der Gruppe der Familien Dinophyceae, Turaniellidae oder Oxytrichidae wie die Gattungen und Arten: Crypthecodinium cohnii, Phaeodactylum tricornutum, Stylonychia mytilus, Stylonychia pustulata, Stylonychia putrina, Stylonychia notophora, Stylonychia sp., Colpidium campylum oder Colpidium sp.

Die Erfindung betrifft des weiteren ein Verfahren zur Herstellung eines Stoffes, der die Struktur aufweist, die in der folgenden allgemeinen Formel I gezeigt wird 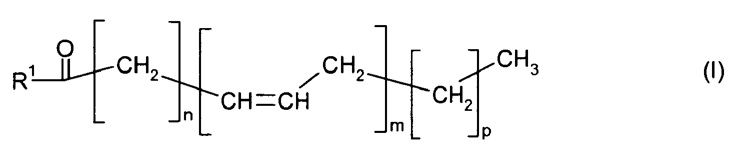 wobei die Variablen und Substiuenten die folgenden sind
- R¹ =: Hydroxyl, Coenzym A (Thioester), Lysophosphatidylcholin, Ly- sophosphatidylethanolamin, Lysophosphatidylglycerol, Lyso- diphosphatidylglycerol, Lysophosphatidylserin, Lysophosphatidylino- sitol, Sphingo- Base oder ein Radikal der Formel II 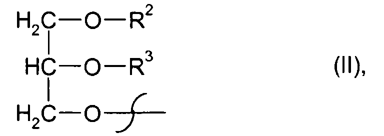
R² = Wasserstoff, Llysophosphatidylcholin, Lysophosphatidylethanolamin, Lysophosphatidylglycerol, Llysodiphosphatidylglycerol, Lysophospha- tidylserin, Lysophosphatidylinositol oder gesättigtes oder ungesättigtes C₂-C₂₄-Alkylcarbonyl,
R³ = Wasserstoff, gesättigtes oder ungesättigtes C₂-C₂₄-Alkylcarbonyl, oder R² und R³ sind unabhängig voneinander ein Radikal der Formel Ia: 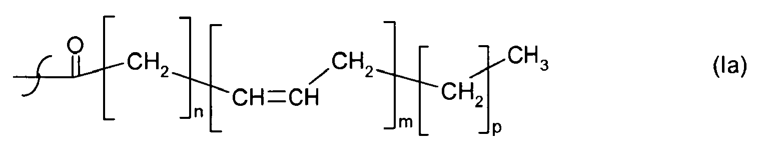
n = 2, 3, 4, 5, 6, 7 or 9, m = 2, 3, 4, 5 oder 6 und p = 0 oder 3;
und wobei das Verfahren das Kultivieren von (i) einer erfindungsgemäßen Wirtszelle oder (ii) eines erfindungsgemäßen transgenen, nicht-humanen Organismus unter Bedingungen umfasst, die die Biosynthese des Stoffes erlauben. Vorzugsweise wird dabei der zuvor genannte Stoff in einer Menge von mindestens 1 Gew.-% bezogen auf den Gesamtgehalt der Lipide in der Wirtszelle oder dem transgenen Organismus bereitgestellt.

R¹ bedeutet in der allgemeinen Formel Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

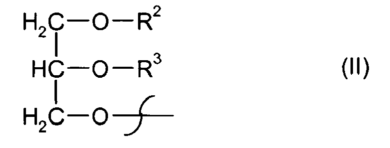

Die oben genannten Reste von R¹ sind immer in Form ihrer Thioester an die Verbindungen der allgemeinen Formel I gebunden.
R² bedeutet in der allgemeinen Formel II Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes C₂-C₂₄-Alkylcarbonyl.

Als Alkylreste seien substituiert oder unsubstituiert, gesättigt oder ungesättigte C₂-C₂₄-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl- genannt, die eine oder mehrere Doppelbindung(en) enthalten. Gesättigte oder ungesättigte C₁₀-C₂₂-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-, die eine oder mehrere Doppelbindung(en) enthalten, sind bevorzugt. Besonders bevorzugt sind gesättigte und/oder ungesättigte C₁₀-C₂₂-Alkylcarbonylreste wie C₁₀-Alkylcarbonyl-, C₁₁-Alkylcarbonyl-, C₁₂-Alkylcarbonyl-, C₁₃-Alkylcarbonyl-, C₁₄-Alkylcarbonyl-, C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Ganz besonders bevorzugt sind gesättigte oder ungesättigte C₁₆-C₂₂-Alkylcarbonylreste wie C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

R³ bedeutet in der allgemeinen Formel II Wasserstoff-, gesättigtes oder ungesättigtes C₂-C₂₄-Alkylcarbonyl.

Als Alkylreste seien substituiert oder unsubstituiert, gesättigt oder ungesättigte C₂-C₂₄-Alkylcarbonyl-Ketten wie Ethylcarbonyl-, n-Propylcarbonyl-, n-Butylcarbonyl-, n-Pentylcarbonyl-, n-Hexylcarbonyl-, n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl- genannt, die eine oder mehrere Doppelbindung(en) enthalten. Gesättigte oder ungesättigte C₁₀-C₂₂-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-, die eine oder mehrere Doppelbindung(en) enthalten, sind bevorzugt. Besonders bevorzugt sind gesättigte und/oder ungesättigte C₁₀-C₂₂-Alkylcarbonylreste wie C₁₀-Alkylcarbonyl-, C₁₁-Alkylcarbonyl-, C₁₂-Alkylcarbonyl-, C₁₃-Alkylcarbonyl-, C₁₄-Alkylcarbonyl-, C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Ganz besonders bevorzugt sind gesättigte oder ungesättigte C₁₆-C₂₂-Alkylcarbonylreste wie C₁₆-Alkylcarbonyl-, C₁₈-Alkylcarbonyl-, C₂₀-Alkylcarbonyl- oder C₂₂-Alkylcarbonylreste, die eine oder mehrere Doppelbindung(en) enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

Die oben genannten Reste von R¹, R² and R³ können mit Hydroxyl- und/oder Epoxygruppen substituierte sein und/oder können Dreifachbindungen enthalten.

Vorteilhaft enthalten die im erfindungsgemäßen Verfahren hergestellten mehrfach ungesättigten Fettsäuren mindestens zwei, vorteilhaft drei, vier, fünf oder sechs Doppelbindungen. Besonders vorteilhaft enthalten die Fettsäuren vier, fünf oder sechs Doppelbindungen. Im Verfahren hergestellte Fettsäuren haben vorteilhaft 18-, 20- oder 22-C-Atome in der Fettsäurekette, bevorzugt enthalten die Fettsäuren 20 oder 22 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, dass im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen.

Vorteilhaft bedeuten die Substituenten R² oder R³ in den allgemeinen Formeln I und II unabhängig voneinander gesättigtes oder ungesättigtes C₁₈-C₂₂-Alkylcarbonyl-, besonders vorteilhaft bedeuten sie unabhängig voneinander ungesättigtes C₁₈-, C₂₀- oder C₂₂-Alkylcarbonyl- mit mindestens zwei Doppelbindungen.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschiedenen Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren, besonders bevorzugt sind die langkettigen Fettsäuren LCPUFAs von C₁₈-, C₂₀- und/oder C₂₂-Fettsäuren.

Im erfindungsgemäßen Verfahren werden vorteilhaft Fettsäureester mit mehrfach ungesättigten C₁₈-, C₂₀- und/oder C₂₂-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft von mindestens fünf oder sechs Doppelbindungen im Fettsäureester hergestellt und führen vorteilhaft zur Synthese von Linolsäure (=LA, C18:2^{Δ9,12}), γ-Linolensäure (= GLA, C18:3^{Δ6,9,12}), Stearidonsäure (= SDA, C18:4 ^{Δ6,9,12,15}), Dihomo-γ-Linolensäure (= DGLA, 20:3,^{Δ8,11,14}), ω-3-Eicosatetraensäure (= ETA, C20:4 ^{Δ5,8,11,14}), Arachidonsäure (ARA, C20:4^{Δ5,8,11,14}), Eicosapentaensäure (EPA, C20:5^{Δ5,8,11,14,17}), ω-6-Docosapentaensäure (C22:5^{Δ4,7,10,13,16}), ω-6-Docosatetraensäure (C22:4^{Δ,7,10,13,16}), ω-3-Docosapentaensäure (= DPA, C22:5^{Δ7,10,13,16,19}), Docosahexaensäure (= DHA, C22:6^{Δ4,7,10,13,16,19}) oder deren Mischungen, bevorzugt ARA, EPA und/oder DHA. Ganz besonders bevorzugt werden, ω-3-Fettsäuren wie EPA und/oder DHA hergestellt.

Die Fettsäureester mit mehrfach ungesättigten C₁₈-, C₂₀- und/oder C₂₂-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei, drei, vier, fünf oder sechs, bevorzugt fünf oder sechs Doppelbindungen enthalten, isoliert werden, vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen, vorteilhaft den Pflanzen, enthalten. In der Regel liegen die verschiedenen vorgenannten Verbindungen (Fettsäureester und freie Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im Verfahren werden die hergestellten LCPUFAs mit einem Gehalt von mindestens 3 Gew.-%, vorteilhaft von mindestens 5 Gew.-%, bevorzugt von mindestens 8 Gew.-%, besonders bevorzugt von mindestens 10 Gew.-%, ganz besonders bevorzugt von mindestens 15 Gew.-% bezogen auf die gesamten Fettsäuren in den transgenen Organismen vorteilhaft in einer transgenen Pflanze hergestellt. Dabei werden vorteilhaft C₁₈- und/oder C₂₀-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie DPA oder DHA, um nur zwei beispielhaft zu nennen, umgesetzt. Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endproduktes betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxyoctadeca-9,11-dienonsäure), Vernonsäure (9,1 0-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11 t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}).
Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Ausgangsorganismus beispielsweise einer Hefe, einer Alge, einem Pilz oder einer Pflanze wie Arabidopsis oder Lein beim Vergleich in der GC-Analyse erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Im Prinzip können alle Gene des Fettsäure- oder Lipidstoffwechsels vorteilhaft in Kombination mit der (den) erfinderischen Polynukleotiden (im Sinne dieser Anmeldung soll der Plural den Singular und umgekehrt beinhalten) im Verfahren zur Herstellung mehrfach ungesättigter Fettsäuren verwendet werden. Vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) verwendet. Bevorzugt werden Gene ausgewählt aus der Gruppe der Δ-4-Desaturasen, Δ-5-Desaturasen, Δ-6-Desaturasen, Δ-8-Desaturasen, Δ-9-Desaturasen, Δ-12-Desaturasen, Δ-15-Desaturasen, Δ-12- und Δ-15-Desaturasen, ω-3-Desaturasen, Δ-6-Elongasen, Δ-9-Elongasen oder Δ-5-Elongasen in Kombination mit den erfindungsgemäßen Polynukleotiden verwendet, wobei einzelne Gene oder mehrere Gene in Kombination verwendet werden können. Hierbei wird für besonders bevorzugte Gen-Kombinationen auf die in den Beipielen gezeigten Tabellen 5 und 6 und die Beispiele verwiesen.

Vorteilhaft setzen die im Verfahren verwendeten Desaturasen ihre jeweiligen Substrate in Form der CoA-Fettsäureester um. Dies führt, wenn vorher ein Elongationsschritt stattgefunden hat, vorteilhaft zu einer erhöhten Produktausbeute. Die jeweiligen Desaturierungsprodukte werden dadurch in höheren Mengen synthetisiert, da der Elongationsschritt in der Regel an den CoA-Fettsäureestern erfolgt, während der Desaturierungsschritt überwiegend an den Phospholipiden oder an den Triglyceriden erfolgt. Eine Ausstauschreaktion, die eine weitere möglicherweise limitierende Enzymreaktion erforderlich machen würde, zwischen den CoA-Fettsäureestern und den Phospholipiden oder Triglyceriden ist somit nicht erforderlich.

Durch die enzymatische Aktivität der im Verfahren verwendeten Polypeptide können unterschiedlichste mehrfach ungesättigte Fettsäuren im erfindungsgemäßen Verfahren hergestellt werden. Je nach Auswahl der für das erfindungsgemäße Verfahren verwendeten Organismen wie den bevorzugte Pflanzen lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder solche, die sich von C18:3-Fettsäuren ableiten, wie SDA, ETA oder EPA. Liegt in der für das Verfahren verwendeten Pflanze als ungesättigte Fettsäure nur Linolsäure (= LA, C18:2^{Δ9,12}) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:3^{Δ9,12,15}) vorhanden, so können als Produkte des Verfahrens nur SDA, ETA, EPA und/oder DHA entstehen, die wie oben beschrieben als freie Fettsäuren oder gebunden vorliegen können. Durch Modifikation der Aktivität der an der Synthese beteiligten Enzyme Δ-5-Desaturase, Δ-6-Desaturase, Δ-4-Desaturase, Δ-12-Desaturase, Δ15-Desaturase, ω3-Desaturase, Δ-5-Elongase und/oder Δ-6-Elongase lassen sich gezielt in den vorgenannten Organismen vorteilhaft in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturase, die Δ-5-Elongase und die Δ-4-Desaturase zusätzlich in die Organismen vorteilhaft in die Pflanze eingebracht, so entstehen zusätzlich ARA, EPA und/oder DHA. Vorteilhaft werden nur ARA, EPA oder DHA oder deren Mischungen synthetisiert, abhängig von den im Organismus bzw. in der Pflanze vorliegenden Fettsäuren, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA, DHA oder deren Mischungen vorteilhaft EPA oder DHA oder deren Mischungen.

Neben der Produktion der Ausgangsfettsäuren für die im erfindungsgemäßen Verfahren verwendeten Polypeptide direkt im Organismus können die Fettsäuren auch von außen gefüttert werden. Aus Kostengründen ist die Produktion im Organismus bevorzugt. Bevorzugte Substrate sind die Linolsäure (C18:2^{Δ9,12}), die y-Linolensäure (C18:3^{Δ6,9,12}), die Eicosadiensäure (C20:2^{Δ11,14}), die Dihomo-y-linolensäure (C20:3^{Δ8,11,14}), die Arachidonsäure (C20:4^{Δ5,8,11,14}), die Docosatetraensäure (C22:4^{Δ7,10,13,16}) und die Docosapentaensäure (C22:5^{Δ4,7,10,13,15}).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit einer erfindungsgemäßen Δ-12-Desaturase und/oder Δ15-Desaturase kodiert, erreicht werden. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art Olea europaea oder der Familie Fabaceae wie der Gattung Glycine z.B. die Gattung und Art Glycine max, die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten erfindungsgemäßen Δ-12-Desaturasen und/oder Δ15-Desaturasen zur Herstellung des Ausgangsprodukts Linolsäure vorteilhaft.

Vorteilhaft werden im Verfahren die vorgenannten Nukleinsäuresequenzen oder deren Derivate oder Homologe eingesetzt, die für Polypeptide codieren, die noch die enzymatische Aktivität der durch Nukleinsäuresequenzen kodierten Proteine besitzen. Diese Sequenzen werden einzeln oder in Kombination mit den erfindungsgemäßen Polynukleotiden in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismus', der die im Verfahren verwendeten Nukleinsäuresequenzen enthält, wobei die Zelle und/oder der Organismus mit einem erfindungsgemäßen Polynukleotiden, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus dem Organismus oder aus der Kultur. Bei der Kultur kann es sich beispielsweise um eine Fermentationskultur beispielsweise im Falle der Kultivierung von Mikroorganismen wie z.B. Mortierella, Thalassiosira, Mantoniella, Ostreococcus, Saccharomyces oder Thraustochytrium oder um eine Treibhaus- oder Feldkultur einer Pflanze handeln. Die so hergestellte Zelle oder der so hergestellte Organismus ist vorteilhaft eine Zelle eines Öl-produzierenden Organismus, wie einer Ölfruchtpflanze wie beispielsweise Erdnuss, Raps, Canola, Lein, Hanf, Erdnuss, Soja, Färberdistel, Hanf, Sonnenblumen oder Borretsch.

Unter Anzucht ist beispielsweise die Kultivierung im Falle von Pflanzenzellen, - gewebe oder -organe auf oder in einem Nährmedium oder der ganzen Pflanze auf bzw. in einem Substrat beispielsweise in Hydrokultur, Blumentopferde oder auf einem Ackerboden zu verstehen.

Als Organismen bzw. Wirtszellen für das erfindungsgemäße Verfahren sind solche geeignet, die in der Lage sind Fettsäuren, speziell ungesättigte Fettsäuren, zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne, Mikroorganismen wie Pilze, beispielsweise die Gattung Mortierella, Thraustochytrium, Saprolegnia, Phytophtora oder Pythium, Bakterien wie die Gattung Escherichia oder Shewanella, Hefen wie die Gattung Saccharomyces, Cyanobakterien, Ciliaten, Algen wie Mantoniella oder Ostreococcus oder Protozoen wie Dinoflagellaten wie Thalassiosira oder Crypthecodinium genannt. Bevorzugt werden Organismen, die natürlicherweise Öle in größeren Mengen synthetisieren können wie Pilze wie Mortierella alpina, Pythium insidiosum, Phytophtora infestans oder Pflanzen wie Soja, Raps, Kokosnuss, Ölpalme, FärberSaflor, Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, Färberdistel, Sonnenblume, Calendula, Mortierella oder Saccharomyces cerevisiae. Prinzipiell sind als Wirtsorganismen neben den vorgenannten transgenen Organismen auch transgene Tiere vorteilhaft nicht-humane Tiere geeignet beispielsweise Caenorhabditis elegans. Weitere geeignete Wirtszellen und Organismen wurden bereits zuvor ausführlich beschrieben.

Transgene Pflanzen, die die im Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze ableiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigte Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch so genanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmes Hexan extrahiert werden. Anschließend wird das Lösungsmittel wieder entfernt. Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf desodoriert.

Vorzugsweise sind die durch dieses Verfahren produzierten PUFAs bzw. LCPUFAs C₁₈-, C₂₀- oder C₂₂-Fettsäuremoleküle, vorteilhaft C₂₀- oder C₂₂-Fettsäuremoleküle mit mindestens zwei Doppelbindungen im Fettsäuremolekül, vorzugsweise drei, vier, fünf oder sechs Doppelbindungen. Diese C₁₈-, C₂₀- oder C₂₂-Fettsäuremoleküle lassen sich aus dem Organismus in Form eines Öls, Lipids oder einer freien Fettsäure isolieren. Geeignete Organismen sind beispielsweise die vorstehend erwähnten. Bevorzugte Organismen sind transgene Pflanzen.

Eine Ausführungsform der Erfindung sind deshalb Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.
Diese Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester- bzw. Fettsäuregemischen bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester- bzw. Fettsäuregemischen in der Regel vorteilhaft nur in Spuren vor, das heißt, sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. Vorteilhaft enthalten die nach dem erfindungsgemäßen Verfahren hergestellten Fettsäureester bzw. Fettsäuregemische weniger als 0,1 % bezogen auf die Gesamtfettsäuren oder keine Buttersäure, kein Cholesterin, keine Clupanodonsäure (= Docosapentaensäure, C22:5^{Δ4,8,12,15,21}) sowie keine Nisinsäure (Tetracosahexaensäure, C23:6^{Δ3,8,12,15,18,21}).

Bevorzugt enthalten die erfindungsgemäßen Öle, Lipide oder Fettsäuren mindestens 0,5%, 1%, 2%, 3%, 4% oder 5%, vorteilhaft mindestens 6%, 7%, 8%, 9% oder 10%, besonders vorteilhaft mindestens 11 %, 12%, 13%, 14% oder 15% ARA oder mindestens 0,5%, 1 %, 2%, 3%, 4% oder 5%, vorteilhaft mindestens 6%, oder 7%, besonders vorteilhaft mindestens 8%, 9% oder 10% EPA und/oder DHA bezogen auf den Gesamtfettsäuregehalt des Produktionsorganismus vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen.

Eine weitere erfindungsgemäße Ausführungsform ist die Verwendung des Öls, Lipids, der Fettsäuren und/oder der Fettsäurezusammensetzung in Futtermitteln, Nahrungsmitteln, Kosmetika oder Pharmazeutika. Die erfindungsgemäßen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, y-Linolensäure, Dihomo-y-Linolensäure, Arachidonsäure, α-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangsorganismus der Anteil der verschiedenen Fettsäuren in dem Öl oder Fett schwanken.

Bei den im Verfahren hergestellten mehrfach ungesättigten Fettsäuren mit vorteilhaft mindestens zwei Doppelbindungen handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wässrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. H₂SO₄. Die Freisetzung der Fettsäuren kann auch direkt ohne die vorhergehend beschriebene Aufarbeitung erfolgen.

Die im Verfahren verwendeten Nukleinsäuren können nach Einbringung in einen Organismus vorteilhaft einer Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in "trans", so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Als Substrate der erfindungsgemäßen Polypeptide des Fettsäure- oder Lipidstoffwechsels, ausgewählt aus der Gruppe Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) eignen sich bevorzugt C₁₆-, C₁₈- oder C₂₀-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der erfindungsgemäßen langkettigen PUFAs müssen die mehrfach ungesättigten C₁₈-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu C₂₀-Fettsäuren, und nach zwei Elongationsrunden zu C₂₂-Fettsäuren. Die Aktivität der erfindungsgemäßen Verfahren verwendeten Desaturasen und Elongasen führt vorzugsweise zu C₁₈-, C₂₀- und/oder C₂₂-Fettsäuren vorteilhaft mit mindestens zwei Doppelbindungen im Fettsäuremolekül, vorzugsweise mit drei, vier, fünf oder sechs Doppelbindungen, besonders bevorzugt zu C₂₀- und/oder C₂₂-Fettsäuren mit mindestens zwei Doppelbindungen im Fettsäuremolekül, vorzugsweise mit drei, vier, fünf oder sechs Doppelbindungen, ganz besonders bevorzugt mit fünf oder sechs Doppelbindungen im Molekül. Nachdem eine erste Desaturierung und die Verlängerung stattfand, können weitere Desaturierungs- und Elongierungsschritte wie z.B. eine solche Desaturierung in Δ-5- und Δ-4-Position erfolgen. Besonders bevorzugt als Produkte des erfindungsgemäßen Verfahrens sind Dihomo-y-linolensäure, Arachidonsäure, Eicosapentaensäure, Docosapetaensäure und/oder Docosahexaensäure. Die C₂₀-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, desaturiert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Werden im erfindungsgemäßen Verfahren als Organismen Mikroorganismus wie Hefen wie Saccharomyces oder Schizosaccharomyces, Pilze wie Mortierella, Aspergillus, Phytophtora, Entomophthora, Mucor oder Thraustochytrium Algen wie Isochrysis, Mantoniella, Ostreococcus, Phaeodactylum oder Crypthecodiniu verwendet, so werden diese Organismen vorteilhaft fermentativ angezogen.

Durch die Verwendung der erfindungsgemäßen Nukleinsäuren, die für eine Desaturase codieren, können im Verfahren die hergestellten mehrfach ungesättigten Fettsäuren mindestens um 5 %, bevorzugt mindestens um 10 %, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Werden im erfindungsgemäßen Verfahren als Organismen Mikroorganismen verwendet, so werden sie je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikontinuierlich oder kontinuierlich nachgefüttert werden. Die hergestellten mehrfach ungesättigten Fettsäuren können nach dem Fachmann bekannten Verfahren wie oben beschrieben aus den Organismen isoliert werden. Beispielsweise über Extraktion, Destillation, Kristallisation, ggf. Salzfällung und/oder Chromatographie. Die Organismen können dazu vorher noch vorteilhaft aufgeschlossen werden.

Das erfindungsgemäße Verfahren wird, wenn es sich bei den Wirtsorganismen um Mikroorganismen handelt, vorteilhaft bei einer Temperatur zwischen 0°C bis 95°, bevorzugt zwischen 10°C bis 85°C, besonders bevorzugt zwischen 15°C bis 75°C, ganz besonders bevorzugt zwischen 15°C bis 45°C durchgeführt.

Der pH Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z.B. Sojaöl, Sonnenblumenöl, Erdnussöl und/oder Kokosfett, Fettsäuren wie z.B. Palmitinsäure, Stearinsäure und/oder Linolsäure, Alkohole und/oder Polyalkohole wie z. B. Glycerin, Methanol und/oder Ethanol und/oder organische Säuren wie z.B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger oder Gas-Form oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natriumhaltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Zitronensäure.

Die zur Kultivierung von Mikroorganismen eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzu gegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder sauerstoffhaltige Gasmischungen, wie z.B. Umgebungsluft, in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere mehrfach ungesättigte Fettsäuren enthaltenden, Fermentationsbrühen haben üblicherweise eine Trockenmasse von 7,5 bis 25 Gew.-%.

Die Fermentationsbrühe kann anschließend weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann schließlich zur Gewinnung der darin enthaltenen Fettsäuren aufgearbeitet werden.

Die Polynukleotide bzw. Polypeptide der vorliegenden Erfindung, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen vorteilhaft in Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Besonders zur Herstellung von PUFAs, beispielsweise Stearidonsäure, Eicosapentaensäure und Docosahexaensäure eignen sich Brasicaceae, Boraginaceen, Primulaceen, oder Linaceen. Besonders vorteilhaft eignet sich Lein (Linum usitatissimum) zur Herstellung von PUFAS mit den erfindungsgemäßen Nukleinsäuresequenzen vorteilhaft, wie beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese C₁₈-Kohlenstoff-Fettsäuren müssen auf C₂₀ und C₂₂ verlängert werden, damit Fettsäuren vom Eicosa- und Docosa-Kettentyp erhalten werden. Mithilfe der im Verfahren verwendeten Desaturasen wie der Δ-12-, Δ-15-, Δ-12- und Δ-15, ω-3-, Δ-4-, Δ-5- und Δ-6-Desaturasen und/oder der Δ-5-, Δ-6-Elongasen können Arachidonsäure, Eicosapentaensäure, Docosapentaensäure oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können C₂₀- und/oder C₂₂-Fettsäuren mit mindestens zwei vorteilhaft mindestens drei, vier, fünf oder sechs Doppelbindungen im Fettsäuremolekül, vorzugsweise C₂₀- oder C₂₂-Fettsäuren mit vorteilhaft vier, fünf oder sechs Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von C₂₀ zu C₂₂-Fettsäuren, zu Fettsäuren wie γ-Linolensäure, Dihomo-γ-linolensäure, Arachidonsäure, Stearidonsäure, Eicosatetraensäure oder Eicosapentaensäure. Substrate der verwendeten Desaturasen und Elongasen im erfindungsgemäßen Verfahren sind C₁₆-, C₁₈- oder C₂₀-Fettsäuren wie zum Beispiel Linolsäure, γ-Linolensäure, α-Linolensäure, Dihomo-γlinolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die synthetisierten C₂₀- oder C₂₂-Fettsäuren mit mindestens zwei, drei, vier, fünf oder sechs Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden. Beispielsweise können Katzen Arachidonsäure nicht mehr synthetisieren.

Unter "Phospholipiden" im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin. Die Begriffe "Produktion oder Produktivität" sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Es umfasst auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff "Effizienz der Produktion" umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff "Ausbeute oder Produkt/Kohlenstoff-Ausbeute" ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe "Biosynthese oder Biosyntheseweg" sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe "Abbau oder Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle), beispielsweise in einem Mehrschritt- und stark regulierten Prozess. Der Begriff "Stoffwechsel" ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Durch den Einsatz der Polynukleotide und optional weiterer Polynukleotide, die für Enzyme des Lipid oder Fettsäurestoffwechsels kodieren, in dem Verfahren können verschiedene vorteilhafte Effekte erzielt werden. So kann die Ausbeute, Produktion und/oder Effizienz der Produktion der mehrfach ungesättigten Fettsäuren in einer Pflanze, bevorzugt in einer Ölfruchtpflanze, oder einem Mikroorganismus beeinflusst werden. Die Anzahl oder Aktivität der erfindungsgemäßen Polypeptide bzw. Polynukleotide kann erhöht werden, so dass größere Mengen der Genprodukte und damit letztlich größere Mengen der Verbindungen der allgemeinen Formel I hergestellt werden. Auch eine de novo Synthese in einem Organismus, dem die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines erfindungsgemäßen Polynukleotids in einen Organismus, allein oder in Kombination mit anderen Genen in eine Zelle kann nicht nur der Biosynthesefluss zum Endprodukt erhöht, sondern auch die entsprechende Triacylglycerin- Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs weiter gesteigert wird. Durch Optimierung der Aktivität oder Erhöhung der Anzahl einer oder mehrerer erfindungsgemäßer Polynukleotide bzw. Polypeptide, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen insbesondere aus Pflanzen zu steigern. Die im Verfahren gewonnenen Fettsäuren eignen sich als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Aus den zuvor gemachten Ausführungen versteht sich, dass die Erfindung auch ein Verfahren zur Herstellung einer Öl-, Lipid- oder Fettsäurezusammensetzung umfassend die Schritte des erfindungsgemäßen Verfahrens und den weiteren Schritt des Formulierens des Stoffes als Öl-, Lipid- oder Fettsäurezusammensetzung betrifft.

In einer bevorzugten Ausführungsform dieses Verfahren wird die Öl-, Lipid- oder Fettsäurezusammensetzung weiter formuliert zu einem Arzneimittel, zu Kosmetik, zu einem Nahrungsmittel, zu einem Futtermittel, vorzugsweise Fischfutter, oder zu einem Nahrungsergänzungsmittel.

Schließlich betrifft die Erfindung grundsätzlich die Verwendung des Polynukleotids, des Vektors, der Wirtszelle, des Polypeptids oder des transgenen, nicht-humanen Organsismus der vorliegenden Erfindung für die Herstellung einer Öl-, Lipid- oder Fettsäurezusammensetzung. Diese ist dann bevorzugt als Arzneimittel, Kosmetik, Nahrungsmittel, Futtermittel, vorzugsweise Fischfutter, oder Nahrungsergänzungsmittel einzusetzen.

Der Inhalt sämtlicher in dieser Patentanmeldung zitierten Literaturstellen, Patentanmeldungen, Patente und veröffentlichten Patentanmeldungen ist hiermit durch Bezugnahme auf den jeweiligen speziellen Offenbarungsgehalt aufgenommen.

### Figuren

Figur 1: Biosynthesewege zur Herstellung langkettiger, mehrfach ungesättigter Fettsäuren, wie Arachidonsäure (= ARA, C20:4^{Δ5,8,11,14}), Eicosapentaensäure (= EPA, C20:5^{Δ5,8,11,14,17}) oder Docosahexaensäure (= DHA, C22:6^{Δ4,7,10,13,16,19}).

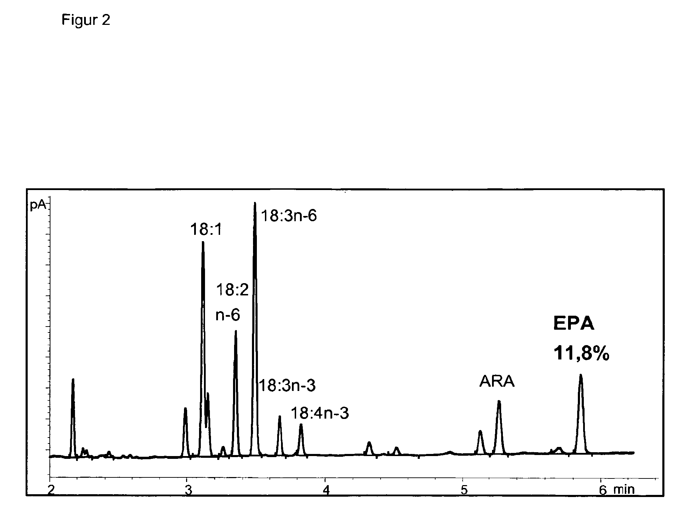

Figur 2: Chromatogramm von Samen einer Rapspflanze transformiert mit dem in den Beispielen beschriebenen Konstrukt LJB765. In nicht-transformierten Rapssamen kann nur 16:0, 16:1, 18:0, 18:1, 18:2n-6, 18:3n-3, 20:0, 20:1, 20:2n-6 und 22:0 detektiert werden. Das Chromatogramm belegt die Herstellung von neuen Fettsäuren in den transgenen Rapssamen. Diese neuen Fettsäuren gehen auf die Aktivität der eingebrachten Gene zurück (siehe EPA, Eicosapentaensäure). Im Vergleich zur nicht-transformierten Kontrolle zeigen die Samen der transgenen Pflanze erhöhte Gehalte an d12-desaturiertem Produkt (C18:2n-6, Linolsäure) und d15-desaturierten Produkten (18:3n-3; 18:4n-3; EPA), die auf die Aktivität der eingebrachten d12- und d15-Desaturase zurück gehen.
Figur 3: Chromatogramm von Samen transgener Arabidopsis Pflanzen, transformiert mit dem in den Beispielen beschriebenen Plasmid LJB765. Ähnliche Werte für die omega3-Fettsäure EPA wie im Raps konnten erhalten werden.
Figur 4: Gaschromatographische Bestimmung der Fettsäuren aus Hefen, die mit dem Plasmid pYES (A) bzw. pYES-d12Des(Nh) (B), pYES-d12Des(Mb) (C), pYES-d12Des(Mg) (D) und pYES-d12Des(Pb) (E) transformiert wurden.
Figur 5: Gaschromatographische Bestimmung der Fettsäuren aus Hefen, die mit dem Plasmid pYES (A) bzw. pYES-d15Des(Nh)2 (B), pYES-d15Des(Mg) (C), pYES-d15Des(Hr) (D), pYES-d15Des(Lg) (E) und pYES-d15Des(Mc) (F) transformiert wurden. Die Fettsäure 18:2n-6 wurde zugefüttert.
Figur 6: Gaschromatographische Bestimmung der Fettsäuren aus Hefen, die mit dem Plasmid pYES (A) bzw. pYES-d15Des(Hr) (B), pYES-d15Des(Lg) (C) und pYES-d15Des(Mc) (D) transformiert wurden. Die Fettsäure 20:2n-6 wurde zugefüttert.
Figur 7: Gaschromatographische Bestimmung der Fettsäuren aus Hefen, die mit dem Plasmid pYES (A) bzw. pYES-d15Des(Hr) (B), pYES-d15Des(Lg) (C) und pYES-d15Des(Mc) (D) transformiert wurden. Die Fettsäure 22:4n-6 wurde zugefüttert.
Figur 8: Gaschromatographische Bestimmung der Fettsäuren aus Arabidopsis Samen der fad3-Mutante, die mit dem Plasmid pSUN (A) bzw. pSUN-d15Des(Mg) (SEQ ID Nr: 183; B) und pYES-d15Des(Nh)2 (SEQ ID Nr: 184; C) transformiert wurden.

### Beispiele

### Beispiel 1: Allgemeine Klonierungsverfahren

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

### Beispiel 2: Sequenzanalyse rekombinanter DNA

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

### Beispiel 3: Lipidextraktion aus Hefen

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der eindeutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (z. B. Sigma), definiert werden.

### Beispiel 4: Klonierung von Desaturase Genen

Durch Suche nach konservierten Bereichen in Proteinsequenzen der Organismen Nectria haematococca, Trichoderma resii, Monosiga brevicollis, *Mycosphaerella fijiensis* Mycospaerella graminicola, Naegleria gruberi, Phycomyces blakesleeanus, Laccaria bicolor, *Helobdella robusta, Lottia gigantea, Microcoleus chthonoplastes,* Nematostella vectensis , Physcomitrella patens, Postia placenta, Selaginella moellendorffii, und Microdochium nivale konnten Sequenzen mit entsprechenden Motiven für Δ12- und/oder Δ15-Desaturasen identifiziert werden: Diese Motive sind insbesondere das Desaturase Motiv 1 "LXXITXLXH" (SEQ ID NR: 83), das Desaturase Motiv 2 "GAXXTXDR" (SEQ ID NR: 84) und das Desaturase Motiv 3 "HVXHH" (SEQ ID NR: 85), wobei X jede Aminosäure bedeutet.

Zur Unterscheidung von Δ12, Δ15 und omega 3 Desaturasen wurden die folgenden Motive benutzt, die an derselben Position im Alignment der Proteinsequenzen dieser Desaturasen lokalisiert sind: Anhand des Motiv 4 "TXXQHXX" (SEQ ID NR: 113), insbesondere anhand des Motiv 5 "T(YF)(LM)QH(TSQ)(DHN)" (SEQ ID NR: 114), noch spezifischer anhand Motiv 6 "T(YF)LQH(TS)(DH)" (SEQ ID NR: 115) wurden Δ12 Desaturasen identifiziert.

Anhand des Motiv 7 "TXXHHX(G--)(X--)X" (SEQ ID NR: 116), insbesondere anhand Motiv 8 "T(YF)(LM)HH(HTSV)(G--)(HY--)(HDEG)" (SEQ ID NR: 117), insbesondere anhand Motiv 9 "T(YF)(LM)HH(HTS)(HDE)" (SEQ ID NR: 118) oder alternativ anhand Motiv 10 "T(YF)(LM)HH(HTSV)G(HY) (HDEG)" (SEQ ID 119), wurden d15 Desaturasen identifiziert.

Omega 3 Desaturasen wurden vorzugsweise anhand von Motiv 11 "TXXHHNX" (SEQ ID NR: 120), insbesondere anhand des Motiv 12 "T(YF)(LM)HHN(HDEG)" (SEQ ID NR: 121) identifiziert.

In einem ersten Schritt wurden vollständige genomische Sequenzen entsprechend von Datenbankeinträgen identifiziert. In einem weiteren Schritt wurde anhand von bioinformatischen Methoden die kodierende Sequenz extrahiert. Um die entsprechende kodierende Sequenz aus den Organismen zu erhalten, können aus cDNA-Präparationen mit den in Tabelle 1 definierten Primersequenzen in einer PCR Reaktion amplifiziert werden. Dabei können Fragmente erhalten werden, wie sie in Tabelle 2 beschrieben sind.

**Tabelle 1: Primersequenzen für die Klonierung der identifizierten Desaturasen.**

| Gen-Name | Organismus | Primer-Sequenz (5'-3') | SEQ ID NR: |
|---|---|---|---|
| D12Des(Nh) | Nectria haematococca | Forward: atggcttctacggctgtgcc | 34 |
| | | Reverse: ttaggcggcgggagggtcg | 35 |
| D15Des(Nh) | Nectria | Forward: atggctactcgacagcgtac | 36 |
| | haematococca | | |
| | | Reverse : ctactccttggcccatcgcatg | 37 |
| D15Des(Tr) | Trichoderma resii | Forward : atggctactaccacgacggt | 38 |
| | | | 39 |
| D12Des(Mb) | Monosiga brevicollis | Forward : atgacggtggcttcccaggtg | 40 |
| | | Reverse : ttacctgtccttgaaccaaag | 41 |
| D15Des(Mg) | Mycospaerella graminicola | Forward : atgagcagaacagtcacatta | 42 |
| | | Reverse : tcacgcgctcttaacatgcg | 43 |
| D12Des(Mg) | Mycospaerella graminicola | Forward: atgagcaccaccgccctctc | 44 |
| | | Reverse: ctattcggaatcatcctca | 45 |
| D15Des(Ng) | Naegleria gruberi | Forward: atgtcagctgccacatcaga | 46 |
| | | Reverse: ttaatgatcccaccacaaaa | 47 |
| D12Des(Pb) | Phycomyces blakesleeanus | Forward: atgtcggataacactgaatc | 48 |
| | | Reverse: ttaattcttgaggaaacgaa | 49 |
| D15Des(Nv) | Nematostella vectensis | Forward: atgccgccgtgtcacgcaac | 50 |
| | | Reverse: ttagtccttttcacagttttc | 51 |
| D15Des(Lb) | Laccaria bicolor | Forward: atggctgttaaaacggac | 88 |
| | | Reverse: ctatttttcaacctcaatac | 89 |
| d15Des(Hr) | *Helobdella robusta* | Forward: atgaactgtgtaactgagg | 93 |
| | | Reverse: tatttgtaataaacctctaac | 94 |
| d15Des(Lg)1 | *Lottia gigantea* | Forward: atggaaaccaaatcaggaag | 95 |
| | | Reverse: ttatgtataaatatgtac | 96 |
| d15Des(Lg)2 | *Lottia gigantea* | Forward: atgaatgaagccaataaccac | 97 |
| | | Reverse: ttatttatagtaatgaattttg | 98 |
| d15Des(Mc) | *Microcoleus chthonoplastes* | Forward: atgcaatcaaacacagttc | 99 |
| | | Reverse: ttagtttgatcgcggatgtttg | 100 |
| d15Des(Mf) | *Mycosphaerella fijiensis* | Forward: atgtttctcgccggaagtgatg | 101 |
| | | Reverse: tcaggctcctagatctttcc | 102 |
| d15Des(Pp) | *Physcomitrella patens* | Forward: atggatcaagccagtaagattg | 159 |
| | | Reverse: ttatctgcaattgcactgttttg | 160 |
| d15Des(Pp2) | *Physcamitrella patens* | | 161 |
| | | Reverse: tcactgctttacatcgttctg | 162 |
| d15Des(Pp3) | *Physcomitrella patens* | Forward: atgtccgtgaagcatgagat | 163 |
| | | Reverse: tcacttagcatttgtgctcttc | 164 |
| d15Des(Ppla) | *Postia placenta* | Forward: atggctaccacggctgattctg | 165 |
| | | Reverse: tcacttagcatttgtgctcttc | 166 |
| d15Des(Sm) | *Selaginella moellendorffii* | Forward: atggtcgctgttccactccg | 167 |
| | | Reverse: ttacttcaagcctggatcgctc | 168 |
| d15Des(Mn) | *Microdochium nivale* | | 169 |
| | | Reverse: ctaaaggtccttgcggggtg | 170 |

**Tabelle 2: Codierende Polynukleotid- bzw. Aminosäuresequenzen der identifizierten Desaturasen.**

| Gen-Name | Organismus | Nukleotide in bp | SEQ ID NR: | Aminosäuren | SEQ ID |
|---|---|---|---|---|---|
| D12Des(Nh) | Nectria | 1437 | 2 | 478 | 3 |
| | haematococca | | | | |
| D15Des(Nh) | Nectria haematococca | 1203 | 5 | 400 | 6 |
| D15Des(Tr) | Trichoderma resii | 1173 | 8 | 390 | 9 |
| D12Des(Mb) | Monosiga brevicollis | 1149 | 11 | 382 | 12 |
| D15Des(Mg) | Mycospaerella graminicola | 1191 | 14 | 396 | 15 |
| D12Des(Mg) | Mycospaerella graminicola | 1449 | 17 | 482 | 18 |
| D15Des(Ng) | Naegleria gruberi | 1197 | 20 | 398 | 21 |
| D12Des(Pb) | Phycomyces blakesleeanus | 1170 | 23 | 389 | 24 |
| D15Des(Nv) | Nematostella vectensis | 990 | 26 | 329 | 27 |
| D15Des(Lb) | Laccaria bicolor | 1323 | 91 | 440 | 92 |
| d15Des(Hr) | *Helobdella robusta* | 1128 | 123 | 375 | 124 |
| d15Des(Lg)1 | *Lottia gigantea* | 1101 | 126 | 366 | 127 |
| d15Des(Lg)2 | *Lottia gigantea* | 1113 | 129 | 370 | 130 |
| d15Des(Mc) | *Microcoleus chthonoplastes* | 1077 | 132 | 358 | 133 |
| d15Des(Mf) | *Mycosphaerella fijiensis* | 1191 | 135 | 396 | 136 |
| d15Des(Pp) | *Physcomitrella patens* | 1185 | 143 | 394 | 144 |
| d15Des(Pp2) | *Physcomitrella patens* | 1143 | 146 | 380 | 147 |
| d15Des(Pp3) | *Physcomitrella patens* | 1095 | 149 | 364 | 150 |
| d15Des(Ppla) | *Postia placenta* | 1284 | 152 | 427 | 153 |
| d15Des(Sm) | *Selaginella moellendorffii* | 1125 | 155 | 374 | 156 |
| d15Des(Mn) | *Microdochium nivale* | 1209 | 157 | 402 | 158 |

**Tabelle 3: Genomische Sequenzen der identifizierten Desaturasen.**

| Gen-Name | Organismus | Nukleotide in bp | SEQ ID |
|---|---|---|---|
| D12Des(Nh) | Nectria haematococca | 2258 | 1 |
| D15Des(Nh) | Nectria haematococca | 1996 | 4 |
| D15Des(Tr) | Trichoderma resii | 1988 | 7 |
| D12Des(Mb) | Monosiga brevicollis | 2520 | 10 |
| D15Des(Mg) | Mycospaerella graminicola | 1179 | 13 |
| D12Des(Mg) | Mycospaerella graminicola | 2301 | 16 |
| D15Des(Ng) | Naegleria gruberi | 1906 | 19 |
| D12Des(Pb) | Phycomyces blakesleeanus | 1740 | 22 |
| D15Des(Nv) | Nematostella vectensis | 2243 | 25 |
| D15Des(Lb) | Laccaria bicolor | 2336 | 90 |
| d15Des(Hr) | *Helobdella robusta* | 1975 | 122 |
| d15Des(Lg)1 | *Lottia gigantea* | 1390 | 125 |
| d15Des(Lg)2 | *Lottia gigantea* | 1513 | 128 |
| d15Des(Mc) | *Microcoleus chthonoplastes* | 3060 | 131 |
| d15Des(Mf) | *Mycosphaerella fijiensis* | 1191 | 134 |
| d15Des(Pp) | *Physcomitrella patens* | 3201 | 142 |
| d15Des(Pp2) | *Physcomitrella patens* | 1543 | 145 |
| d15Des(Pp3) | *Physcomitrella patens* | 1495 | 148 |
| d15Des(Ppla) | *Postia placenta* | 2468 | 151 |
| d15Des(Sm) | *Selaginella moellendorffii* | 4066 | 154 |

Zur Charakterisierung der Funktionen der einzelnen Sequenzen wird der offene Leserahmen der DNA (Tab. 2) stromabwärts des Galactose-induzierbaren GAL1-Promotors von pYES2.1/V5-His-TOPO (Invitrogen) kloniert, wobei die Plasmide pYES-D12Des(Nh), pYES-D15Des(Nh), pYES-D15Des(Tr), pYES-D12Des(Mb), pYES-D15Des(Mg), pYES-D12Des(Mg), pYES-D15Des(Ng), pYES-D12Des(Pb), pYES-D15Des(Nv), pYES-D15Des(Lb), pYES-D15Des(Hr), pYES-D15Des(Lg)1, pYES-D15Des(Lg)2, pYES-D15Des(Mc), pYES-D15Des(Mf), pYes-D15Des(Pp), pYes-D15Des(Pp2), pYes-D15Des(Pp3), pYes-D15Des(Ppla), oder pYes-D15Des(Sm), entstehen. Diese Plasmide können dann nach Herstellerangaben in den Hefestamm INVSC-1 (Invitrogen) transformiert und auf Platten mit DOB-U Agar basierend auf Uracil Auxotrophie selektioniert werden. Positive Kolonien werden durch PCR identifiziert. Dazu wird jeweils mit 1 µl aufgetauten Zellen, 200 µM dNTPs, 2,5 U Taq-Polymerase und 100 pmol eines jeden Primers in einem Gesamtvolumen von 50 µl durchgeführt. Die Bedingungen für die PCR sind wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten. Parallel wird der leere Vektor pYES2.1/V5-His-TOPO in der beschriebenen Art und Weise in kompetente Hefezellen des Stamms INVSC-1 transformiert. Hefezellen mit den Plasmiden pYES-D12Des(Nh), pYES-D15Des(Nh), pYES-D15Des(Tr), pYES-D12Des(Mb), pYES-D15Des(Mg), pYES-D12Des(Mg), pYES-D15Des(Ng), pYES-D12Des(Pb), pYES-D15Des(Nv), pYES-D15Des(Lb), pYES-D15Des(Hr), pYES-D15Des(Lg)1, pYES-D15Des(Lg)2, pYES-D15Des(Mc), pYES-D15Des(Mf), pYes-D15Des(Pp), pYes-D15Des(Pp2), pYes-D15Des(Pp3), pYes-D15Des(Ppla), oder pYes-D15Des(Sm), werden 12 h in flüssigem DOB-U Medium bei 28 °C, 200 rpm inkubiert und dann weitere 12 h in Induktionsmedium (DOB-U+2% (w/v) Galaktose+ 2% (w/v) Raffinose) sowie 250 µM von ins Medium zugegebenen Fettsäuren angezogen. Anhand der zugegebenen Fettsäuren lässt sich die Spezifität und Aktivität des zu charakterisierenden Gens bestimmen.

Hefen, die mit den Plasmiden pYES2/V5-His-TOPO oder pYES-D12Des(Nh), pYES-D15Des(Nh), pYES-D15Des(Tr), pYES-D12Des(Mb), pYES-D15Des(Mg), pYES-D12Des(Mg), pYES-D15Des(Ng), pYES-D12Des(Pb), pYES-D15Des(Nv), pYES-D15Des(Lb), pYES-D15Des(Hr), pYES-D15Des(Lg)1, pYES-D15Des(Lg)2, pYES-D15Des(Mc), pYES-D15Des(Mf), pYes-D15Des(Pp), pYes-D15Des(Pp2), pYes-D15Des(Pp3), pYes-D15Des(Ppla), oder pYes-D15Des(Sm), transformiert werden, werden folgendermaßen analysiert:

Die Hefezellen aus den Hauptkulturen werden durch Zentrifugation (100 x g, 5 min, 20°C) geerntet und mit 100 mM NaHCO₃ pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgt durch zweimalige Extraktion mit Petrolether (PE). Zur Entfernung nicht derivatisierter Fettsäuren werden die organischen Phasen je einmal mit 2 ml 100 mM NaHCO₃, pH 8,0 und 2 ml Aqua dest. gewaschen. Anschließend wurden die PE-Phasen mit Na₂SO₄ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wurde von 50°C bis 250°C mit einer Rate von 5°C/min und schließlich 10 min bei 250°C (halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

### Aktivitäts- und Substratbestimmung der identifizierten Desaturasen

Die Substratspezifität von D12Des(Nh), D15Des(Nh), D15Des(Tr), D12Des(Mb), D15Des(Mg), D12Des(Mg), D15Des(Ng), D12Des(Pb), D15Des(Nv), D15Des(Lb), D15Des(Hr), D15Des(Lg)1, D15Des(Lg2), D15Des(Mc), D15Des(Mf),D15Des(Pp), D15Des(Pp2), D15Des(Pp3), D15Des(Ppla), oder D15Des(Sm), kann nach Expression und Fütterung verschiedener Fettsäuren ermittelt werden. Ausgehend von den konservierten Elementen wurde folgende Aktivität für die kodierenden Sequenzen gefunden (Tab. 4).

**Tabelle 4: Aktivität der identifizierten Desaturasen**

| Gen-Name | Organismus | Aktivität | Polynukleotid SEQ ID NR: | Polypeptid SEQ ID NR: |
|---|---|---|---|---|
| D12Des(Nh) | Nectria haemato-cocca | Δ12-Desaturase- | 2 | 3 |
| D15Des(Nh) | Nectria haemato-cocca | Δ15-Desaturase- | 5 | 6 |
| D15Des(Tr) | Trichoderma resii | Δ15-Desaturase | 8 | 9 |
| D12Des(Mb) | Monosiga brevicollis | Δ12-Desaturase | 11 | 12 |
| D15Des(Mg) | Mycospaerella graminicola | Δ15-Desaturase | 14 | 15 |
| D12Des(Mg) | Mycospaerella graminicola | Δ12-Desaturase | 17 | 18 |
| D15Des(Ng) | Naegleria gruberi | Δ15-Desaturase- | 20 | 21 |
| D12Des(Pb) | Phycomyces blakesleeanus | Δ12-Desaturase | 23 | 24 |
| D15Des(Nv) | Nematostella vectensis | Δ15-Desaturase- | 26 | 27 |
| D15Des(Lb) | Laccaria bicolor | Δ15-Desaturase | 91 | 92 |
| d15Des(Hr) | *Helobdella robusta* | Δ15-Desaturase | 123 | 124 |
| d15Des(Lg)1 | Lottia gigantea | Δ15-Desaturase | 126 | 127 |
| d15Des(Lg)2 | Lottia gigantea | Δ15-Desaturase | 129 | 130 |
| d15Des(Mc) | Microcoleus chthonoplastes | Δ15-Desaturase- | 132 | 133 |
| d15Des(Mf) | Mycosphaerella fijiensis | Δ15-Desaturase | 135 | 136 |
| d15Des(Pp) | *Physcomitrella patens* | (15-Desaturase | 143 | 144 |
| d15Des(Pp2) | Physcomitrella patens | (15-Desaturase | 146 | 147 |
| d15Des(Pp3) | Physcomitrella patens | (15-Desaturase | 149 | 150 |
| d15Des(Ppla) | *Postia placenta* | Δ15-Desaturase | 152 | 153 |
| d15Des(Sm) | *Selaginella moellendorffii* | Δ15-Desaturase | 155 | 156 |
| d15Des(Mn) | *Microdochium nivale* | Δ15-Desaturase | 157 | 158 |

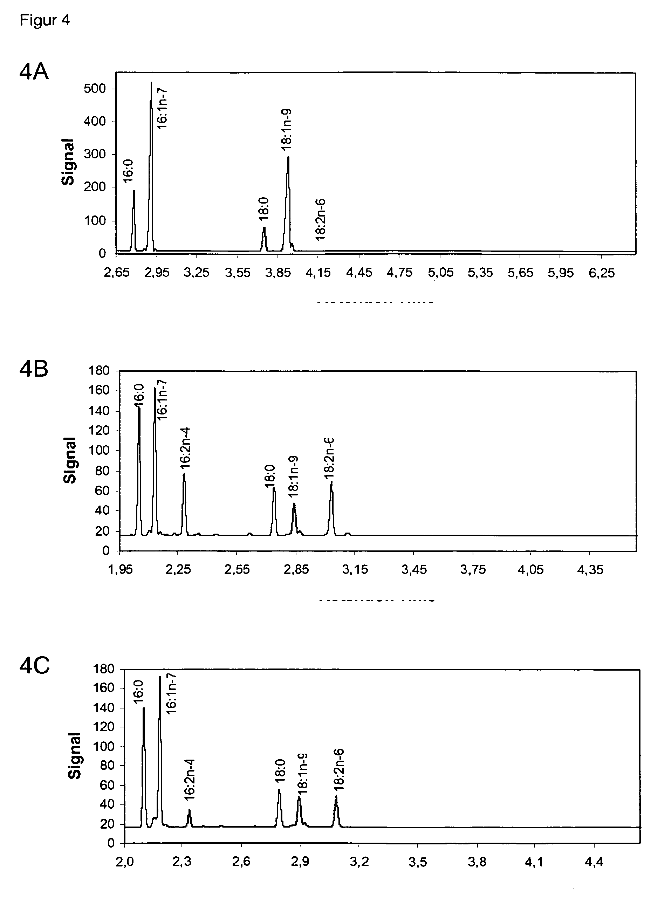

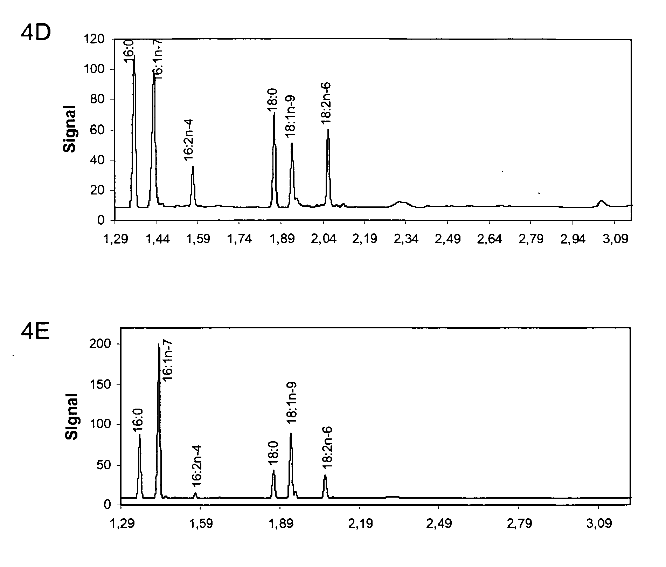

Diese gefundenen Aktivitäten wurden mittels Expression der Desaturasen in Hefe zusätzlich überprüft. In Tabelle 4A ist die Umsetzung verschiedener Fettsäure-Substrate zu den erwarteten Fettsäure Produkten aufgelistet. Außer der Fettsäure 18:1 n-9 wurden alle Substrate im Experiment gefüttert und sind daher im Überschuss vorhanden. Abbildung 4, 5, 6 und 7 zeigen die Chromatogramme der einzelnen Experimente.

**Tabelle 4A: Fütterung von Hefen**

| **Proben-name/gefütterte Fettsäure** | **Beobachteter Reaktionsschritt** | | | | **Umsetzungsrate (%)** | | **Beob. Aktivität** | **Abb.** |
|---|---|---|---|---|---|---|---|---|
| | Substrat (Flächenein-heiten) | | Produkt (Flächenein-heiten) | | Erwar-tet | Beob. | | |
| pYES2 Leervektor | 18:1 n-9 | 139,3 | 18:2n-6 | 0,7 | - | - | - | **4a** |
| d12Des(Nh) | 18:1n-9 | 10,5 | 18:2n-6 | 14,8 | > 0 | 59 | Δ12-Des. | **4b** |
| d12Des(Mb) | 18:1n-9 | 12,0 | 18:2n-6 | 9,9 | > 0 | 45 | A12-Des. | **4c** |
| d12Des(Mg) | 18:1n-9 | 33,8 | 18:2n-6 | 36,0 | > 0 | 52 | Δ12-Des. | **4d** |
| d12Des(Pb) | 18:1n-9 | 55,3 | 18:2n-6 | 18,0 | > 0 | 25 | Δ12-Des. | **4e** |
| pYES2 Leervektor / 18:2n-9 | 18:2n-6 | 49,3 | 18:3n-3 | 0,0 | - | - | - | **5a** |
| d15Des(Nh)2 / 18:2n-9 | 18:2n-6 | 79,3 | 18:3n-3 | 9,1 | > 0 | 10 | Δ15-Des. | **5b** |
| d15Des(Mg) / 18:2n-9 | 18:2n-6 | 66,0 | 18:3n-3 | 15,3 | > 0 | 19 | Δ15-Des. | **5c** |
| d15Des(Hr) / 18:2n-9 | 18:2n-6 | 3,8 | 18:3n-3 | 1,5 | > 0 | 29 | Δ15-Des. | **5d** |
| d15Des(Lg)2 / 18:2n-9 | 18:2n-6 | 4,3 | 18:3n-3 | 2,2 | > 0 | 34 | Δ15-Des. | **5e** |
| d15Des(Mc) / 18:2n-9 | 18:2n-6 | 3,2 | 18:3n-3 | 2,1 | > 0 | 40 | Δ5-Des. | **5f** |
| pYES2 Leervektor / 20:2n-6 | 20:2n-6 | 27,7 | 20:3n-6 | 0,0 | - | - | - | **6a** |
| d15Des(Hr) / 20:2n-6 | 20:2n-6 | 17,4 | 20:3n-6 | 2,3 | > 0 | 12 | ω3-Des. | **6b** |
| d15Des(Lg)2 / 20:2n-6 | 20:2n-6 | 17,0 | 20:3n-6 | 4,6 | > 0 | 21 | ω3-Des. | **6c** |
| d15Des(Mc)/20:2n-6 | 20:2n-6 | 11,2 | 20:3n-6 | 7,5 | >0 | 40 | ω3-Des. | **6d** |
| pYES2 Leervektor / 20:4n-6 | 20:4n-6 | 14,1 | 20:5n-3 | - | - | - | - | **7a** |
| d15Des(Hr) / 20:4n-6 | 20:4n-6 | 15,6 | 20:5n-3 | 2,8 | > 0 | 15 | ω3-Des. | **7b** |
| d15Des(Lg)2/20:4n-6 | 20:4n-6 | 9,3 | 20:5n-3 | 10,7 | > 0 | 54 | ω3-Des. | **7c** |
| d15Des(Mc) / 20:4n-6 | 20:4n-6 | 19,4 | 20:5n-3 | 2,6 | > 0 | 12 | ω3-Des. | **7d** |

Als Kontrolle für den Test auf Δ12-Desaturase Aktivität wurden Hefen nur mit dem pYES Leervektor transformiert und das Fettsäureprofil analysiert (Abbildung 4A). Im Vergleich hierzu ist bei Hefen, welche die Desaturasen d12Des(Nh), d12Des(Mb), d12Des(Mg) und d12Des(Pb) exprimieren, die zusätzliche Fettsäure 18:2n-6 zu beobachten (Abbildung 4B, 4C, 4D, 4E). Diese getesteten Desaturasen haben daher Δ12-Desaturase Aktivität.

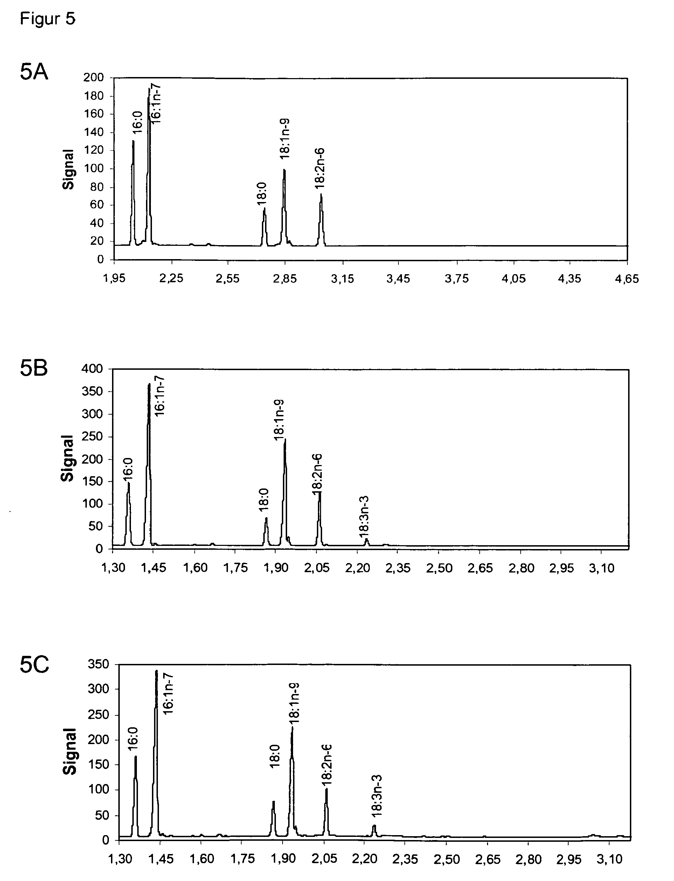

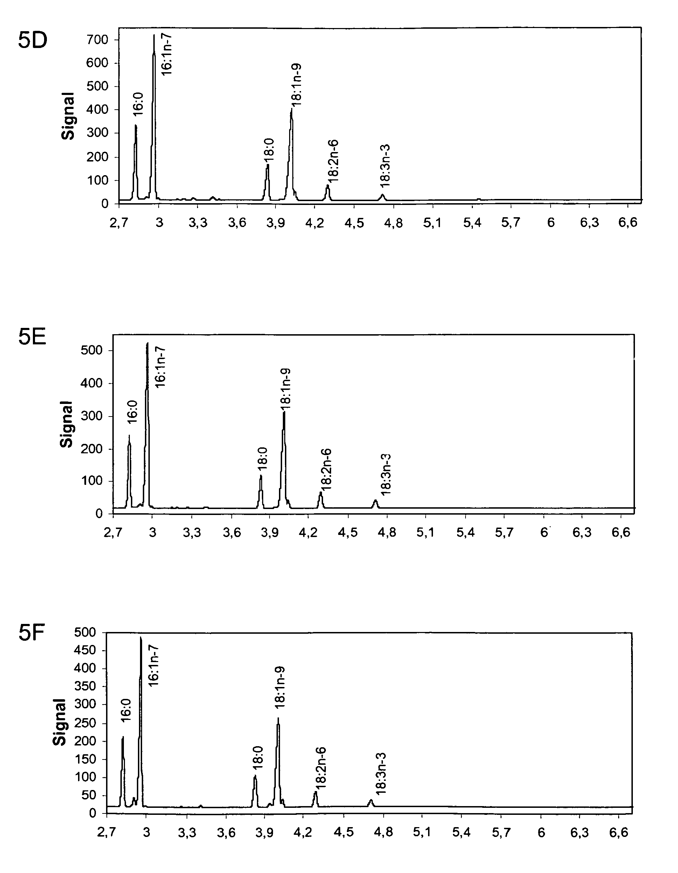

Als Kontrolle für den Test auf Δ15-Desaturase Aktivität wurden Hefen mit dem pYES Leervektor transformiert, die Fettsäure 18:2n-6 gefüttert und das Fettsäureprofil analysiert (Abbildung 5A). Im Vergleich hierzu ist bei Hefen, welche die Desaturasen d15Des(Nh)2, d15Des(Mg), d15Des(Hr), d15Des(Lg) und d15Des(Mc) exprimieren, die zusätzliche Fettsäure 18:3n-3 zu beobachten (Abbildung 5B, 5C, 5D, 5E, 5F). Diese getesteten Desaturasen haben daher Δ15-Desaturase Aktivität.

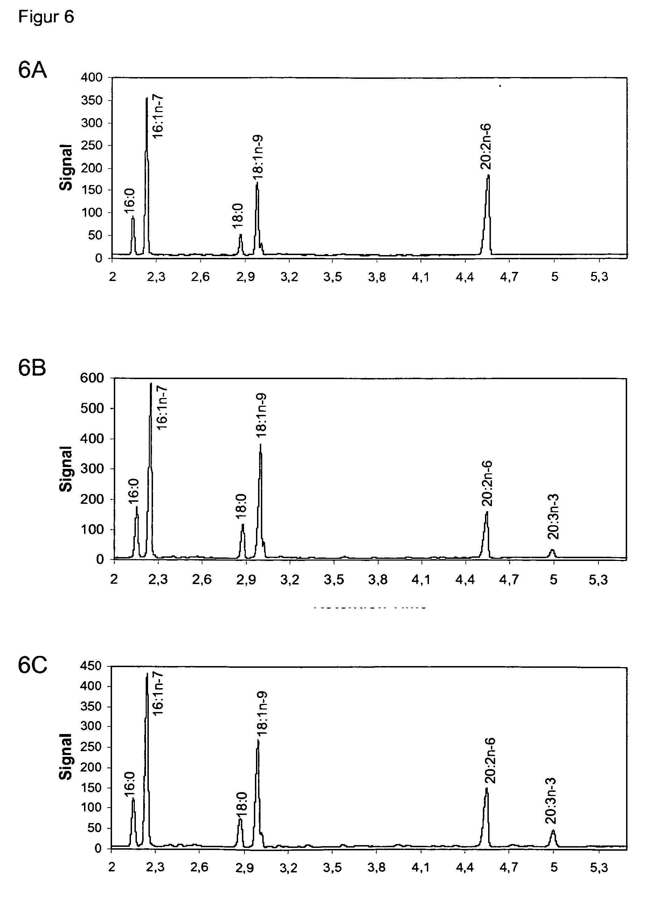

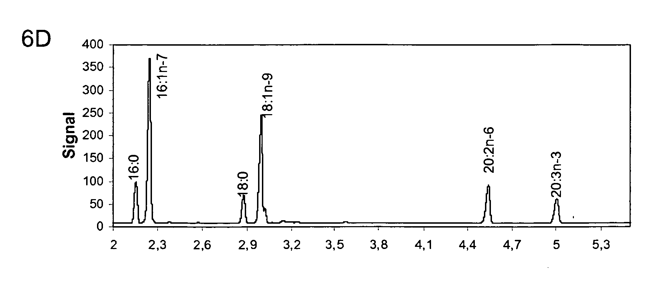

Als Kontrolle für den Test auf ω3-Desaturase Aktivität bei Verwendung von 20:2n-6 als Substrat wurden Hefen mit dem pYES Leervektor transformiert, die Fettsäure 20:2n-6 gefüttert und das Fettsäureprofil analysiert (Abbildung 6A). Im Vergleich hierzu ist bei Hefen, welche die Desaturasen d15Des(Hr), d15Des(Lg), d15Des(Mc) exprimieren, die zusätzliche Fettsäure 20:3n-3 zu beobachten (Abbildung 6B, 6C, 6D). Diese getesteten Desaturasen haben daher ω3-Desaturase Aktivität denen 20:2n-6 als Substrat dient.

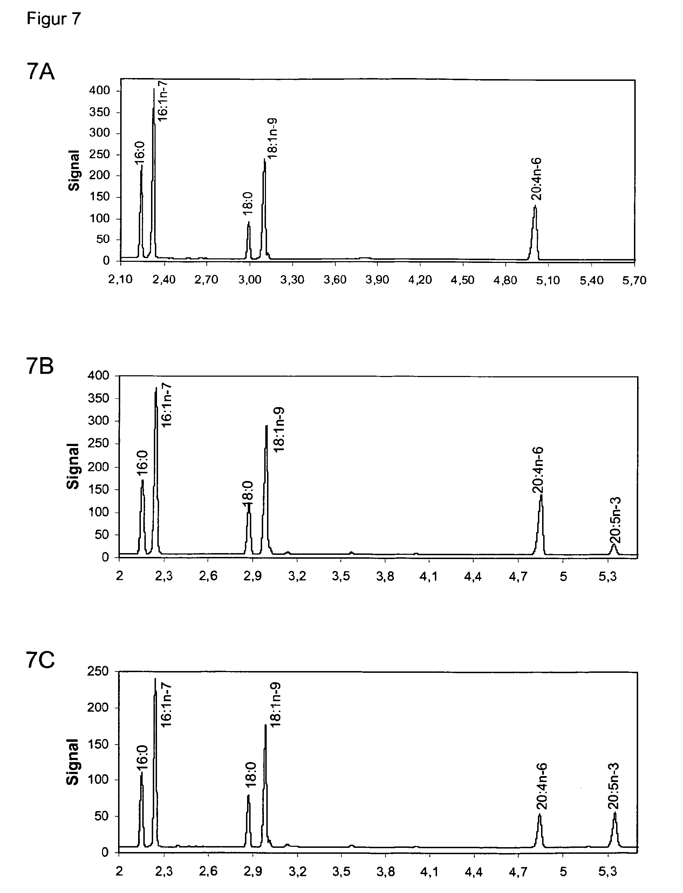

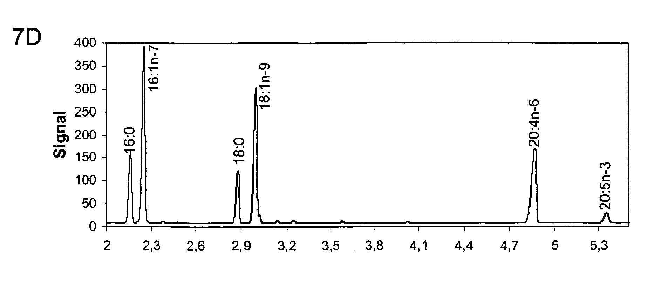

Als Kontrolle für den Test auf ω3-Desaturase Aktivität bei Verwendung von 22:4n-6 als Substrat wurden Hefen mit dem pYES Leervektor transformiert, die Fettsäure 20:4n-6 gefüttert und das Fettsäureprofil analysiert (Abbildung 7A). Im Vergleich hierzu ist bei Hefen, welche die Desaturasen d15Des(Hr), d15Des(Lg), d15Des(Mc) exprimieren, die zusätzliche Fettsäure 22:5n-3 zu beobachten (Abbildung 7B, 7C, 7D). Diese getesteten Desaturasen haben daher ω3-Desaturase Aktivität denen 20:4n-6 als Substrat dient.

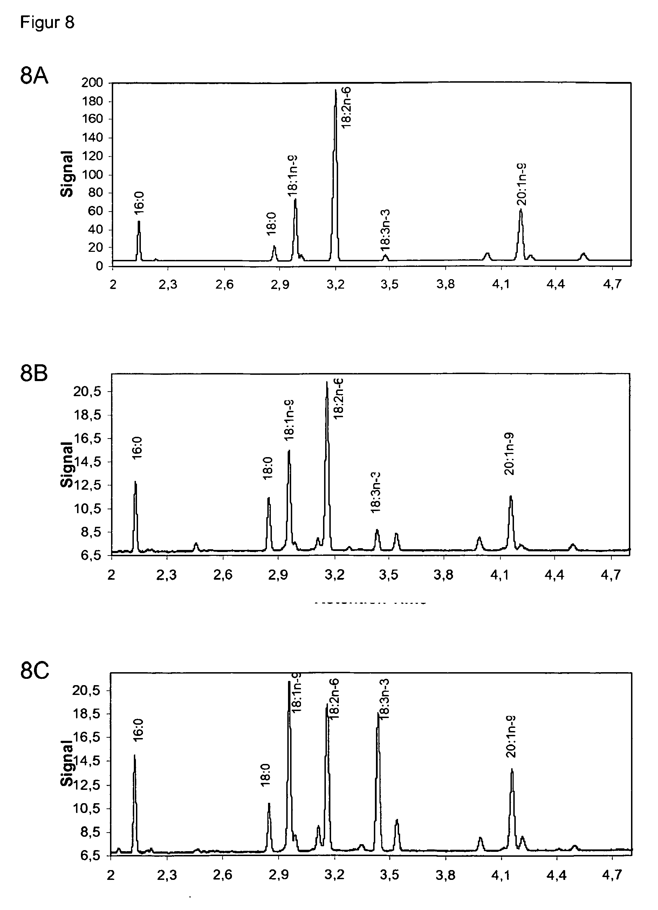

Weiterhin wurden die in Hefe nachgewiesenen Aktivitäten in Pflanzen überprüft, welche die Desaturasen samenspezifisch exprimierten. Hierzu wurde die fad3 Mutante von Arabidopsis verwendet, welche im Vergleich zu dem Col-0 Wildtyp nur sehr wenig 18:3n-3 im Samen produziert (siehe Tabelle 4A und Abbildung 8A). Im Vergleich hierzu ist bei fad3 Mutanten, welche die Desaturasen d15Des(Mg) (siehe Tabelle 4B) und d15Des(Nh) (siehe Tabelle 4C) im Samen exprimieren stark erhöht Mengen von 18:3n-3 zu beobachten (Abbildung 8B, 8C). Diese getesteten Desaturasen haben daher in Pflanzen d15-Desaturase Aktivität.

Tabelle 4B: Position des Bereichs vom binären Vektors pSUN-d15Des(Mg) (16093 bp, DNA, circular), der die Desaturase d15Des(Mg) (SEQ ID Nr: 15) kodiert, sowie die Position des die Expression regulierenden zugehörigen Promotors und Terminators. Der zugrunde liegende Basisvektor ist pSUN. Die angegebene Position beschreibt den Start- und Endpunkt des entsprechenden Elements in Basenpaaren (bp) bezogen auf SEQ ID NR: 183. Folgende Elemente sind durch einen Präfix charakterisiert: p- (Promoter), : c- (codierende Sequenz), t- (Terminator).

| Element | Beschreibung | Position |
|---|---|---|
| p-SBP | Promotor vom VfSBP aus Vicia faba | 263-2061 |
| c-d15Des(Mg_GA) | Kodiert Δ15-Desaturase aus Mycospaerella graminicola (SEQ ID Nr: 15) | 2071-3261 |
| t-CatpA | Terminator aus Vicia faba | 3291-3526 |

Tabelle 4C: Position des Bereichs vom binären Vektors pSUN-d15Des(Nh)2 (16097 bp, DNA, circular), der die Desaturase d15Des(Nh)2 (SEQ ID Nr: 6) kodiert, sowie die Position des die Expression regulierenden zugehörigen Promotors und Terminators. Der zugrunde liegende Basisvektor ist pSUN. Die angegebene Position beschreibt den Start- und Endpunkt des entsprechenden Elements in Basenpaaren (bp) bezogen auf SEQ ID NR: 184. Folgende Elemente sind durch einen Präfix charakterisiert: p- (Promoter), : c- (codierende Sequenz), t- (Terminator).

| Element | Beschreibung | Position |
|---|---|---|
| p-SBP | Promotor vom VfSBP aus Vicia faba | 263-2061 |
| c-d15Des(Nh_GA)2 | Kodiert Δ15-Desaturase aus Nectria haematococca (SEQ ID Nr: 6) | 2063-3265 |
| t-CatpA | Terminator aus Vicia faba | 3296-3530 |

### Beispiel 5: Herstellung transgener Pflanzen zur Produktion von langkettigen mehrfach ungesättigten Fettsäuren.

Zur Herstellung von langkettigen mehrfach ungesättigten Fettsäuren in Pflanzen werden verschiedenen Gene des Stoffwechselweges auf einem binären Vektor kombiniert. Zur Herstellung der Fettsäure Eicosapentaensäure (20:5Δ5,8,11,14,17) werden Gene wie in Tabelle 5 beschrieben, kombiniert. Entsprechend werden zur Herstellung der Fettsäure Docosahexaensäure (22:6Δ4,7,10,13,16,19) die Gene wie in Tabelle 6 beschrieben, kombiniert.

**Tabelle 5: Genkombination zur Herstellung von Eicosapentaensäure**

| Gene | Aktivität | SEQ ID NR: |
|---|---|---|
| D6Des(Pir) | Δ6-Desaturase | 28 |
| D6Elo(Pp) | Δ6-Elongase | 31 |
| D5Des(Tc) | Δ5-Desaturase | 29 |
| ω3-Des(Pi) | Omega 3-Desaturase | 30 |
| D12Des(Nh) | Δ12-Desaturase | 2 |
| D15Des(Nh) | Δ15-Desaturase | 5 |
| D15Des(Tr) | Δ12-/Δ15-Desaturase | 8 |
| D12Des(Mb) | Δ12-Desaturase | 11 |
| D15Des(Mg) | Δ15-Desaturase | 14 |
| D12Des(Mg) | Δ12-Desaturase | 17 |
| D15Des(Ng) | Δ15-Desaturase | 20 |
| D12Des(Pb) | Δ12-Desaturase | 23 |
| D15Des(Nv) | Δ15-Desaturase | 26 |
| D15Des(Lb) | Δ15-Desaturase | 91 |
| d15Des(Hr) | Δ15-Desaturase | 123 |
| d15Des(Lg)1 | Δ15-Desaturase | 126 |
| d15Des(Lg)2 | Δ15-Desaturase | 129 |
| d15Des(Mc) | Δ15-Desaturase | 132 |
| d15Des(Mf) | Δ15-Desaturase | 135 |
| d15Des(Pp) | Δ15-Desaturase | 143 |
| d15Des(Pp2) | Δ15-Desaturase | 146 |
| d15Des(Pp3) | Δ15-Desaturase | 149 |
| d15Des(Ppla) | Δ15-Desaturase | 152 |
| d15Des(Sm) | Δ15-Desaturase | 155 |
| d15Des(Mn) | Δ15-Desaturase | 157 |

**Tabelle 6: Genkombination zur Herstellung von Docosahexaensäure**

| Gene | Aktivität | SEQ ID NR: |
|---|---|---|
| D6Des(Pir) | Δ6-Desaturase | 28 |
| D6Elo(Pp) | Δ6-Elongase | 31 |
| D5Des(Tc) | Δ5-Desaturase | 29 |
| ω3-Des(Pi) | Omega 3-Desaturase | 30 |
| D12Des(Nh) | Δ12-Desaturase | 2 |
| D15Des(Nh) | Δ15-Desaturase | 5 |
| D15Des(Tr) | Δ12-/Δ15-Desaturase | 8 |
| D12Des(Mb) | Δ12-Desaturase | 11 |
| D15Des(Mg) | Δ15-Desaturase | 14 |
| D12Des(Mg) | Δ12-Desaturase | 17 |
| D15Des(Ng) | Δ15-Desaturase | 20 |
| D12Des(Pb) | Δ12-Desaturase | 23 |
| D15Des(Nv) | Δ15-Desaturase | 26 |
| D15Des(Lb) | Δ15-Desaturase | 91 |
| d15Des(Hr) | Δ15-Desaturase | 123 |
| d15Des(Lg)1 | Δ15-Desaturase | 126 |
| d15Des(Lg)2 | Δ15-Desaturase | 129 |
| d15Des(Mc) | Δ15-Desaturase | 132 |
| d15Des(Mf) | Δ15-Desaturase | 135 |
| d15Des(Pp) | Δ15-Desaturase | 143 |
| d15Des(Pp2) | Δ15-Desaturase | 146 |
| d15Des(Pp3) | Δ15-Desaturase | 149 |
| d15Des(Ppla) | Δ15-Desaturase | 152 |
| d15Des(Sm) | Δ15-Desaturase | 155 |
| d15Des(Mn) | Δ15-Desaturase | 157 |
| D5Elo(Ot) | Δ5-elongase | 32 |
| D4Des(Tc) | Δ4-desaturase | 33 |

Für die Transformation von Pflanzen werden weiterere Transformationsvektoren auf Basis von pSUN-USP erzeugt. Dazu werden mit folgenden Primerpaaren Notl-Schnittstellen am 5' und 3'-Ende der kodierenden Sequenz eingefügt (siehe Tabelle 7).

Zusammensetzung des PCR-Ansatzes (50 µL):
5,00 µL Template cDNA
5,00 µL 10x Puffer (Advantage-Polymerase)+ 25mM MgCl₂
5,00 µL 2mM dNTP
1,25 µL je Primer (10 pmol/µL)
0,50 µL Advantage-Polymerase
Die Advantage-Polymerase von Clontech wird eingesetzt.

Reaktionsbedingungen der PCR:
Anlagerungstemperatur: 1 min 55°C
Denaturierungstemperatur: 1 min 94°C
Elongationstemperatur: 2 min 72°C
Anzahl der Zyklen: 35

**Tabelle 7: Primersequenzen (für die Klonierung von Transformationsvektoren auf Basis von pSUN-USP)**

| Gen | Primer | SEQ ID NR: |
|---|---|---|
| D6-Des(Pir) | Fwd: gcggccgcgccatggtggacctcaagcctgg | 52 |
| | Rvs: gcggccgttacatcgctgggaactcgg | 53 |
| D5-Des(Tc) | | 54 |
| | Rvs: gcggccgcgcctcagtcctgcttcttggtgtc | 55 |
| O3-Des(Pi) | Fwd: gcggccgcgccatggcgacgaaggaggcgta | 56 |
| | Rvs: gcggccgcgttacgtggacttggtcttggcc | 57 |
| D6-Elo(Pp) | Fwd: gcggccgcgccatggaggtcgtggagagattc | 58 |
| | Rvs: gcggccgcgtcactcagttttagctccc | 59 |
| D12Des(Nh) | Fwd: gcggccgcgccatggcttctacggctgtgcc | 60 |
| | Rvs: gcggccgcgttaggcggcgggagggtcga | 61 |
| D15Des(Nh) | Fwd: gcggccgcgccatggctactcgacagcgtac | 62 |
| | Rvs: gcggccgcgctactccttggcccatcgcatg | 63 |
| D15Des(Tr) | Fwd: gcggccgcgccatggctactaccacgacggtc | 64 |
| | Rvs: gcggccgcgtcattgcgcccagtgcagag | 65 |
| D12Des(Mb) | Fwd: gcggccgcgccatgacggtggcttcccaggtg | 66 |
| | Rvs: gcggccgcgttacctgtccttgaaccaaag | 67 |
| D15Des(Mg) | Fwd: gcggccgcgccatgagcagaacagtcacatta | 68 |
| | Rvs: gcggccgcgtcacgcgctcttaacatgcg | 69 |
| D12Des(Mg) | Fwd: gcggccgcgccatgagcaccaccgccctctc | 70 |
| | Rvs: gcggccgcgctattcggaatcatcctcaac | 71 |
| D15Des(Ng) | Fwd: gcggccgcgccatgtcagctgccacatcag | 72 |
| | Rvs: gcggccgcgttaatgatcccaccacaaaa | 73 |
| D12Des(Pb) | Fwd : gcggccgcgccatgtcggataacactgaatc | 74 |
| | Rvs: gcggccgcgttaattcttgaggaaacgaa | 75 |
| D15Des(Nv) | Fwd: gcggccgcgccatgccgccgtgtcacgcaac | 76 |
| | Rvs: gcggccgcgttagtccttttcacagttttc | 77 |
| D15Des(Lb) | Fwd: gcggccgcgccatggctgttaaaacggac | 86 |
| | Rvs: gcggccgcgctatttttcaacctcaatac | 87 |
| d15Des(Hr) | Fwd: gcggccgcgccatgaactgtgtaactgagg | 103 |
| | Rvs: gcggccgcgtatttgtaataaacctctaac | 104 |
| d15Des(Lg)1 | Fwd: gcggccgcgccatggaaaccaaatcaggaag | 105 |
| | Rvs: gcggccgcgttatgtataaatatgtac | 106 |
| d 15Des(Lg)2 | Fwd: gcggccgcgccatgaatgaagccaataaccac | 107 |
| | Rvs: gcggccgcgttatttatagtaatgaattttg | 108 |
| d15Des(Mc) | Fwd: gcggccgcgccatgcaatcaaacacagttc | 109 |
| | Rvs: gcggccgcgttagtttgatcgcggatgtttg | 110 |
| d15Des(Mf) | Fwd: gcggccgcgccatgtttctcgccggaagtgatg | 111 |
| | Rvs: gcggccgcgtcaggctcctagatctttcc | 112 |
| d15Des(Pp) | | 171 |
| | Rvs: gcggccgcgttatctgcaattgcactgttttg | 172 |
| d15Des(Pp2) | | 173 |
| | Rvs: gcggccgcgt cactgcttta catcgttctg | 174 |
| d15Des(Pp3) | Fwd: gcggccgcgc catgtccgtg aagcatgaga t | 175 |
| | Rvs: gcggccgcgt cacttagcat ttgtgctctt c | 176 |
| d15Des(Ppla) | | 177 |
| | Rvs: gcggccgcgt caacgagaca cgctcgctg | 178 |
| d15Des(Sm) | Fwd: gcggccgcgc catggtcgct gttccactcc g | 179 |
| | Rvs: gcggccgcgt tacttcaagc ctggatcgct c | 180 |
| d15Des(Mn) | | 181 |
| | Rvs: gcggccgcgc taaaggtcct tgcggggtg | 182 |
| DSEIo(Ot) | Fwd: gcggccgcgccatgagcgcctccggtgcgctg | 78 |
| | Rvs: gcggccgcgttagtcaatttttc | 79 |
| D4Des(Tc) | Fwd: gcggccgcgccatgacggtcggctacgacgag | 80 |
| | Rvs: gcggccgcgtcaggcagcgcgctgccagg | 81 |

Die PCR Produkte werden für 4 h bei 37°C mit dem Restriktionsenzym NotI inkubiert. Der Pflanzen-Expressionsvektor pSUN300-USP wird in gleicherweise inkubiert. Anschließend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel purification Kit gemäss Herstellerangaben. Anschliessend werden Vektor und PCR-Produkte ligiert. Dazu wird das Rapid Ligation Kit von Roche verwendet. Die entstandene Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des OCS-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: Das PCR-Fragment wurde mit EcoRI/Sall nachgeschnitten und in den Vektor pSUN300 mit OCS Terminator eingesetzt. Es entstand das Plasmid mit der Bezeichnung pSUN-USP, das für die Pflanzen-Transformation mittels Agrobacterium tumefaciens eingesetzt werden kann.

### a) Erzeugung transgener Rapspflanzen (verändert nach Moloney et al., 1992, Plant Cell Reports, 8:238-242)

Zur Erzeugung transgener Rapspflanzen wurden binäre Vektoren wie die weiter oben beschrieben pSUN Plasmide mit den entsprechend kombinierten Genen in Agrobacterium tumefaciens C58C1:pGV2260 transformiert (Deblaere et al, 1984, Nucl. Acids. Res. 13, 4777-4788). Beispielhaft zur Verwendung und Analyse von d15-Desaturasen und d12 Desaturasen soll die Herstellung von Pflanzen transformiert mit dem Konstrukt LJB765 (SEQ ID 137), das eine d15 Desaturase und eine d12 Desaturase enthält (Elemente c-d15Des(Fm_GA) mit SEQ ID NR: 140 und cd12-Des(Co) mit SEQ ID NR: 141 in unten folgender Tabelle 8), beschrieben werden. Die in dem Konstrukt verwendete d15-Desaturase bzw. d12 Desaturase können durch die erfindungsgemäßen d15-Desaturasen bzw. d12 Desaturasen ersetzt werden. Der binäre Vektor LJB765 mit der SEQ ID NR: 137 enthält die in Tabelle 8 aufgeführten Elemente.

**Tabelle 8: Beschreibung des binären Vektors LJB765: (28346 bp, DNA, circular). Die angegebene Position beschreibt den Start- und Endpunkt des entsprechenden Elements in Basenpaaren (bp) bezogen auf SEQ ID NR: 137. Folgende Elemente sind durch einen Präfix charakterisiert: c- (codierende Sequenz), o- (Replikationsursprung), p- (Promoter), t- (Terminator).**

| Element | Beschreibung | Position |
|---|---|---|
| c-d6-Elo(Pp_GA) SEQ ID NR: 31 1 | D6-Elongase aus Physcomitrella patens | 980-1852 |
| c-d6-Des(Pir) SEQ ID NR: 28 | D6-Desaturase aus Pythium irregulare | 2953-4332 |
| c-03Des(Pi_GA2) SEQ ID NR: 30 | Omega3-Desaturase aus Phytophthora infestans | 5402-6487 |
| c-d6Elo(Tp_GA) SEQ ID NR: 138 | D6-Elongase aus Thalassiosira pseudonana | 8854-9672 |
| c-d6Des(Ot_GA2) SEQ ID 139 | D6-Desaturase aus Ostreococcus tauri | 10543-11913 |
| c-d15Des(Fm_GA) SEQ ID 140 | D15-Desaturase aus Fusarium monoliforme | 12939-14147 |
| c-d5Des(Tc_GA) SEQ ID NR: 29 | D5-Desaturase aus Thraustochytrium ssp. | 15582-16901 |
| c-d12-Des(Co) SEQ ID NR: 141 | D12-Desaturase aus Calendula officinalis | 18900-20051 |
| c-Nptll | Neomycine phosphotransferase | 21425-22219 |
| c-PVS1 | Partitioning protein | 23248-24248 |
| c-StaA | PVS partioning protein | 23487-24116 |
| c-VS1 | VS1 orf3 | 24137-24352 |
| c-RepA | Replication protein | 24511-25618 |
| c-aadA | Adenylattransferase | 27382-28173 |
| o-BOM | pBR322 replication origin | 26252-26512 |
| o-ColE1 | Origin of replication | 26367-27213 |
| p-VfUSP | Promoter VfUSP aus Vicia faba | 299-972 |
| p-LeB4 | Promoter LeB4 aus Vicia faba | 2163-2918 |
| p-Napin | Promoter Napin aus Brassica napus | 4735-5398 |
| p-VfSBP | Promoter VfSBP aus Vicia faba | 7054-8852 |
| p-BnGLP | Promoter BnGLP aus Brassica napus | 9914-10539 |
| p-VfUSP | Promoter VfUSP aus Vicia faba | 12250-12923 |
| p-Conlinin | Promoter Conlinin aus Linum usitatissimum | 14505-15543 |
| p-LuPXR | Promoter LuPXR aus Linum usitatissimum | 17150-18876 |
| p-NOS | Promoter from Agrobacterium tumefaciens | 21106-21393 |
| RB | Rechte Border des T-Plasmids | 83-106 |
| LB | Linke Border des T-Plasmids | 22732-22756 |
| t-35S | Terminator CamV35S | 1903-2124 |
| t-LeB | Terminator aus Vicia faba | 4375-4671 |
| t-E9 | Terminator aus Pisum sativum | 6488-7045 |
| t-CatpA | Terminator aus Vicia faba | 9673-9907 |
| t-AtGLP | Terminator aus Arabidopsis thaliana | 11914-12186 |
| t-35S | Terminator CamV35S | 14201-14416 |
| t-OCS | Terminator aus Agrobacterium tumefaciens | 16949-17147 |
| t-AtPXR | Terminator aus Arabidopsis thaliana | 20117-20516 |
| t-NOS | Terminator aus Agrobacterium tumefaciens | 20743-20998 |
| t-NOS | Terminator aus Agrobacterium tumefaciens | 22318-22570 |

Nach Transformation des binären Plasmids LJB765 in Agrobacterium (siehe oben), wurden zur Transformation von Rapspflanzen (Var. Westar), eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 cm²) wurden in einer Petrischale mit einer 1:50 Agrobakterienverdünnung für 5-10 Minuten inkubiert. Es folgte eine 3-tägige Co-Inkubation in Dunkelheit bei 25°C auf 3MS-Medium mit 0,8 % Bacto-Agar. Die Kultivierung wurde nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln zum Medium gegeben.

Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Expression der Desaturase- bzw. Elongase-Gene mittels Lipidanalysen untersucht wie beispielhaft in Qiu et al. 2001, J. Biol. Chem. 276, 31561-31566 beschrieben. Figur 2 zeigt ein Chromatogramm von Raps-Samen geerntet von Pflanzen, die mit dem Konstrukt LJB765 transformiert wurden. Dabei kann die Herstellung von neuen Fettsäuren in den Rapssamen beobachtet werden. Diese neuen Fettsäuren gehen auf die Aktivität der eingebrachten Gene zurück (siehe EPA, Eicosapentaensäure). Im Vergleich zur nicht-transformierten Kontrolle zeigen die Samen der transgenen Pflanze (Figur 2) erhöhte Gehalte an d12-desaturiertem Produkt (C18:2n-6, Linolsäure) und d15-desaturierten Produkten (18:3n-3; 18:4n-3; EPA), die auf die Aktivität der eingebrachten d12- und d15-Desaturase zurück gehen. Insgesamt konnten 18,4% omega3 Fettsäuren produziert werden, davon 11,8% EPA, 3,0% 18:4n-3 und 3,6% 18:3n-3. Die nicht transformierte Kontrollpflanze enthält zwischen 5 und 6% omega3-Fettsäuren, d.h. in den transgenen Pflanzen wurde der Gehalt an omega3-Fettsäuren verdreifacht.

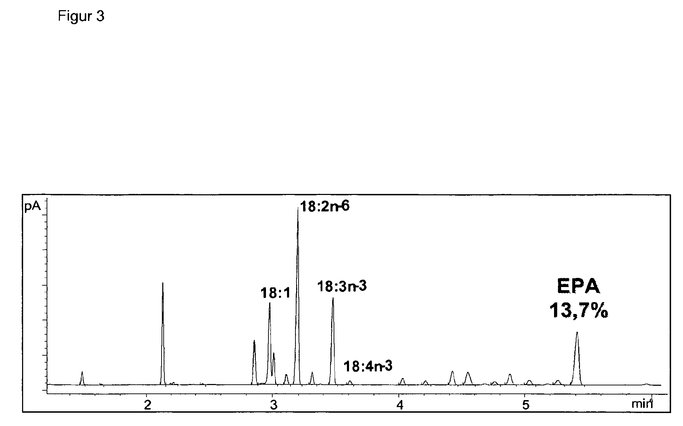

In einem weiteren Beispiel wurde das Konstrukt LJB765 in Arabidopsis thaliana transformiert. Dazu wurde die Floral Dip Methode von (Clough and Bent, 1998, Plant Journal 16: 735-743) verwendet. Transgene Pflanzen wurden auf AgarPlatten mit Kanamycine selektiert und entsprechend Sayanova et al., 2003, FEBS Letters, 542,100-104 wurden die geernteten Samen durch gaschromatographische Analyse untersucht. Hierbei wurden ähnliche Werte wie im Raps erhalten (Figur 3). Im Vergleich zur nicht-transformierten Kontrolle zeigen die Samen der transgenen Pflanze erhöhte Gehalte an d12-desaturiertem Produkt (C18:2n-6, Linolsäure). Auch zeigen die transformierten Pflanzen einen gegenüber den nicht transformierten Pflanzen deutlich erhöhten Gehalt (i. e. eine Verdoppelung) an omega3 Fettsäuren. So wurden beispielsweise 13,7% EPA erhalten. Insgesamt konnten 28,6% omega3 Fettsäuren produziert werden, davon 13,7% EPA, 0,6% 18:3n-3, 13,3% 18:3n-3 und 1,0% 20:4n-3.

In ähnlicher Weise lassen sich auch die erfindungsgemäßen d12- und d15-Desaturasen charakterisieren und zur Herstellung von mehrfach ungesättigten langkettigen omega3-Fettsäuren (wie Eicosapentaensäure und/oder Docosahexaensäure) verwenden.

### b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 hergestellt werden.

## Patentansprüche

1. Polynukleotid umfassend eine Nukleinsäuresequenz, die ausgewählt ist aus der Gruppe bestehend aus:
(a) Nukleinsäuresequenz wie in einer der SEQ ID NR: 1 oder 2 gezeigt;
(b) Nukleinsäuresequenz, die ein Polypeptid kodiert, das eine Aminosäuresequenz wie in einer der SEQ ID NR: 3 gezeigt aufweist;
(c) Nukleinsäuresequenz, die mindestens 95% identisch zu einer der Nukleinsäuresequenzen aus (a) oder (b) ist und ein Polypeptid mit einer Δ12-Desaturase Aktivität kodiert; und
(d) Nukleinsäuresequenz für ein Fragment einer Nukleinsäure aus (a), (b) oder (c), wobei das Fragment ein Polypeptid mit Δ12-Desaturase Aktivität kodiert.

2. Polynukleotid nach Anspruch 1, wobei das Polynukleotid aus RNA oder DNA besteht.

3. Vektor umfassend das Polynukleotid nach Anspruch 1 oder 2.

4. Vektor nach Anspruch 3, wobei der Vektor ein Expressionsvektor ist.

5. Vektor nach Anspruch 3 oder 4, wobei der Vektor mindestens ein weiteres Polynukleotid umfasst, das ein weiteres Enzym kodiert, das in die Biosynthese von Lipiden oder Fettsäuren eingebunden ist.

6. Wirtszelle, umfassend das Polynukleotid nach Anspruch 1 oder 2 oder den Vektor nach einem der Ansprüche 3 bis 5.

7. Wirtszelle nach Anspruch 6, wobei die Wirtszelle zusätzlich mindestens ein weiteres Enzym umfasst, das in die Biosynthese von Lipiden oder Fettsäuren eingebunden ist.

8. Vektor nach Anspruch 5 oder Wirtszelle nach Anspruch 7 wobei das Enzym ausgewählt ist aus der Gruppe bestehend aus: Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n), Fettsäure-Elongase(n), Δ4-Desaturase(n), Δ5-Desaturase(n), Δ6-Desaturase(n), Δ8-Desaturase(n), Δ9-Desaturase(n), Δ12-Desaturase(n), Δ5-Elongase(n), Δ6-Elongase(n) und Δ9-Elongase(n).

9. Verfahren zur Herstellung eines Polypeptids mit Δ12-Desaturase Aktivität, umfassend die Schritte:
(a) Exprimieren eines Polynukleotids nach Anspruch 1 oder 2 in einer Wirtszelle; und
(b) Gewinnen des Polypeptids, das von dem Polynukleotid nach (a) kodiert wird, aus der Wirtszelle.

10. Polypeptid, das von dem Polynukleotid gemäß Anspruch 1 oder 2 kodiert wird oder das erhältlich durch das Verfahren nach Anspruch 9 ist.

11. Polypeptid nach Anspruch 10, das eine Aminosäuresequenz wie in SEQ ID NO: 3 aufweist oder eine Aminosäuresequenz, die dazu mindestens 95% identisch ist, wobei das Polypeptid Δ12-Desaturase-Aktivität aufweist.

12. Antikörper, der das Polypeptid nach Anspruch 10 oder 11 spezifisch erkennt.

13. Transgener, nicht-humaner Organismus umfassend das Polynukleotid nach Anspruch 1 oder 2, den Vektor nach einem der Ansprüche 3 bis 5 oder die Wirtszelle nach Anspruch 6 oder 7.

14. Transgener, nicht-humaner Organismus nach Anspruch 13, wobei der Organismus ein Tier, eine Pflanze oder ein multizellulärer Mikroorganismus ist.

15. Verfahren zur Herstellung eines Stoffes, der die Struktur aufweist, die in der folgenden allgemeinen Formel I gezeigt wird 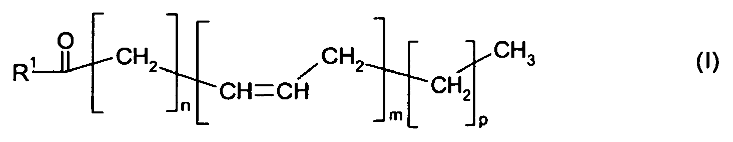 wobei die Variablen und Substiuenten die folgenden sind:
R¹ = Hydroxyl, Coenzym A (Thioester), Lysophosphatidylcholin, Ly- sophosphatidylethanolamin, Lysophosphatidylglycerol, Lyso- diphosphatidylglycerol, Lysophosphatidylserin, Lysophosphatidylino- sitol, Sphingo- Base oder ein Radikal der Formel II 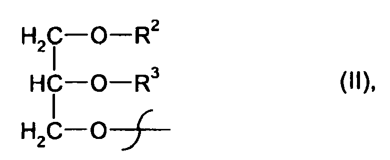
R² = Wasserstoff, Llysophosphatidylcholin, Lysophosphatidylethanolamin, Lysophosphatidylglycerol, Llysodiphosphatidylglycerol, Lysophospha- tidylserin, Lysophosphatidylinositol oder gesättigtes oder ungesättigtes C₂-C₂₄-Alkylcarbonyl,
R³ = Wasserstoff, gesättigtes oder ungesättigtes C₂-C₂₄-Alkylcarbonyl, oder R² und R³ sind unabhängig voneinander ein Radikal der Formel Ia: 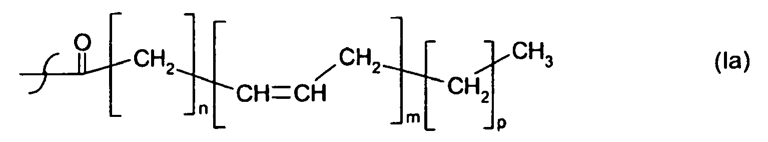
n = 2, 3, 4, 5, 6, 7 or 9, m = 2, 3, 4, 5 oder 6 und p = 0 oder 3;
und
wobei das Verfahren das Kultivieren von (i) einer Wirtszelle nach einem der Ansprüche 3 bis 5 oder (ii) eines transgenen, nicht-humanen Organismus nach Anspruch 13 oder 14 unter Bedingungen umfasst, die die Biosynthese des Stoffes erlauben.

16. Verfahren zur Herstellung einer Öl-, Lipid- oder Fettsäurezusammensetzung, umfassend die Schritte des Verfahrens nach Anspruch 15 und den weiteren Schritt des Formulierens des Stoffes als Öl-, Lipid- oder Fettsäurezusammensetzung.

17. Verfahren nach Anspruch 16, wobei die Öl-, Lipid- oder Fettsäurezusammensetzung weiter formuliert wird zu einem Arzneimittel, zu Kosmetik, zu einem Nahrungsmittel, zu einem Futtermittel, vorzugsweise Fischfutter, oder zu einem Nahrungsergänzungsmittel.

18. Verwendung des Polynukleotids nach Anspruch 1 oder 2, des Vektors nach einem der Ansprüche 3 bis 5, der Wirtszelle nach Anspruch 6 oder 7, des Vektors oder der Wirtszelle nach Anspruch 8, des Polypeptids nach Anspruch 10 oder 11 oder des transgenen, nicht-humanen Organismus nach Anspruch 13 oder 14 für die Herstellung einer Öl-, Lipid- oder Fettsäurezusammensetzung.

19. Verwendung nach Anspruch 18, wobei die Öl-, Lipid- oder Fettsäurezusammensetzung einzusetzen ist als Arzneimittel, Kosmetik, Nahrungsmittel, Futtermittel, vorzugsweise Fischfutter, oder Nahrungsergänzungsmittel.

## Claims

1. A polynucleotide comprising a nucleic acid sequence which is selected from the group consisting of:
(a) nucleic acid sequence as shown in either of SEQ ID No. 1 and 2;
(b) nucleic acid sequence which codes for a polypeptide which features an amino acid sequence as shown in SEQ ID No. 3;
(c) nucleic acid sequence which has at least 95% identity to one of the nucleic acid sequences of (a) or (b), and which codes for a polypeptide with a Δ12-desaturase activity; and
(d) nucleic acid sequence for a fragment of a nucleic acid of (a), (b) or (c), where the fragment codes for a polypeptide with Δ12-desaturase activity.

2. The polynucleotide according to claim 1, wherein the polynucleotide consists of RNA or DNA.

3. A vector comprising the polynucleotide according to claim 1 or 2.

4. The vector according to claim 3, wherein the vector is an expression vector.

5. The vector according to claim 3 or 4, wherein the vector comprises at least one further polynucleotide which codes for a further enzyme which is involved in the biosynthesis of lipids or fatty acids.

6. A host cell comprising the polynucleotide according to claim 1 or 2 or the vector according to any of claims 3 to 5.

7. The host cell according to claim 6, wherein the host cell additionally comprises at least one further enzyme which is involved in the biosynthesis of lipids or fatty acids.

8. The vector according to claim 5 or the host cell according to claim 7, wherein the enzyme is selected from the group consisting of: acyl-CoA dehydrogenase(s), acyl-ACP [= acyl carrier protein] desaturase(s), acyl-ACP thioesterase(s), fatty acid acyltransferase(s), acyl-CoA:lysophospholipid acyltransferase(s), fatty acid synthase(s), fatty acid hydroxylase(s), acetyl-coenzyme A carboxylase(s), acyl-coenzyme A oxidase(s), fatty acid desaturase(s), fatty acid acetylenase(s), lipoxygenase(s), triacylglycerol lipase(s), allene oxide synthase(s), hydroperoxide lyase(s), fatty acid elongase(s), Δ4-desaturase(s), Δ5-desaturase(s), Δ6-desaturase(s), Δ8-desaturase(s), Δ9-desaturase (s), Δ12-desaturase (s), Δ5-elongase(s), Δ6-elongase(s) and Δ9-elongase(s).

9. A method of generating a polypeptide with Δ12-desaturase activity, comprising the steps:
(a) expressing a polynucleotide according to claim 1 or 2 in a host cell; and
(b) obtaining, from the host cell, the polypeptide which is encoded by the polynucleotide according to (a).

10. A polypeptide which is encoded by the polynucleotide according to claim 1 or 2 or which is obtainable by the method according to claim 9.

11. The polypeptide according to claim 10 which has an amino acid sequence as in SEQ ID No. 3 or an amino acid sequence which has at least 95% identity thereto, wherein the polypeptide has Δ12-desaturase activity.

12. An antibody which specifically recognizes the polypeptide according to claim 10 or 11.

13. A transgenic, nonhuman organism comprising the polynucleotide according to claim 1 or 2, the vector according to any of claims 3 to 5 or the host cell according to claim 6 or 7.

14. The transgenic, nonhuman organism according to claim 13, wherein the organism is an animal, a plant or a multicellular microorganism.

15. A process for the production of a substance which has the structure shown in the general formula I hereinbelow 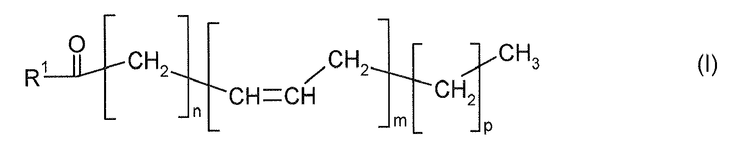 where the variables and substituents are as
follows:
R¹ = hydroxyl, coenzyme A (thioester), lysophosphatidylcholine, lysophosphatidylethanolamine, lysophosphatidylglycerol, lysodiphosphatidylglycerol, lysophosphatidylserine, lysophosphatidylinositol, sphingo base or a radical of the formula II 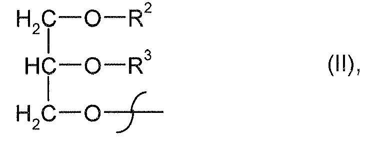
R² = hydrogen, lysophosphatidylcholine, lysophosphatidylethanolamine, lysophosphatidylglycerol, lysodiphosphatidylglycerol, lysophosphatidyl- serine, lysophosphatidylinositol or saturated or unsaturated C₂-C₂₄-alkylcarbonyl,
R³ = hydrogen, saturated or unsaturated C₂-C₂₄- alkylcarbonyl, or R² and R³ independently of one another are a radical of the formula Ia: 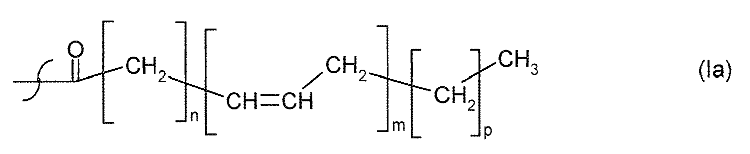
n = 2, 3, 4, 5, 6, 7 or 9, m = 2, 3, 4, 5 or 6 and p = 0 or 3;
and
wherein the process comprises the cultivation of (i) a host cell according to any of claims 3 to 5 or (ii) of a transgenic, nonhuman organism according to claim 13 or 14 under conditions which permit the biosynthesis of the substance.

16. A process for the production of an oil, lipid or fatty acid composition, comprising the steps of the process according to claim 15 and the further step of formulating the substance as an oil, lipid or fatty acid composition.

17. The process according to claim 16, wherein the oil, lipid or fatty acid composition is formulated further to give a drug, a cosmetic product, a foodstuff, a feedstuff, preferably fish food, or a food supplement.

18. The use of the polynucleotide according to claim 1 or 2, of the vector according to any of claims 3 to 5, of the host cell according to claim 6 or 7, of the vector or the host cell according to claim 8, of the polypeptide according to claim 10 or 11 or of the transgenic, nonhuman organism according to claim 13 or 14 for the production of an oil, lipid or fatty acid composition.

19. The use according to claim 18, wherein the oil, lipid or fatty acid composition is to be employed as a drug, cosmetic product, foodstuff, feedstuff, preferably fish food, or food supplement.

## Revendications

1. Polynucléotide comprenant une séquence d'acide nucléique qui est choisie dans le groupe constitué par :
(a) une séquence d'acide nucléique telle que présentée dans l'une des séquences SEQ ID n° 1 ou 2 ;
(b) une séquence d'acide nucléique qui code un polypeptide qui présente une séquence d'aminoacides telle que présentée dans la séquence SEQ ID n° 3 ;
(c) une séquence d'acide nucléique qui est identique à raison d'au moins 95 % à l'une des séquences d'acide nucléique de (a) ou (b) et code un polypeptide ayant une activité Δ12-désaturase ; et
(d) une séquence d'acide nucléique pour un fragment d'un acide nucléique de (a), (b) ou (c), le fragment codant un polypeptide à activité Δ12-désaturase.

2. Polynucléotide selon 1a revendication 1, le polynucléotide consistant en ARN ou ADN.

3. Vecteur comprenant le polynucléotide selon la revendication 1 ou 2.

4. Vecteur selon la revendication 3, le vecteur étant un vecteur d'expression.

5. Vecteur selon la revendication 3 ou 4, le vecteur comprenant au moins un autre polynucléotide qui code une autre enzyme qui est impliquée dans la biosynthèse des lipides ou des acides gras.

6. Cellule hôte, comprenant le polynucléotide selon la revendication 1 ou 2 ou le vecteur selon l'une quelconque des revendications 3 à 5.

7. Cellule hôte selon la revendication 6, la cellule hôte comprenant en outre au moins une autre enzyme qui est impliquée dans la biosynthèse des lipides ou des acides gras.

8. Vecteur selon la revendication 5 ou cellule hôte selon la revendication 7, l'enzyme étant choisie dans le groupe constitué par les enzymes suivante :
acyl-CoA-déshydrogénase(s), acyl-ACP[= acyl carrier protein]-désaturase(s), acyl-ACP-thioestérase(s), acide gras-acyl-transférase(s), acyl-COA:lysophospholipide-acyltransférase(s), acide gras-synthase(s), acide gras-hydroxylase(s), acétyl-coenzyme A-carboxylase(s), acyl-coenzyme A-oxydase(s), acide gras-désaturase(s), acide gras-acétylénase(s), lipoxygénase(s), triacylglycérol-lipase(s), allène-oxyde-synthase(s), hydroperoxyde-lyase(s), acide gras-élongase(s), Δ4-désaturase(s), Δ5-désaturase(s), Δ6-désaturase(s), Δ8-désaturase(s), Δ9-désaturase(s), Δ12-désaturase(s), Δ5-élongase(s), Δ6-élongase(s) et Δ9-élongase(s).

9. Procédé pour la production d'un polypeptide à activité Δ12-désaturase, comprenant les étapes :
(a) expression d'un polynucléotide selon la revendication 1 ou 2 dans une cellule hôte ; et
(b) obtention du polypeptide qui est codé par le polynucléotide selon (a), à partir de la cellule hôte.

10. Polypeptide, qui est codé par le polynucléotide selon la revendication 1 ou 2 ou qui peut être obtenu par le procédé selon la revendication 9.

11. Polypeptide selon la revendication 10, qui présente une séquence d'aminoacides telle que dans SEQ ID n° 3 ou une séquence d'aminoacides qui est identique à celle-ci à raison d'au moins 95 %, le polypeptide présentant une activité Δ12-désaturase.

12. Anticorps, qui reconnaît spécifiquement le polypeptide selon la revendication 10 ou 11.

13. Organisme non humain transgénique, comprenant le polynucléotide selon la revendication 1 ou 2, le vecteur selon l'une quelconque des revendications 3 à 5 ou la cellule hôte selon la revendication 6 ou 7.

14. Organisme non humain transgénique selon la revendication 13, l'organisme étant un animal, une plante ou un micro-organisme multicellulaire.

15. Procédé pour la production d'une substance ayant la structure qui est présentée dans la formule générale I suivante 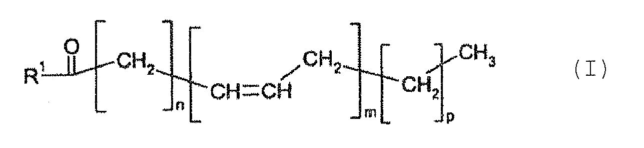 les variables et les substituants étant les suivantes :
R¹ = hydroxy, coenzyme A (thioester), lysophosphatidyl- choline, lysophosphatidyléthanolamine, lysophosphatidylglycérol, lysodiphosphatidyl- glycérol, lysophosphatidylsérine, lysophosphatidylinositol, sphingo-base ou un radical de formule II 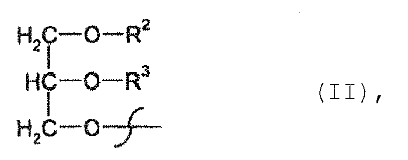
R² = hydrogène, lysophosphatidylcholine, lysophosphatidyléthanolamine, lysophosphatidyl- glycérol, lysodiphosphatidylglycérol, lysophosphatidylsérine, lysophosphatidylinositol ou alkyl(C₂-C₂₄)carbonyle saturé ou insaturé,
R³ = hydrogène, alkyl(C₂-C₂₄)carbonyle saturé ou insaturé, ou R² et R³ représentent, indépendamment l'un de l'autre, un radical de formule la : 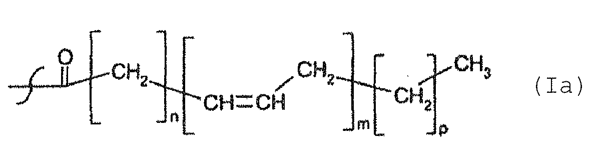
n = 2, 3, 4, 5, 6, 7 ou 9, m = 2, 3, 4, 5 ou 6 et p = 0 ou 3 ;
et
le procédé comprenant la culture (i) d'une cellule hôte selon l'une quelconque des revendications 3 à 5 ou (ii) d'un organisme non humain transgénique selon la revendication 13 ou 14, dans des conditions permettant la biosynthèse de la substance.

16. Procédé pour la production d'une composition d'huile, de lipide ou d'acide gras, comprenant les étapes du procédé selon la revendication 15 et l'étape ultérieure de la formulation de la substance sous forme de composition d'huile, de lipide ou d'acide gras.

17. Procédé selon la revendication 16, dans lequel la composition d'huile, de lipide ou d'acide gras est ultérieurement formulée en un médicament, en cosmétique, en un produit alimentaire, en un aliment pour animaux, de préférence un aliment pour poissons, ou en un complément nutritionnel.

18. Utilisation du polynucléotide selon la revendication 1 ou 2, du vecteur selon l'une quelconque des revendications 3 à 5, de la cellule hôte selon la revendication 6 ou 7, du vecteur ou de la cellule hôte selon la revendication 8, du polypeptide selon la revendication 10 ou 11 ou de l'organisme non humain transgénique selon la revendication 13 ou 14, pour la production d'une composition d'huile, de lipide ou d'acide gras.

19. Utilisation selon la revendication 18, la composition d'huile, de lipide ou d'acide gras étant utilisée sous forme de médicament, cosmétique, produit alimentaire, aliment pour animaux, de préférence aliment pour poissons, ou complément nutritionnel.
